# EUROPEAN PATENT APPLICATION

(11) **EP 3 009 421 A1**
(43) Date of publication of application: **20.04.2016**
(21) Application number: 14810255.1
(22) Date of filing: 09.06.2014
(51) Int. Cl.: C07C 233/73, A61K 31/166, A61K 31/277, A61K 31/415, A61K 31/44, A61K 31/4439, A61K 31/497, A61K 31/505, A61K 31/506, A61P 1/00, A61P 21/00, A61P 25/14, A61P 25/16, A61P 25/18, A61P 25/20, A61P 25/22, A61P 25/24, A61P 25/28, A61P 25/30, A61P 29/00

(54) **GLYCINE TRANSPORTER INHIBITOR**

(30) Priority: 10.06.2013 JP 2013122243
(71) Applicant: Taisho Pharmaceutical Co., Ltd., Tokyo 170-8633 (JP)
(72) Inventor: YAMADA, Yousuke, Tokyo 170-8633 (JP); OHTA, Hiroshi, Tokyo 170-8633 (JP); TAMITA, Tomoko, Tokyo 170-8633 (JP); ABE, Kumi, Tokyo 170-8633 (JP); YAMAMOTO, Shuji, Tokyo 170-8633 (JP); SHIROKAWA, Shin-ichi, Tokyo 170-8633 (JP); ABE, Masahito, Tokyo 170-8633 (JP); MATSUDA, Yohei, Tokyo 170-8633 (JP); ARAKI, Yuko, Tokyo 170-8633 (JP)
(74) Representative: Hoffmann Eitle
(86) International application number: PCT/JP2014/065269
(87) International publication number: WO 2014/199960

(57) **Abstract**

The present invention provides novel compounds of formula [IA] or pharmaceutically acceptable salts thereof: 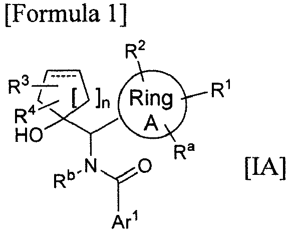 which are useful in the prevention or treatment of diseases such as schizophrenia, Alzheimer's disease, cognitive impairment, dementia, anxiety disorders (e.g., generalized anxiety disorder, panic disorder, obsessive-compulsive disorder, social anxiety disorder, post-traumatic stress disorder, specific phobias, acute stress disorder), depression, drug dependence, spasm, tremor, pain, Parkinson's disease, attention deficit hyperactivity disorder, bipolar disorder, eating disorder, or sleep disorders, which is based on the glycine uptake-inhibiting action.

## Description

### TECHNICAL FIELD

The present invention relates to compounds having a glycine transporter-inhibiting action.

### BACKGROUND ART

The NMDA receptor, which is one of glutamate receptors, is located on the nerve cell membranes in the brain and involved in various neurophysiologic events such as neuronal plasticity, cognition, attention, and memory. The NMDA receptor has a plurality of allosteric binding sites, one of which is the glycine binding site (glycine binding site on NMDA receptor complex). It has been reported that the glycine binding site on NMDA receptor complex is involved in the activation of NMDA receptors (Non-Patent Document 1).

Action potential arriving at the presynaptic terminals of glycinergic nerves triggers the release of glycine into synaptic clefts. The released glycine binds to the postsynaptic receptors or the like and is then removed from the synaptic clefts by transporters. Based on this fact, glycine transporters are believed to regulate the functions of NMDA receptors through regulation of the amount of glycine in the extracellular fluid.

Glycine transporters (GlyTs) are proteins involved in the reuptake of extracellular glycine into cells, and two subtypes, GlyT1 and GlyT2, have so far been identified. GlyT1, which is expressed primarily in the cerebral cortex, hippocampus, thalamus and the like, has been reported to be associated with diseases such as schizophrenia, Alzheimer's disease, cognitive impairment, dementia, anxiety disorders (e.g., generalized anxiety disorder, panic disorder, obsessive-compulsive disorder, social anxiety disorder, post-traumatic stress disorder, specific phobias, acute stress disorder), depression, drug dependence, spasm, tremor, pain, Parkinson's disease, attention deficit hyperactivity disorder, bipolar disorder, eating disorder, and sleep disorders (Non-Patent Documents 2-4).

Compounds having a GlyT1-inhibiting action and having a benzamide structure have been reported in the document shown below (Patent Document 1). The compounds described in Patent Document 1 are characterized by having a sulfone-containing group.

### CITATION LIST

### PATENT DOCUMENTS

Patent Document 1: WO2006134341

### NON-PATENT DOCUMENTS

Non-Patent Document 1: Molecular Psychiatry (2004) 9, 984-997
Non-Patent Document 2: Current Medicinal Chemistry, 2006, 13, 1017-1044
Non-Patent Document 3: Neuropsychopharmacology (2005), 30, 1963-1985
Non-Patent Document 4: Expert Opinion on Therapeutic Patents (2004) 14 (2) 201-214

### SUMMARY OF INVENTION

### TECHNICAL PROBLEM

The present invention aims to provide novel compounds or pharmaceutically acceptable salts thereof which are useful in the prevention or treatment of diseases such as schizophrenia, Alzheimer's disease, cognitive impairment, dementia, anxiety disorders (e.g., generalized anxiety disorder, panic disorder, obsessive-compulsive disorder, social anxiety disorder, post-traumatic stress disorder, specific phobias, acute stress disorder), depression, drug dependence, spasm, tremor, pain, Parkinson's disease, attention deficit hyperactivity disorder, bipolar disorder, eating disorder, or sleep disorders, which is based on the glycine uptake-inhibiting action.

### SOLUTION TO PROBLEM

As a result of extensive and intensive studies on structurally novel compounds with an inhibitory action against GlyT1, the present inventors found that the compounds having a hydroxy group which are represented by the following formula, are superior GlyT1-inhibiting substances. This finding has led to the completion of the present invention.

The present invention will be described below in detail. Embodiments of the present invention (hereinafter each referred to as "the inventive compound") are as shown below.
(1) A compound of formula [IA] or a pharmaceutically acceptable salt thereof: 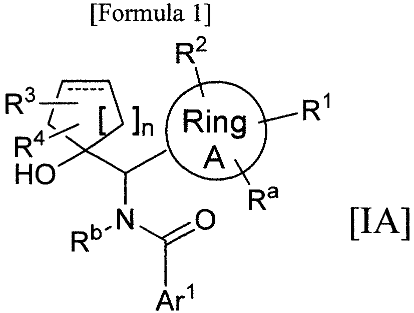
   wherein the double line with one line being dotted represents a single bond or a double bond, Ar¹ represents a naphthyl group, an isoquinolyl group, a naphtylidinyl group, a cinnolinyl group, a phenyl group, a pyrazinyl group, a pyrazolopyridyl group, an isothiazolyl group, a pyridazinyl group, a pyrazolyl group, a tetrahydroindazolyl group, an imidazopyridyl group, an isoxazolopyridyl group, (the phenyl group, the pirazinyl group, the pirazolopyridyl group, the isothiazolyl group, the pyridazinyl group, the pyrazolyl group, the tetrahydroindazolyl group, the imidazopyridyl group, and the isoxazolopyridyl group are optionally substituted with 1 to 3 substituents selected from substituent group 1 A), or a pyridyl group substituted with 1 to 2 substituents selected from substituent group 1A,
      substituent group 1A is the group consisting of a halogen atom, a C₁₋₆ alkyl group, a C₁₋₆ haloalkyl group, a C₁₋₆ alkoxy group, a C₁₋₆ haloalkoxy group, a C₃₋₆ cycloalkyl group, a C₁₋₆ alkylsulfonyl group, a hydroxy group, a pyrazolyl group, a triazolyl
      group, a tetrazolyl group, and a pyrimidinyl group,
   ring A represents a phenyl group or a pyridyl group,
   R¹ and R² are the same or different and are each a hydrogen atom, a halogen atom, a cyano group, a C₁₋₆ alkyl group optionally substituted with a substituent selected from substituent group 2A, a C₁₋₆ haloalkyl group, a C₁₋₆ alkoxy group, a C₁₋₆ haloalkoxy group, a C₂₋₇ alkoxycarbonyl group, a carbamoyl group optionally substituted with one or two C₁₋₆ alkyl groups, a phenyl group optionally substituted with a substituent selected from substituent group 3A, or a monocyclic heteroaryl group optionally substituted with a substituent selected from substituent group 4A,
      substituent group 2A is the group consisting of a hydroxy group, and an amino group optionally substituted with one or two C₁₋₆ alkyl groups,
      substituent group 3A is the group consisting of a cyano group and a C₁₋₆ alkanoyl group,
      substituent group 4A is the group consisting of a C₁₋₆ alkyl group and a C₁₋₆
      haloalkyl group,
   R^{a} represents a hydrogen atom or a halogen atom,
   R³ and R⁴ are the same or different and each represent a hydrogen atom, a halogen atom, or a hydroxy group, or together form an oxo group,
   R^{b} represents a hydrogen atom or a C₁₋₆ alkyl group, and
   n represents 0, 1, or 2.
(2) The compound according to (1) or a pharmaceutically acceptable salt thereof wherein ring A is a pyridyl group,
   R^{a} is a hydrogen atom, and
   R^{b} is a hydrogen atom.
(3) The compound according to (1) or (2) or a pharmaceutically acceptable salt thereof, wherein Ar¹ is a phenyl group optionally substituted with 1 to 3 substituents selected from substituent group 1A, or a pyridyl group substituted with 1 to 2 substituents selected from substituent group 1A.
(4) The compound according to any one of (1) to (3) or a pharmaceutically acceptable salt thereof, wherein Ar¹ is a pyridyl group substituted with 1 to 2 substituents selected from substituent group 1A.
(5) The compound according to any one of (1) to (3) or a pharmaceutically acceptable salt thereof, wherein Ar¹ is a pyridyl group substituted with 1 to 2 substituents selected from a halogen atom, a C₁₋₆ haloalkyl group, a C₁₋₆ alkylsulfonyl group, a pyrazolyl group, and a triazolyl group.
(6) The compound according to any one of (1) to (5) or a pharmaceutically acceptable salt thereof, wherein R¹ and R² are the same or different and are each a hydrogen atom, a halogen atom, or a monocyclic heteroaryl group optionally substituted with a substituent selected from substituent group 4A.
(7) The compound according to any one of (1) to (6) or a pharmaceutically acceptable salt thereof, wherein R¹ and R² are the same or different and are each a hydrogen atom or a halogen atom.
(8) The compound according to any one of (1) to (7) or a pharmaceutically acceptable salt thereof, wherein the double line with one line being dotted is a single bond,
   R³ and R⁴ are the same or different and are each a hydrogen atom or a halogen atom, and n is 1 or 2.
(9) The compound according to (1) or a pharmaceutically acceptable salt thereof, wherein the compound represented by formula [IA] or a pharmaceutically acceptable salt thereof is represented by the following formula [I]: 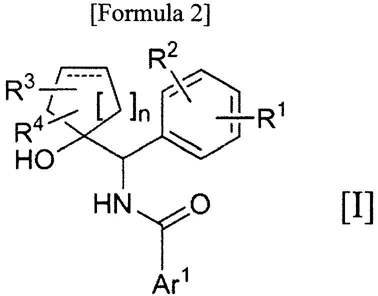
   wherein
   the double line with one line being dotted represents a single bond or a double bond,
   Ar¹ represents a naphthyl group, an isoquinolyl group, a phenyl group optionally substituted with 1 to 3 substituents selected from substituent group 1, a pyrazinyl group optionally substituted with 1 to 2 substituents selected from substituent group 1, or a pyridyl group substituted with 1 to 2 substituents selected from substituent group 1,
   substituent group 1 is the group consisting of a halogen atom, a C₁₋₆ alkyl group, a C₁₋₆ haloalkyl group, a C₁₋₆ alkoxy group, a pyrazolyl group, and a triazolyl group,
   R¹ and R² are the same or different and each represent a hydrogen atom, a halogen atom, a cyano group, a C₁₋₆ alkyl group optionally substituted with a substituent selected from substituent group 2, a C₁₋₆ haloalkyl group, a C₁₋₆ alkoxy group, a C₁₋₆ haloalkoxy group, a C₂₋₇ alkoxycarbonyl group, a carbamoyl group optionally substituted with one or two C₁₋₆ alkyl groups, a phenyl group optionally substituted with a substituent selected from substituent group 3, or a monocyclic heteroaryl group optionally substituted with a substituent selected from substituent group 4,
      substituent group 2 is the group consisting of a hydroxy group, and an amino group optionally substituted with one or two C₁₋₆ alkyl groups,
      substituent group 3 is the group consisting of a cyano group and a C₁₋₆ alkanoyl group,
      substituent group 4 is the group consisting of a C₁₋₆ alkyl group and a C₁₋₆ haloalkyl group,
   R³ and R⁴ are the same or different and each represent a hydrogen atom, a halogen atom, or a hydroxy group, or together form an oxo group, and
   n represents 0, 1, or 2.
(10) The compound according to (9) or a pharmaceutically acceptable salt thereof, wherein Ar¹ is a phenyl group optionally substituted with 1 to 3 substituents selected from substituent group 1, or a pyridyl group substituted with 1 to 2 substituents selected from substituent group 1.
(11) The compound according to (9) or a pharmaceutically acceptable salt thereof, wherein Ar¹ is a pyridyl group substituted with 1 to 2 substituents selected from substituent group 1.
(12) The compound according to (9) or a pharmaceutically acceptable salt thereof, wherein Ar¹ is a pyridyl group substituted with 1 to 2 substituents selected from a halogen atom and a C₁₋₆ haloalkyl group.
(13) The compound according to any one of (9) to (12) or a pharmaceutically acceptable salt thereof, wherein R¹ and R² are the same or different and are each a hydrogen atom, a halogen atom, or a monocyclic heteroaryl group optionally substituted with a substituent selected from substituent group 4.
(14) The compound according to any one of (9) to (12), or a pharmaceutically acceptable salt thereof, wherein R¹ and R² are the same or different and are each a hydrogen atom or a halogen atom.
(15) The compound according to any one of (9) to (14) or a pharmaceutically acceptable salt thereof, wherein the double line with one line being dotted is a single bond,
   R³ and R⁴ are the same or different and are each a hydrogen atom or a halogen atom, and n is 1 or 2.
(16) The compound according to (1) or a pharmaceutically acceptable salt thereof selected from the group consisting of
   3-chloro-N-{[3-(1-ethyl-1H-pyrazol-4-yl)phenyl](1-hydroxycyclohexyl)methyl}-4-(trifluoromethyl)pyridine-2-carboxamide,
   3-chloro-N-[(6-chloropyridin-2-yl)(1-hydroxycyclohexyl)methyl]-4-(trifluoromethyl)pyridine-2-carboxamide,
   3-chloro-N-{[3-(1-ethyl-1H-pyrazol-4-yl)phenyl](1-hydroxycyclopentyl)methyl}4-(trifluoromethyl)pyridine-2-carboxamide,
   3-chloro-N-{[3-(1-ethyl-1H-pyrazol-4-yl)phenyl](1-hydroxycyclopentyl)methyl}-4-(trifluoromethyl)pyridine-2-carboxamide,
   3-chloro-N-{(1-hydroxycyclopentyl)[3-(1H-pyrazol-4-yl)phenyl]methyl}-4-(trifluoromethyl)pyridine-2-carboxamide,
   3-chloro-N-[(6-chloropyridin-2-yl)(1-hydroxycyclopentyl)methyl]-4-(trifluoromethyl)pyridine-2-carboxamide,
   3-chloro-N-{(1-hydroxycyclopentyl)[3-(pyridin-3-yl)phenyl]methyl}-4-(trifluoromethyl)pyridine-2-carboxamide,
   3-chloro-N-{(1-hydroxycyclohexyl)[3-(pyridin-3-yl)phenyl]methyl}-4-(trifluoromethyl)pyridine-2-carboxamide,
   3-chloro-N-[(6-chloropyridin-2-yl)(1-hydroxycyclopentyl)methyl]-4-(trifluoromethyl)pyridine-2-carboxamide,
   3-chloro-N-[(6-chloropyridin-2-yl)(1-hydroxycyclohexyl)methyl]-2-(trifluoromethyl)pyridine-4-carboxamide,
   N-[(2-chloro-3-fluorophenyl)(cis-4-fluoro-1-hydroxycyclohexyl)methyl]-2-(1H-tetrazol-1-yl)-4-(trifluoromethyl)benzamide,
   3-chloro-N-{(4,4-difluoro-1-hydroxycyclohexyl)[3-(1-ethyl-1H-pyrazol-4-yl)phenyl]methyl}-4-(trifluoromethyl)pyridine-2-carboxamide,
   3-chloro-N-[(3-chlorophenyl)(1-hydroxycyclohexyl)methyl]-4-(trifluoromethyl)pyridine-2-carboxamide,
   3-chloro-N-[(3-fluorophenyl)(1-hydroxycyclohexyl)methyl]-4-(trifluoromethyl)pyridine-2-carboxamide,
   3-chloro-N-[(2,3-dichlorophenyl)(1-hydroxycyclopentyl)methyl]-4-(trifluoromethyl)pyridine-2-carboxamide,
   3-chloro-N-[(4,4-difluoro-1-hydroxycyclohexyl){3-[1-(propan-2-yl)-1H-pyrazol-4-yl]phenyl}methyl]-4-(trifluoromethyl)pyridine-2-carboxamide,
   3-chloro-N-{(1-hydroxycyclopentyl)[3-(pyridin-4-yl)phenyl]methyl}-4-(trifluoromethyl)pyridine-2-carboxamide,
   N-[(2-chloro-3-fluorophenyl)(cis-4-fluoro-l-hydroxycyclohexyl)methyl]-5-(methylsulfonyl)-2-(trifluoromethyl)pyridine-4-carboxamide,
   N-[(2-chloro-3-fluorophenyl)(cis-4-fluoro-1-hydroxycyclohexyl)methyl]-2-(methylsulfonyl)-5-(trifluoromethyl)pyridine-3-carboxamide,
   3-chloro-N-[(2-chloro-3-fluorophenyl)(cis-4-fluoro-1-hydroxycyclohexyl)methyl]-4-(trifluoromethyl)pyridine-2-carboxamide,
   N-[(3-bromophenyl)(1-hydroxycyclohexyl)methyl]-3-chloro-4-(trifluoromethyl)pyridine-2-carboxamide,
   3-chloro-N-[(2,3-dichlorophenyl)(1-hydroxycyclopentyl)methyl]-4-(trifluoromethyl)pyridine-2-carboxamide,
   N-[(2-chloro-3-fluorophenyl)(cis-4-fluoro-1-hydroxycyclohexyl)methyl]-2-(2H-1,2,3-triazol-2-yl)-6-(trifluoromethyl)pyridine-3-carboxamide,
   N-[(2-chloro-3-fluorophenyl)(cis-4-fluoro-1-hydroxycyclohexyl)methyl]-2-(methylsulfonyl)-5-(trifluoromethyl)benzamide,
   2-chloro-N-[(S)-[3-(1-ethyl-1H-pyrazol-4-yl)phenyl](1-hydroxycyclopentyl)methyl]-3-(trifluoromethyl)benzamide,
   2-chloro-N-{[3-(1-ethyl-1H-pyrazol-4-yl)phenyl](1-hydroxycyclohexyl)methyl -3 - (trifluoromethyl)benzamide,
   3-chloro-N-[(2-chlorophenyl)(4-fluoro-1-hydroxycyclohexyl)methyl]-4-(trifluoromethyl)pyridine-2-carboxamide,
   3-chloro-N-[(4-chloropyridin-2-yl)(cis-4-fluoro-1-hydroxycyclohexyl)methyl]-2-(trifluoromethyl)pyridine-4-carboxamide,
   3-chloro-N-[(2-chloro-3-fluorophenyl)(cis-4-fluoro-1-hydroxycyclohexyl)methyl]-2-(trifluoromethyl)pyridine-4-carboxamide,
   3-chloro-N-[(3-chlorophenyl)(1-hydroxycyclopentyl)methyl]-4-(trifluoromethyl)pyridine-2-carboxamide,
   3-chloro-N-[(3-fluorophenyl)(1-hydroxycyclohexyl)methyl]-4-(trifluoromethyl)pyridine-2-carboxamide,
   3-chloro-N-[(3-chloro-2-fluorophenyl)(4,4-difluoro-1-hydroxycyclohexyl)methyl]-4-(trifluoromethyl)pyridine-2-carboxamide,
   3-chloro-N-[(2-chloro-4-fluorophenyl)(1-hydroxycyclopentyl)methyl]-4-(trifluoromethyl)pyridine-2-carboxamide,
   3-chloro-N-[(3,5-dichlorophenyl)(1-hydroxycyclopentyl)methyl]-4-(trifluoromethyl)pyridine-2-carboxamide,
   N-[(2-bromophenyl)(1-hydroxycyclopentyl)methyl]-3-chloro-4-(trifluoromethyl)pyridine-2-carboxamide,
   N-[(3-bromophenyl)(1-hydroxycyclohexyl)methyl]-3-chloro-4-(trifluoromethyl)pyridine-2-carboxamide,
   3-chloro-N-[(2,3-dichlorophenyl)(4,4-difluoro-1-hydroxycyclohexyl)methyl]-4-(trifluoromethyl)pyridine-2-carboxamide,
   3-chloro-N-[(3-chloro-2-fluorophenyl)(4-fluoro-1-hydroxycyclohexyl)methyl]-4-(trifluoromethyl)pyridine-2-carboxamide,
   3-chloro-N-[(2,5-dichlorophenyl)(1-hydroxycyclopentyl)methyl]-4-(trifluoromethyl)pyridine-2-carboxamide,
   3-chloro-N-[(8)-(3-chlorophenyl)(cis-4-fluoro-1-hydroxycyclohexyl)methyl]-4-(trifluoromethyl)pyridine-2-carboxamide,
   3-chloro-N-[(2-chlorophenyl)(1-hydroxycyclopentyl)methyl]-4-(trifluoromethyl)pyridine-2-carboxamide,
   3-chloro-N-[(2,5-dichlorophenyl)(1-hydroxycyclopentyl)methyl]-4-(trifluoromethyl)pyridine-2-carboxamide,
   3-chloro-N-{(1-hydroxycyclopentyl)[3-(pyridin-3-yl)phenyl]methyl}-4-(trifluoromethyl)pyridine-2-carboxamide,
   3-chloro-N-[(4,4-difluoro-1-hydroxycyclohexyl){3-[1-(2,2,2-trifluoroethyl)-1H-pyrazol-4-yl]phenyl}methyl]-4-(trifluoromethyl)pyridine-2-carboxamide,
   3-chloro-N-{(4,4-difluoro-1-hydroxycyclohexyl)[3-(1-ethyl-1H-pyrazol-4-yl)phenyl]methyl}-4-(trifluoromethyl)pyridine-2-carboxamide,
   3-chloro-N-{(4,4-difluoro-l-hydroxycyclohexyl)[3-(pyridin-2-yl)phenyl]methyl}-4-(trifluoromethyl)pyridine-2-carboxamide,
   3 -chloro-N- {(S)-(1-hydroxycyclopentyl)[3-(pyridin-2-yl)phenyl]methyl} -4-(trifluoromethyl)pyridine-2-carboxamide,
   N-[(S)-(3-bromophenyl)(1-hydroxycyclopentyl)methyl]-2-chloro-3-(trifluoromethyl)benzamide,
   3-chloro-N-[(6-chloropyridin-2-yl)(4,4-difluoro-1-hydroxycyclohexyl)methyl]-4-(trifluoromethyl)pyridine-2-carboxamide,
   3-chloro-N-[(6-chloropyridin-2-yl)(cis-4-fluoro-1-hydroxycyclohexyl)methyl]-4-(trifluoromethyl)pyridine-2-carboxamide,
   3-chloro-N-{(1-hydroxycyclohexyl)[3-(pyridin-3-yl)phenyl]methyl}-4-(trifluoromethyl)pyridine-2-carboxamide,
   2-chloro-N-[(6-chloropyridin-2-yl)(cis-4-fluoro-1-hydroxycyclohexyl)methyl]-3-(trifluoromethyl)benzamide,
   N-[(S)-(3-bromophenyl)(1-hydroxycyclopentyl)methyl]-3-chloro-4-(trifluoromethyl)pyridine-2-carboxamide,
   3-chloro-N-[(3-chloro-2-fluorophenyl)(4-fluoro-1-hydroxycyclohexyl)methyl]-4-(trifluoromethyl)pyridine-2-carboxamide,
   3-chloro-N-[(3-chlorophenyl)(1-hydroxycyclohexyl)methyl]-4-(trifluoromethyl)pyridine-2-carboxamide,
   2-chloro-N-[(4-chloropyridin-2-yl)(cis-4-fluoro-1-hydroxycyclohexyl)methyl]-3-(trifluoromethyl)benzamide,
   3-chloro-N-{(1-hydroxycyclopentyl)[3-(1H-pyrazol-1-yl)phenyl]methyl}-4-(trifluoromethyl)pyridine-2-carboxamide,
   3-chloro-N-[(3-chlorophenyl)(1-hydroxycyclopentyl)methyl]-4-(trifluoromethyl)pyridine-2-carboxamide,
   3-chloro-N-{(1-hydroxycyclopentyl)[3-(pyrimidin-5-yl)phenyl]methyl}-4-(trifluoromethyl)pyridine-2-carboxamide,
   N-[(S)-(3-bromophenyl)(cis-4-fluoro-1-hydroxycyclohexyl)methyl]-3-chloro-4-(trifluoromethyl)pyridine-2-carboxamide,
   3-chloro-N-[(3-chloro-2-fluorophenyl)(4,4-difluoro-1-hydroxycyclohexyl)methyl]-2-(trifluoromethyl)pyridine-4-carboxamide,
   3-chloro-N-[(6-chloropyridin-2-yl)(cis-4-fluoro-1-hydroxycyclohexyl)methyl]-2-(trifluoromethyl)pyridine-4-carboxamide,
   3-chloro-N-[(3-fluorophenyl)(1-hydroxycyclopentyl)methyl]-4-(trifluoromethyl)pyridine-2-carboxamide,
   3-chloro-N-[(3-chlorophenyl)(cis-4-fluoro-1-hydroxycyclohexyl)methyl]-4-(trifluoromethyl)pyridine-2-carboxamide,
   2-chloro-N-[(2-chloro-3-fluorophenyl)(cis-4-fluoro-1-hydroxycyclohexyl)methyl]-3-(trifluoromethyl)benzamide,
   3-chloro-N-[(4,4-difluoro-1-hydroxycyclohexyl){3-[1-(propan-2-yl)-1H-pyrazol-4-yl]phenyl}methyl]-4-(trifluoromethyl)pyridine-2-carboxamide,
   3-chloro-N-[(2-chloro-3-fluorophenyl)(4,4-difluoro-1-hydroxycyclohexyl)methyl]-4-(trifluoromethyl)pyridine-2-carboxamide,
   3-chloro-N-[(2,3-difluorophenyl)(4-fluoro-1-hydroxycyclohexyl)methyl]-4-(trifluoromethyl)pyridine-2-carboxamide,
   3-chloro-N-[-(cis-4-fluoro-1-hydroxycyclohexyl)(3-fluorophenyl)methyl]-4 (trifluoromethyl)pyridine-2-carboxamide,
   N-[(2-chloro-3-fluorophenyl)(cis-4-fluoro-1-hydroxycyclohexyl)methyl]-2-(methylsulfonyl)-4-(trifluoromethyl)benzamide,
   2-chloro-N-{[3-(1-methyl-1H-pyrazol-4-yl)phenyl](1-hydroxycyclopentyl)methyl}-3 - (trifluoromethyl)benzamide,
   2-chloro-N-{(1-hydroxycyclopentyl)[3-(1H-pyrazol-4-yl)phenyl]methyl}-3 - (trifluoromethyl)benzamide,
   N-{[3-(1-ethyl-1H-pyrazol-4-yl)phenyl](1-hydroxycyclopentyl)methyl isoquinoline-1-carboxamide,
   3-chloro-N-[(3-fluorophenyl)(1-hydroxycyclopentyl)methyl]-4-(trifluoromethyl)pyridine-2-carboxamide,
   3-chloro-N-[(3-chloro-5-fluorophenyl)(1-hydroxycyclopentyl)methyl]-4-(trifluoromethyl)pyridine-2-carboxamide,
   3-chloro-N-[(2-chlorophenyl)(4,4-difluoro-1-hydroxycyclohexyl)methyl]-4-(trifluoromethyl)pyridine-2-carboxamide,
   N-[(2-chloro-3-fluorophenyl)(cis-4-fluoro-1-hydroxycyclohexyl)methyl]-3-(2H-1,2,3-triazol-2-yl)-5-(trifluoromethyl)pyridine-2-carboxamide,
   3-chloro-N-[(2-chloro-3-fluorophenyl)(cis-4-fluoro-1-hydroxycyclohexyl)methyl]-4-(difluoromethyl)pyridine-2-carboxamide,
   3-chloro-N-[(4,4-difluoro-1-hydroxycyclohexyl)(2,3-difluorophenyl)methyl]-4-(trifluoromethyl)pyridine-2-carboxamide,
   N-[(2-chlorophenyl)(4,4-difluoro-1-hydroxycyclohexyl)methyl]-5-(methylsulfonyl)-2-(trifluoromethyl)pyridine-4-carboxamide,
   2-chloro-N-[(2-chloro-3-fluorophenyl)(cis-4-fluoro-l-hydroxycyclohexyl)methyl]-5-(trifluoromethyl)benzamide,
   3-chloro-N-[(4,4-difluoro-1-hydroxycyclohexyl)(3-fluorophenyl)methyl]-4-(trifluoromethyl)pyridine-2-carboxamide,
   N-[(2-chloro-3-fluorophenyl)(cis-4-fluoro-1-hydroxycyclohexyl)methyl]isoquinoline-1-carboxamide,
   N-[(2-chloro-3-fluorophenyl)(cis-4-fluoro-1-hydroxycyclohexyl)methyl]-2-(1H-pyrazol-1-yl)-4-(trifluoromethyl)benzamide,
   3-chloro-N-{(S)-(1-hydroxycyclopentyl)[3-(pyrimidin-2-yl)phenyl]methyl}-4-(trifluoromethyl)pyridine-2-carboxamide,
   3-chloro-N-[(2-chloro-4-fluorophenyl)(1-hydroxycyclopentyl)methyl]-4-(trifluoromethyl)pyridine-2-carboxamide,
   3-chloro-N-[(4,4-difluoro-1-hydroxycyclohexyl){{3-[1-(2,2,2-trifluoroethyl)-1H-pyrazol-4-yl]phenyl}}methyl]-4-(trifluoromethyl)pyridine-2-carboxamide,
   3-chloro-N-[(2-chloro-3-fluorophenyl)(4,4-difluoro-1-hydroxycyclohexyl)methyl]-2-(trifluoromethyl)pyridine-4-carboxamide,
   3-chloro-N-[(3-chloro-2-fluorophenyl)(4-fluoro-1-hydroxycyclohexyl)methyl]-2-(trifluoromethyl)pyridine-4-carboxamide,
   N-[(6-chloropyridin-2-yl)(4,4-difluoro-1-hydroxycyclohexyl)methyl]isoquinoline-1-carboxamide,
   3-chloro-N-[(4-chloropyridin-2-yl)(cis-4-fluoro-1-hydroxycyclohexyl)methyl]-4-(trifluoromethyl)pyridine-2-carboxamide,
   3-chloro-N-{{(4,4-difluoro-1-hydroxycyclohexyl)[3-(pyridin-3-yl)phenyl]methyl}-4-(trifluoromethyl)pyridine-2-carboxamide,
   3-chloro-N-[(3-chlorophenyl)(1-hydroxycyclopentyl)methyl]-2-(trifluoromethyl)pyridine-4-carboxamide,
   2-chloro-N-[(6-chloropyridin-2-yl)(cis-4-fluoro-1-hydroxycyclohexyl)methyl]-6-(trifluoromethyl)pyridine-3-carboxamide,
   3-chloro-N-[(cis-4-fluoro-1-hydroxycyclohexyl)(6-fluoropyridin-2-yl)methyl]-4-(trifluoromethyl)pyridine-2-carboxamide,
   3-chloro-N-[(4,4-difluoro-1-hydroxycyclohexyl)(6-fluoropyridin-2-yl)methyl]-4-(trifluoromethyl)pyridine-2-carboxamide,
   3-chloro-N-[(1-hydroxycyclopentyl)(3-methoxyphenyl)methyl]-4-(trifluoromethyl)pyridine-2-carboxamide,
   N-[(S)-(3-bromophenyl)(trans-4-fluoro-1-hydroxycyclohexyl)methyl]-3-chloro-4-(trifluoromethyl)pyridine-2-carboxamide,
   3-chloro-N-[(S)-(3-chlorophenyl)(cis-4-fluoro-1-hydroxycyclohexyl)methyl]-2-(trifluoromethyl)pyridine-4-carboxamide,
   5-chloro-N-[(2-chloro-3-fluorophenyl)(cis-4-fluoro-1-hydroxycyclohexyl)methyl]-2-(trifluoromethyl)pyridine-4-carboxamide,
   2-chloro-N-[(2-chloro-3-fluorophenyl)(cis-4-fluoro-1-hydroxycyclohexyl)methyl]-5-(trifluoromethyl)pyridine-3-carboxamide,
   5-chloro-N-[(6-chloropyridin-2-yl)(4,4-difluoro-1-hydroxycyclohexyl)methyl]-2-(trifluoromethyl)pyridine-4-carboxamide,
   2-chloro-N-[(6-chloropyridin-2-yl)(4,4-difluoro-1-hydroxycyclohexyl)methyl]-3-(trifluoromethyl)benzamide,
   2-chloro-N-[(4-chloropyridin-2-yl)(4,4-difluoro-1-hydroxycyclohexyl)methyl]-3-(trifluoromethyl)benzamide,
   3-chloro-N-{(1-hydroxycyclopentyl)[3-(1H-pyrazol-1-yl)phenyl]methyl}-4-(trifluoromethyl)pyridine-2-carboxamide,
   3-chloro-N-[(2,3-dichlorophenyl)(4,4-difluoro-1-hydroxycyclohexyl)methyl]-4-(trifluoromethyl)pyridine-2-carboxamide,
   3-chloro-N-{(4,4-difluoro-1-hydroxycyclohexyl)[3-(1-ethyl-1H-pyrazol-4-yl)phenyl]methyl}-2-(trifluoromethyl)pyridine-4-carboxamide,
   3-chloro-N-[(2-chloro-5-fluorophenyl)(cis-4-fluoro-1-hydroxycyclohexyl)methyl]-4-(trifluoromethyl)pyridine-2-carboxamide,
   N-[(2-chlorophenyl)(4,4-difluoro-1-hydroxycyclohexyl)methyl]-2-(methylsulfonyl)-5-(trifluoromethyl)pyridine-3-carboxamide,
   N-[(3-bromophenyl)(1-hydroxycyclopentyl)methyl]-3-chloro-4-(trifluoromethyl)pyridine-2-carboxamide,
   3-chloro-N-[(2-chlorophenyl)(4,4-difluoro-1-hydroxycyclohexyl)methyl]-2-(trifluoromethyl)pyridine-4-carboxamide,
   3-chloro-N-[(2-chlorophenyl)(cis-4-fluoro-1-hydroxycyclohexyl)methyl]-2-(trifluoromethyl)pyridine-4-carboxamide,
   3-chloro-N-[(2-fluorophenyl)(1-hydroxycyclopentyl)methyl]-4-(trifluoromethyl)pyridine-2-carboxamide,
   3-chloro-N-[(3-chloro-5-fluorophenyl)(1-hydroxycyclopentyl)methyl]-4-(trifluoromethyl)pyridine-2-carboxamide,
   3-chloro-N-[(3-chlorophenyl)(4,4-difluoro-1-hydroxycyclohexyl)methyl]-4-(trifluoromethyl)pyridine-2-carboxamide,
   2,6-dichloro-N-{(4,4-difluoro-1-hydroxycyclohexyl)[3-(1-ethyl-1H-pyrazol-4-yl)phenyl]methyl}-3-(trifluoromethyl)benzamide,
   2-chloro-N-[(S)-(6-chloropyridin-2-yl)(cis-4-fluoro-1-hydroxycyclohexyl)methyl]-5-(trifluoromethyl)benzamide,
   3-chloro-N-[(2-fluorophenyl)(1-hydroxycyclopentyl)methyl]-4-(trifluoromethyl)pyridine-2-carboxamide,
   3-chloro-N-{(4,4-difluoro-1-hydroxycyclohexyl)[3-(pyridin-2-yl)phenyl]methyl}-2-(trifluoromethyl)pyridine-4-carboxamide,
   3-chloro-N-[(2-chlorophenyl)(4-fluoro-1-hydroxycyclohexyl)methyl]-4-(trifluoromethyl)pyridine-2-carboxamide,
   5-chloro-N-[(2-chloropyridin-3-yl)(cis-4-fluoro-1-hydroxycyclohexyl)methyl]-2-(trifluoromethyl)pyridine-4-carboxamide,
   3-chloro-N-[(6-chloropyridin-2-yl)(cis-4-fluoro-1-hydroxycyclohexyl)methyl]-4-(difluoromethyl)pyridine-2-carboxamide,
   N-[(2-chlorophenyl)(4,4-difluoro-1-hydroxycyclohexyl)methyl]-2-(methylsulfonyl)-4-(trifluoromethyl)benzamide,
   3-chloro-N-{(4,4-difluoro-1-hydroxycyclohexyl)[3-(pyridin-2-yl)phenyl]methyl}-4-(trifluoromethyl)pyridine-2-carboxamide,
   3-chloro-N-[(4,4-difluoro-1-hydroxycyclohexyl)(2,3-difluorophenyl)methyl]-2-(trifluoromethyl)pyridine-4-carboxamide,
   3-chloro-N-[(3-chlorophenyl)(4,4-difluoro-1-hydroxycyclohexyl)methyl]-2-(trifluoromethyl)pyridine-4-carboxamide,
   2-chloro-N-{(4,4-difluoro-1-hydroxycyclohexyl)[3-(1-ethyl-1H-pyrazol-4-yl)phenyl]methyl}-5-(trifluoromethyl)benzamide,
   3-chloro-N-[(S)-(3-chlorophenyl)(trans-4-fluoro-1-hydroxycyclohexyl)methyl]-4-(trifluoromethyl)pyridine-2-carboxamide,
   N-{(4,4-difluoro-1-hydroxycyclohexyl)[3-(1-ethyl-1H-pyrazol-4-yl)phenyl]methyl -4-fluoro-2-(trifluoromethyl)benzamide,
   2-chloro-N-[(3-fluorophenyl)(1-hydroxycyclopentyl)methyl]-3-(trifluoromethyl)benzamide,
   3-chloro-N-[(3,5-dichlorophenyl)(1-hydroxycyclopentyl)methyl]-4-(trifluoromethyl)pyridine-2-carboxamide,
   2-chloro-N-{(4,4-difluoro-1-hydroxycyclohexyl)[3-(1-ethyl-1H-pyrazol-4-yl)phenyl]methyl}-3-(trifluoromethyl)benzamide,
   2-chloro-N-[(6-chloropyridin-2-yl)(4,4-difluoro-1-hydroxycyclohexyl)methyl]-5-(trifluoromethyl)pyridine-3-carboxamide,
   3-chloro-N-[(cis-4-fluoro-1-hydroxycyclohexyl)(phenyl)methyl]-4-(trifluoromethyl)pyridine-2-carboxamide,
   3-chloro-N-[(4,4-difluoro-1-hydroxycyclohexyl)(3-fluorophenyl)methyl]-2-(trifluoromethyl)pyridine-4-carboxamide,
   N-[(2-chloro-3-fluorophenyl)(4,4-difluoro-1-hydroxycyclohexyl)methyl]-2-(methylsulfonyl)-4-(trifluoromethyl)benzamide,
   3-chloro-N-[(trans-4-fluoro-1-hydroxycyclohexyl)(2-fluorophenyl)methyl]-4-(trifluoromethyl)pyridine-2-carboxamide,
   3-chloro-N-[(5-chloropyridin-3-yl)(cis-4-fluoro-1-hydroxycyclohexyl)methyl]-4-(trifluoromethyl)pyridine-2-carboxamide,
   3-chloro-N-[(1-hydroxycyclopentyl)(3-methylphenyl)methyl]-4-(trifluoromethyl)pyridine-2-carboxamide,
   3-chloro-N-[(4,4-difluoro-1-hydroxycyclohexyl)(3-fluorophenyl)methyl]-4-(trifluoromethyl)pyridine-2-carboxamide,
   2-chloro-N-[(6-chloropyridin-2-yl)(cis-4-fluoro-1-hydroxycyclohexyl)methyl]-4-(trifluoromethyl)benzamide,
   3-chloro-N-[(3-chloropyridin-2-yl)(4,4-difluoro-1-hydroxycyclohexyl)methyl]-4-(trifluoromethyl)pyridine-2-carboxamide,
   3-chloro-N-[(4,4-difluoro-1-hydroxycyclohexyl)(6-fluoropyridin-2-yl)methyl]-2-(trifluoromethyl)pyridine-4-carboxamide,
   3-chloro-N-[(5-chloro-2-fluorophenyl)(cis-4-fluoro-1-hydroxycyclohexyl)methyl]-4-(trifluoromethyl)pyridine-2-carboxamide,
   3-chloro-N-[(4,4-difluoro-1-hydroxycyclohexyl)(3-fluorophenyl)methyl]-4-(trifluoromethyl)pyridine-2-carboxamide,
   N-[(3-bromophenyl)(4-fluoro-1-hydroxycyclohexyl)methyl]-3-chloro-4-(trifluoromethyl)pyridine-2-carboxamide,
   3-chloro-N-[(2-chlorophenyl)(4,4-difluoro-1-hydroxycyclohexyl)methyl]-4-(trifluoromethyl)pyridine-2-carboxamide,
   2-chloro-N-[(6-chloropyridin-2-yl)(4,4-difluoro-1-hydroxycyclohexyl)methyl]-6-(trifluoromethyl)pyridine-3-carboxamide,
   3-chloro-N-[4,4-difluoro-1-hydroxycyclohexyl)(2-fluorophenyl)methyl]-4-(trifluoromethyl)pyridine-2-carboxamide,
   3-chloro-N-[(S)-(3-cyanophenyl)(1-hydroxycyclopentyl)methyl]-4-(trifluoromethyl)pyridine-2-carboxamide,
   2-chloro-N-{(4,4-difluoro-1-hydroxycyclohexyl)[3-(1-ethyl-1H-pyrazol-4-yl)phenyl]methyl}-3-(trifluoromethyl)benzamide,
   3-chloro-N-[(2,5-dichlorophenyl)(4,4-difluoro-1-hydroxycyclohexyl)methyl]-4-(trifluoromethyl)pyridine-2-carboxamide,
   N-[(2-chloro-3-fluorophenyl)(cis-4-fluoro-1-hydroxycyclohexyl)methyl]-2-(methylsulfonyl)-6-(trifluoromethyl)pyridine-3-carboxamide,
   3-chloro-N-[(4-chloropyridin-2-yl)(4,4-difluoro-1-hydroxycyclohexyl)methyl]-4-(trifluoromethyl)pyridine-2-carboxamide,
   N-[(2-chloro-3-fluorophenyl)(cis-4-fluoro-1-hydroxycyclohexyl)methyl]-2-(2H-1,2,3-triazol-2-yl)-4-(trifluoromethyl)benzamide,
   2-chloro-N-[(4-chloropyridin-2-yl)(cis-4-fluoro-1-hydroxycyclohexyl)methyl]-6-(trifluoromethyl)pyridine-3-carboxamide,
   2-chloro-N-[(3-fluorophenyl)(1-hydroxycyclopentyl)methyl]-3-(trifluoromethyl)benzamide,
   3-chloro-N-[(3-chloro-4-fluorophenyl)(1-hydroxycyclopentyl)methyl]-4-(trifluoromethyl)pyridine-2-carboxamide,
   4-chloro-N-[(2-chloro-3-fluorophenyl)(cis-4-fluoro-1-hydroxycyclohexyl)methyl]-3-methyl pyridine-2-carboxamide,
   3-chloro-N-[(1-hydroxycyclopentyl)(2-methylphenyl)methyl]-4-(trifluoromethyl)pyridine-2-carboxamide,
   2,6-dichloro-N-{(4,4-difluoro-1-hydroxycyclohexyl)[3-(1-ethyl-1H-pyrazol-4-yl)phenyl]methyl}-3-(trifluoromethyl)benzamide,
   3-chloro-N-[(2,5-dichlorophenyl)(4,4-difluoro-1-hydroxycyclohexyl)methyl]-4-(trifluoromethyl)pyridine-2-carboxamide,
   2-chloro-N-[(2-chloro-3-fluorophenyl)(cis-4-fluoro-1-hydroxycyclohexyl)methyl]-6-(trifluoromethyl)pyridine-3-carboxamide,
   N-[(4-chloropyridin-2-yl)(cis-4-fluoro-1-hydroxycyclohexyl)methyl]-2-(methylsulfonyl)-5-(trifluoromethyl)benzamide,
   2-chloro-N-{(1-hydroxycyclopentyl)[3-(pyrimidin-5-yl)phenyl]methyl}-3-(trifluoromethyl)benzamide,
   N-{(4,4-difluoro-1-hydroxycyclohexyl)[3-(1-ethyl-1H-pyrazol-4-yl)phenyl]methyl}naphthalene-1-carboxamide,
   3-chloro-N-[(3-cyanophenyl)(1-hydroxycyclopentyl)methyl]-4-(trifluoromethyl)pyridine-2-carboxamide,
   3-chloro-N-[(3-ethoxyphenyl)(1-hydroxycyclopentyl)methyl]-4-(trifluoromethyl)pyridine-2-carboxamide,
   N-[(3-bromophenyl)(4,4-difluoro-1-hydroxycyclohexyl)methyl]-3-chloro-4-(trifluoromethyl)pyridine-2-carboxamide,
   3-chloro-N-[(3-chloropyridin-2-yl)(cis-4-fluoro-1-hydroxycyclohexyl)methyl]-4-(trifluoromethyl)pyridine-2-carboxamide,
   3-chloro-N-{(1-hydroxycyclopentyl)[3-(2H-1,2,3-triazol-2-yl)phenyl]methyl}-4-(trifluoromethyl)pyridine-2-carboxamide,
   3-chloro-N-{(4,4-difluoro-1-hydroxycyclohexyl)[3-(1-ethyl-1H-pyrazol-4-yl)phenyl]methyl}-2-methylbenzamide,
   5-chloro-N-[(2-chlorophenyl)(cis-4-fluoro-1-hydroxycyclohexyl)methyl]-2-(trifluoromethyl)pyridine-4-carboxamide,
   N-[(3-bromophenyl)(4,4-difluoro-1-hydroxycyclohexyl)methyl]-3-chloro-4-(trifluoromethyl)pyridine-2-carboxamide,
   3-chloro-N-{(4,4-difluoro-1-hydroxycyclohexyl)[3-(pyrazin-2-yl)phenyl]methyl}-4-(trifluoromethyl)pyridine-2-carboxamide,
   N-[(2-chlorophenyl)(4,4-difluoro-1-hydroxycyclohexyl)methyl]-2-(methylsulfonyl)-6-(trifluoromethyl)pyridine-3-carboxamide,
   3-chloro-N-[(2-chloropyridin-3-yl)(4,4-difluoro-1-hydroxycyclohexyl)methyl]-4-(trifluoromethyl)pyridine-2-carboxamide,
   3-chloro-N-[(2-chloropyridin-3-yl)(cis-4-fluoro-1-hydroxycyclohexyl)methyl]-4-(trifluoromethyl)pyridine-2-carboxamide,
   2-chloro-N-[(2-chloropyridin-3-yl)(4,4-difluoro-1-hydroxycyclohexyl)methyl]-3-(trifluoromethyl)benzamide,
   N-[(2-chlorophenyl)(4,4-difluoro-1-hydroxycyclohexyl)methyl]-2-(methylsulfonyl)-5-(trifluoromethyl)benzamide,
   N-{(4,4-difluoro-1-hydroxycyclohexyl)[3-(-ethyl-1H-pyrazol-4-yl)phenyl]methyl}-4-fluoro-2-(trifluoromethyl)benzamide,
   N-{(4,4-difluoro-1-hydroxycyclohexyl)[3-(1-ethyl-1H-pyrazol-4-yl)phenyl]methyl}-2-methyl-3-(trifluoromethyl)benzamide,
   3-chloro-N-[(cis-4-fluoro-1-hydroxycyclohexyl)(phenyl)methyl]-4-(trifluoromethyl)pyridine-2-carboxamide,
   2-chloro-N-[(2-chloropyridin-3-yl)(cis-4-fluoro-1-hydroxycyclohexyl)methyl]-3-(trifluoromethyl)benzamide,
   3-chloro-N-[(4-chloropyridin-2-yl)(4,4-difluoro-1-hydroxycyclohexyl)methyl]-2-(trifluoromethyl)pyridine-4-carboxamide,
   2-chloro-N-{(1-hydroxycyclopentyl)[3-(pyridin-3-yl)phenyl]methyl}-3-(trifluoromethyl)benzamide,
   3-chloro-N-[(1-hydroxycyclopentyl)(3-methoxyphenyl)methyl]-4-(trifluoromethyl)pyridine-2-carboxamide,
   3-chloro-N-[(4-chloropyridin-3-yl)(cis-4-fluoro-1-hydroxycyclohexyl)methyl]-4-(trifluoromethyl)pyridine-2-carboxamide,
   3-chloro-N-[(6-chloropyridin-2-yl)(4,4-difluoro-1-hydroxycyclohexyl)methyl]-2-(trifluoromethyl)pyridine-4-carboxamide,
   2-chloro-N-[(2-chlorophenyl)(cis-4-fluoro-1-hydroxycyclohexyl)methyl]-5-(trifluoromethyl)pyridine-3-carboxamide,
   3-chloro-N-[(3-chlorophenyl)(cis-4-fluoro-1-hydroxycyclohexyl)methyl]-2-(trifluoromethyl)pyridine-4-carboxamide,
   3-chloro-N-[(3-chloro-4-fluorophenyl)(cis-4-fluoro-1-hydroxycyclohexyl)methyl]-4-(trifluoromethyl)pyridine-2-carboxamide,
   3-chloro-N-[(cis-4-fluoro-1-hydroxycyclohexyl)(2-fluorophenyl)methyl]-4-(trifluoromethyl)pyridine-2-carboxamide,
   2-chloro-N-{(1-hydroxycyclopentyl)[3-(pyridin-4-yl)phenyl]methyl}-3-(trifluoromethyl)benzamide,
   2-chloro-N-[(3-cyanophenyl)(1-hydroxycyclopentyl)methyl]-3-(trifluoromethyl)benzamide,
   2-chloro-N-[(2-chloro-3-fluorophenyl)(cis-4-fluoro-1-hydroxycyclohexyl)methyl]-4-(trifluoromethyl)benzamide,
   N-[(3-bromophenyl)(1-hydroxycyclohexa-3-en-1-yl)methy1]-3-chloro-4-(trifluoromethyl)pyridine-2-carboxamide,
   5-chloro-N-[(2-chloro-3-fluorophenyl)(cis-4-fluoro-1-hydroxycyclohexyl)methyl]-2-(trifluoromethyl)benzamide,
   2,5-dichloro-N-[(2-chloro-3-fluorophenyl)(cis-4-fluoro-1-hydroxycyclohexyl)methyl]benzamide,
   3 -chloro-N-[(3-chloro-4-fluorophenyl)(1-hydroxycyclopentyl)methyl]-4-(trifluoromethyl)pyridine-2-carboxamide,
   N-{(4,4-difluoro-1-hydroxycyclohexyl)[3-(1-ethyl-1H-pyrazol-4-yl)phenyl]methyl}isoquinoline-1-carboxamide,
   2-chloro-N-[(2-chloropyridin-3-yl)(cis-4-fluoro-1-hydroxycyclohexyl)methyl]-5-(trifluoromethyl)benzamide,
   N-[(2-chlorophenyl)(4,4-difluoro-1-hydroxycyclohexyl)methyl]-3-(2H-1,2,3-triazol-2-yl)-5-(trifluoromethyl)pyridine-2-carboxamide,
   2-chloro-N-[(2-chloro-3-fluorophenyl)(cis-4-fluoro-1-hydroxycyclohexyl)methyl]-5-methoxybenzamide,
   N-[(3-chloro-2-fluorophenyl)(4,4-difluoro-1-hydroxycyclohexyl)methyl]-2-(methylsulfonyl)-4-(trifluoromethyl)benzamide,
   3-chloro-N-{(1-hydroxycyclopentyl)[3-(1H-1,2,3-triazol-1-yl)phenyl]methyl}-4-(trifluoromethyl)pyridine-2-carboxamide,
   3-chloro-N-[(2-chloro-3-fluorophenyl)(cis-4-fluoro-1-hydroxycyclohexyl)methyl]-4-(trifluoromethyl)pyridine-2-carboxamide,
   2,3-dichloro-N-{(4,4-difluoro-1-hydroxycyclohexyl)[3-(1-ethyl-1H-pyrazol-4-yl)phenyl]methyl}benzamide,
   3-chloro-N-[(1-hydroxycyclopentyl)(phenyl)methyl]-4-(trifluoromethyl)pyridine-2-carboxamide,
   5-chloro-N-[(4-chloropyridin-2-yl)(4,4-difluoro-1-hydroxycyclohexyl)methyl]-2-(trifluoromethyl)pyridine-4-carboxamide,
   2-chloro-N-{(4,4-difluoro-1-hydroxycyclohexyl)[3-(pyridin-2-yl)phenyl]methyl}-5-(trifluoromethyl)benzamide,
   3-chloro-N-[(2-chlorophenyl)(4,4-difluoro-1-hydroxycyclohexyl)methyl]-2-(trifluoromethyl)pyridine-4-carboxamide.
(17) A pharmaceutical composition comprising, as an active ingredient, the compound or pharmaceutically acceptable salt thereof according to any one of (1) to (16).
(18) An agent for preventing or treating diseases of schizophrenia, Alzheimer's disease, cognitive impairment, dementia, anxiety disorders, depression, drug dependence, spasm, tremor, pain, Parkinson's disease, attention deficit hyperactivity disorder, bipolar disorder, eating disorder, or sleep disorders, which comprises, as an active ingredient, the compound or pharmaceutically acceptable salt thereof according to any one of (1) to (16).

### ADVANTAGEOUS EFFECTS OF INVENTION

The inventive compounds have glycine transporter (GlyT1)-inhibiting activity.

### DESCRIPTION OF EMBODIMENTS

The term "C₁₋₆ alkyl group" as used herein refers to a straight-chain or branched-chain alkyl group having 1 to 6 carbon atoms, and includes, for example, a methyl group, an ethyl group, a n-propyl group, an isopropyl group, a n-butyl group, an isobutyl group, a tert-butyl group, a n-pentyl group, an isopentyl group, and a n-hexyl group.

The term "C₁₋₆ alkoxy group" as used herein refers to a straight-chain or branched-chain alkoxy group having 1 to 6 carbon atoms, and includes, for example, a methoxy group, an ethoxy group, a n-propoxy group, an isopropoxy group, a n-butoxy group, an isobutoxy group, a n-pentyloxy group, an isopentyloxy group, and a n-hexyloxy group.

The term "C₂₋₇ alkoxycarbonyl group" as used herein refers to a group composed of "C₁₋₆ alkoxy group" defined above and a carbonyl group and includes, for example, methoxycarbonyl, and ethoxycarbonyl.

The term "C₁₋₆ alkanoyl group" as used herein refers to a straight-chain or branched-chain alkanoyl group having 1 to 6 carbon atoms, and includes, for example, a formyl group, an acetyl group, a propanoyl group, a butanoyl group, and a pivaloyl group.

The term "halogen (halo)" as used herein refers to fluorine, chlorine, bromine, or iodine.

The term "haloC₁₋₆ alkyl group" as used herein refers to a straight-chain or branched-chain alkyl group which has 1 to 6 carbon atoms and which has been substituted by a halogen atom or halogen atoms. The preferred number of the substituting halogen atom(s) is 1 to 3. Examples of the haloC₁₋₆ alkyl group include a fluoromethyl group, a difluoromethyl group, a trifluoromethyl group, and a trichloromethyl group.

The term "haloC₁₋₆ alkoxy group" as used herein refers to a straight-chain or branched-chain alkoxy group which has 1 to 6 carbon atoms and which has been substituted by a halogen atom or halogen atoms. The preferred number of the substituting halogen atom(s) is 1 to 3. Examples of the haloC₁₋₆ alkoxy group include a fluoromethoxy group, a difluoromethoxy group, and a trifluoromethoxy group.

The term "C₁₋₆ alkylsulfonyl group" as used herein refers to a group composed of "C₁₋₆ alkoxy group" defined above and a sulfonyl group and includes, for example, a methylsulfonyl group, an ethylsulfonyl group, a n-propylsulfonyl group, an isopropylsulfonyl group, a n-butylsulfonyl group, an isobutylsulfonyl group, a tert-butylsulfonyl group, a n-pentylsulfonyl group, an isopentylsulfonyl group, and a n-hexylsulfonyl group.

The term "C₃₋₆ cycloalkyl group" as used herein refers to a cycloalkyl group having 3 to 6 carbon atoms, and includes, for example, a cyclopropyl group, a cyclobutyl group, a cyclopentyl group, and a cyclohexyl group.

The term "monocyclic heteroaryl group" as used herein refers to a monocyclic heteroaryl group having in the ring at least one atom selected from the group consisting of a nitrogen atom, an oxygen atom, and a sulfur atom. When the monocyclic heteroaryl group has a nitrogen atom or nitrogen atoms in the ring, the each nitrogen atom may be an N-oxide.

The monocyclic heteroaryl group is preferably a 5- or 6-membered heteroaryl group, and includes, for example, a pyridyl group, a pyridazyl group, a pyrimidyl group, a pyrazyl group, a pyrazolyl group, a thiazolyl group, an imidazolyl group, an oxazolyl group, an isoxazolyl group, a thienyl group, a triazolyl group, an oxadiazolyl group, and a thiadiazolyl group.

The term "pharmaceutically acceptable salt" as used herein refers to an acid addition salt that may be accepted in pharmaceutical terms. Examples of the acid that may be used include inorganic acids such as sulfuric acid, hydrochloric acid, hydrobromic acid, nitric acid and phosphoric acid, and organic acids such as acetic acid, oxalic acid, lactic acid, citric acid, malic acid, gluconic acid, tartaric acid, fumaric acid, maleic acid, methanesulfonic acid, ethanesulfonic acid, benzenesulfonic acid and p-toluenesulfonic acid. The free forms may be converted to these salts in a conventional manner.

One of preferable embodiments of the compounds according to the present invention will be described below. Of the following preferable embodiments, compounds satisfying two or more conditions are more preferable.

Preferable are compounds wherein the double line with one line being dotted is a single bond.

Preferable are compounds wherein Ar¹ is a phenyl group optionally substituted with one to three identical or different substituents selected from substituent group 1A, a pyridyl group substituted with one or two identical or different substituents selected from substituent group 1A, or an isoquinolyl group; more preferable are compounds wherein Ar¹ is a pyridyl group substituted with one or two identical or different substituents selected from substituent group 1A; yet more preferable are compounds wherein Ar¹ is a pyridyl group substituted with one or two identical or different substituents selected from a halogen atom, a C₁₋₆ haloalkyl group, a C₁₋₆ alkylsulfonyl group, a pyrazolyl group and a triazolyl group; and most preferable are compounds wherein Ar¹ is a pyridyl group substituted with two identical or different substituents selected from a halogen atom and a C₁₋₆ haloalkyl group.

Note that, in the aforementioned "substituent group 1A", more preferable groups are a halogen atom, a C₁₋₆ haloalkyl group, a C₁₋₆ alkylsulfonyl group, a pyrazolyl group and a triazolyl group.

Further, in the aforementioned "pyridyl group substituted with two identical or different substituents selected from a halogen atom and a C₁₋₆ haloalkyl group", more preferable is a pyridyl group substituted with two substituents selected from a halogen atom and a trifluoromethyl group, and yet more preferable is 2-chloropyridin-4-yl substituted with one trifluoromethyl group.

Preferable are compounds wherein ring A is a phenyl group or a pyridyl group, and more preferable are compounds wherein ring A is a pyridyl group.

Preferable are compounds wherein R¹ and R² are the same or different and are each a hydrogen atom, a halogen atom, or a monocyclic heteroaryl group optionally substituted with one substituent selected from substituent group 4A; more preferable are compounds wherein R¹ and R² are the same or different and are each a hydrogen atom or a halogen atom, or a pyrazolyl group, a triazolyl group, a pyridyl group or a pyrazinyl group which are optionally substituted with one substituent selected from substituent group 4A; yet more preferable are compounds wherein R¹ and R² are the same or different and are each a hydrogen atom or a halogen atom; particularly preferable are compounds wherein R¹ is a hydrogen atom and R² is a halogen atom; and most preferable are compounds wherein R¹ is a hydrogen atom and R² is a chlorine atom.

Preferable are compounds wherein R^{a} is a hydrogen atom.

Preferable are compounds wherein R³ and R⁴ are the same or different and are each a hydrogen atom or a halogen atom; more preferable are compounds wherein R³ and R⁴ are the same or different and are each a hydrogen atom or a fluorine atom; yet more preferable are compounds wherein R³ is a fluorine atom, and R⁴ is a hydrogen atom or a fluorine atom.

Preferable are compounds wherein R^{b} is a hydrogen atom.

Preferable are compounds wherein n is 1 or 2; and more preferable are compounds wherein n is 2.

The carbon atom to which the nitrogen atom in the amide structure is attached preferably has the following configuration.

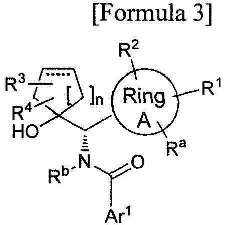

Other preferable embodiments of the compounds according to the present invention will be described below. Of the following preferable embodiments, compounds satisfying two or more conditions are more preferable.

Preferable are compounds wherein the double line with one line being dottedis a single bond, are preferable.

Preferable are compounds wherein Ar¹ is a phenyl group optionally substituted with one to three substituents selected from substituent group 1, or a pyridyl group substituted with one to two substituents selected from substituent group 1; more preferable are compounds wherein Ar¹ is a pyridyl group substituted with one to two substituents selected from substituent group 1; yet more preferable are compounds wherein Ar¹ is a pyridyl group substituted with one to two substituents selected from a halogen atom and a C₁₋₆ haloalkyl group; and most preferable are compounds wherein Ar¹ is a pyridyl group substituted with two substituents selected from a halogen atom and a C₁₋₆ haloalkyl group.

Preferable are compounds wherein R¹ and R² are the same or different and are each a hydrogen atom, a halogen atom, or a monocyclic heteroaryl group optionally substituted with a substituent selected from substituent group 4; more preferable are compounds wherein R¹ and R² are the same or different and are each a hydrogen atom or a halogen atom; yet more preferable are compounds wherein R¹ is a hydrogen atom and R² is a halogen atom.

Preferable are compounds wherein R³ and R⁴ are the same or different and are each a hydrogen atom or a halogen atom.

Preferable are compounds wherein n is 1 or 2.

The carbon atom to which the nitrogen atom in the amide structure is attached preferably has the following configuration.

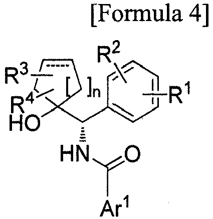

A preferable embodiment of the compounds according to the present invention is the compound represented by the following general formula (IA-a), or a pharmaceutically acceptable salt thereof.

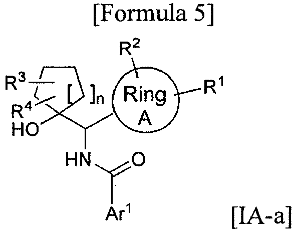

wherein preferable embodiments of Ar¹, ring 1, R¹, R², R³ R⁴, and n are as described above.

More preferable embodiments are those wherein
Ar¹ is a phenyl group optionally substituted with one or two identical or different substituents selected from a halogen atom, a C₁₋₆ haloalkyl group, a C₁₋₆ alkylsulfonyl group, a pyrazolyl group and a triazolyl group, or a pyridyl group substituted with one or two identical or different substituents selected from a halogen atom, a C₁₋₆ haloalkyl group, a C₁₋₆ alkylsulfonyl group, a pyrazolyl group and a triazolyl group,
ring A is a phenyl group or a pyridyl group,
R¹ and R² are the same or different and are each a hydrogen atom or a halogen atom, or a pyrazolyl group, a triazolyl group, a pyridyl group or a pyrazinyl group which are optionally substituted with one substituent selected from substituent group 4A,
R³ and R⁴ are the same or different and are each a hydrogen atom or a halogen atom,
n is 1 or 2, and
the carbon atom to which the nitrogen atom in the amide structure is attached has the following configuration.

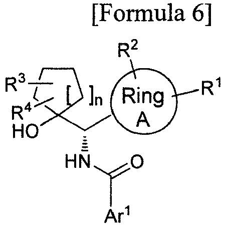

Furthermore preferable embodiments are those wherein
Ar¹ are the same or different and is a pyridyl groups substituted with two substituents selected from a halogen atom and a trifluoromethyl group,
ring A is a pyridyl group,
R¹ and R² are the same ore different and are each a hydrogen atom or a halogen atom,
R³ and R⁴ are the same or different and are each a hydrogen atom or a fluorine atom,
n is 2, and
the carbon atom to which the nitrogen atom in the amide structure is attached preferably has the following configuration,

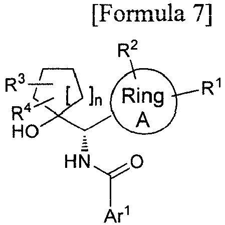

Particularly preferable embodiments are those wherein
Ar¹ is a 2-chloropyridin-4-yl group substituted with one trifluoromethyl group,
ring A is a pyridyl group,
R¹ is a hydrogen atom,
R² is a chlorine atom,
R³ is a fluorine atom,
R⁴ is a hydrogen atom or a fluorine atom,
n is 2, and
the carbon atom to which the nitrogen atom in the amide structure is attached has the following configuration,

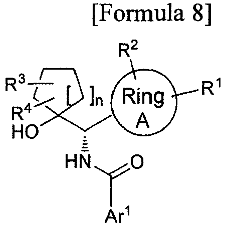

The above compound groups wherein Ar¹ is a 2-chloropyridin-4-yl group substituted with one trifluoromethyl group have much stronger glycine transporter-inhibiting action. Further, the above compound groups having a cyclohexane ring wherein R³ is a fluorine atom are metabolically stable and have useful properties to be a pharmaceutical product.

The inventive compounds may contain a plurality of asymmetric centers. Thus, the aforementioned compounds may exist not only in optically active forms but also in their racemates. Further, a plurality of diastereomers may also exist. All of these forms are included in the scope of the present invention. Individual isomers may be obtained by known methods, for example, by use of optically active starting materials or intermediates, by an optically selective reaction or a diastereoselective reaction in the preparation of intermediates or final products, or by chromatographic separation or the like in the preparation of intermediates or final products. If the inventive compounds form hydrates or solvates, these hydrates or solvates are also included in the scope of the present invention. Likewise, pharmaceutically acceptable salts of hydrates or solvates of the inventive compounds are also included in the scope of the present invention.

The compounds according to the present invention also encompass compounds in which one or more hydrogen atoms, carbon atoms, nitrogen atoms, oxygen atoms or halogen atoms are replaced by their radioisotopes or stable isotopes. These labeled compounds are useful for metabolism and/or pharmacokinetics study, biological analysis as receptor ligands, or other purposes.

The compound according to the present invention may be administered orally or parenterally. In addition, an agent comprising the compound according to the present invention as an active ingredient may be administered orally or parenterally. The parenteral administration includes, for example, intravenous, nasal, transdermal, subcutaneous, intramuscular or sublingual administration.

The compounds according to the present invention may be administered in doses which, in the case of treating adults, range from 1 to 2000 mg per day, preferably from 1 to 200 mg per day, either once daily or in divided portions. The dose may be increased or decreased as appropriate, depending on the age, body weight and symptom of a patient and an administration route.

The compounds of the present invention may be administered alone or in combination with one or more pharmaceutically acceptable carriers or diluents.

The dosage forms are tablets, capsules, granules, dispersions, powders, lozenges, ointments, creams, emulsions, suspensions, suppositories, injections and the like, all of which may be produced by conventional formulation techniques (for example, the methods set forth in the 15th revised Japanese Pharmacopoeia). These dosage forms may be selected as appropriate, according to the symptoms and age of patients and the purpose of treatment.

To produce these preparations, a composition containing the compound of the present invention may be blended with one or more pharmacologically acceptable carriers, namely, excipients (e.g., crystalline cellulose, starch, lactose, mannitol), binders (e.g., hydroxypropylcellulose, polyvinylpyrrolidone), lubricants (e.g., magnesium stearate, talc), disintegrants (e.g., carboxymethylcellulose calcium), and/or various other pharmacologically acceptable additives.

Also, the compounds of the present invention may be formulated by forming an inclusion compound with α-, β- or γ-cyclodextrin or methylated cyclodextrin.

The compounds of the present invention may be used in combination with one or more other therapeutic agents, namely, various antipsychotics, antidepressants, for example, 5HT3 antagonists, 5HT2 antagonists, serotonin agonists, NK-1 antagonists, selective serotonin reuptake inhibitors (SSRIs), serotonin noradrenaline reuptake inhibitors (SNRIs), tricyclic antidepressants, dopaminergic antidepressants, H3 antagonists, 5HT1A antagonists, 5HT1B antagonists, 5HT1D antagonists, D1 agonists, M1 agonists, anticonvulsants, cognitive enhancement drugs, and other psychoactive drugs.

Examples of other therapeutic agents that may be used in combination with the compounds of the present invention include ondansetron, granisetron, metoclopramide, sumatriptan, rauwolscine, yohimbine, fluoxetine, citalopram, escitalopram, femoxetine, fluvoxamine, paroxetine, indalpine, sertraline (registered trademark), zimeldine, venlafaxine, reboxetine, Milnacipran, duloxetine, imipramine, amitriptiline, chlomipramine, nortriptiline, bupropion, amineptine, divalproex, carbamazepine, diazepam, risperidone, olanzapine, ziprasidone, aripiprazole, quetiapine, perospirone, clozapine, haloperidol, pimozide, droperidol, chlorpromazine, thioridazine, mesoridazine, trifluoperazine, perphenazine, fluphenazine, thiflupromazine, prochlorperazine, acetophenazine, thiothixene, chlorprothixene, lamotrigine, loxapine, molindone, and the like. Such combinations may be administered simultaneously (in the same pharmaceutical formulation or in different pharmaceutical formulations), separately, or sequentially.

Particular advantages associated with the use of, and methods for treatment with, combinations of the compounds of the present invention include comparable or improved effects achieved by using individual ingredients at lower doses than their usual doses. Such use and treatment methods are also expected to further enhance the therapeutic effects on positive and/or negative symptoms of psychiatric disorders and/or cognitive impairment. The use of and methods for treatment with combinations of the compounds of the present invention also may provide benefits in the treatment of patients who do not sufficiently respond to, or who are resistant to, treatment with some type of neuroleptic.

The compounds of formula [IA] and the compounds of formula [I] may be produced by various methods of synthesis. The methods described below are only illustrative of the process for producing the inventive compounds and should not be taken as limiting.

In the general production processes, the term "inert solvent" refers to, for example, an alcohol such as methanol, ethanol, isopropanol, n-butanol, tert-butanol,or ethylene glycol; an ether such as diethyl ether, tert-butyl methyl ether, diisopropyl ether, tetrahydrofuran (THF), 1,4-dioxane, or 1,2-dimethoxyethane; a hydrocarbon such as pentane, hexane, heptane, toluene, benzene, or xylene; an ester such as ethyl acetate or ethyl formate; a ketone such as acetone or methyl ethyl ketone; a halogenated carbon-based solvent such as chloroform or dichloromethane; an amide such as N,N-dimethylformamide (DMF) or N-methylpyrrolidone; acetonitrile; dimethyl sulfoxide; water; or a mixed solvent thereof. These solvents are selected as appropriate, according to various reaction conditions known to skilled artisans.

The term "base" refers to, for example, an alkali metal or alkaline earth metal hydride such as lithium hydride, sodium hydride, potassium hydride, or calcium hydride; an alkali metal or alkaline earth metal amide such as lithium amide, sodium amide, lithium diisopropylamide, lithium dicyclohexylamide, lithium hexamethyldisilazide, sodium hexamethyldisilazide (NaHMDS), or potassium hexamethyldisilazide; an alkali metal or alkaline earth metal lower alkoxide such as sodium methoxide, sodium ethoxide, or potassium tert-butoxide; an alkyl lithium such as butyl lithium, sec-butyl lithium, tert-butyl lithium, or methyl lithium; an alkali metal or alkaline earth metal hydroxide such as sodium hydroxide, potassium hydroxide, lithium hydroxide, or barium hydroxide; an alkali metal or alkaline earth metal carbonate such as sodium carbonate, potassium carbonate, or cesium carbonate; an alkali metal or alkaline earth metal hydrogencarbonate such as sodium hydrogen carbonate or potassium hydrogen carbonate; an amine such as triethylamine, N-methylmorpholine, N,N-diisopropylethylamine (DIPEA), 1,8-diazabicyclo[5.4.0]undec-7-ene (DBU), 1,5-diazabicyclo[4.3.0]non-5-ene (DBN), or N,N-dimethylaniline; or a basic heterocyclic compound such as pyridine, imidazole, or 2,6-lutidine. These bases are selected as appropriate, according to various reaction conditions known to skilled artisans.

The term "acid" refers to, for example, an inorganic acid such as hydrochloric acid, hydrobromic acid, sulfuric acid, nitric acid, or phosphoric acid; or an organic acid such as p-toluenesulfonic acid, methanesulfonic acid, trifluoroacetic acid, formic acid, acetic acid, citric acid, or oxalic acid. These acids are selected as appropriate, according to various reaction conditions known to skilled artisans.

The term "Lewis acid" refers to, for example, boron trifluoride, aluminum trichloride, titanium tetrachloride, iron trichloride, zinc chloride, or tin tetrachloride. These Lewis acids are selected as appropriate, according to various reaction conditions known to skilled artisans.

In the general production processes, X represents an organic phosphorus functional group such as a phosphonium salt substituted with an aryl group, etc., or a phosphonic acid derivative substituted with an alkoxy group, an aryloxy group, etc. X¹ represents a halogen atom or a hydroxy group, X² and X³ represent a chlorine atom, a bromine atom, an iodine atom or a trifluoromethanesulfonyloxy group, and R⁵, R⁶ and R⁷ represent each an optionally substituted phenyl group or an optionally substituted alkyl group. Further, P¹ is a protective group of a nitrogen atom such as a tert-butoxycarbonyl group or an allyl group (Theodora W. Greene, Peter G.M. Wuts, "Greene's Protective Groups in Organic Synthesis, Forth Edition"; see Wiley Interscience), P² is a hydrogen atom, or a protective group of a hydroxy group such as trimethylsilyl group, tert-butoxycarbonyl group (see the above document), other are defined as above.

In the general production processes, R³ and R⁴ may also be converted or introduced as appropriate by various functional group transformation or functional group introduction reaction during the steps.

### General production process 1

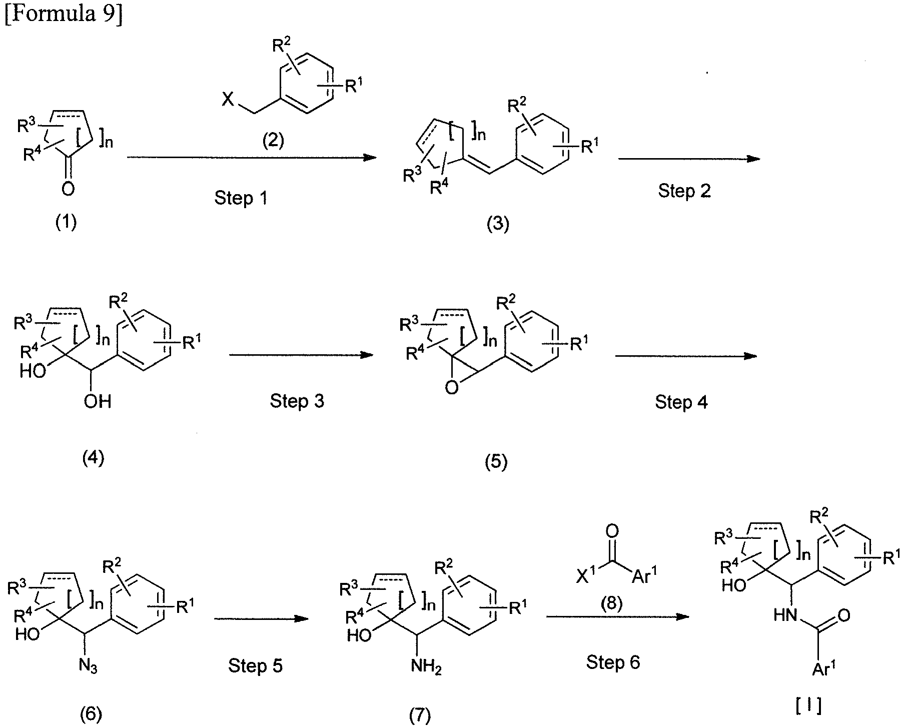

Step 1: Compound (1) may be reacted with compound (2) in an inert solvent in the presence or absence of a base to obtain compound (3).. Compound (2) used herein refers to an organic phosphorus reagent commonly used in Wittig reaction and Horner-Wadsworth-Emmons reaction, and examples include phosphonium salts such as (3-chlorobenzyl)(triphenyl)phosphonium bromide and phosphonate esters.

Step 2: Compound (3) may be reacted with an oxidizing agent in an inert solvent in the presence or absence of a chiral ligand to obtain compound (4).. The oxidizing agent used herein refers to a reagent commonly used for forming diols with multiple bonds, and examples include osmium tetroxide. The chiral ligand used herein refers to a reagent commonly used for forming asymmetric diols with multiple bonds, and examples include bis(dihydroquininyl)phthalazine and bis(dihydroquinidinyl)phthalazine. Reagents obtained by mixing these reagents such as AD mix α may also be used. In the present step, the reaction may also be conducted using additives as appropriate such as methanesulfonamide.

Step 3: Compound (4)may be subjected to anintramolecular cyclization reaction in an inert solvent in the presence or absence of a base to obtain compound (5). An example of the intramolecular cyclization reaction in this case is anintramolecular cyclization reaction wherein the hydroxy group of compound (4) is converted to a leaving group by methanesulfonylation, toluenesulfonylation, halogenation or the like. Alternatively, an intramolecular cyclization reaction may be conducted by Mitsunobu reaction using an organic phosphorous compound and an azo compound or a phosphorus ylide reagent.

Step 4: Compound (5)may be subjected to a ring cleavage reaction in an inert solvent in the presence or absence of acid or Lewis acid to obtain compound (6). As an example of the ring cleavage reaction in this case, the epoxide of compound (5) may be subjected to a ring cleavage reaction using a reagent commonly used for the synthesis of an azide compound such as sodium azide. Alternatively, compound (7) may also be synthesized, without proceeding through step 5, by subjecting to the similar reaction using amines such as ammonia.

Step 5: Compound (6) may be subjected to a reduction reaction in an inert solvent to obtain compound (7).. The reduction reaction used herein refers to a reaction commonly used for reduction of an azide group, and examples include reduction reactions using triphenyl phosphine, trimethylphosphine or the like. Further, the reduction by hydrogenation reaction using a metal catalyst such as a palladium catalyst or a platinum catalyst may also be employed, and the metal catalysts include, in addition to palladium carbon and platinum carbon, palladium carbon with an adjusted activity by various additives.

Step 6: Compound (7) and compound (8) wherein X¹ is a halogen atom may be subjected to an amidation reaction in an inert solvent in the presence or absence of a base to obtain the compound [I] of the present invention. Alternatively, compound (7) and compound (8) wherein X¹ is a hydroxyl group may be subjected to various amidation reactions known by those skilled in the art to obtain the compound [I] of the present invention. The amidation reaction used herein refers to, for example, an amidation reaction conducted in an inert solvent in the presence or absence of a base using a condensation agent such as 0-(7-azabenzotriazol-l-yl)-N,N,N',N'-tetramethyluronium hexafluorophosphate (HATU), O-(benzotriazol-1-yl)-N,N,N',N'-tetramethyluronium hexafluorophosphate (HBTU), N,N'-dicyclohexylcarbodiimide (DCC), 1-ethyl-3-(3-dimethylaminopropyl)carbodiimide hydrochloride (EDC·HCl), diphenylphosphoryl azide (DPPA) or carbonyldiimidazole (CDI), or the amidation reaction through a mixed acid anhydride using ethyl chlorocarbonate, isobutyl chlorocarbonate or trimethylacetyl chloride. During the amidation reaction using a condensation agent, an additive such as 1-hydroxybenzotriazol (HOBt) or hydroxysuccinimide (HOSu) may be used as appropriate.

### General production process 2

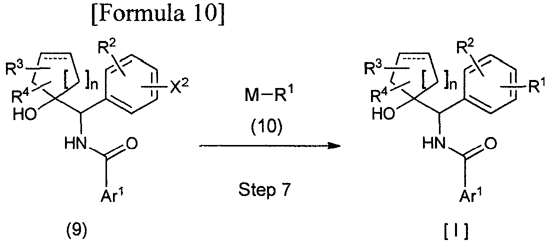

Step 7: Compound (9) and compound (10) may be subjected to various cross-coupling reactions known by those skilled in the art in an inert solvent in the presence or absence of a base using a palladium catalyst and as appropriate a ligand of the palladium catalyst, obtain the compound [I] of the present invention. The cross-coupling reaction used herein may be conducted by many standard procedures known by those skilled in the art, and examples include reaction of compound (9) with compound (10), which is wherein M is boric acid, borate ester, or tin or zinc substituted with trialkyl, etc. Examples of the palladium catalyst used herein include palladium acetate, tris(dibenzylideneacetone)dipalladium(0), tetrakis(triphenylphosphine)palladium(0), (1,3-diisopropylimidazol-2-ylidene)(3-chloropyridyl)palladium (II) dichloride, [1,3-bis(2,6-diisopropylphenyl)imidazol-2-ylidene](3-chloropyridyl) palladium (II) dichloride, and [1,1'-bis(diphenylphosphino)ferrocene]palladium chloride, and examples of the ligand include triphenylphosphine, 2,2-bis(diphenylphosphino)-1,1-binaphthyl (BINAP), 2-(di-tert-butylphosphino)biphenyl, and 9,9-dimethyl-4,5-bis(diphenylphosphino)xanthene (Xantphos).

Compound (25) including the aforementioned compound (7) may be produced in accordance with the following method.

### General production process 3

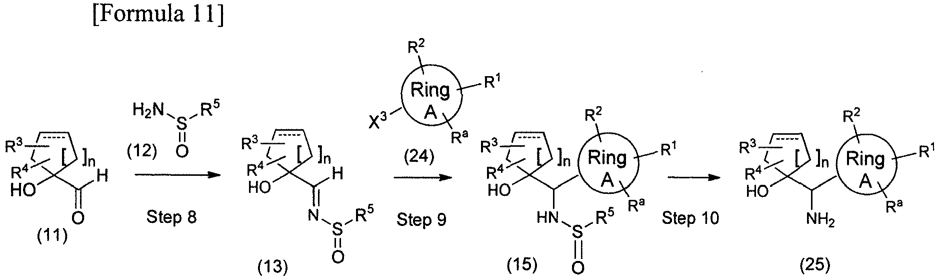

Step 8: Compound (11) and compound (12) may be subjected to a condensation reaction in an inert solvent in the presence of acid or Lewis acid in the presence or absence of a dehydrating agent, to obtain compound (13).

Step 9: Compound (24) may be converted using a metal or an alkyllithium reagent to a metal reagent, which may be subsequently reacted with compound (13) in an inert solvent to obtain compound (15). Examples of the metal used herein include magnesium and zinc, and examples of the alkyllithium reagent include n-butyllithium, sec-butyllithium, tert-butyllithium and phenyllithium reagents.

Step 10: Compound (15) may be subjected to a reaction in an inert solvent in the presence of acid or Lewis acid, or a base to obtain compound (25).

The compound [IA] (compound (26)) of the present invention wherein R^{b} is a hydrogen atom may be produced from compound (25) in accordance with the following method.

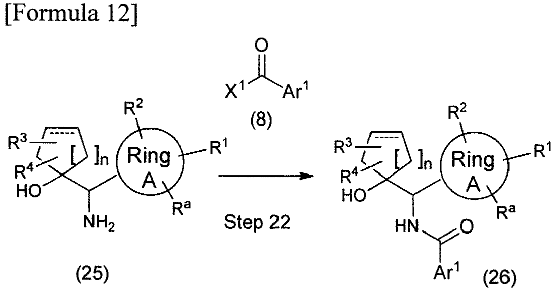

Step 22: Compound (25) obtained in the above step 10 may be subjected to an amidation reaction with compound (8) wherein X¹ is a halogen atom or compound (8) wherein X¹ is a hydroxy group, as in step 6 of General production process 1, obtain compound (26).

Note that the method described in step 6 of General production process 1 may be used for the amidation reaction.

The aforementioned compound (7) may also be produced in accordance with the following method.

### General production process 4

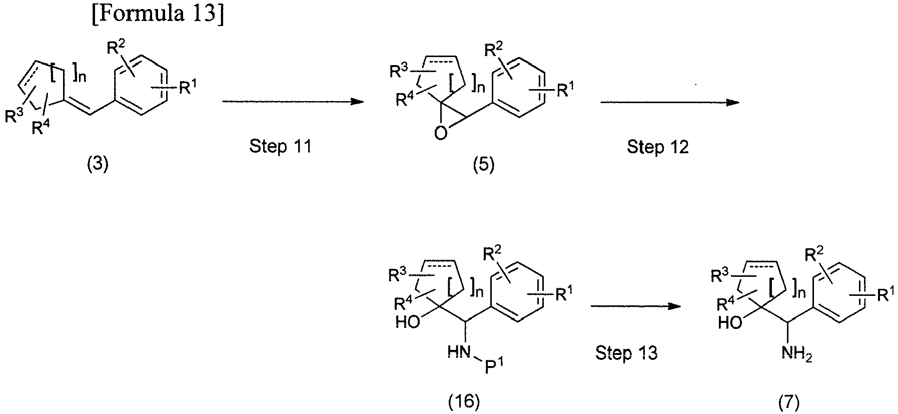

Step 11: e Compound (3) may be reacted with an oxidizing agent in an inert solvent in the presence or absence of a base to obtain compound (5). The oxidizing agent used herein refers to a reagent commonly used for epoxidating multiple bonds, and examples include 3-chloroperbenzoic acid. Further, the reaction may be conducted using a chiral ligand with a suitable oxidizing agent.

Step 12: Compound (5) may be subjected to a ring cleavage reaction in an inert solvent in the presence or absence of acid or Lewis acid to obtain compound (16). As an example of the ring cleavage reaction in this case, the epoxide of compound (5) may be subjected to a ring cleavage reaction using amines such as allylamines **which** may be protected by a protective group described in Theodora W. Greene, Peter G.M. Wuts, "Greene's Protective Groups in Organic Synthesis, Forth Edition". Alternatively, compound (7) may also be directly synthesized, without proceeding through step 13, by subjecting to the similar reaction using amines such as ammonia.

Step 13: Compound (16) may be subjected to a deprotection reaction described in Theodora W. Greene and Peter G. M. Wuts, "Protective Groups in Organic Synthesis Third Edition", in an inert solvent to obtain compound (7). For example, in the case where P¹ is an allyl group, the deprotection reaction may be conducted using an N,N-dimethylbarbituric acid and a palladium catalyst. Examples of the palladium catalyst used herein include palladium acetate, tris(dibenzylideneacetone)dipalladium(0), and tetrakis(triphenylphosphine)palladium(0).

The aforementioned compound (7) may also be produced in accordance with the following method.

### General production process 5

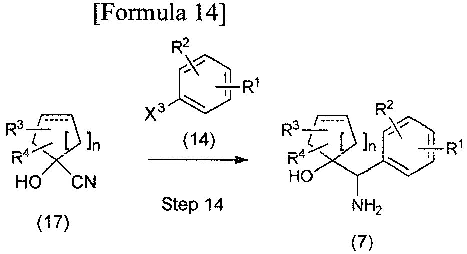

Step 14: Compound (14) may be converted using a metal or an alkyllithium reagent to a metal reagent, which may be subsequently reacted with compound (17) in an inert solvent, followed by reacting with a reducing agent to obtain compound (7). Examples of the metal used herein include magnesium or zinc, and examples of the alkyllithium reagent include n-butyllithium, sec-butyllithium, tert-butyllithium and phenyllithium reagents. The reducing agent used herein is generally a reagent capable of converting an imino group to an amino group by reduction, and examples include lithium borohydride, sodium borohydride, calcium borohydride, lithium triethylborohydride, tri-sec-lithium butylborohydride, tri-sec-potassium butylborohydride, zinc borohydride, borane, lithium trimethoxyborohydride, lithium triacetoxyborohydride, tetramethylammonium borohydride, lithium aluminum hydride, sodium aluminum hydride, sodium bis(2-methoxyethoxy)aluminum hydride, diisobutylaluminum hydride and trichlorosilane.

The aforementioned compound (7) may also be produced in accordance with the following method.

### General production process 6

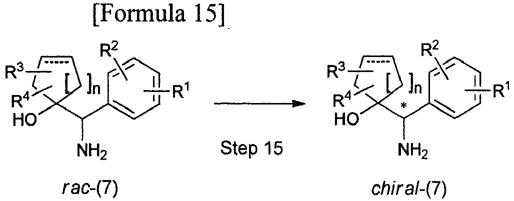

Step 15: Optically inactive compound (7) or compound (7) having a low optical purity may be reacted with an optically active acid; subsequently, a recrystallization operation is conducted to obtain a salt of optically active compound (7) or a salt of compound (7) having a high optical purity.

### Examples of the optically active acid used herein include (S)-methoxyphenylacetate.

The aforementioned compound (3) may also be produced in accordance with the following method.

### General production process 7

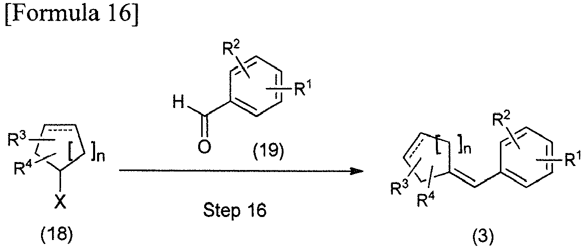

Step 16: Compound (18) may be reacted with compound (19) in an inert solvent in the presence or absence of a base to obtain compound (3). Compound (18) herein is an organic phosphorus reagent commonly used in Wittig reaction and Horner-Wadsworth-Emmons reaction, and examples include phosphonium salts such as cyclopentyl(triphenyl)phosphonium bromide and phosphonate esters.

The aforementioned compound (11) may also be produced in accordance with the following method.

### General production process 8

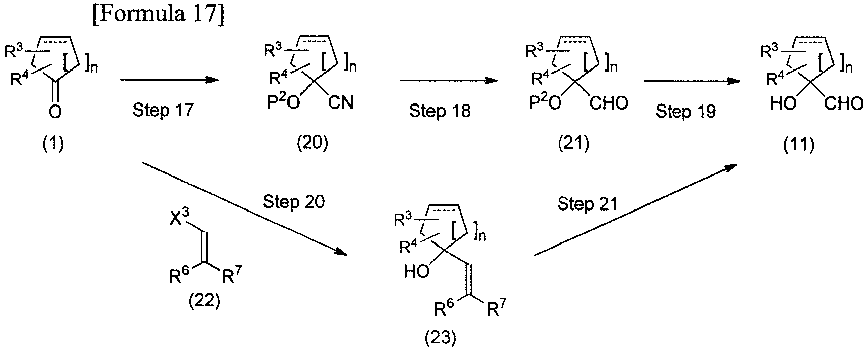

Step 17: Compound (1) may be subjected to a cyanation reaction in an inert solvent using various inorganic cyanating reagents or organic cyanating reagents known by those skilled in the art to obtain compound (20). The cyanation reaction used herein may be conducted by many standard procedures known by those skilled in the art, and a cyanating agent such as, for example, potassium cyanide or sodium cyanide as the inorganic cyanating reagent, or trimethylsilanecarbonitrile as the organic cyanating reagent, may be used. Further, the reaction may also be conducted using a reagent capable of activating the reaction. For example, N-methylmorpholine oxide or the like may be used.

Step 18: Compound (20) may be reacted with a reducing agent in an inert solvent to obtain compound (21). The reducing agent used herein is a reagent capable of converting a cyano group to aldehyde by reduction, and examples include diisobutylaluminum hydride.

Step 19: Compound (21), wherein P² is a protective group of a hydroxy group, may be subjected to hydrolyzation in an inert solvent using an acid or a base, or a deprotection reaction described in Theodora W. Greene and Peter G.M. Wuts, "Protective Groups in Organic Synthesis Third Edition" to obtain compound (11).

Step 20: Compound (22) may be converted using a metal or an alkyllithium reagent to a metal reagent, which may be subsequently reacted to compound (1) in an inert solvent to obtain compound (23). Examples of the metal used herein include magnesium or zinc, and examples of the alkyllithium reagent include n-butyllithium, sec-butyllithium, tert-butyllithium and phenyllithium.

Step 21: Compound (23) may be reacted with an oxidizing agent in an inert solvent to obtain compound (11). The oxidizing agent used herein generally refers to a reagent used for synthesizing a carbonyl compound by oxidatively cleaving a double bond, and examples include ozone, osmium tetroxide and ruthenium tetraoxide. Further, additives selected as appropriate may be added together with these oxidizing agents, and examples include dimethylsulfide or triphenyl phosphine used for ozone oxidation, and sodium periodate used for osmium tetroxide oxidation.

The compound [IA] (compound (27)) of the present invention wherein R^{b} is a C₁₋₆ alkyl group may be produced in accordance with the following method.

### General production process 9

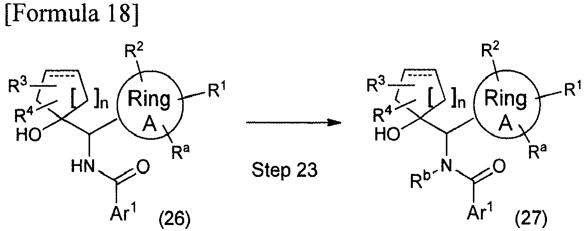

Step 23: Compound (26) obtained in the step 22 of the aforementioned General production process 3 may be reacted with an alkyl halide in an inert solvent in the presence of a base to obtain compound (27). Examples of the base used herein include sodium hydride.

### EXAMPLES

Next, the present invention will be further described in more detail with reference to Production Examples, Examples and Test Examples, which are not intended to limit the scope of the present invention.

In the following Production Examples and Examples, the microwave reactor used is Biotage Initiator.

In the following Production Examples and Examples, the "NH silica gel cartridge" used for purification by column chromatography was a Biotage SNAPCartridge KP-NH or GRACE REVELERIS Amino, and the "silica gel cartridge" was a Biotage SNAP Cartridge KP-Sil, HP-Sil, or GRACE REVELERIS Silica.

In the following Production Examples and Examples, the "NH silica gel" used for purification by preparative thin-layer chromatography (PTLC) was Wako NH2 Silica Gel 60F254 Plate-Wako, 20 cm x 20 cm, and the "silica gel" was Merck Silica Gel 60F254, 20 cm x 20 cm.

In the following Production Examples and Examples, purification by preparative high performance liquid chromatography (HPLC) was conducted under the following conditions. It should be noted that when trifluoroacetic acid was used in the main procedure for producing compounds having a basic functional group, neutralization operation or the like was conducted as appropriate for obtaining the compounds in free form.

### Apparatus: Gilson Trilution LC

Column: YMC-Actus triart C18 5 µm 20 x 50 mm or Waters SunFire Prep C18 OBD 5 µm 30 x 50 mm
Solvent: A-liquid; 0.1 % trifluoroacetic acid containing water, B-liquid; 0.1 % trifluoroacetic acid containing acetonitrile
Gradient conditions: 0 min (A-liquid/B-liquid = 90/10), 11 min (A-liquid/B-liquid = 20/80), 12 to 13.5 min (A-liquid/B-liquid = 5/95), flow rate 40 mL/min
Detection method: UV 254 nm

In the following Production Examples and Examples, mass spectra (MS) were measured using the following apparatuses.
MS: Shimadzu LCMS-2010EV, micromass Platform LC, Shimadzu LCMS-IT-TOF, micromass GCT, 1290 Infinity and Agilent 6150

In the following Production Examples and Examples, nuclear magnetic resonance spectra (NMR) were used for structure confirmation. NMR was measured using the following apparatuses.
NMR spectra: [1H-NMR] 600MHz: JNM-ECA600 (JOEL Ltd.), 500MHz: JNM-ECA500 (JOEL Ltd.), 300MHz: UNITYNOVA300 (Varian Inc.), 200MHz: GEMINI2000/200 (Varian Inc.)

The RT (Retention Time (min)) in the following Production Examples and Table 1 are values measured using a high performance liquid chromatography mass spectrometer (LCMS) under any of the following conditions.

### Condition A

Measuring instrument: Agilent Agilent 1290 Infinity and Agilent 6150
Column: Waters Acquity CSH C18, 1.7 µm, φ2.1 x 50 mm
Solvent: A-liquid; 0.1% formic acid containing water, B-liquid; 0.1% formic acid containing acetonitrile,
Gradient: 0 min (A-liquid/B-liquid = 80/20), 1.2 to 1.4 min (A-liquid /B-liquid = 1/99)
Flow rate: 0.8 mL/min, Detection method: 254 nm

### Condition B

Measuring instrument: Agilent Agilent 1290 Infinity and Agilent 6150
Column: Waters Acquity CSH C18, 1.7 µm, φ2.1 x 50 mm
Solvent: A-liquid; 0.1 % formic acid containing water, B-liquid; 0.1 % formic acid containing acetonitrile
Gradient: 0 min (A-liquid/B-liquid = 95/5), 1.2 min (A-liquid/B-liquid = 50/50), 1.38 min
(A-liquid/B-liquid = 3/97)
flow rate: 0.8 mL/min (0 to 1.2 min), 0.8 mL/min (1.2 to 1.38 min)
Detection method: 254 nm

In the following Production Examples and Examples, optical isomer analysis of racemic compounds and optically active compounds was measured using the following instruments, and the RT (min) was shown in the following Production Examples and Table 1.
Measuring instrument: Agilent Agilent 1100 (chiral HPLC)
Measuring Instrument: Waters Waters 2695 and 2998 (chiral HPLC)
Measuring instrument: Shimadzu LC-30AD (chiral HPLC)
Measuring instrument: Nihon Waters SFC30 system (chiral super critical fluid chromatography (SFC))

In the following Production Examples and Examples, optical rotation analysis was measured using the following instrument.
Measuring instrument: JASCO JASCO P-2300

In the following Production Examples and Examples, X-ray crystal structure analysis was measured using the following instrument.
Measuring instrument: Rigaku R-AXIS RAPID II

In the following Production Examples and Examples, compounds were named in accordance with ACD/Name (ACD/Labs 12.01, Advanced Chemistry Development Inc.).

In the following Production Examples and Examples, chirality confirmation of compounds was conducted by any of chiral HPLC analysis, chiral SFC analysis, optical rotation analysis, or X-ray crystal structure analysis, or by a combination thereof.

In the following Production Examples and Examples, a pair of diastereomers derive from the cis or trans of the substituent at 4-position on the cyclohexane ring in formula (II) to which the hydroxyl group binds (the substituent is a fluorine atom in formula (II)) and of the hydroxyl group per se, and if no determination can be made as to cis or trans, one diastereomer shall be referred to as diastereomer 1 and the other diastereomer that pairs with it shall be referred to as diastereomer 2. Without either indication, a mixture of two diastereomers is meant. A pair of enantiomers derive from the chirality on the asymmetric carbon at the benzyl position of formula (II), and if no determination can be made as to its steric configuration, one enantiomer shall be referred to as enantiomer 1 and the other enantiomer that pairs with it shall be referred to as enantiomer 2. Without either indication, a racemate is meant. It should, however, be noted that the structure represented by formula (II) is just an illustration and the substituents at the 4-position of the cyclohexane ring and on the phenyl ring are by no means limited to formula (II). It should also be noted that even in the case where the cyclohexane ring moiety is replaced with a cyclobutane ring, the substituents on the hydroxyl group and at the 3-position of the cycobutane ring can cause cis- or trans-isomerism. For compounds that were not determined for cis or trans and for the steric configuration of the benzyl position, the indication of (diastereomer 1, enantiomer 1), for example, was adopted.

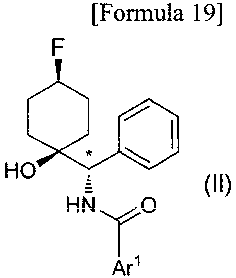

### Production Example 1 1-Chloro-3-[(4-fluoroxcyclohexylidene)methyl]benzene

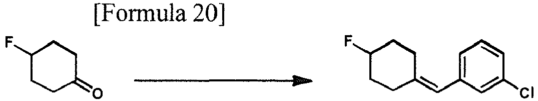

A solution of 1.9 M sodium hexamethyldisilazide in hexane (2.7 mL) was added to a suspension of (3-chlorobenzyl)(triphenyl)phosphonium bromide (2.4 g) in diethyl ether (10 mL) and stirred at 0°C for 30 minutes.
A solution of 4-fluorocyclohexanone (600 mg) in diethyl ether (10 mL) was added dropwise to the reaction mixture and stirred at room temperature for 3 hours. Water was added to the reaction mixture, filtrated, and subsequently the filtrate was extracted with diethyl ether. The organic layer was concentrated under reduced pressure, and subsequently the residue was purified by column chromatography (silica gel cartridge, hexane) to obtain the title compound (85 mg).
(EI) m/z : 224(M+)
1H NMR (600 MHz, CHLOROFORM-d) d ppm 1.72 - 2.00 (m, 4 H) 2.18 - 2.26 (m, 1 H) 2.32 - 2.40 (m, 1 H) 2.48 - 2.60 (m, 2 H) 4.75 - 4.89 (m, 1 H) 6.25 (s, 1 H) 7.03 - 7.36 (m, 4 H)

The following compounds were synthesized by the similar method. 1-[(4,4-Difluorocyclohexylidene)methyl]-3-iodobenzene
(EI) m/z : 334(M+)
1-[(4,4-Difluorocyclohexylidene)methyl]-3-fluorobenzene
(EI) m/z : 226(M+)
1H NMR (600 MHz, CHLOROFORM-d) d ppm 1.84 - 2.15 (m, 4 H) 2.38 - 2.60 (m, 4 H) 6.34 (s, 1 H) 6.82 - 6.98 (m, 3 H) 7.23 - 7.32 (m,1 H)
1-Bromo-3-[(4,4-difluorocyclohexylidene)methyl]benzene
(EI) m/z : 286(M+)
1H NMR (600 MHz, CHLOROFORM-d) d ppm 1.87 - 1.98 (m, 2 H) 2.00 - 2.09 (m, 2 H) 2.42 - 2.48 (m, 2 H) 2.49 - 2.58 (m, 2 H) 6.31 (s, 1 H) 7.06 - 7.13 (m,1 H) 7.20 (t, J=7.6 Hz, 1 H) 7.28 - 7.40 (m, 2 H)
1-Chloro-2-[(4,4-difluorocyclohexylidene)methyl]benzene
(EI) m/z : 242(M+)
1H NMR (600 MHz, CHLOROFORM-d) d ppm 1.88 - 1.99 (m, 2 H) 2.02 - 2.13 (m, 2 H)
2.37 - 2.43 (m, 2 H) 2.46 - 2.54 (m, 2 H) 6.37 (s, 1 H) 7.14 - 7.25 (m, 3 H) 7.35 - 7.41 (m, 1 H)
1,2-Dichloro-3-[(4,4-difluorocyclohexylidene)methyl]benzene
(EI) m/z : 276(M+)
1,4-Dichloro-2-[(4,4-difluorocyclohexylidene)methyl]benzene
(EI) m/z : 276(M+)
1-[(4,4-Difluorocyclohexylidene)methyl]-2,3,4-trifluorobenzene
1H NMR (600 MHz, CHLOROFORM-d) d ppm 1.90 - 2.00 (m, 2 H), 2.03 - 2.09 (m, 2 H), 2.36 - 2.40 (m, 2 H), 2.46 - 2.52 (m, 2 H), 6.19 (s, 1 H), 6.82 - 6.88 (m, 1 H), 6.89 - 6.97 (m, 1 H)
1-[(4,4-Difluorocyclohexylidene)methyl]-2,3-difluorobenzene
1H NMR (600 MHz, CHLOROFORM-d) d ppm 1.90 - 2.13 (m, 4 H) 2.35 - 2.54 (m, 4 H) 6.26 (s, 1 H) 6.84 - 6.94 (m, 1 H) 6.98 - 7.10 (m, 2 H)
1-Chloro-3-[(4,4-difluorocyclohexylidene)methyl]benzene
1H NMR (600 MHz, CHLOROFORM-d) d ppm 1.85 - 2.15 (m, 4 H) 2.39 - 2.57 (m, 4 H) 6.32 (s, 1 H) 7.00 - 7.29 (m, 4 H)
1-Chloro-3-[(4,4-difluorocyclohexylidene)methyl]-2-fluorobenzene
1H NMR (600 MHz, CHLOROFORM-d) d ppm 1.90 - 2.12 (m, 4 H) 2.37 - 2.53 (m, 4 H) 6.26 (s, 1 H) 7.00 - 7.09 (m, 2 H) 7.26 - 7.34 (m, 1 H)
2-Chloro-1-[(4,4-difluorocyclohexylidene)methyl]-3-fluorobenzene
1H NMR (600 MHz, CHLOROFORM-d) d ppm 1.89 - 1.99 (m, 2 H) 2.02 - 2.14 (m, 2 H) 2.35 - 2.44 (m, 2 H) 2.47 - 2.56 (m, 2 H) 6.34 (s, 1 H) 6.93 - 7.22 (m, 3 H)
{[4-(3-Bromobenzylidene)cyclohexyl]oxy}(tert-butyl)dimethyl silane
(EI) m/z : 380(M+)
1H NMR (600 MHz, CHLOROFORM-d) d ppm 0.04 - 0.12 (m, 6 H) 0.86 - 0.95 (m, 9 H) 1.22 - 2.72 (m, 8 H) 3.88 - 3.95 (m, 1 H) 6.15 - 6.20 (m, 1 H) 7.08 - 7.13 (m, 1 H) 7.14 - 7.19 (m, 1 H) 7.29 - 7.36 (m, 2 H)
tert-Butyl {[4-(3-chlorobenzylidene)cyclohexyl]oxy}dimethyl silane
(EI) m/z : 336(M+)
1H NMR (600 MHz, CHLOROFORM-d) d ppm 0.02 - 0.10 (m, 6 H) 0.89 - 0.94 (m, 9 H) 1.52 (dtd, J=12.5, 8.2, 8.2, 4.5 Hz, 1 H) 1.58 - 1.65 (m, 1 H) 1.67 - 1.75 (m, 1 H) 1.77 - 1.84 (m, 1 H) 2.12 - 2.24 (m, 2 H) 2.46 - 2.54 (m, 1 H) 2.57 - 2.65 (m, 1 H) 3.86 - 3.96 (m, 1 H) 6.12 - 6.23 (m, 1 H) 7.06 (d, J=7.8 Hz, 1 H) 7.14 - 7.18 (m, 2 H) 7.20 - 7.24 (m, 1 H)
1-Chloro-2-[(4-fluorocyclohexylidene)methyl]benzene
1H NMR (600 MHz, CHLOROFORM-d) d ppm 1.71 - 2.72 (m, 8 H) 4.75 - 4.90 (m, 1 H) 6.30 (s, 1 H) 7.06 - 7.41 (m, 4 H)
1-Chloro-2-fluoro-3-[(4-fluorocyclohexylidene)methyl]benzene
(ESI neg.) m/z : 241(M-H)-
1H NMR (600 MHz, CHLOROFORM-d) d ppm 1.73 - 2.03 (m, 4 H) 2.20 - 2.32 (m, 2 H) 2.40 - 2.49 (m, 1 H) 2.53 - 2.61 (m, 1 H) 4.75 - 4.92 (m, 1 H) 6.19 (s, 1 H) 6.99 - 7.10 (m, 2 H) 7.22 - 7.31 (m, 1 H)
1,2-Difluoro-3-[(4-fluorocyclohexylidene)methyl]benzene
(EI) m/z : 226(M+)
1H NMR (600 MHz, CHLOROFORM-d) d ppm 1.71 - 2.34 (m, 6 H) 2.40 - 2.50 (m, 1 H) 2.52 - 2.63 (m, 1 H) 4.73 - 4.94 (m, 1 H) 6.11 - 6.28 (m, 1 H) 6.75 - 7.41 (m, 3 H)
1-Fluoro-3-[(4-fluorocyclohexylidene)methyl]benzene
1H NMR (600 MHz, CHLOROFORM-d) d ppm 1.68 - 2.62 (m, 8 H) 4.73 - 4.91 (m, 1 H) 6.27 (s, 1 H) 6.83 - 7.35 (m, 4 H)
1-[(4,4-Difluorocyclohexylidene)methyl]-2-fluoroben
1H NMR (600 MHz, CHLOROFORM-d) d ppm 1.90 - 1.99 (2 H, m) 2.01 - 2.12 (2 H, m) 2.40 - 2.46 (2 H, m) 2.47 - 2.51 (2 H, m) 6.29 (1 H, s) 7.01 - 7.25 (4 H, m)
1-Fluoro-2-[(4-fluorocyclohexylidene)methyl]benzene
1 H NMR (600 MHz, CHLOROFORM-d) d ppm 1.74 - 2.60 (8 H, m) 4.73 - 4.90 (1 H, m) 6.22 (1 H, s) 7.00 - 7.37 (4 H, m)

### Production Example 2 1-[(S)-(3-Chlorophenyl)(hydroxy)methyl]-4-fluorocyclohexanol

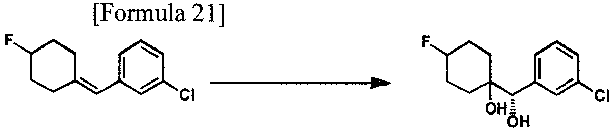

AD mix α (500 mg) and methanesulfonamide (34 mg) were added to a solution of 1-chloro-3-[(4-fluorocyclohexylidene)methyl]benzene (80 mg) dissolved in a mixture of tert-butanol (1.6 mL) and water (1.6 mL), and stirred at 0°C for 2 days. An aqueous saturated sodium thiosulfate solution was added to the reaction mixture, the reaction mixture was extracted with ethyl acetate, and dried over anhydrous magnesium sulfate. After filtering off the desiccating agent, the organic layer was concentrated under reduced pressure, and the residue was purified by column chromatography (silica gel cartridge, hexane/ethyl acetate = 100 : 0 to 70 : 30) to obtain the title compound (72 mg).
1H NMR (600 MHz, CHLOROFORM-d) d ppm 1.15 - 1.99 (m, 7 H) 2.31 - 2.47 (m, 1 H) 4.30 - 4.91 (m, 2 H) 7.19 - 7.40 (m, 4 H)

The following compounds were synthesized by the similar method.
(Enantiomer 1) 1-[(3-Chlorophenyl)(hydroxy)methyl]cyclopentanol
1H NMR (600 MHz, CHLOROFORM-d) d ppm 1.30 - 1.35 (m, 1 H) 1.59 - 1.66 (m, 2 H) 1.71 - 1.86 (m, 4 H) 2.50 - 2.54 (m, 1 H) 4.57 - 4.61 (m, 1 H) 7.27 - 7.29 (m, 3 H) 7.41 - 7.44 (m, 1 H)
(Enantiomer 1) 1-[(2-Chlorophenyl)(hydroxy)methyl]-4,4-difluorocyclohexanol (ESI neg.) m/z : 275(M-H)-
1H NMR (600 MHz, CHLOROFORM-d) d ppm 1.36 - 1.43 (m, 1 H) 1.66 - 1.80 (m, 2 H) 1.84 - 2.21 (m, 4 H) 2.33 (d, J=3.7 Hz, 1 H) 5.11 - 5.14 (m, 1 H) 7.22 - 7.26 (m, 1 H) 7.29 - 7.38 (m, 2 H) 7.59 - 7.66 (m, 1 H)

Chiral analysis conditions
Column: Daicel CHIRALPAK AD3, 4.6*150
Solvent system: Hexane : EtOH = 95 : 5
Flow rate: 1 mL/min
RT: 9.28, 10.26, Later
(Enantiomer 2) 1-[(2-Chlorophenyl)(hydroxy)methyl]-4,4-difluorocyclohexanol

Chiral analysis conditions
Column: Daicel CHIRALPAK AD3, 4.6*150
Solvent system: Hexane : EtOH = 95 : 5
Flow rate: 1 mL/min
RT: 9.28, 10.26, Faster

With the proviso that AD mix β was used as the reaction agent.
(Enantiomer 1) 1-[(2,3-Difluorophenyl)(hydroxy)methyl]-4,4-difluorocyclohexanol
1H NMR (600 MHz, CHLOROFORM-d) d ppm 1.41 - 1.50 (m, 1 H) 1.58 - 1.73 (m, 2 H) 1.86 - 2.22 (m, 4 H) 2.39 - 2.45 (m, 1 H) 4.93 (d, J=4.1 Hz, 1 H) 7.08 - 7.17 (m, 2 H) 7.25 - 7.31 (m, 1 H)
(Enantiomer 1) 1-[(3-Chlorophenyl)(hydroxy)methyl]-4,4-difluorocyclohexanol
1H NMR (600 MHz, CHLOROFORM-d) d ppm 1.41 - 1.59 (m, 2 H) 1.63 - 1.73 (m, 1 H) 1.84 - 2.21 (m, 4 H) 2.35 (d, J=3.7 Hz, 1 H) 4.48 (d, J=3.7 Hz, 1 H) 7.20 - 7.3 (m, 3 H) 7.35 - 7.39 (m, 1 H)
(Enantiomer 1) 1-[(3-Chloro-2-fluorophenyl)(hydroxy)methyl]-4,4-difluorocyclohexanol
1H NMR (600 MHz, CHLOROFORM-d) d ppm 1.42 - 1.50 (m, 1 H) 1.58 - 1.72 (m, 2 H) 1.85 - 2.22 (m, 4 H) 2.42 (d, J=4.1 Hz, 1 H) 4.93 (d, J=4.5 Hz, 1 H) 7.08 - 7.18 (m, 1 H) 7.33 - 7.48 (m, 2 H)
(Enantiomer 1) 1-[(2-Chloro-3-fluorophenyl)(hydroxy)methyl]-4,4-difluorocyclohexanol
1H NMR (600 MHz, CHLOROFORM-d) d ppm 1.34 - 1.42 (m, 1 H) 1.66 - 1.77 (m, 2 H) 1.85 - 2.22 (m, 4 H) 2.40 (d, J=3.7 Hz, 1 H) 5.10 - 5.14 (m, 1 H) 7.09 - 7.15 (m, 1 H) 7.27-7.32 (m, 1 H) 7.40 - 7.45 (m, 1 H)
(Enantiomer 1) 4,4-Difluoro-1-{hydroxy[3-(pyridin-2-yl)phenyl]methyl}cyclohexanol (ESI pos.) m/z : 320(M+H)+
LCMS RT 0.52, Condition A
1H NMR (600 MHz, CHLOROFORM-d) d ppm 1.49 - 1.75 (m, 3 H), 1.84 - 2.21 (m, 5 H), 2.26 (s, 1 H), 2.55 (br. s., 1 H), 4.61 (s, 1 H), 7.23 - 7.29 (m, 1 H), 7.39 - 7.44 (m, 1 H), 7.47 (t, J=7.6 Hz, 1 H), 7.71 - 7.80 (m, 2 H), 7.86 - 7.95 (m, 1 H), 7.96 - 8.06 (m, 1 H), 8.66 - 8.73 (m, 1 H)
(Enantiomer 1) cis-1-[(2-Chlorophenyl)(hydroxy)methyl]-4-fluorocyclohexanol, and
(Enantiomer 1) trans-1-[(2-Chlorophenyl)(hydroxy)methyl]-4-fluorocyclohexanol
Data of (cis isomer, enantiomer 1)
(ESI neg.) m/z : 257(M-H)-
1H NMR (600 MHz, CHLOROFORM-d) d ppm 1.38 - 1.54 (m, 3 H) 1.74 - 2.04 (m, 4 H) 2.09 - 2.18 (m, 1 H) 4.37 - 4.53 (m, 1 H) 5.08 (s, 1 H) 7.20 - 7.37 (m, 3 H) 7.58 - 7.65 (m, 1 H)

Chiral analysis conditions
Column: Daicel CHIRALPAK AD3, 4.6*150
Solvent system: Hexane : EtOH = 90 : 10
Flow rate: 1 mL/min
RT: 8.18, 8.88, Faster
Data of (trans isomer, enantiomer 1)
(ESI neg.) m/z : 257(M-H)-
1H NMR (600 MHz, CHLOROFORM-d) d ppm 1.12 - 1.21 (m, 1 H) 1.70 - 1.96 (m, 7 H) 4.75 - 4.89 (m, 1 H) 5.11 (s, 1 H) 7.20 - 7.38 (m, 3 H) 7.58 - 7.65 (m, 1 H)

Chiral analysis conditions
Column: Daicel CHIRALPAK AD3, 4.6*150
Solvent system: Hexane : EtOH = 80 : 20
Flow rate: 1 mL/min
RT: 3.97, 4.39, Later
(Diastereomer mixture, enantiomer 1) 1-[(3-Chloro-2-fluorophenyl)(hydroxy)methyl]-4-fluorocyclohexanol
(ESI neg.) m/z : 275(M-H)-
1HNMR (600 MHz, CHLOROFORM-d) d ppm 1.17 - 2.11 (m, 7 H) 2.39 - 2.51 (m, 1 H) 4.36 - 4.93 (m, 2 H) 7.09 - 7.15 (m, 1 H) 7.31 - 7.39 (m, 1 H) 7.40 - 7.47 (m, 1 H)

Chiral analysis conditions
Column: Daicel CHIRALPAK AD3, 4.6* 150
Solvent system: Hexane : EtOH = 80 : 20
Flow rate: 1 mL/min
Data of (diastereomer 1, enantiomer 1)
RT: 4.81, 8.64, Faster
Data of (diastereomer 2, enantiomer 1)
RT: 3.82, 4.22, Later
(Diastereomer 1, enanthiomer 1) 1-[(2,3-Difluorophenyl)(hydroxy)methyl]-4-fluorocyclohexanol
1H NMR (600 MHz, CHLOROFORM-d) d ppm 1.30 - 2.12 (m, 8 H) 4.35 - 4.54 (m, 1 H) 4.86 - 4.94 (m, 1 H) 7.05 - 7.35 (m, 3 H)
(Enantiomer 1) 4,4-Difluoro-1-[(3-fluorophenyl)(hydroxy)methyl]cyclohexanol
1 H NMR (600 MHz, CHLOROFORM-d) d ppm 1.41 - 1.60 (2 H, m) 1.65 - 1.73 (1 H, m) 1.84 - 2.21 (5 H, m) 2.34 (1 H, d, J=3.3 Hz) 4.51 (1 H, d, J=2.9 Hz) 7.00 - 7.04 (1 H, m) 7.08 - 7.13 (2 H, m) 7.30 - 7.34 (1 H, m)
(Diastereomer mixture, enanthiomer 1) 4-Fluoro-1-[(3-fluorophenyl)(hydroxy)methyl]cyclohexanol
(Enantiomer 1) 4,4-Difluoro-1-[(2-fluorophenyl)(hydroxy)methyl]cyclohexanol
1H NMR (600 MHz, CHLOROFORM-d) d ppm 1.43 - 1.50 (1 H, m) 1.60 - 1.73 (2 H, m) 1.85 - 2.22 (5 H, m) 2.37 (1 H, d, J=4.5 Hz) 4.93 (1 H, d, J=4.5 Hz) 7.02 - 7.06 (1 H, m) 7.16 - 7.21 (1 H,m)7.27-7.33 (1 H, m) 7.50 - 7.54 (1 H, m)
(Diastereomer mixture, enanthiomer 1) 4-Fluoro-1-[(2-fluorophenyl)(hydroxy)methyl]cyclohexanol

### Production Example 3 (2R)-2-(3-Chlorophenyl)-6-fluoro-1-oxaspiro[2.5]octane

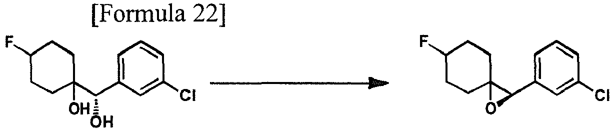

Triethylamine (84 µL) and methanesulfonic acid anhydride (62 mg) were added under ice-cooling to a solution of 1-[(S)-(3-chlorophenyl)(hydroxy)methyl]-4-fluorocyclohexanol (71 mg) in chloroform (1.0 mL), and the mixture was stirred at room temperature for 4 hours. Water was added to the reaction mixture, extracted with chloroform, and the organic layer was separated by a phase separation cartridge (Biotage, Isolute Phase Separator) and concentrated under reduced pressure. The obtained crude product was dissolved in methanol (1.0 mL), potassium carbonate (130 mg) was added thereto, and stirred at room temperature overnight. Water was added to the reaction mixture, extracted with chloroform, and the organic layer was separated by a phase separation cartridge and concentrated under reduced pressure.

The residue was purified by column chromatography (silica gel cartridge, hexane/ethyl acetate = 100 : 0 to 70 : 30) to obtain the title compound (47 mg).
1H NMR (600 MHz, CHLOROFORM-d) d ppm 1.19 - 1.28 (m, 1 H) 1.50 - 1.66 (m, 2 H) 1.68 - 1.76 (m, 1 H) 1.77 - 1.97 (m, 2 H) 1.98 - 2.18 (m, 2 H) 3.87 - 3.91 (m, 1 H) 4.67 - 4.93 (m, 1 H) 7.17 - 7.21 (m, 1 H) 7.23 - 7.31 (m, 3 H)

The following compounds were synthesized by the similar method.
(Enantiomer 1) 2-(3-Chlorophenyl)-1-oxaspiro[2.4]heptane
1H NMR (600 MHz, CHLOROFORM-d) d ppm 1.36 - 2.09 (m, 8 H) 3.98 (s, 1 H) 7.11 - 7.36 (m, 4 H)
(Enantiomer 1) 2-(2-Chlorophenyl)-6,6-difluoro-1-oxaspiro[2.5]octane
1H NMR (600 MHz, CHLOROFORM-d) d ppm 1.42 - 1.50 (m, 2 H) 1.81 - 2.03 (m, 4 H) 2.16 - 2.29 (m, 2 H) 4.05 (s, 1 H) 7.22 - 7.39 (m, 4 H)
(Enanthiomer 2) 2-(2-Chlorophenyl)-6,6-difluoro-1-oxaspiro[2.5]octane
(Enantiomer 1) 2-(2,3-Difluorophenyl)-6,6-difluoro-1-oxaspiro[2.5]octane
1H NMR (600 MHz, CHLOROFORM-d) d ppm 1.45 - 1.60 (m, 2 H) 1.81 - 2.08 (m, 4 H) 2.10 - 2.29 (m, 2 H) 4.07 (s, 1 H) 7.00 - 7.19 (m, 3 H)
(Enantiomer 1) 2-(3-Chlorophenyl)-6,6-difluoro-l-oxaspiro [2.5]octane
1 H NMR (600 MHz, CHLOROFORM-d) d ppm 1.43 - 1.62 (m, 2 H) 1.73 - 1.82 (m, 1 H) 1.85 - 2.29 (m, 5 H) 3.96 (s, 1 H) 7.15 - 7.33 (m, 4 H)
(Enantiomer 1) 2-(3-Chloro-2-fluorophenyl)-6,6-difluoro-1-oxaspiro[2.5]octane
1H NMR (600 MHz, CHLOROFORM-d) d ppm 1.45 - 1.60 (m, 2 H) 1.83 - 2.08 (m, 4 H) 2.10 - 2.28 (m, 2 H) 4.06 (s, 1 H) 7.07 - 7.13 (m, 1 H) 7.16 - 7.22 (m, 1 H) 7.32 - 7.38 (m, 1 H)
(Enantiomer 1) 2-(2-Chloro-3-fluorophenyl)-6,6-difluoro-1-oxaspiro[2.5]octane
(EI) m/z : 276(M+)
(Enantiomer 1) 2-{3-[6,6-Difluoro-l-oxaspiro[2,5]oct-2-yl]phenyl}pyridine
(ESI pos.) m/z : 302(M+H)+
LCMS RT 0.92, Condition A
(Enantiomer 1) (3S,6S)-2-(2-Chlorophenyl)-6-fluoro-1-oxaspiro[2.5]octane
(EI) m/z : 240(M+)
1H NMR (600 MHz, CHLOROFORM-d) d ppm 1.06 - 1.15 (m, 1 H) 1.49 - 1.67 (m, 2 H) 1.70 - 1.78 (m, 1 H) 1.82 - 1.92 (m, 1 H) 1.96 - 2.09 (m, 1 H) 2.12 - 2.22 (m, 2 H) 3.99 (s, 1 H) 4.71 - 4.87 (m, 1 H) 7.22 - 7.31 (m, 2 H) 7.32 - 7.38 (m, 2 H)
(Enantiomer 1) (3R,6R)-2-(2-Chlorophenyl)-6-fluoro-1-oxaspiro[2.5]octane
(EI) m/z : 240(M+)
1H NMR (600 MHz, CHLOROFORM-d) d ppm 1.12 - 1.22 (m, 1 H) 1.48 - 1.92 (m, 4 H) 1.99 - 2.21 (m, 3H) 4.01 (s, 1 H) 4.74 - 4.90 (m, 1 H) 7.19 - 7.42 (m, 4 H)
(Enantiomer 1) 2-(3-Chloro-2-fluorophenyl)-6-fluoro-1-oxaspiro[2.5]octane
(EI) m/z : 258(M+)
1H NMR (600 MHz, CHLOROFORM-d) d ppm 1.11 - 1.24 (m, 1 H) 1.48 - 2.22 (m, 7 H) 3.96 - 4.05 (m, 1 H) 4.71 - 4.92 (m, 1 H) 7.05 - 7.12 (m, 1 H) 7.15 - 7.24 (m, 1 H) 7.32 - 7.37 (m, 1 H)
(Diastereomer 1, enanthiomer 1) 2-(2,3-Difluorophenyl)-6-fluoro-1-oxaspiro[2.5]octane
(ESI pos.) m/z : 265(M+Na)+
1H NMR (600 MHz, CHLOROFORM-d) d ppm 1.12 - 1.21 (m, 1 H) 1.53 - 1.66 (m, 2 H) 1.71 - 1.82 (m, 1 H) 1.86 - 2.02 (m, 2 H) 2.09 - 2.23 (m, 2 H) 4.67 - 4.88 (m, 1 H) 7.00 - 7.19 (m, 3 H)
(Enantiomer 1) 6,6-Difluoro-2-(3-fluorophenyl)-1-oxaspiro[2.5]octane
1HNMR (600 MHz, CHLOROFORM-d) d ppm 1.45 - 1.61 (2 H, m) 1.75 - 1.81 (1 H, m) 1.85-1.94 (1 H, m) 1.96 - 2.29 (4 H, m) 3.97 (1 H, s) 6.96 - 7.04 (2 H, m) 7.05 - 7.13 (1 H, m) 7.29 - 7.36 (1 H, m)
(Diastereomer mixture, enanthiomer 1) 6-Fluoro-2-(3-fluorophenyl)-1-oxaspiro[2.5]octane (Enantiomer 1) 6,6-Difluoro-2-(2-fluorophenyl)-1-oxaspiro[2.5]octane
1H NMR (600 MHz, CHLOROFORM-d) d ppm 1.49 -1.55 (2 H, m) 1.83 - 1.92 (2 H, m) 1.95 - 2.05 (2 H, m) 2.10 - 2.28 (2 H, m) 4.07 (1 H, s) 7.03 - 7.09 (1 H, m) 7.12 - 7.18 (1 H, m) 7.26 - 7.32 (2 H, m)
(Diastereomer mixture, enanthiomer 1) 6-Fluoro-2-(2-fluorophenyl)-1-oxaspiro[2.5]octane

### Production Example 4 cis-1-[(S)-Azide(3-chlorophenyl)methyl]-4-fluorocyclohexanol

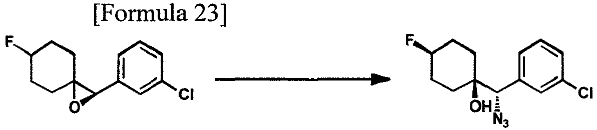

(2R)-2-(3-Chlorophenyl)-6-fluoro-1-oxaspiro[2.5]octane (47 mg), sodium azide (95 mg) and ammonium chloride (21 mg) were dissolved in a mixture of methanol (1.6 mL) and water (0.20 mL), and the mixture was stirred in a sealed tube at 120°C overnight. Water was added to the reaction mixture, extracted with chloroform, and the organic layer was separated by a phase separation cartridge and concentrated under reduced pressure. The residue was purified by column chromatography (silica gel cartridge, hexane/ethyl acetate = 100 : 0 to 70 : 30) to obtain the title compound (27 mg) and trans-1-[(S)-azide(3-chlorophenyl)methyl]-4-fluorocyclohexanol (24 mg).
1 H NMR (600 MHz, CHLOROFORM-d) d ppm 1.23 - 1.53 (m, 3 H) 1.76 - 1.97 (m, 5 H) 4.33 - 4.51 (m, 2 H) 7.20 - 7.39 (m, 4 H)
trans-1-[(S)-Azide(3-chlorophenyl)methyl]-4-fluorocyclohexanol
1H NMR (600 MHz, CHLOROFORM-d) d ppm 1.14 - 1.22 (m, 1 H) 1.48 - 2.00 (m, 7 H) 4.38 (s, 1 H) 4.73 - 4.89 (m, 1 H) 7.19 - 7.41 (m, 4 H)

The following compounds were synthesized by the similar method. cis-1-[Azide(3-chlorophenyl)methyl]-4-fluorocyclohexanol, and
trans-1-[Azide(3-chlorophenyl)methyl]-4-fluorocyclohexanol
1-[Azide(3-chlorophenyl)methyl]cyclohex-3-en-1-ol
(Diastereomer 1, enanthiomer 1) 1-[Azide(2-chlorophenyl)methyl]-4-fluorocyclohexanol
1H NMR (600 MHz, CHLOROFORM-d) d ppm 1.36 - 1.52 (m, 2 H) 1.69 - 2.00 (m, 5 H) 2.03 - 2.13 (m, 1 H) 4.36 - 4.54 (m, 1 H) 5.10 (s, 1 H) 7.22 - 7.45 (m, 3 H) 7.62 (dd, J=7.8, 1.7 Hz, 1H)
(Diastereomer 2, enanthiomer 1) 1-[Azide(2-chlorophenyl)methyl]-4-fluorocyclohexanol
1H NMR (600 MHz, CHLOROFORM-d) d ppm 1.07 - 2.18 (m, 8 H) 4.73 - 4.89 (m, 1 H) 5.14 (s, 1 H) 7.20 - 7.46 (m, 3 H) 7.57 - 7.64 (m, 1 H)
(Diastereomer 1, enantiomer 1) 1-[Azide(3-chloro-2-fluorophenyl)methyl]-4-fluorocyclohexanol, and (diastereomer 2, enantiomer 1) 1-[Azide(3-chloro-2-fluorophenyl)methyl]-4-fluorocyclohexanol
Data of (diastereomer 1, enantiomer 1)
1H NMR (600 MHz, CHLOROFORM-d) d ppm 1.31 - 2.06 (m, 8 H) 4.36 - 4.54 (m, 1 H) 4.86 (s, 1 H) 7.13 - 7.20 (m, 1 H) 7.36 - 7.47 (m, 2 H)
Data of (diastereomer 2, enantiomer 1)
1H NMR (600 MHz, CHLOROFORM-d) d ppm 1.15 - 1.22 (m, 1 H) 1.65 - 1.97 (m, 7 H) 4.74 - 4.87 (m, 1 H) 4.88 (s, 1 H) 7.13 - 7.19 (m, 1 H) 7.37 - 7.46 (m, 2 H)
(Diastereomer 1, enanthiomer 1) 1-[Azide(2,3-difluorophenyl)methyl]-4-fluorocyclohexanol (ESI neg.) m/z : 320(M+Cl)-
1H NMR (600 MHz, CHLOROFORM-d) d ppm 1.31 - 2.07 (m, 8 H) 4.37 - 4.56 (m, 1 H) 4.86 (s, 1 H) 7.10 - 7.23 (m, 2 H) 7.26 - 7.32 (m, 1 H)
(Diastereomer 1, enanthiomer 1) 1-[Azide(3-fluorophenyl)methyl]-4-fluorocyclohexanol
1H NMR (600 MHz, CHLOROFORM-d) d ppm 1.16 - 2.03 (m, 8 H) 4.29 - 4.51 (m, 2 H) 6.98 - 7.50 (m, 4 H)
(Diastereomer mixture, enanthiomer 1) 1-[Azide(2-fluorophenyl)methyl]-4-fluorocyclohexanol

### Production Example 5 cis-1-[Amino(3-chlorophenyl)methyl]-4-fluorocyclohexanol

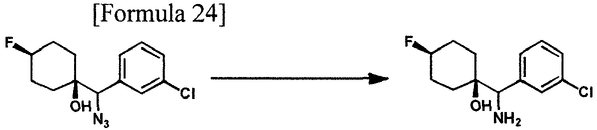

cis-1-[Azide(3-chlorophenyl)methyl]-4-fluorocyclohexanol (300 mg) was dissolved in a mixture (5.3 mL) of tetrahydrofuran : water = 4 : 1, a solution of 1.0 M trimethylphosphine in tetrahydrofuran (1.6 mL) was added thereto under ice-cooling, and the mixture was stirred at room temperature overnight. 1.0 M Hydrochloric acid was added to acidify the reaction mixture, followed by separation with diethyl ether. The obtained aqueous layer was basified with an aqueous solution of 2.0 M sodium hydroxide and subsequently extracted with chloroform. The organic layer was separated by a phase separation cartridge and concentrated under reduced pressure to obtain the title compound (270 mg).

The following compound was synthesized by the similar method.
(Diastereomer 1, enanthiomer 1) 1-[Amino(2,3-difluorophenyl)methyl]-4-fluorocyclohexanol (ESI pos.) m/z : 260(M+H)+
1H NMR (600 MHz, CHLOROFORM-d) d ppm 1.21 - 2.01 (m, 8 H) 4.20 (s, 1 H) 4.35 - 4.54 (m, 1 H) 7.04 - 7.14 (m, 2 H) 7.19 - 7.25 (m, 1 H)

### Production Example 6 cis-1-[Amino(3-chlorophenyl)methyl]-4-fluorocyclohexanol

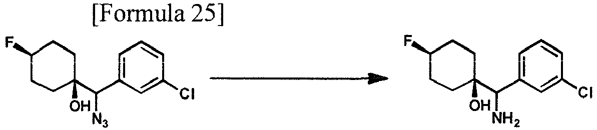

cis-1-[Azido(3-chlorophenyl)methyl]-4-fluorocyclohexanol (17 mg) was dissolved in ethanol (1.2 mL), palladium 10% on carbon (20 mg) was added thereto, and the mixture was stirred under hydrogen atmosphere at normal pressure and room temperature for 30 minutes. Further, cis-1-[azide(3-chlorophenyl)methyl]-4-fluorocyclohexanol (17 mg) was dissolved in ethanol (1.2 mL), palladium 5% on carbon (20 mg) was added thereto, and the mixture was stirred under hydrogen atmosphere at normal pressure and room temperature for 30 minutes. The reaction mixtures were each filtered and subsequently the filtrates were each concentrated under reduced pressure and combined to obtain a mixture (52 mg) of the title compound and cis-1-[amino(phenyl)methyl]-4-fluorocyclohexanol.
(ESI pos.) m/z : 258(M+H)+
LCMS RT 0.67, Condition B

The following compound was synthesized by the similar method.
cis-1-[Amino(phenyl)methyl]-4-fluorocyclohexanol
(ESI pos.) m/z : 224(M+H)+
LCMS RT 0.52, Condition B
(Enantiomer 1) cis-1-[Amino(phenyl)methyl]-4-fluorocyclohexanol

### Production Example 7 cis-4-Fluoro-l-[(trimethylsilyl)oxy]cyclohexanecarbonitrile

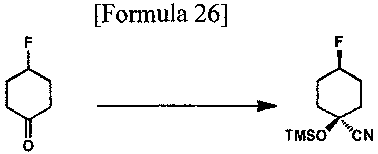

Trimethylsilanecarbonitrile (5.4 mL) was added to a solution of 4-fluorocyclohexanone (1.0 g) and 4-methylmorpholine 4-oxide (250 mg) in chloroform (43 mL) at room temperature and the mixture was stirred at 50°C for 2 hours. An aqueous saturated sodium hydrogen carbonate solution was added to the reaction mixture, extracted with chloroform, and the organic layer was separated by a phase separation cartridge and concentrated under reduced pressure. The residue was purified by column chromatography (silica gel cartridge, hexane/ethyl acetate = 98 : 2 to 80 : 20) to obtain a crude product (1.5 g) containing the title compound.
(EI) m/z : 215 (M+)
1H NMR (600 MHz, CHLOROFORM-d) d ppm 0.26 (s, 9 H) 1.74 - 2.20 (m, 8 H) 4.68 - 4.81 (m, 1 H)

The following compounds were synthesized by the similar method.
1-[(Trimethylsilyl)oxy](²H₁₀)cyclohexane carbonitrile
4,4-Difluoro-1-[(trimethylsilyl)oxy]cyclohexane carbonitrile
1H NMR (600 MHz, CHLOROFORM-d) d ppm 0.27 (9 H, s) 1.94 - 2.24 (8 H, m)
1-[(Trimethylsilyl)oxy]cyclohexane carbonitrile
1H NMR (600 MHz, CHLOROFORM-d) d ppm 0.24 (9 H, s) 1.20 - 1.29 (1 H, m) 1.50 - 1.67 (5 H, m) 1.70 - 1.80 (2 H, m) 1.97 - 2.09 (2 H, m)

### Production Example 8 cis-4-Fluoro-1-hydroxycyclohexanecarbaldehyde

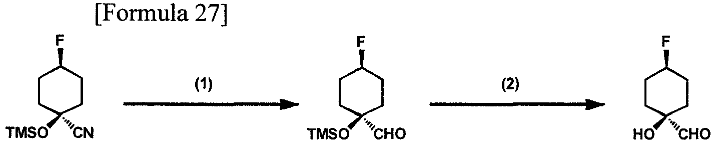

(1) A solution of 1.0 M diisobutylaluminum hydride in hexane (14 mL) was added at -40°C to a solution of cis-4-fluoro-1-[(trimethylsilyl)oxy]cyclohexanecarbonitrile (1.5 g) in hexane (71 mL), stirred for 10 minutes, and subsequently stirred at 0°C for 1 hour. An aqueous saturated sodium potassium tartrate solution was added to the reaction mixture, and stirred at room temperature overnight. The reaction mixture was extracted with chloroform, the organic layer was separated by a phase separation cartridge, and subsequently concentrated under reduced pressure. The residue was purified twice by column chromatography (silica gel cartridge, hexane/ethyl acetate = 95 : 5 to 60 : 40) to obtain cis-4-fluoro-1-[(trimethylsilyl)oxy]cyclohexanecarbaldehyde (750 mg).
(2) 2.0 M Hydrochloric acid (17 mL) was added at room temperature to a solution of cis-4-fluoro-1-[(trimethylsilyl)oxy]cyclohexanecarbaldehyde (750 mg) in tetrahydrofuran (34 mL), and stirred for 2 hours. An aqueous saturated sodium hydrogen carbonate solution was added to the reaction mixture, extracted with chloroform and ethyl acetate, and the organic layer was concentrated under reduced pressure. The residue was purified by column chromatography (silica gel cartridge, hexane/ethyl acetate = 92 : 8 to 34 : 66) to obtain a crude product (250 mg) containing the title compound.
1H NMR (600 MHz, CHLOROFORM-d) d ppm 1.30 - 2.15 (m, 8 H) 2.91 (s, 1 H) 4.51 - 4.70 (m, 1 H) 9.51 (s, 1 H)

The following compounds were synthesized by the similar method.
1-Hydroxy(²H₁₀)cyclohexanecarbaldehyde 4,4-difluoro-1-hydroxycyclohexanecarbaldehyde
1H NMR (600 MHz, CHLOROFORM-d) d ppm 1.16 - 2.38 (8 H, m) 3.09 (1 H, s) 9.53 (1 H, s)
1-Hydroxycyclohexanecarbaldehyde
1H NMR (600 MHz, CHLOROFORM-d) d ppm 1.13 - 1.94 (8 H, m) 9.51 (1 H, s)

### Production Example 9 cis-1-Ethenyl-4-fluorocyclohexanol

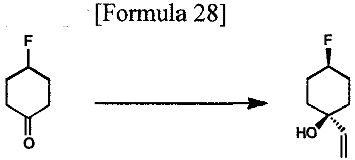

A solution of 1.0 M vinylmagnesium bromide in tetrahydrofuran (14 mL) was added at -78°C to a solution of 4-fluorocyclohexanone (1.0 g) in tetrahydrofuran (43 mL), and the mixture was stirred for 1 hour. An aqueous saturated ammonium chloride solution was added to the reaction mixture, extracted 3 times with chloroform, and subsequently the organic layer was separated by a phase separation cartridge and concentrated under reduced pressure. The residue was purified by column chromatography (silica gel cartridge, hexane/ethyl acetate = 92 : 8 to 34 : 66) to obtain a mixture of the title compound and trans-1-ethenyl-4-fluorocyclohexanol (4.3 : 1 = 430 mg).
(EI) m/z : 144(M+)
1H NMR (600 MHz, CHLOROFORM-d) d ppm 1.44 - 2.02 (m, 8 H) 4.49 - 4.63 (m, 1 H) 5.10 (d, J=10.7 Hz, 1 H) 5.26 - 5.33 (m, 1 H) 5.91 - 5.98 (m, 1 H)

### Production Example 10 cis-4-Fluoro-1-hydroxycyclohexanecarbaldehyde

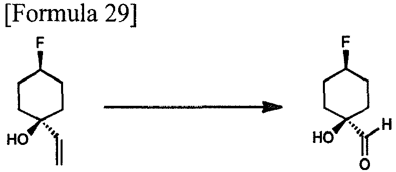

Ozone gas was bubbled at -78°C to a solution of 1-ethenyl-4-fluorocyclohexanol (1.3 g) in methanol (45 mL) and stirred for 30 minutes. After bubbling oxygen for 2 minutes and nitrogen for 5 minutes to the reaction mixture, a mixed solution of triphenyl phosphine (4.7 g) in hexane (18 mL) and chloroform (9.0 mL) was added dropwise and the mixture was stirred at room temperature for 1 hour. The reaction mixture was concentrated under reduced pressure, and the residue was purified by column chromatography (silica gel cartridge, hexane/ethyl acetate = 92 : 8 to 34 : 66) to obtain a crude product (830 mg) containing the title compound.

### Production Example 11 (S)-N-[(E)-(cis-4-Fluoro-1-hydroxycyclohexyl)methylidene]-2-methylpropane-2-sulfinamide

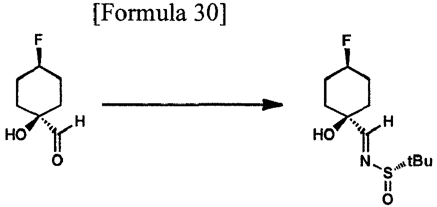

(S)-t Butylsulfinamide (1.3 g) and tetraethoxytitanium (2.4 g) were added to a solution of cis-4-fluoro-1-hydroxycyclohexanecarbaldehyde (510 mg) in tetrahydrofuran (35 mL), and heated to reflux for 5 hours. The reaction mixture was brought back to room temperature, an aqueous saturated sodium hydrogen carbonate solution was added thereto, and the mixture was filtered through Celite. The filtrate was extracted 3 times with chloroform, subsequently the organic layer was separated by a phase separation cartridge and concentrated under reduced pressure. The residue was purified twice by column chromatography (silica gel cartridge, hexane/ethyl acetate = 92 : 8 to 34 : 66) and subsequently purified by PTLC (silica gel, hexane/ethyl acetate = 2 : 1 x 2) to obtain the title compound (350 mg).
(ESI pos.) m/z : 250(M+H)+
LCMS RT 0.72, Condition A
1H NMR (600 MHz, CHLOROFORM-d) d ppm 1.21 (s, 9 H) 1.66 - 1.80 (m, 4 H) 1.90 - 2.16 (m, 4 H) 3.15 (s, 1 H) 4.51 - 4.69 (m, 1 H) 8.06 (s, 1 H)

The following compounds were synthesized by the similar method.
(S)-N-{(E)-[1-Hydroxy(²H₁₀)cyclohexyl]methylidene}-2-methylpropane-2-sulfinamide(S)-N-[(E)-(4,4-difluoro-1-hydroxycyclohexyl)methylidene]-2-methylpropane-2-sulfinamide
(ESI pos.) m/z : 268(M+H)+
1H NMR (600 MHz, CHLOROFORM-d) d ppm 1.22 (9 H, s) 1.58 - 1.72 (2 H, m) 1.94 - 2.32 (6 H, m) 3.21 (1 H, br. s.) 8.07 (1 H, s)
(S)-N-[(E)-(1-Hydroxycyclohexyl)methylidene]-2-methylpropane-2-sulfinamide
(ESI pos.) m/z : 232(M+H)+

### Production Example 12 (S)-N-[(S)-(3-Chlorophenyl)(cis-4-fluoro-1-hydroxycyclohexyl)methyl]-2-methylpropane-2-sulfinamide

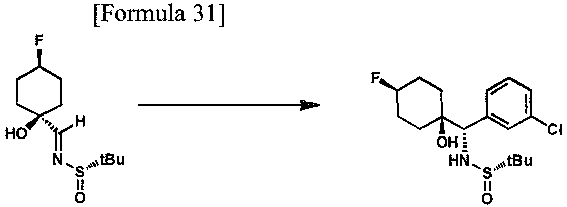

1,2-Dibromoethane (50 µL), iodine (1 piece) and 1-bromo-3-chlorobenzene (3.3 g) were added to a solution of magnesium (420 mg) in tetrahydrofuran (9.1 mL), and heated to reflux for 30 minutes. After cooling the reaction mixture to -78°C, a solution of (S)-N-[(E)-(cis-4-fluoro-1-hydroxycyclohexyl)methylidene]-2-methylpropane-2-sulfinamide (530 mg) in tetrahydrofuran (34 mL) was slowly added dropwise. The mixture was stirred at -40°C for 4 hours, an aqueous saturated ammonium chloride solution was added to the reaction mixture, extracted 3 times with chloroform, and subsequently the organic layer was separated by a phase separation cartridge and concentrated under reduced pressure. The residue was purified by column chromatography (silica gel cartridge, hexane/ethyl acetate = 88 : 12 to 0 : 100) and subsequently purified 3 times by preparative HPLC to obtain the title compound (240 mg).
(ESI pos.) m/z : 362(M+H)+
LCMS RT 1.02, Condition A
1H NMR (600 MHz, CHLOROFORM-d) d ppm 1.11 - 2.06 (m, 8 H) 1.24 (s, 9 H) 4.19 - 4.38 (m, 2 H) 4.75 (br. s., 1 H) 7.16 - 7.32 (m, 4 H)

The following compounds were synthesized by the similar method.
(Diastereomer 1) (S)-N-[(2-Chlorophenyl)(cis-4-fluoro-1-hydroxycyclohexyl)methyl]-2-methylpropane-2-sulfmamide, and
(diastereomer 2) (S)-N-[(2-Chlorophenyl)(cis-4-fluoro-1-hydroxycyclohexyl)methyl]-2-methylpropane-2-sulfinamide
Data of (diastereomer 1)
(ESI pos.) m/z : 362(M+H)+
LCMS RT 0.97, Condition A
Data of (diastereomer 2)
(ESI pos.) m/z : 362(M+H)+
LCMS RT 0.93, Condition A
(Diastereomer 1) (S)-N-[(2-Chloro-3-fluorophenyl)(cis-4-fluoro-1-hydroxycyclohexyl)methyl]-2-methylpropane-2-sulfinamide, and
(diastereomer 2) (S)-N-[(2-Chloro-3-fluorophenyl)(cis-4-fluoro-1-hydroxycyclohexyl)methyl]-2-methylpropane-2-sulfinamide
Data of (diastereomer 1)
(ESI pos.) m/z : 380(M+H)+
LCMS RT 1.00, Condition A
Data of (diastereomer 2)
(ESI pos.) m/z : 380(M+H)+
LCMS RT 0.97, Condition A
(Diastereomer 1) (S)-N-[3-Chloro-4-fluorophenyl)(cis-4-fluoro-1-hydroxycyclohexyl)methyl]-2-methylpropane-2-sulfinamide
(ESI pos.) m/z : 380(M+H)+
LCMS RT 1.03, Condition A
(Diastereomer 1) (S)-N-[(3-Chloro-5-fluorophenyl)(cis-4-fluoro-1-hydroxycyclohexyl)methyl]-2-methylpropane-2-sulfinamide
(ESI pos.) m/z : 380(M+H)+
LCMS RT 0.99, Condition A
1H NMR (600 MHz, CHLOROFORM-d) d ppm 1.10 - 2.24 (m, 8 H) 1.18 (s, 9 H) 4.05 - 4.19 (m, 1 H) 4.41 - 4.61 (m, 1 H) 4.73 (d, J=8.7 Hz, 1 H) 6.97 - 7.07 (m, 1 H) 7.13 (dd, J=8.5, 2.7 Hz, 1 H) 7.62 - 7.73 (m, 1 H)
(Diastereomer mixture) (S)-N-{(3-Chlorophenyl)[1-hydroxy(²H₁₀)cyclohexyl]methyl}-2-methylpropane-2-sulfinamide
(Diastereomer 1) (S)-N-[(5-Chloropyridin-3-yl)(cis-4-fluoro-l-hydroxycyclohexyl)methyl]-2-methylpropane-2-sulfinamide
1H NMR (600 MHz, CHLOROFORM-d) d ppm 1.09 - 2.03 (m, 17 H) 4.24 - 4.43 (m, 2 H) 4.62 (br. s., 1 H) 7.64 (t, J=2.1 Hz, 1 H) 8.42 (d, J=2.1 Hz, 1 H) 8.52 (d, J=2.1 Hz, 1 H)
(Diastereomer 2) (S)-N-[(5-Chloropyridin-3-yl)(cis-4-fluoro-1-hydroxycyclohexyl)methyl]-2-methylpropane-2-sulfinamide
1H NMR (600 MHz, CHLOROFORM-d) d ppm 0.88 - 2.31 (m, 17 H) 3.99 - 4.29 (m, 2 H) 4.39 - 4.74 (m, 1 H) 7.71 - 7.90 (m, 1 H) 8.27 - 8.75 (m, 2 H)
(Diastereomer 1) (S)-N-[(cis-4-Fluoro-1-hydroxycyclohexyl) (pyridin-2-yl)methyl]-2-methylpropane-2-sulfinamide
(ESI pos.) m/z : 329 (M+H)+
(Diastereomer 1) (S)-N-[(2-Chloropyridin-4-yl)(cis-4-fluoro-1-hydroxycyclohexyl)methyl]-2-methylpropane-2-sulfinamide
1H NMR (600 MHz, CHLOROFORM-d) d ppm 0.74 - 2.00 (m, 17 H) 4.24 - 4.40 (m, 2 H) 4.70 (br. s., 1 H) 7.16 (br. d, J=5.4 Hz, 1 H) 7.29 (br. s, 1 H) 8.36 (d, J=5.4 Hz, 1 H)
(Diastereomer 2) (S)-N-[(2-Chloropyridin-4-yl)(cis-4-fluoro-1-hydroxycyclohexyl)methyl]-2-methylpropane-2-sulfinamide
1H NMR (600 MHz, CHLOROFORM-d) d ppm 0.79 - 2.13 (m, 17 H) 4.08 (d, J=9.1 Hz, 1 H) 4.23 (br. d, J=9.1 Hz, 1 H) 4.43 - 4.61 (m, 1 H) 7.23 (br. d, J=5.2 Hz, 1 H) 7.34 (br. s, 1 H) 8.36 (d, J=5.2 Hz, 1 H)
(Diastereomer 1) (S)-N-[(cis-4-Fluoro-1-hydroxycyclohexyl)(pyridin-3-yl)methyl]-2-methylpropane-2-sulfinamide
(ESI pos.) m/z : 329(M+H)+
(Diastereomer 2) (S)-N-[(cis-4-Fluoro-1-hydroxycyclohexyl)(pyridin-3-yl)methyl]-2-methylpropane-2-sulfinamide
(ESI pos.) m/z : 329(M+H)+
(Diastereomer 1) (S)-N-[(2-Chloropyridin-3-yl)(cis-4-fluoro-1-hydroxycyclohexyl)methyl]-2-methylpropane-2-sulfinamide
(ESI pos.) m/z : 363(M+H)+
(Diastereomer 1) (S)-N-[(6-Chloropyridin-2-yl)(cis-4-fluoro-1-hydroxycyclohexyl)methyl]-2-methylpropane-2-sulfinamide
1 H NMR (600 MHz, CHLOROFORM-d) d ppm 1.14 (s, 9 H) 1.22 - 2.08 (m, 8 H) 3.96 (br. s., 1 H) 4.22 (d, J=8.3 Hz, 1 H) 4.34 - 4.51 (m, 1 H) 4.66 (br. d, J=8.3 Hz, 1 H) 7.13 (d, J=7.4 Hz, 1 H) 7.27 (d, J=7.4 Hz, 1 H) 7.65 (t, J=7.4 Hz, 1 H)
(Diastereomer 1) (S)-N-[(6-Chloropyridin-3-yl)(4,4-difluoro-1-hydroxycyclohexyl)methyl]-2-methylpropane-2-sulfinamide
1H NMR (600 MHz, CHLOROFORM-d) d ppm 1.16 - 2.20 (8 H, m) 4.34 - 4.44 (2 H, m) 4.76 - 4.81 (1 H, m) 7.30 - 7.36 (1 H, m) 7.56 - 7.62 (1 H, m) 8.25 - 8.40 (1 H, m)
(Diastereomer 2) (S)-N-[(6-Chloropyridin-3-yl)(4,4-difluoro-1-hydroxycyclohexyl)methyl]-2-methylpropane-2-sulfinamide
1H NMR (600 MHz, CHLOROFORM-d) d ppm 1.23 - 2.20 (8 H, m) 4.10 - 4.20 (2 H, m) 7.31 - 7.36 (1 H, m) 7.80 - 7.86 (1 H, m) 8.34 - 8.40 (1 H, m)
(Diastereomer 1) (S)-N-[(4-chloropyridin-2-yl)(cis-4-fluoro-1-hydroxycyclohexyl)methyl]-2-methylpropane-2-sulfinamide
(ESI pos.) m/z : 363(M+H)+
(Diastereomer 1) (S)-N-[(6-Chloropyridin-3-yl)(cis-4-fluoro-1-hydroxycyclohexyl)methyl]-2-methylpropane-2-sulfinamide
1 H NMR (600 MHz, CHLOROFORM-d) d ppm 1.08 - 1.98 (m, 17 H) 4.22 - 4.41 (m, 2 H) 4.56 (d, J=2.9 Hz, 1 H) 7.32 (d, J=8.3 Hz, 1 H) 7.60 (dd, J=8.3, 2.5 Hz, 1 H) 8.32 (d,
J=2.5 Hz, 1 H)
(Diastereomer 1) (S)-N-[(3-Chloropyridin-2-yl)(cis-4-fluoro-1-hydroxycyclohexyl)methyl]-2-methylpropane-2-sulfinamide
(ESI pos.) m/z : 363(M+H)+
(Diastereomer 1) (S)-N-[(4-Chloropyridin-2-yl)(cis-4-fluoro-1-hydroxycyclohexyl)methyl]-2-methylpropane-2-sulfinamide
(ESI pos.) m/z : 363(M+H)+
(Diastereomer 1) (S)-N-[(6-Chloropyridin-2-yl)(4,4-difluoro-1-hydroxycyclohexyl)methyl]-2-methylpropane-2-sulfinamide
(ESI pos.) m/z : 381(M+H)+
1H NMR (600 MHz, CHLOROFORM-d) d ppm 1.44 - 2.19 (m, 17 H) 4.21 - 4.26 (m, 1 H) 4.71 (d, J=8.7 Hz, 1 H) 7.13 (dd, J=7.6, 0.8 Hz, 1 H) 7.28 - 7.30 (m, 1 H) 7.66 (t, J=7.6 Hz, 1 H)
(Diastereomer 1) (S)-N-[(3-Chloropyridin-2-yl)(4,4-difluoro-1-hydroxycyclohexyl)methyl]-2-methylpropane-2-sulfinamide
(ESI pos.) m/z : 381(M+H)+
(Diastereomer 2) (S)-N-[(3-Chloropyridin-2-yl)(4,4-difiuoro-1-hydroxycyclohexyl)methyl]-2-methylpropane-2-sulfinamide
(ESI pos.) m/z : 381(M+H)+
(Diastereomer 1) (S)-N-[(cis-4-Fluoro-1-hydroxycyclohexyl)(6-fluoropyridin-2-yl)methyl]-2-methylpropane-2-sulfinamide
(ESI pos.) m/z : 347(M+H)+
(Diastereomer 1) (S)-N-[(2-Chloropyridin-3-yl)(4,4-difluoro-1-hydroxycyclohexyl)methyl]-2-methylpropane-2-sulfinamide
(ESI pos.) m/z : 381(M+H)+
(Diastereomer 1) (S)-N-[(4-Chloropyridin-2-yl)(4,4-difluoro-1-hydroxycyclohexyl)methyl]-2-methylpropane-2-sulfinamide
(ESI pos.) m/z : 381(M+H)+
(Diastereomer 1) (S)-N-[(4,4-Difluoro-1-hydroxycyclohexyl)(6-fluoropyridin-2-yl)methyl]-2-methylpropane-2-sulfinamide
(ESI pos.) m/z : 365(M+H)+
(Diastereomer 1) (S)-N-[(3-Chloropyridin-2-yl)(cis-4-fluoro-1-hydroxycyclohexyl)methyl]-2-methylpropane-2-sulfinamide
(ESI pos.) m/z : 363(M+H)+
(Diastereomer 1) (S)-N-[(2-Chloro-5-fluorophenyl)(cis-4-fluoro-1-hydroxycyclohexyl)methyl]-2-methylpropane-2-sulfinamide
(ESI pos.) m/z : 380(M+H)+
(Diastereomer 1) (S)-N-[(5-Chloro-2-fluorophenyl)(cis-4-fluoro-1-hydroxycyclohexyl)methyl]-2-methylpropane-2-sulfinamide
(ESI pos.) m/z : 380(M+H)+
(Diastereomer 1) (S)-N-[(6-Chloropyridin-2-yl)(1-hydroxycyclohexyl)methyl]-2-methylpropane-2-sulfinamide
(ESI pos.) m/z : 345(M+H)+
(Diastereomer 2) (S)-N-[(3-Chloro-4-fluorophenyl)(cis-4-fluoro-1-hydroxycyclohexyl)methyl]-2-methylpropane-2-sulfinamide
(ESI pos.) m/z : 380(M+H)+

### Production Example 13 cis-1-[(S)-Amino(3-chlorophenyl)methyl]-4-fluorocyclohexanol

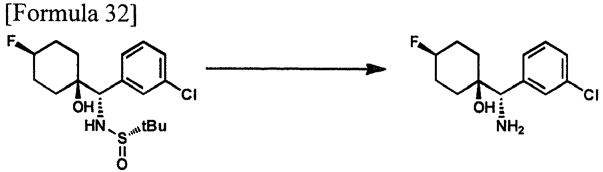

A solution of 4.0 M hydrogen chloride in 1,4-dioxane (0.4 mL) was added to a solution of (S) -N-[(S)-(3-chlorophenyl)(cis-4-fluoro-1-hydroxycyclohexyl)methyl]-2-methylpropane-2-sulfinamide (180 mg) in methanol (5.0 mL), and the mixture was stirred at room temperature for 1 hour. An aqueous saturated sodium hydrogen carbonate solution was added to the reaction mixture, extracted 3 times with chloroform, and the organic layer was separated by a phase separation cartridge and concentrated under reduced pressure. A mixture (120 mg) containing the title compound was obtained from the residue.

The following compounds were synthesized by the similar method.
(Enantiomer 1) cis-1-[Amino(2-chlorophenyl)methyl]-4-fluorocyclohexanol
(Enantiomer 2) cis-1-[Amino(2-chlorophenyl)methyl]-4-fluorocyclohexanol
(Enantiomer 1) cis-1-[Amino(2-chloro-3-fluorophenyl)methyl]-4-fluorocyclohexanol
(ESI pos.) m/z : 276(M+H)+
(Enanthiomer 2) cis-1-[Amino(2-chloro-3-fluorophenyl)methyl]-4-fluorocyclohexanol
(ESI pos.) m/z : 276(M+H)+
(Enantiomer 1) cis-1-[Amino(3-chloro-4-fluorophenyl)methyl]-4-fluorocyclohexanol
1H NMR (600 MHz, CHLOROFORM-d) d ppm 1.08 - 2.18 (8 H, m), 3.72 (1 H, s), 4.36 - 4.50 (1 H, m), 7.06 - 7.12 (1 H, m), 7.16 - 7.22 (1 H, m), 7.35 - 7.42 (1 H, m)
(Enantiomer 1) cis-1-[Amino(3-chloro-5-fluorophenyl)methyl]-4-fluorocyclohexanol
(ESI pos.) m/z : 276(M+H)±
LCMS RT 0.72, Condition B
(Enanthiomer 2) cis-1-[Amino(3-chloro-4-fluorophenyl)methyl]-4-fluorocyclohexanol
1H NMR (600 MHz, CHLOROFORM-d) d ppm 1.11 - 1.95 (8 H, m) 3.72 (1 H, s) 4.35 - 4.51 (1 H, m) 7.07 - 7.11 (1 H, m) 7.17 - 7.21 (1 H, m) 7.37 - 7.40 (1 H, m)
(Enantiomer 1) cis-1-[Amino(pyridin-2-yl)methyl]-4-fluorocyclohexanol
1H NMR (600 MHz, CHLOROFORM-d) d ppm 0.74 - 2.21 (8 H, m) 3.75 (1 H, s) 4.34 - 4.53 (1 H, m) 7.19 - 7.28 (2 H, m) 7.63 - 7.72 (1 H, m) 8.41 - 8.64 (1 H, m)
(Enantiomer 1) cis-1-[Amino(2-chloropyridin-3-yl)methyl]-4-fluorocyclohexanol
1H NMR (600 MHz, CHLOROFORM-d) d ppm 1.22 - 2.06 (8 H, m) 2.92 (1 H, br. s.) 3.80 (1 H, s) 4.34 - 4.58 (2 H, m) 7.23 - 7.30 (1 H, m) 7.98 - 8.05 (1 H, m) 8.29 - 8.34 (1 H, m)
(Enantiomer 1) 1-[Amino(2-chloropyridin-3-yl)methyl]-4,4-difluorocyclohexanol
(ESI pos.) m/z : 277(M+H)+
(Enantiomer 1) 1-[Amino(4-chloropyridin-2-yl)methyl]-4,4-difluorocyclohexanol
(ESI pos.) m/z : 277(M+H)+
(Enantiomer 1) 1-[Amino(6-fluoropyridin-2-yl)methyl]-4,4-difluorocyclohexanol
(ESI pos.) m/z : 261 (M+H)+
(Enantiomer 1) cis-1-[Amino(2-chloro-5-fluorophenyl)methyl]-4-fluorocyclohexanol
1H NMR (600 MHz, CHLOROFORM-d) d ppm 1.11 - 2.07 (8 H, m) 4.31 - 4.56 (2 H, m) 6.86 - 6.98 (1 H, m) 7.28 - 7.38 (2 H, m)
(Enantiomer 1) cis-1-[Amino(5-chloro-2-fluorophenyl)methyl]-4-fluorocyclohexanol
(ESI pos.) m/z : 276(M+H)+

### Production Example 14 {[2-(3-Chlorophenyl)-1-oxaspiro[2.5]octa-6-yl]oxy}(tert-butyl)dimethylsilane

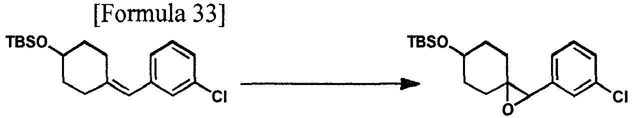

3-Chloroperbenzoic acid (6.6 g) was added under ice-cooling to a solution of {[4-(3-chlorobenzyliene)cyclohexyl]oxy}(tert-butyl) dimethylsilane (5.6 g) in chloroform (100 mL), and the mixture was stirred at room temperature for 18 hours. An aqueous saturated sodium thiosulfate solution was added to the reaction mixture, stirred, and the solution was extracted 3 times with chloroform, and subsequently washed twice with an aqueous saturated sodium hydrogen carbonate solution. The organic layer was separated by a phase separation cartridge and concentrated under reduced pressure. The residue was purified by column chromatography (silica gel cartridge, hexane/ethyl acetate = 98 : 2 to 88 : 12) to obtain the title compound (3.2 g).
(ESI pos.) m/z : 375(M+Na)+
1H NMR (600 MHz, CHLOROFORM-d) d ppm 0.01 - 0.07 (m, 6 H) 0.83 - 0.93 (m, 9 H) 1.12 - 2.00 (m, 8 H) 3.85 (s, 1 H) 3.88 - 3.92 (m, 1 H) 7.17 - 7.32 (m, 4 H)

The following compounds were synthesized by the similar method.
2-(3-Fluorophenyl)-1-oxaspiro[2.3]hexane
(ESI pos.) m/z : 179(M+H)+
1H NMR (600 MHz, CHLOROFORM-d) d ppm 1.64 - 1.76 (m, 1 H) 1.84 - 2.01 (m, 2 H) 2.37 - 2.55 (m, 2 H) 2.58 - 2.69 (m, 1 H) 3.84 (s, 1 H) 6.82 - 6.89 (m, 1 H) 6.95 - 7.02 (m, 2 H) 7.28 - 7.34 (m, 1 H)
2-(3-Fluorophenyl)-1-oxaspiro[2.4]heptane
(ESI pos.) m/z : 193(M+H)+
2-(2-Fluorophenyl)-1-oxaspiro[2.4]heptane
(ESI pos.) m/z : 193(M+H)+
2-(2-Chlorophenyl)-1-oxaspiro[2.4]heptane
(ESI pos.) m/z : 209(M+H)+
2-(3-Chlorophenyl)-1-oxaspiro[2.4]heptane
2-(4-Chlorophenyl)-1-oxaspiro[2.4]heptane
(ESI pos.) m/z : 209(M+H)+
2-(2-Methylphenyl)-1-oxaspiro[2.4]heptane
(ESI pos.) m/z : 189(M+H)+
2-(3-Methylphenyl)-i-oxaspiro[2.4]heptane
(ESI pos.) m/z : 189(M+H)+
1H NMR (600 MHz, CHLOROFORM-d) d ppm 1.37 - 2.13 (m, 8 H) 2.35 (s, 3 H) 3.98 (s, 1 H) 7.03 - 7.11 (m, 3 H) 7.20 - 7.27 (m, 1 H)
2-(4-Methylphenyl)-1-oxaspiro[2.4]heptane
(ESI pos.) m/z : 189(M+H)+
2-(2-Methoxyphenyl)-1-oxaspiro[2.4]heptane
(ESI pos.) m/z : 205(M+H)+
2-(3-Methoxyphenyl)-1-oxaspiro[2.4]heptane
(ESI pos.) m/z : 205(M+H)+
1H NMR (600 MHz, CHLOROFORM-d) d ppm 1.42 - 2.09 (m, 8 H) 3.81 (s, 3 H) 3.99 (s, 1 H) 6.78 - 6.89 (m, 3 H) 7.23 - 7.28 (m, 1 H)
1-[3-(1-Oxaspiro[2.4]hepta-2-yl)phenyl]-1H-1,2,3-triazole
(ESI pos.) m/z : 242(M+H)+
2-[3-(1-Oxaspiro[2.4]hepta-2-yl)phenyl]-2H-1,2,3-triazole
(ESI pos.) m/z : 242(M+H)+
1H NMR (600 MHz, CHLOROFORM-d) d ppm 1.43 - 2.11 (m, 8 H) 4.10 (s, 1 H) 7.23 - 7.29 (m, 1 H) 7.46 (t, J=7.8 Hz, 1 H) 7.79 - 7.85 (m, 2 H) 7.97 - 8.05 (m, 2 H)
1-[3-(1-Oxaspiro[2.4]hepta-2-yl)phenyl]-1H-pyrazole
(ESI pos.) m/z : 241(M+H)+
2-[3-(Trifluoromethyl)phenyl]-1-oxaspiro[2.4]heptane
(ESI pos.) m/z : 265(M+Na)+
2-[3-(Trifluoromethoxy)phenyl]-1-oxaspiro[2.4]heptane
(ESI pos.) m/z : 259(M+H)+
2-(3-Ethoxyphenyl)-1-oxaspiro[2.4]heptane
(ESI pos.) m/z : 219(M+H)+
2-(3-Chloro-5-fluorophenyl)-1-oxaspiro[2.4]heptane
(EI) m/z : 226(M+)
2-(3-Chloro-4-fluorophenyl)-1-oxaspiro[2.4]heptane
(EI) m/z : 226(M+)
2-(2-Chloro-4-fluorophenyl)-1-oxaspiro[2.4]heptane
(EI) m/z : 226(M+)
Methyl 3-(1-Oxaspiro[2.4]hepta-2-yl)benzoate
(ESI pos.) m/z : 255(M+Na)+
2-(2,3-Dichlorophenyl)-1-oxaspiro[2.4]heptane
(ESI pos.) m/z : 265(M+Na)+
2-(2,5-Dichlorophenyl)-1-oxaspiro[2.4]heptane
(ESI pos.) m/z : 265(M+Na)+
2-(3,5-Dichlorophenyl)-1-oxaspiro[2.4]heptane
(ESI pos.) m/z : 265(M+Na)+
2-(3-Bromophenyl)-1-oxaspiro[2.5]octane
(EI) m/z : 266(M+)
1H NMR (600 MHz, CHLOROFORM-d) d ppm 1.21 - 1.90 (m, 10 H) 3.81 (s, 1 H) 7.17 - 7.26 (m, 2 H) 7.38 - 7.43 (m, 1 H) 7.46 (s, 1 H)
2-(3-Fluorophenyl)-1-oxaspiro [2.5]octane
(ESI pos.) m/z : 207(M+H)+
4-[3-(6,6-Difluoro-1-oxaspiro[2.5]oct-2-yl)phenyl]-1-ethyl-1H-pyrazole
(ESI pos.) m/z : 319(M+H)+
1 H NMR (600 MHz, CHLOROFORM-d) d ppm 1.22 - 2.29 (m, 11 H) 4.01 (s, 1 H) 4.22 (q, J-7.3 Hz, 2 H) 7.15 (d, J=7.4 Hz, 1 H) 7.24 - 7.45 (m, 3 H) 7.67 (s, 1 H) 7.78 (s, 1 H)
6,6-Difluoro-2-(3-fluorophenyl)-1-oxaspiro[2.5]octane
(EI) m/z : 242(M+)
1H NMR (600 MHz, CHLOROFORM-d) d ppm 1.43 - 2.32 (m, 8 H) 3.97 (s, 1 H) 6.95 - 7.04 (m, 2 H) 7.09 (d, J=7.4 Hz, 1 H) 7.29 - 7.36 (m, 1 H)
2-(3-Bromophenyl)-6,6-difluoro-1-oxaspiro[2.5]octane
(EI) m/z : 302(M+)
1H NMR (600 MHz, CHLOROFORM-d) d ppm 1.43 - 1.61 (m, 2 H) 1.70 - 1.82 (m, 1 H) 1.84 - 2.28 (m, 5 H) 3.95 (s, 1 H) 7.20 - 7.27 (m, 2 H) 7.40 - 7.47 (m, 2 H)
2-(2-Chlorophenyl)-6,6-difluoro-1-oxaspiro[2.5]octane
4-[3-(6,6-Difluoro-1-oxaspiro[2.5]oct-2-yl)phenyl]-1-(propane-2-yl)-1 H-pyrazole
(ESI pos.) m/z : 333(M+H)+
4-[3-(6,6-Difluoro-1-oxaspiro [2.5] oct-2-yl)phenyl]-1-(2,2,2-trifluoroethyl)-1 H-pyrazole
(ESI pos.) m/z : 373(M+H)+
2-(2,3-Dichlorophenyl)-6,6-difluoro-1-oxaspiro[2.5]octane
(EI) m/z: 292(M+)
6,6-Difluoro-2-(3-iodophenyl)-1-oxaspiro[2.5]octane
(EI) m/z : 350(M+)
1H NMR (600 MHz, CHLOROFORM-d) d ppm 1.42 - 2.28 (m, 8 H) 3.92 (s, 1 H) 7.07 - 7.12 (m, 1 H) 7.21 - 7.31 (m, 1 H) 7.62 - 7.67 (m, 2 H)
2-(2,5-Dichlorophenyl)-6,6-difluoro-1-oxaspiro[2.5] octane
(EI) m/z : 292(M+)
6,6-Difluoro-2-(2,3,4-trifluorophenyl)-1-oxaspiro[2.5]octane
1H NMR (600 MHz, CHLOROFORM-d) d ppm 1.42 - 1.51 (m, 1 H), 1.52 - 1.58 (m, 1 H), 1.80 - 1.95 (m, 2 H), 1.97 - 2.07 (m, 2 H), 2.11 - 2.26 (m, 2 H), 4.02 (s, 1 H), 6.92 - 7.04 (m, 2 H)
{[2-(3-Bromophenyl)-1-oxaspiro[2.5]oct-6-yl]oxy}(tert-butyl)dimethyl silane (ESI pos.) m/z : 419(M+Na)+
1H NMR (600 MHz, CHLOROFORM-d) d ppm 0.01 - 0.07 (m, 6 H) 0.83 - 0.92 (m, 9 H) 1.11 - 2.06 (m, 8 H) 3.75 - 3.90 (m, 2 H) 7.18 - 7.28 (m, 2 H) 7.38 - 7.43 (m, 1 H) 7.43 - 7.48 (m, 1 H)

### Production Example 15 2-(3-Chlorophenyl)-l-oxaspiro[2.5]octan-6-ol

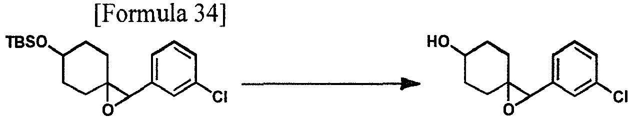

A solution of 1.0 M tetra-n-butylammonium fluoride in tetrahydrofuran (120 mL) was added under ice-cooling to a solution of {[2-(3-chlorophenyl)-1-oxaspiro[2.5]octa-6-yl]oxy}(tert-butyl)dimethylsilane (14 g) in tetrahydrofuran (79 mL), and the mixture was stirred at room temperature overnight. The reaction mixture was diluted with water, subsequently extracted 3 times with ethyl acetate, and the obtained organic layer was dried over anhydrous magnesium sulfate. After filtering off the desiccating agent, the organic layer was concentrated under reduced pressure, and the residue was purified by column chromatography (silica gel cartridge, hexane/ethyl acetate = 88 : 12 to 0 : 100) to obtain the title compound (11 g).
(ESI pos.) m/z : 261 (M+Na)+
1H NMR (600 MHz, CHLOROFORM-d) d ppm 1.28 - 2.14 (m, 8 H) 3.87 (s, 1 H) 3.88 - 3.94 (m, 1 H) 7.16 - 7.34 (m, 4 H)
The following compound was synthesized by the similar method.
2-(3-Bromophenyl)-1-oxaspiro[2.5]octan-6-ol
(ESI pos.) m/z : 305(M+Na)+
1H NMR (600 MHz, CHLOROFORM-d) d ppm 1.23 - 2.13 (m, 8 H) 3.80 - 3.95 (m, 2 H) 7.19 - 7.27 (m, 2 H) 7.46 (s, 2 H)

### Production Example 16 2-(3-Chlorophenyl)-6-fluoro-1-oxaspiro[2.5]octane

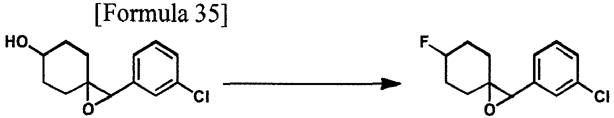

Triethylamine (17 mL), triethylamine trihydrogen fluoride (6.6 mL) and 1,1,2,2,3,3,4,4,4-nonafluorobutane-1-sulfonyl fluoride (7.1 mL) were sequentially added dropwise under ice-cooling to a solution of 2-(3-chlorophenyl)-1-oxaspiro[2.5]octan-6-ol (3.2 g) in tetrahydrofuran (41 mL), and the mixture was stirred at room temperature for 5 hours. The reaction mixture was diluted with water, extracted 3 times with chloroform, subsequently the organic layer was separated by a phase separation cartridge and concentrated under reduced pressure. The residue was purified by column chromatography (silica gel cartridge, hexane/ethyl acetate = 98 : 2 to 0 : 100) to obtain a mixture of the title compound and 2-(3-chlorophenyl)-1-oxaspiro[2.5]oct-5-ene (3.2 g). 2-(3-Chlorophenyl)-1-oxaspiro[2.5]oct-5-ene
(EI) m/z : 220(M+)

### Production Example 17 2-(3-Bromophenyl)-6-fluoro-1-oxaspiro[2.5]octane

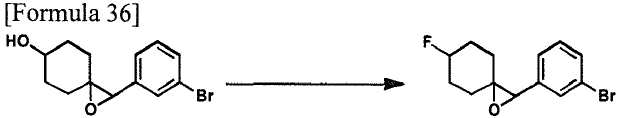

1,1,2,2,3,3,4,4,4-Nonafluorobutane-1-sulfonyl fluoride (480 µL) was added dropwise at -15°C to a solution of 2-(3-bromophenyl)-l-oxaspiro[2.5]octan-6-ol (490 mg) and 1,8-diazabicyclo[5.4.0]undec-7-ene (780 µL) in toluene (17 mL), and the mixture was stirred at -17°C for 30 minutes. The reaction mixture was diluted with water, extracted 3 times with chloroform, subsequently the organic layer was separated by a phase separation cartridge and concentrated under reduced pressure. The residue was purified by column chromatography (silica gel cartridge, hexane/ethyl acetate = 98 : 2 to 82 : 18) and subsequently purified by PTLC (silica gel, hexane/ethyl acetate = 10 : 1) to obtain the title compound (230 mg) and 2-(3-bromophenyl)-1-oxaspiro[2.5]oct-5-ene (330 mg).
(ESI pos.) m/z : 307(M+Na)+
1H NMR (600 MHz, CHLOROFORM-d) d ppm 1.15 - 2.23 (m, 8 H) 3.87 (s, 1 H) 4.65 - 4.79 (m, 1 H) 7.17 - 7.29 (m, 2 H) 7.37 - 7.49 (m, 2 H)
2-(3-Bromophenyl)-1-oxaspiro[2.5]oct-5-en
(ESI pos.) m/z : 287(M+Na)+
1H NMR (600 MHz, CHLOROFORM-d) d ppm 1.36 - 2.50 (m, 8 H) 3.84 - 3.95 (m, 1 H) 5.45 - 5.84 (m, 2 H) 7.17 - 7.52 (m, 4 H)

### Production Example 18 4-{3-[(4,4-Difluorocyclohexylidene)methyl]phenyl}-1-ethyl-1 H-pyrazole

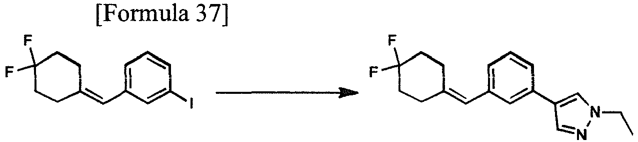

1-Ethyl-4-(4,4,5,5-tetramethyl-1,3,2-dioxaborolan-2-yl)-1H-pyrazole (10 g), potassium carbonate (6.2 g) and tetrakis(triphenylphosphine)palladium(0) (1.7 g) were added to a solution of 1-[(4,4-difluorocyclohexylidene)methyl]-3-iodobenzene (5.0 g) in N,N-dimethylformamide (50 mL) and ethanol (25 mL), and the mixture was stirred at 90°C overnight. The reaction mixture was left to cool to room temperature, water was added thereto, and subsequently extracted 3 times with chloroform. After washing the extract with saline, the organic layer was separated by a phase separation cartridge and concentrated under reduced pressure. The residue was purified by column chromatography (silica gel cartridge, hexane/ethyl acetate = 93 : 7 to 82 : 18) to obtain the title compound (4.1 g).
(ESI pos.) m/z : 303(M+H)+
1H NMR (600 MHz, CHLOROFORM-d) d ppm 1.50 - 1.59 (m, 3 H) 1.88 - 1.99 (m, 2 H) 2.00 - 2.10 (m, 2 H) 2.44 - 2.51 (m, 2 H) 2.57 (t, J=6.6 Hz, 2 H) 4.15 - 4.26 (m, 2 H) 6.40 (s, 1 H) 7.04 (d, J=7.0 Hz, 1 H) 7.23 - 7.41 (m, 3 H) 7.63 (s, 1 H) 7.76 (s, 1 H)

The following compound was synthesized by the similar method.
4-{3-[(4,4-Difluorocyclohexylidene)methyl]phenyl }-1 H-pyrazole
(ESI pos.) m/z : 275(M+H)+

With the proviso that tert-butyl 4-(4,4,5,5-tetramethyl-1,3,2-dioxaborolan-2-yl)-1H-pyrazole-1-carboxylate was used as the starting material.

### Production Example 19 4-{3-[(4,4-Difluorocyclohexylidene)methyl]phenyl}-1-(propane-2-yl)-1 H-pyrazole

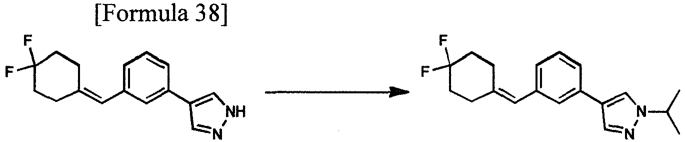

2-Iodopropane (140 µL) and potassium carbonate (190 mg) were added to a solution of 4-{3-[(4,4-difluorocyclohexylidene)methyl]phenyl}-1H-pyrazole (250 mg) in N,N-dimethylformamide (7.0 mL), and the mixture was stirred at room temperature for 3 days. 2-Iodopropane (270 µL) was further added to the reaction mixture and stirred overnight. The reaction mixture was diluted with a mixture of ethyl acetate and hexane (= 1 : 1) and washed twice with water. The obtained organic layer was dried over anhydrous magnesium sulfate. After filtering off the desiccating agent, the organic layer was concentrated under reduced pressure, and the residue was purified by column chromatography (silica gel cartridge, hexane/ethyl acetate = 93 : 7 to 65 : 35) to obtain the title compound (280 mg).
(ESI pos.) m/z : 317(M+H)+

The following compound was synthesized by the similar method.
4-{3-[(4,4-Difluorocyclohexylidene)methyl]phenyl}-1-(2,2,2-trifluoroethyl)-1H-pyrazole
(ESI pos.) m/z : 357(M+H)+

### Production Example 20 2-{3-[(4,4-Difluorocyclohexylidene) methyl]phenyl}pyridine

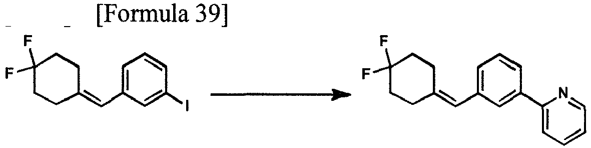

Two pairs of a solution of 1-[(4,4-difluorocyclohexylidene)methyl]-3-iodobenzene (1.0 g), tetrakis(triphenylphosphine)palladium(0) (350 mg) and 2-(tri-n-butylstannyl)pyridine (1.4 mL) in toluene (10 mL) were prepared, respectively irradiated with microwave in a sealed tube, and stirred at 130°C for 2 hours. The reaction mixtures were combined, diluted with ethyl acetate, subsequently adsorbed on NH silica gel, and purified by column chromatography packed with potassium fluoride (silica gel cartridge, hexane/ethyl acetate = 98 : 2 to 48 : 52) to obtain the title compound (1.3 g).
(ESI pos.) m/z : 286(M+H)+
LCMS RT 1.07, Condition A

### Production Example 21 1-Chloro-3-(cyclopentylidenemethyl)benzene

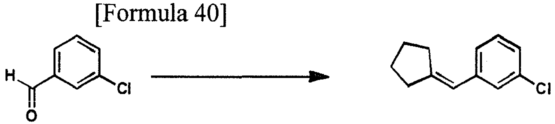

A solution of 1.9 M sodium hexamethyldisilazide in hexane (22 mL) was added to a solution of cyclopentyl (triphenyl) phosphonium bromide (18 g) in tetrahydrofuran (20 mL), and the mixture was stirred at 0°C for 1 hour. A solution of 3-chlorobenzaldehyde (3.0 g) in tetrahydrofuran (20 mL) was added dropwise to the reaction mixture and the mixture was stirred at room temperature for 3 hours. The reaction mixture was diluted with water and extracted 3 times with ethyl acetate, and washed sequentially with an aqueous saturated ammonium chloride solution, water and saline. The obtained organic layer was dried over anhydrous magnesium sulfate. After filtering off the desiccating agent, the organic layer was concentrated under reduced pressure, and the residue was purified by column chromatography (silica gel cartridge, hexane/ethyl acetate = 98 : 2 to 82 : 18) to obtain the title compound (1.6 g).
1H NMR (600 MHz, CHLOROFORM-d) d ppm 1.64 - 1.82 (m, 4 H) 2.46 - 2.56 (m, 4 H) 6.28 - 6.31 (m, 1 H) 7.11 - 7.17 (m, 2 H) 7.21 - 7.25 (m, 1 H) 7.28 (s, 1 H)

The following compounds were synthesized by the similar method.
1-(Cyclobutylidenemethyl)-3-fluorobenzene
(EI) m/z : 162(M+)
1H NMR (600 MHz, CHLOROFORM-d) d ppm 2.07 - 2.18 (m, 2 H) 2.81 - 2.94 (m, 2 H) 2.98 - 3.09 (m, 2 H) 6.02 - 6.08 (m, 1 H) 6.80 - 7.00 (m, 3 H) 7.20 - 7.26 (m, 1 H)
1-(Cyclopentylidenemethyl)-3-fluorobenzene
(EI) m/z : 176(M+)
1-(Cyclopentylidenemethyl)-2-fluorobenzene
(EI) m/z : 176(M+)
1-Chloro-2-(cyclopentylidenemethyl)benzene
(EI) m/z : 192(M+)
1-Chloro-4-(cyclopentylidenemethyl)benzene
(EI) m/z : 192(M+)
1-(Cyclopentylidenemethyl)-2-methylbenzene
(EI) m/z : 172(M+)
1-(Cyclopentylidenemethyl)-3-methylbenzene
(ESI pos.) m/z : 173(M+H)+
1H NMR (600 MHz, CHLOROFORM-d) d ppm 1.61 - 1.71 (m, 2 H) 1.73 - 1.81 (m, 2 H) 2.33 (s, 3 H) 2.43 - 2.60 (m, 4 H) 6.26 - 6.39 (m, 1 H) 6.94 - 7.24 (m, 4 H)
1-(Cyclopentylidenemethyl)-4-methylbenzene
(EI) m/z : 172(M+)
1-(Cyclopentylidenemethyl)-2-methoxybenzene
(ESI pos.) m/z : 189(M+H)+
1-(Cyclopentylidenemethyl)-3 -methoxybenzene
(ESI pos.) m/z : 189(M+H)+
1H NMR (600 MHz, CHLOROFORM-d) d ppm 1.61 - 1.70 (m, 2 H) 1.72 - 1.83 (m, 2 H) 2.41 - 2.63 (m, 4 H) 3.81 (s, 3H) 6.31 - 6.36 (m, 1 H) 6.72 (dd, J=8.1, 2.3 Hz, 1 H) 6.83 - 6.93 (m, 2 H) 7.23 (t, J=8.1 Hz, 1 H)
1-[3-(Cyclopentylidenemethyl)phenyl]-1H-1,2,3-triazole
(ESI pos.) m/z : 226(M+H)+
2-[3-(Cyclopentylidenemethyl)phenyl]-2H-1,2,3-triazole
(ESI pos.) m/z : 226(M+H)+
1H NMR (600 MHz, CHLOROFORM-d) d ppm 1.63 - 1.75 (m, 2 H) 1.77 - 1.86 (m, 2 H) 2.45 - 2.57 (m, 2 H) 2.58 - 2.67 (m, 2 H) 6.43 (m, J=2.1, 2.1 Hz, 1 H) 7.23 - 7.33 (m, 1 H) 7.41 (t, J=8.1 Hz, 1 H) 7.77 - 7.91 (m, 3 H) 8.02 - 8.10 (m, 1 H)
1-[3-(Cyclopentylidenemethyl)phenyl]-1H-pyrazole
(ESI pos.) m/z : 225(M+H)+
1-(Cyclopentylidenemethyl)-3-(trifluoromethyl)benzene
(EI) m/z : 226(M+)
3-(Cyclopentylidenemethyl)phenyl trifluoromethyl ether
(EI) m/z : 242(M+)
1-(Cyclopentylidenemethyl)-3-ethoxybenzene
(ESI pos.) m/z : 203(M+H)+
1-Chloro-3-(cyclopentylidenemethyl)-5-fluorobenzene
(EI) m/z : 210(M+)
2-Chloro-4-(cyclopentylidenemethyl)-1 -fluorobenzene
(EI) m/z : 210(M+)
2-Chloro-1-(cyclopentylidenemethyl)-4-fluorobenzene
(EI) m/z : 210(M+)
Methyl 3-(cyclopentylidenemethyl)benzoate
(ESI pos.) m/z : 217(M+H)+
1,2-Dichloro-3-(cyclopentylidenemethyl)benzene
(EI) m/z : 226(M+)
1,4-Dichloro-2-(cyclopentylidenemethyl)benzene
(EI) m/z : 226(M+)
1,3-Dichloro-5-(cyclopentylidenemethyl)benzene
(EI) m/z : 226(M+)
1-Bromo-3-(cyclohexylidenemethyl)benzene
(EI) m/z : 250(M+)
1H NMR (600 MHz, CHLOROFORM-d) d ppm 1.48 - 1.68 (m, 6 H) 2.17 - 2.28 (m, 2 H) 2.31 - 2.37 (m, 2 H) 6.15 (s, 1 H) 7.06 - 7.13 (m, 1 H) 7.16 (t, J=7.8 Hz, 1 H) 7.28 - 7.36 (m, 2 H)
1-(Cyclohexylidenemethyl)-3-fluorobenzene
(EI) m/z : 190(M+)

### Production Example 22 1-[Amino(3-bromophenyl)methyl]cyclopentanol

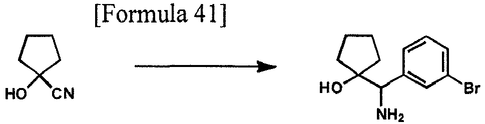

Under nitrogen atmosphere, a solution of 2.6 M n-butyllithium in hexane (70 mL) and a solution of 1-hydroxycyclopentanecarbonitrile (6.8 g) in tetrahydrofuran (100 mL) were sequentially slowly added dropwise at -78°C to a solution of 1,3-dibromobenzene (43 g) in tetrahydrofuran (180 mL). The mixture was stirred while the temperature was elevated to -10°C over a period of 3 hours. An aqueous saturated sodium hydrogen carbonate solution was added to the reaction mixture and phase separation was conducted; subsequently the aqueous layer was extracted twice with diethyl ether, and the obtained organic layers were combined and dried over anhydrous magnesium sulfate. After filtering off the desiccating agent, the organic layer was concentrated under reduced pressure, and the residue was dissolved in methanol (180 mL). Sodium borohydride (4.6 g) was added thereto under ice-cooling and the mixture was stirred at room temperature overnight. Water was added to the reaction mixture, the mixture was concentrated under reduced pressure, diluted with ethyl acetate, and subsequently extracted 3 times with 1.0 M hydrochloric acid. The obtained aqueous layer was washed with ethyl acetate, subsequently basified with an aqueous solution of 3.0 M sodium hydroxide, and then extracted 3 times with chloroform. The obtained organic layers were combined and dried over anhydrous magnesium sulfate. After filtering off the desiccating agent, the organic layer was concentrated under reduced pressure to obtain the title compound (4.6 g).

The following compounds were synthesized by the similar method.
1-[Amino(phenyl)methyl]cyclopentanol
(ESI pos.) m/z : 192(M+H)+
With the proviso that 1-[(trimethylsilyl)oxy]cyclopentanecarbonitrile was used as the starting material.
1-[Amino(2-bromophenyl)methyl]cyclopentanol
(ESI pos.) m/z : 270(M+H)+
1H NMR (600 MHz, CHLOROFORM-d) d ppm 1.17 - 1.96 (m, 8 H) 3.86 (s, 1 H) 4.49 (s, 1 H) 7.08 - 7.14 (m, 1 H) 7.28 - 7.33 (m, 1 H) 7.52 - 7.56 (m, 1 H) 7.72 (dd, J=7.8, 1.8 Hz, 1 H)
With the proviso that 1-[(trimethylsilyl)oxy]cyclopentanecarbonitrile and 1,3-dibromobenzene were used as the starting materials.
1-[Amino(4-fluorophenyl)methyl]cyclopentanol
(ESI pos.) m/z : 210(M+H)+
With the proviso that 1-[(trimethylsilyl)oxy]cyclopentanecarbonitrile was used as the starting material.
1-[Amino(3-chlorophenyl)methyl]-3-{[tert-butyl(dimethyl)silyl]oxy}cyclopentanol
(ESI pos.) m/z : 356(M+H)+
LCMS RT 0.79, Condition A
1H NMR (600 MHz, CHLOROFORM-d) d ppm -0.01 - 0.09 (m, 6 H) 0.83 - 0.86 (m, 9H) 1.46 - 2.06 (m, 6 H) 3.76 - 3.95 (m, 1 H) 4.25 - 4.40 (m, 1H) 7.20 - 7.31 (m, 3 H) 7.43 (s, 1 H)

### Production Example 23 1-[(S)-Amino(3-bromophenyl)methyl]cyclopentanol

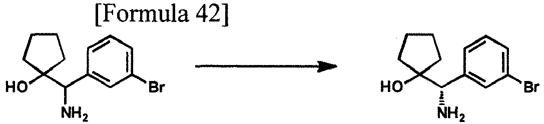

1-[Amino(3-bromophenyl)methyl]cyclopentanol (6.4 g) was dissolved in ethyl acetate (100 mL), (S)-methoxyphenylacetic acid (3.9 g) was added thereto, and stirred at room temperature for 6 days. The precipitated solid was collected by filtration and recrystallized twice with ethyl acetate and ethanol. An aqueous saturated sodium hydrogen carbonate solution was added to the obtained crystal, extracted 3 times with chloroform, and the organic layer was separated by a phase separation cartridge and concentrated under reduced pressure. An aqueous saturated sodium hydrogen carbonate solution was added again to the residue, extracted 3 times with chloroform, and the organic layer was separated by a phase separation cartridge and subsequently concentrated under reduced pressure to obtain the title compound (1.3 g, > 99%ee).
(ESI pos.) m/z : 270(M+H)+
1H NMR (600 MHz, CHLOROFORM-d) d ppm 1.17 - 1.94 (m, 8 H) 3.87 (s, 1 H) 7.16 - 7.21 (m, 1 H) 7.29 - 7.34 (m, 1 H) 7.37 - 7.42 (m, 1 H) 7.52 - 7.58 (m, 1 H)

### Chiral analysis conditions

Column: Daicel CHIRALPAK AD-H, 4.6*250
Solvent system: Hexane : EtOH : IPA : DEA = 60 : 30 : 10 : 0.2
Flow rate: 0.8 mL/min
RT: 6.21, 12.01, Faster

### Production Example 24 3-{[tert-Butyl(dimethyl)silyl]oxy}-1-[(trimethylsilyl)oxy]cyclopentanecarbonitrile

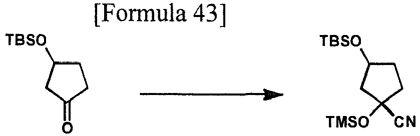

Lithium perchlorate (78 mg) and trimethylsilanecarbonitrile (2.2 mL) were added to 3-{[tert-butyl(dimethyl)silyl]oxy}cyclopentanone (3.2 g), and the mixture was stirred at room temperature overnight. The reaction mixture was diluted with diethyl ether and separated with an aqueous saturated sodium hydrogen carbonate solution, and the obtained organic layer was dried over anhydrous magnesium sulfate. After filtering off the desiccating agent, the organic layer was concentrated under reduced pressure to obtain the title compound (4.1 g).
(ESI pos.) m/z : 336(M+Na)+
1H NMR (600 MHz, CHLOROFORM-d) d ppm 0.03 - 0.29 (m, 15 H) 0.81 - 0.95 (m, 9 H) 1.75 - 2.59 (m, 6 H) 4.28 - 4.37 (m, 1 H)

### Production Example 25 3-[({[3-Chloro-4-(trifluoromethyl) pyridin-2-yl]carbonyl}amino)(1-hydroxycyclopentyl)methyl] benzoic acid

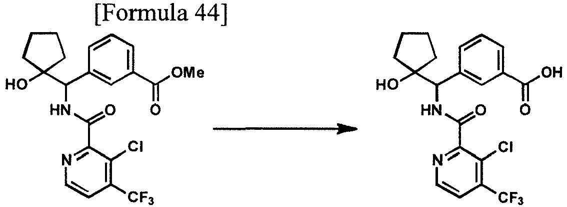

An aqueous solution of 2.0 M sodium hydroxide (10 mL) was added to a solution of methyl 3-[({[3-chloro-4-(trifluoromethyl)pyridin-2-yl]carbonyl}amino)(1-hydroxycyclopentyl)methyl]benzoate (230 mg) in 1,4-dioxane (10 mL), and stirred at room temperature for 18 hours. 2.0 M Hydrochloric acid was added to acidify the reaction mixture, subsequently extracted 3 times with chloroform, and the organic layer was separated by a phase separation cartridge and concentrated under reduced pressure to obtain the title compound (0.31 g).
(ESI pos.) m/z : 443(M+H)+
LCMS RT 0.87, Condition A
1H NMR (600 MHz, DMSO-d6) d ppm 1.12 - 1.84 (m, 8 H) 4.95 - 5.14 (m, 1 H) 7.34 - 7.51 (m, 1 H) 7.62 - 7.73 (m, 1 H) 7.78 - 7.87 (m, 1 H) 7.91 - 8.01 (m, 1 H) 8.07 (s, 1 H) 8.73 - 8.90 (m, 1 H) 9.13 - 9.34 (m, 1 H)

### Production Example 26 Methyl 3-[(1-hydroxycyclopentyl)(prop-2-en-1-ylamino)methyl]benzoate

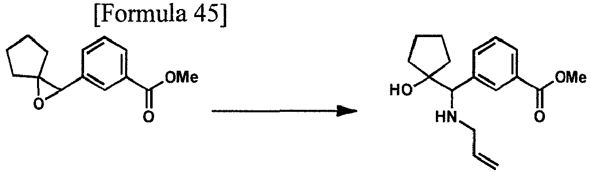

Methyl 3-(1-oxaspiro[2.4]hept-2-yl)benzoate (6.6 g), lithium perchlorate (5.0 g) and allylamine (18 mL) were stirred with heating in a sealed tube at 120°C for 3 hours. Lithium perchlorate (2.5 g) was added thereto and further stirred with heating for 2 hours. The reaction mixture was diluted with ethyl acetate, washed twice with an aqueous saturated ammonium chloride solution, and the aqueous layer was extracted 3 times with ethyl acetate. The obtained organic layers were combined and dried over anhydrous magnesium sulfate. After filtering off the desiccating agent, the organic layer was concentrated under reduced pressure, and the residue was purified by column chromatography (NH silica gel cartridge, hexane/ethyl acetate = 92 : 8 to 34 : 66) to obtain the title compound (640 mg).
(ESI pos.) m/z : 290(M+H)+

### Production Example 27 cis-1-[Amino(3-chlorophenyl)methyl]-4-fluorocyclohexanol

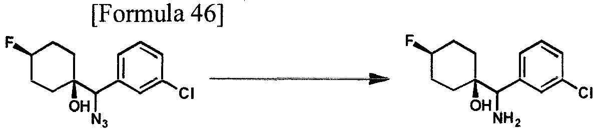

A mixture (1.0 g) of cis-1-[azide(3-chlorophenyl)methyl]-4-fluorocyclohexanol and 1-[azide(3-chlorophenyl)methyl]cyclohex-3-en-1-ol was dissolved in methanol (20 mL), platinum 10% on carbon (100 mg) was added thereto, and stirred under hydrogen atmosphere at normal pressure and 85°C for 4 hours, and subsequently stirred at room temperature overnight. The reaction mixture was filtered, subsequently the filtrate was concentrated under reduced pressure, and the residue was purified by column chromatography (silica gel cartridge, chloroform/methanol = 100 : 0 to 90 : 10) to obtain a mixture of the title compound and 1-[amino(3-chlorophenyl)methyl]cyclohexanol (360 mg).

The following compounds were synthesized by the similar method.
cis-1-[(S)-Amino(3-chlorophenyl)methyl]-4-fluorocyclohexanol
trans-1-[(S)-Amino(3-chlorophenyl)methyl]-4-fluorocyclohexanol
1H NMR (600 MHz, CHLOROFORM-d) d ppm 0.96 - 1.11 (m, 1 H) 1.43 - 2.08 (m, 7 H) 3.75 (s, 1 H) 4.76 - 4.89 (m, 1 H) 7.16 - 7.28 (m, 3 H) 7.31 - 7.35 (m, 1 H)
(Enantiomer 1) cis-1-[Amino(2-chlorophenyl)methyl]-4-fluorocyclohexanol
1H NMR (600 MHz, CHLOROFORM-d) d ppm 1.23 - 1.46 (m, 3 H) 1.73 - 2.07 (m, 5 H) 4.36 - 4.56 (m, 2 H) 7.16 - 7.39 (m, 3 H) 7.59 (dd, J=7.8,1.2 Hz, 1 H)
(Enantiomer 1) trans-1-[Amino(2-chlorophenyl)methyl]-4-fluorocyclohexanol
1H NMR (600 MHz, CHLOROFORM-d) d ppm 0.74 - 2.08 (m, 8 H) 4.48 (br. s., 1 H) 4.74 - 4.92 (m, 1 H) 7.14 - 7.63 (m, 4 H)
(Diastereomer 1, enanthiomer 1) 1-[Amino(3-chloro-2-fluorophenyl)methyl]-4-fluorocyclohexanol
1H NMR (600 MHz, CHLOROFORM-d) d ppm 1.23 - 1.45 (m, 3 H) 1.56 - 2.00 (m, 5 H) 4.17 (s, 1 H) 4.35 - 4.54 (m, 1 H) 7.05 - 7.14 (m, 1 H) 7.29 - 7.42 (m, 2 H)
(Diastereomer 2, enanthiomer 1) 1-[Amino(3-chloro-2-fluorophenyl)methyl]-4-fluorocyclohexanol
1H NMR (600 MHz, CHLOROFORM-d) d ppm 1.06 - 1.17 (m, 1 H) 1.54 - 2.08 (m, 7 H) 4.18 (s, 1 H) 4. 74 - 4.89 (m, 1 H) 7.04 - 7.12 (m, 1 H) 7.28 - 7.40 (m, 2 H)
(Diastereomer 1, enanthiomer 1) 1-[Amino(3-fluorophenyl)methyl]-4-fluorocyclohexanol
1H NMR (600 MHz, CHLOROFORM-d) d ppm 1.07 - 2.04 (m, 8 H) 3.73 (s, 1 H) 4.25 - 4.58 (m, 1 H) 6.87 - 7.41 (m, 4 H)
(Diastereomer mixture, enanthiomer 1) 1-[Amino(2-fluorophenyl)methyl]-4-fluorocyclohexanol

### Production Example 28 (6-Chloropyridin-2-yl)(1-hydroxycyclopentyl)methanone

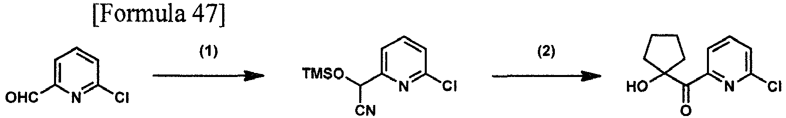

(1) Zinc iodide (II) (56 mg) and trimethylsilanecarbonitrile (540 µl) were added to a solution of 6-chloropicoline aldehyde (500 mg) in chloroform (10 ml), and stirred at room temperature overnight. The reaction mixture was diluted with chloroform, then washed with water, and the organic layer was separated by a phase separation cartridge and concentrated under reduced pressure. The crude product containing 2-(6-chloropyridin-2-yl)-2-((trimethylsilyl)oxy)acetonitrile (840 mg) was obtained.
(2) A solution of 1.3 M lithium bis(trimethylsilyl)amide in tetrahydrofuran (2.4 ml) was added dropwise at -78°C to a solution of 2-(6-chloropyridin-2-yl)-2-((trimethylsilyl)oxy)acetonitrile (700 mg) in tetrahydrofuran (5.8 ml), and the mixture was stirred for 1 hour. Cyclopentanone (260 µl) was added to the reaction mixture and stirred for 3 hours. 1.0 M hydrochloric acid (6.0 ml) was added to the reaction mixture and stirred at room temperature overnight. An aqueous saturated sodium hydrogen carbonate solution was added to basify the reaction mixture and subsequently the reaction mixture was added to water. After extracting with ethyl acetate, washing with saline, and drying over anhydrous sodium sulfate, concentration was carried out under reduced pressure. The residue was purified by column chromatography (silica gel cartridge, hexane/ethyl acetate = 90 : 10 to 50 : 50) to obtain the title compound (63 mg).
(ESI pos.) m/z : 226(M+H)+

### Production Example 29 1-[Amino(6-chloropyridin-2-yl)methyl]cyclopentanol

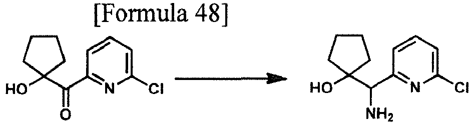

Ammonium acetate (210 mg) was added to a solution of (6-chloropyridin-2-yl)(1-hydroxycyclopentyl)methanone (60 mg) in methanol (2.0 ml), and stirred at 40°C for 2 hours. Sodium triacetoxyborohydride (170 mg) was added to the reaction mixture at 0°C, and the mixture was stirred at room temperature for 1.5 hours. An aqueous saturated sodium hydrogen carbonate solution was added to the reaction mixture and subsequently the reaction mixture was added to water. After extracting with methanol-chloroform, washing with saline and drying over anhydrous sodium sulfate were carried out, followed by concentration under reduced pressure. The residue was purified by column chromatography (silica gel cartridge, chloroform/methanol = 100 : 0 to 90 : 10) to obtain the title compound (14 mg).
(ESI pos.) m/z : 227(M+H)+

### Example 1 3-Chloro-N-[(S)-(3-chlorophenyl)(cis-4-fluoro-1-hydroxycyclohexyl)methyl]-4-(trifluoromethyl)pyridine-2-carboxamide

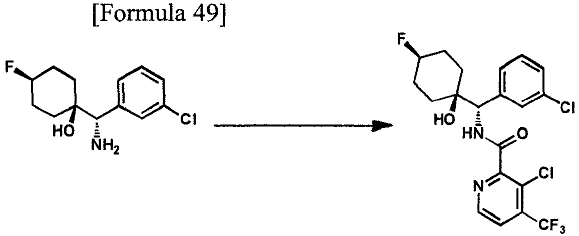

A solution of cis-1-[(S)-amino(3-chlorophenyl)methyl]-4-fluorocyclohexanol (0.9 mg), 3-chloro-4-(trifluoromethyl)pyridine-2-carboxylic acid (1.6 mg), 1-ethyl-3-(3-dimethylaminopropyl)carbodiimide hydrochloride (1.3 mg), 1-hydroxybenzotriazole (1.1 mg) and triethylamine (1.2 µL) in chloroform (0.2 mL) was stirred at room temperature for 1.5 hours. Water was added to the reaction mixture and extracted 3 times with chloroform. The organic layer was concentrated under reduced pressure, and the residue was purified by column chromatography (silica gel cartridge, hexane/ethyl acetate = 90 : 10 to 50 : 50) to obtain the title compound (2.0 mg).

### Example 2 3-Chloro-N-[(3-chlorophenyl)(cis-4-fluoro-1-hydroxycyclohexyl)methyl]-4-(trifluoromethyl)pyridine-2-carboxamide

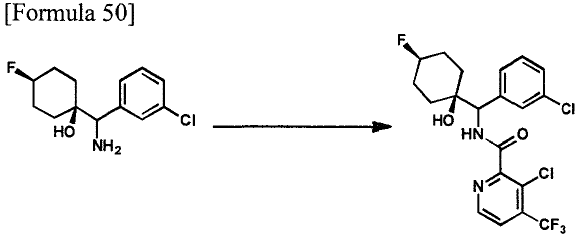

N,N-Diisopropylethylamine (610 µL) was added under ice-cooling to a solution of 3-chloro-4-(trifluoromethyl)pyridine-2-carboxylic acid (340 mg), O-(7-azabenzotriazol-1-yl)-N,N,N',N'-tetramethyluronium hexafluorophosphoric acid (620 mg) in N,N-dimethylformamide (6.6 mL) and stirred for 30 minutes. A solution of cis-1-[amino(3-chlorophenyl)methyl]-4-fluorocyclohexanol (330 mg) in N,N-dimethylformamide (5.0 mL) was added dropwise to the reaction mixture and stirred for 3 hours. The mixture was diluted with water, extracted 3 times with chloroform, and the organic layer was separated by a phase separation cartridge and concentrated under reduced pressure. The residue was purified by preparative HPLC to obtain the title compound (180 mg).

### Example 3 N-[(3-Bromophenyl)(4-fluoro-1-hydroxycyclohexyl)methyl]-3-chloro-4-(trifluoromethyl)pyridine-2-carboxamide

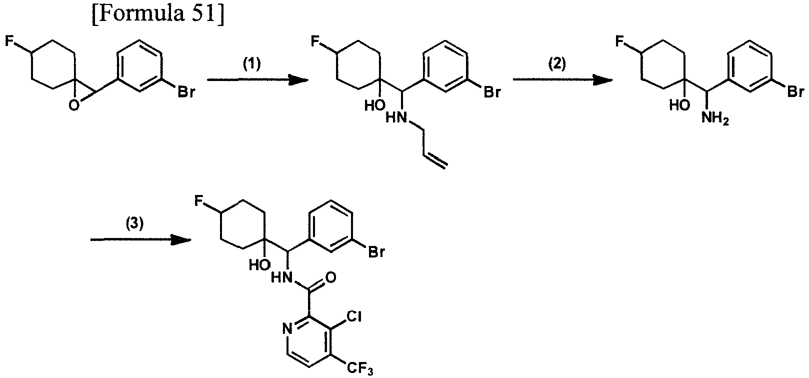

(1) 2-(3-Bromophenyl)-6-fluoro-1-oxaspiro[2.5]octane (250 mg), lithium perchlorate (380 mg) and allylamine (0.99 mL) were stirred with heating in a sealed tube at 120°C for 3 hours. The reaction mixture was diluted with ethyl acetate, washed twice with an aqueous saturated ammonium chloride, and the aqueous layer was extracted 3 times with ethyl acetate. The obtained organic layers were combined and dried over anhydrous magnesium sulfate. After filtering off the desiccating agent, the organic layer was concentrated under reduced pressure to obtain a crude product (290 mg) containing 1-[(3-bromophenyl) (prop-2-en-1-ylamino)methyl]-4-fluorocyclohexanol.
(2) Tetrakis(triphenylphosphine)palladium(0) (29 mg) was added under nitrogen atmosphere to a solution of 1-[(3-bromophenyl)(prop-2-en-1-ylamino)methyl]-4-fluorocyclohexanol (280 mg) and 1,3-dimethylpyrimidine-2,4,6(1H,3H,5H)-trione (380 mg) in chloroform (8.0 mL), and stirred at room temperature for 1.5 hours. An aqueous saturated sodium hydrogen carbonate solution was added to the reaction mixture, extracted 3 times with chloroform, and the organic layer was separated by a phase separation cartridge and concentrated under reduced pressure. A crude product (250 mg) containing 1-[amino(3-bromophenyl)methyl]-4-fluorocyclohexanol was obtained from the residue.
(3) The title compound (130 mg) was obtained from 1-[amino(3-bromophenyl)methyl]-4-fluorocyclohexanol (240 mg) by the same method as in Example 2.

### Example 4

(Enantiomer 1) 3-Chloro-N-[(2-chlorophenyl)(4,4-difluoro-1-hydroxycyclohexyl)methyl]-4-(trifluoromethyl)pyridine-2-carboxamide

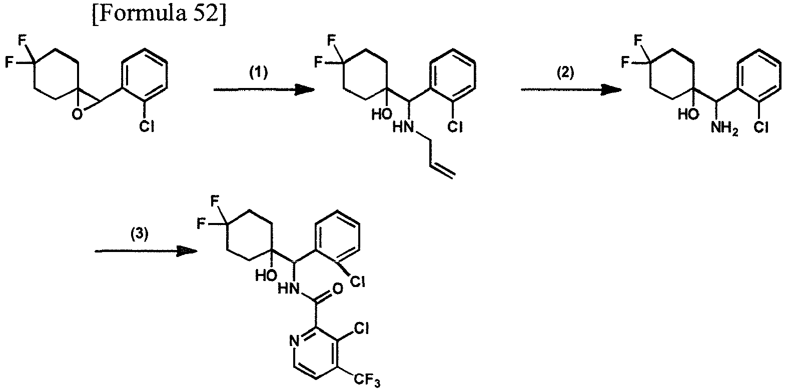

(1) A crude product (130 mg) containing (enantiomer 1) 1-[(2-chlorophenyl)(prop-2-en-1-ylamino)methyl]-4,4-difluorocyclohexanol was obtained from (enantiomer 1) 2-(2-chlorophenyl)-6,6-difluoro-1-oxaspiro[2.5]octane (110 mg) by the same method as in Example 3 (1).
(2) (Enantiomer 1) 1-[amino(2-chlorophenyl)methyl]-4,4-difluorocyclohexanol (48 mg) was obtained from (enantiomer 1) 1-[(2-chlorophenyl) (prop-2-en-1-ylamino)methyl]-4,4-difluorocyclohexanol (130 mg) by the same method as in Example 3 (2).
(3) The title compound (49 mg) was obtained from (enantiomer 1) 1-[amino(2-chlorophenyl)methyl]-4,4-difluorocyclohexanol (47 mg) by the similar method as in Example 1.

### Example 5 N-{(4,4-Difluoro-1-hydroxycyclohexyl) [3-(1-ethyl-1H-pyrazol-4-yl)phenyl]methyl}-4-fluoro-2-(trifluoromethyl)benzamide

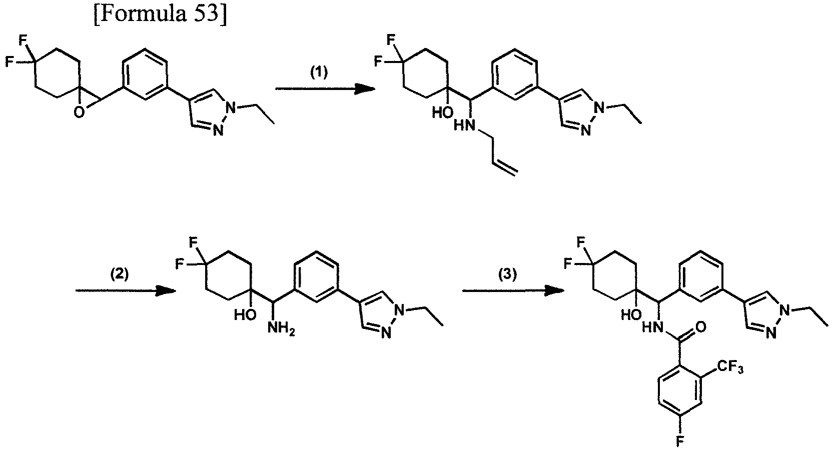

(1) 4-[3-(6,6-Difluoro-1-oxaspiro[2.5]oct-2-yl)phenyl]-1-ethyl-1H-pyrazole (500 mg), lithium perchlorate (670 mg) and allylamine (1.2 mL) were stirred in a sealed tube at 120°C for 1 hour under microwave irradiation. Similarly, 4-[3-(6,6-difluoro-1-oxaspiro[2.5]oct-2-yl)phenyl]-1-ethyl-1H-pyrazole (1.0 g), lithium perchlorate (1.3 g) and allylamine (4.0 mL) were stirred in a sealed tube at 120°C for 1 hour under microwave irradiation. The reaction mixtures were combined, diluted with ethyl acetate, subsequently washed sequentially with an aqueous saturated ammonium chloride solution twice, water and saline. The obtained organic layer was dried over anhydrous magnesium sulfate. After filtering off the desiccating agent, the organic layer was concentrated under reduced pressure, and the residue was purified by column chromatography (silica gel cartridge, hexane/ethyl acetate = 88 : 12 to 0 : 100, chloroform/methanol = 98 : 2 to 87 : 13) to obtain 1-{[3-(1-ethyl-1H-pyrazol-4-yl)phenyl](prop-2-en-1-ylamino)methyl}-4,4-difluorocyclohexanol (1.8 g).
(2) 1-{Amino[3-(1-ethyl-1H-pyrazol-4-yl)phenyl]methyl}-4,4-difluorocyclohexanol (1.1 g) was obtained from 1-{[3-(1-ethyl-1H-pyrazol-4-yl)phenyl](prop-2-en-1-ylamino)methyl}-4,4-difluorocyclohexanol (1.8 g) by the similar method as in Example 3 (2).
(3) 4-Fluoro-2-(trifluoromethyl)benzoyl chloride (27 µL) was added dropwise under ice-cooling to a solution of 1-{amino[3-(1-ethyl-1H-pyrazol-4-yl)phenyl]methyl}-4,4-difluorocyclohexanol (40 mg) and triethylamine (25 µL) in tetrahydrofuran (1.5 mL) and stirred at room temperature overnight. The reaction mixture was concentrated under reduced pressure, the residue was purified by preparative HPLC, and subsequently stirred overnight with diisopropyl ether. The obtained solid was collected by filtration to obtain the title compound (19 mg).

### Example 6 3-Chloro-N-{(4,4-difluoro-1-hydroxycyclohexyl)[3-(1-ethyl-1H-pyrazol-4-yl)phenyl]methyl}-4-(trifluoromethyl)pyridine-2-carboxamide

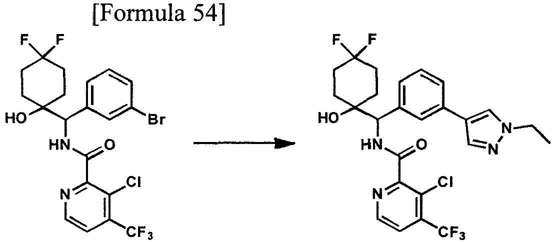

N-[(3-Bromophenyl)(4,4-difluoro-1-hydroxycyclohexyl)methyl]-3-chloro-4-(trifluoromethyl)pyridine-2-carboxamide (150 mg) was dissolved in N,N-dimethylformamide (1.2 mL) and ethanol (0.6 mL), 1-ethyl-4-(4,4,5,5-tetramethyl-1,3,2-dioxaborolan-2-yl)-1H-pyrazole (95 mg), potassium carbonate (59 mg) and tetrakis(triphenylphosphine)palladium(0) (17 mg) were added thereto, and stirred under nitrogen atmosphere in a sealed tube at 90°C for 24 hours. The reaction mixture was brought back to room temperature, water was added thereto, extracted 3 times with chloroform, and subsequently the organic layer was separated by a phase separation cartridge and concentrated under reduced pressure. The residue was purified by preparative HPLC to obtain the title compound (15 mg).

### Example 7 3-Chloro-N-{(4,4-difluoro-1-hydroxycyclohexyl)[3-(pyridin-2-yl)phenyl]methyl}-4-(trifluoromethyl)pyridine-2-carboxamide

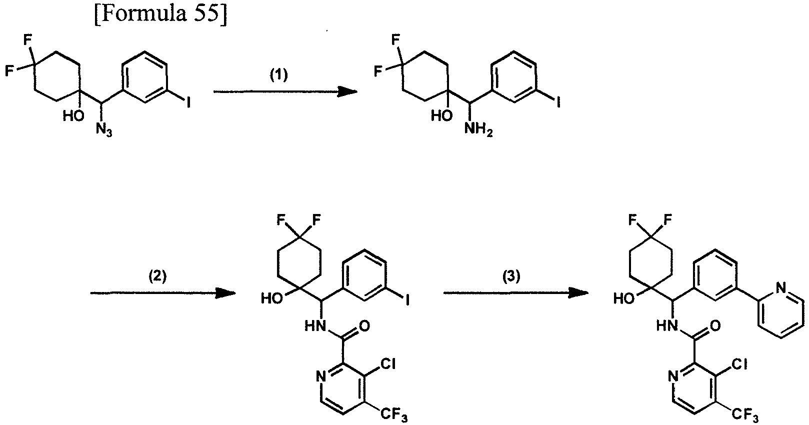

(1) Water (1.0 mL) and triphenyl phosphine (150 mg) were added to a solution of 1-[azide(3-iodophenyl)methyl]-4,4-difluorocyclohexanol (38 mg) in tetrahydrofuran (1.0 mL), and stirred at room temperature for 20 hours. An aqueous solution of 1.0 M sodium hydroxide was added to the reaction mixture, and stirred at room temperature for 5 hours and at 80°C for 22 hours. The reaction mixture was left to cool, extracted 3 times with chloroform, and subsequently the organic layer was separated by a phase separation cartridge and concentrated under reduced pressure. A crude product (300 mg) containing 1-[amino(3-iodophenyl)methyl]-4,4-difluorocyclohexanol was obtained from the residue.
(2) 3-Chloro-N-[(4,4-difluoro-1-hydroxycyclohexyl)(3-iodophenyl)methyl]-4-(trifluoromethyl)pyridine-2-carboxamide (31 mg) was obtained from 1-[amino(3-iodophenyl)methyl]-4,4-difluorocyclohexanol (300 mg) by the similar method as in Example 2.
(3) 2-(Tributylstannyl)pyridine (110 µL) and tetrakis(triphenylphosphine)palladium(0) (27 mg) were added to a solution of 3-chloro-N-[(4,4-difluoro-1-hydroxycyclohexyl) (3-iodophenyl)methyl]-4-(trifluoromethyl)pyridine-2-carboxamide (130 mg) in toluene (1.0 mL) and stirred in a sealed tube under microwave irradiation at 130°C for 2 hours. The reaction mixture was diluted with ethyl acetate, subsequently adsorbed on NH silica gel, and purified by column chromatography packed with potassium fluoride (silica gel cartridge, hexane/ethyl acetate = 88 : 12 to 0 : 100) to obtain the title compound (5.2 mg).

### Example 8 3-Chloro-N-[(S)-(3-cyanophenyl)(1-hydroxycyclopentyl)methyl]-4-(trifluoromethyl)pyridine-2-carboxamide

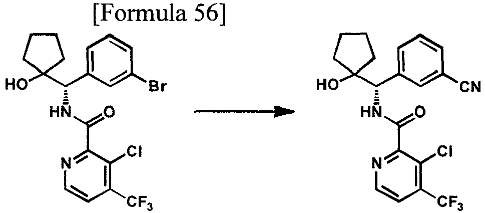

Dicyano zinc (50 mg) and tetrakis(triphenylphosphine)palladium(0) (25 mg) were added to a solution of N-[(S)-(3-bromophenyl)(1-hydroxycyclopentyl)methyl]-3-chloro-4-(trifluoromethyl)pyridine-2-carboxamide (100 mg) in N,N-dimethylformamide (2.0 mL) and stirred in a sealed tube under nitrogen atmosphere at 90°C for 2 hours. Dicyano zinc (51 mg) was added to the reaction mixture and further stirred at 110°C for 18 hours. Water was added to the reaction mixture and subsequently extracted with ethyl acetate. The obtained organic layer was sequentially washed with an aqueous saturated sodium hydrogen carbonate solution and saline, and subsequently dried over anhydrous magnesium sulfate. After filtering off the desiccating agent, the organic layer was concentrated under reduced pressure, and the residue was purified by column chromatography (silica gel cartridge, hexane/ethyl acetate = 88 : 12) and by PTLC (in twice development with silica gel, hexane/ethyl acetate = 1 : 1). The obtained residue was washed with diisopropyl ether, and collected by filtration to obtain the title compound (24 mg).

### Example 9 3-Chloro-N-{(S)-(1-hydroxycyclopentyl)[3-(pyridin-2-yl)phenyl]methyl}-4-(trifluoromethyl)pyridine-2-carboxamide

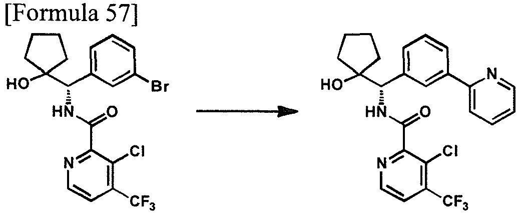

2-(Tributylstannyl)pyridine (100 µL) and tetrakis(triphenylphosphine)palladium(0) (25 mg) were added to a solution of N-[(S)-(3-bromophenyl)(1-hydroxycyclopentyl)methyl]-3-chloro-4-(trifluoromethyl)pyridine-2-carboxamide (100 mg) in toluene (1.0 mL) and stirred in a sealed tube under nitrogen atmosphere at 130°C for 22 hours. The reaction mixture was diluted with ethyl acetate, subsequently adsorbed on NH silica gel, and purified by column chromatography packed with potassium fluoride (silica gel cartridge, hexane/ethyl acetate = 88 : 12 to 0 : 100, chloroform/methanol = 80: 20) and by PTLC (in two-step development with silica gel, hexane/ethyl acetate = 1 : 2) to obtain the title compound (16 mg).

### Example 10 N-[(S)-(3-Bromophenyl)(1-hydroxycyclopentyl)methyl]-3-(1H-pyrazol-1-yl)-4-(trifluoromethyl)pyridine-2-carboxamide

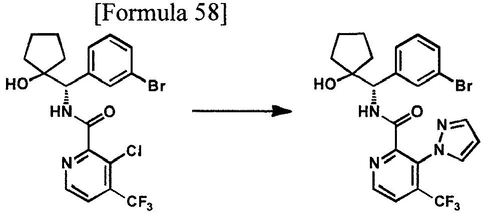

Pyrazole (7.1 mg), copper iodide (I) (2.1 mg), cesium carbonate (54 mg) and N,N'-dimethylcyclohexane-1,2-diamine (3.3 µL) were added to a solution of N-[(S)-(3-bromophenyl)(1-hydroxycyclopentyl)methyl]-3-chloro-4-(trifluoromethyl)pyridine-2-carboxamide (50 mg) in N,N-dimethylformamide (1.0 mL), and stirred at 100°C for 2 hours. The reaction mixture was brought back to room temperature, the insoluble matter was filtered off using a glass filter filtration paper, an aqueous saturated sodium hydrogen carbonate solution was added to the filtrate, and the solution was extracted 3 times with chloroform. The organic layer was separated by a phase separation cartridge and concentrated under reduced pressure, and the residue was purified by preparative HPLC to obtain the title compound (18 mg).

### Example 11 3-Chloro-N-[(3-chlorophenyl)(1,3-dihydroxycyclopentyl)methyl]-4-(trifluoromethyl)pyridine-2-carboxamide

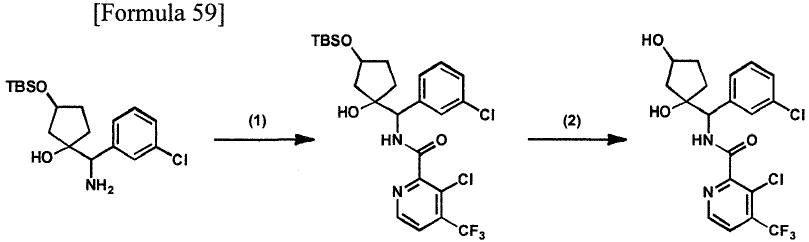

(1) N-[(3-{[tert-Butyl(dimethyl)silyl]oxy}-1-hydroxycyclopentyl)(3-chlorophenyl)methyl]-3-chloro-4-(trifluoromethyl)pyridine-2-carboxamide (440 mg) was obtained from 1-[amino(3-chlorophenyl)methyl]-3-{[tert-butyl(dimethyl)silyl]oxy}cyclopentanol (420 mg) by the similar method as in Example 2.
(2) A solution of 1.0 M tetra-n-butylammonium fluoride in tetrahydrofuran (0.86 mL) was added to a solution of N-[(3-{[tert-butyl(dimethyl)silyl]oxy}-1-hydroxycyclopentyl)(3-chlorophenyl)methyl]-3-chloro-4-(trifluoromethyl)pyridine-2-carboxamide (440 mg) in tetrahydrofuran (5.0 mL), and stirred at room temperature for 1 hour. Water was added to the reaction mixture, the mixture was extracted with ethyl acetate, washed with saline, and concentrated under reduced pressure. The residue was purified by preparative HPLC to obtain the title compound (300 mg).

### Example 12 3-Chloro-N-[(3-chlorophenyl)(1-hydroxy-3-oxocyclopentyl)methyl]-4-(trifluoromethyl)pyridine-2-carboxamide

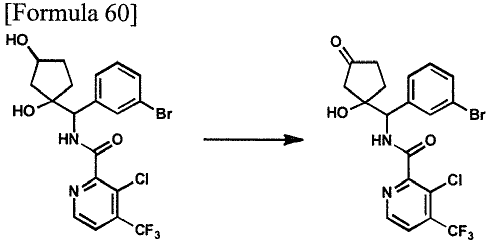

1,1,1-Triacetoxy-1,1-dihydro-1,2-benziodoxol-3(1H)-one (340 mg) was added to a solution of 3-chloro-N-[(3-chlorophenyl)(1,3-dihydroxycyclopentyl)methyl]-4-(trifluoromethyl)pyridine-2-carboxamide (300 mg) in chloroform (5.0 mL), stirred at room temperature overnight, and 1,1,1-triacetoxy-1,1-dihydro-1,2-benziodoxol-3(1H)-one (170 mg) was further added and stirred at room temperature overnight. The reaction mixture was concentrated under reduced pressure and the residue was purified by column chromatography (silica gel cartridge, chloroform/methanol = 100 : 0 to 95 : 5) to obtain the title compound (320 mg).

### Example 13 3-Chloro-N-{(1-hydroxycyclopentyl)[3-(hydroxymethyl)phenyl]methyl}-4-(trifluoromethyl)pyridine-2-carboxamide

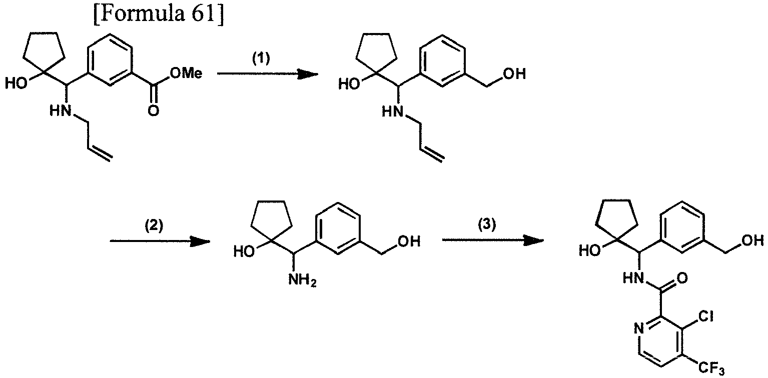

(1) A solution of methyl 3-[(1-hydroxycyclopentyl)(prop-2-en-1-ylamino)methyl]benzoate (30 mg) in tetrahydrofuran (5.0 mL) was added under ice-cooling to a solution of lithium aluminum hydride (7.9 mg) in tetrahydrofuran (5.0 mL), and stirred at 0°C for 3 hours. Acetone and water were sequentially added to the reaction mixture, and after phase separation, three extractions were conducted with ethyl acetate. The obtained organic layers were combined and dried over anhydrous magnesium sulfate, the desiccating agent was filtered off, the filtrate was concentrated under reduced pressure to obtain a crude product (44 mg) containing 1-{[3-(hydroxymethyl)phenyl](prop-2-en-1-ylamino)methyl}cyclopentanol.
(2) A crude product (150 mg) containing 1-{amino[3-(hydroxymethyl)phenyl]methyl}cyclopentanol was obtained from 1-{[3-(hydroxymethyl)phenyl](prop-2-en-1-ylamino)methyl}cyclopentanol (72 mg) by the similar method as in Example 3 (2).
(3) The title compound (7.8 mg) was obtained from 1-{amino[3-(hydroxymethyl)phenyl]methyl}cyclopentanol (250 mg) by the similar method as in Example 2.

### Example 14 3-Chloro-N-[{3-[(dimethylamino) methyl] phenyl}(1-hydroxycyclopentyl)methyl]-4-(trifluoromethyl)pyridine-2-carboxamide

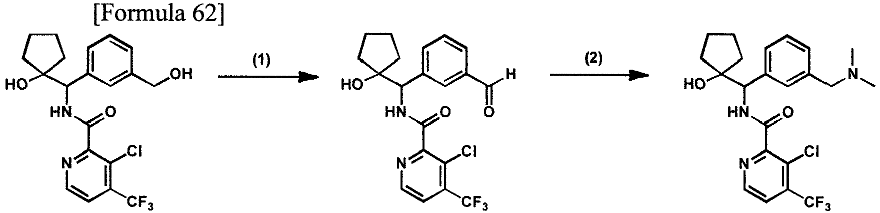

(1) 1,1,1-Triacetoxy-1,1-dihydro-1,2-benziodoxol-3(1H)-one (5.9 mg) was added to a solution of 1-{amino[3-(hydroxymethyl)phenyl]methyl}cyclopentanol (5.0 mg) in chloroform (1.0 mL), and stirred at room temperature for 18 hours. An aqueous saturated sodium thiosulfate solution was added to the reaction mixture and subsequently extracted 3 times with chloroform. The organic layer was separated by a phase separation cartridge and concentrated under reduced pressure to obtain a crude product (13 mg) containing 3-chloro-N-[(3-formylphenyl)(1-hydroxycyclopentyl)methyl]-4-(trifluoromethyl)pyridine-2-carboxamide.
(2) A solution of 2.0 M dimethylamine in tetrahydrofuran (0.68 mL) was added to a solution of 3-chloro-N-[(3-formylphenyl)(1-hydroxycyclopentyl)methyl]-4-(trifluoromethyl)pyridine-2-carboxamide (56 mg) in chloroform (3.0 mL), stirred at room temperature for 18 hours, subsequently a solution of 2.0 M dimethylamine in tetrahydrofuran (1.1 mL) was added thereto and stirred at room temperature for 4 days. Further, dimethylamine hydrochloride (63 mg) was added thereto, stirred at room temperature for 4 days, and subsequently sodium triacetoxyborohydride (30 mg) was added to the reaction mixture and stirred at room temperature for 5 hours. Acetone and water were sequentially added to the reaction mixture, stirred, and extracted 3 times with chloroform. The organic layer was separated by a phase separation cartridge, concentrated under reduced pressure, and purified by PTLC (NH silica gel, ethyl acetate) to obtain the title compound (12 mg).

### Example 15 3-Chloro-N-{[3-(dimethylcarbamoyl)phenyl](1-hydroxycyclopentyl)methyl}-4-(trifluoromethyl)pyridine-2-carboxamide

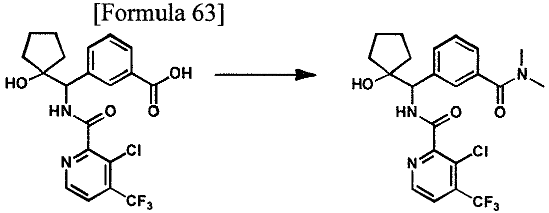

N,N-Diisopropylethylamine (390 µL) was added under ice-cooling to a solution of 3-[({[3-chloro-4-(trifluoromethyl)pyridin-2-yl]carbonyl}amino)(1-hydroxycyclopentyl)methyl]benzoic acid (200 mg) and O-(7-azabenzotriazol-1-yl)-N,N,N',N'-tetramethyluronium hexafluorophosphoric acid (240 mg) in N,N-dimethylformamide (4.0 mL) and stirred for 20 minutes. An aqueous 50% dimethylamine solution (45 µL) was added to the reaction mixture and stirred at room temperature for 18 hours. An aqueous saturated sodium hydrogen carbonate solution was added to the reaction mixture and then extracted 3 times with ethyl acetate. The obtained organic layers were combined, dried over anhydrous magnesium sulfate, the desiccating agent was filtered off, and the filtrate was concentrated under reduced pressure. The residue was purified by column chromatography (NH silica gel cartridge, hexane/ethyl acetate = 88 : 12 to 0 : 100), and the obtained residue was washed with diisopropyl ether and collected by filtration to obtain the title compound (8.9 mg).

### Example 16 3-Chloro-N-{[3-(1-ethyl-1H-pyrazol-4-yl)phenyl](1-hydroxycyclopentyl)methyl}pyridine-2-carboxamide

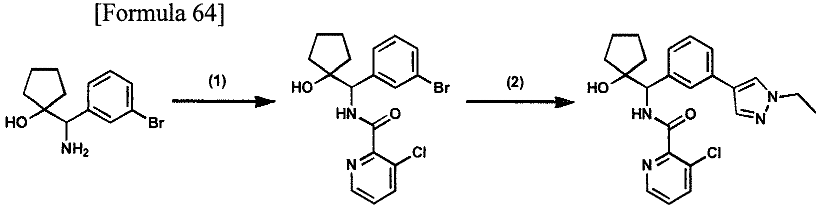

(1) A solution of 3-chloropyridine-2-carboxylic acid (180 mg), 1-ethyl-3-(3-dimethylaminopropyl)carbodiimide hydrochloride (240 mg) and 1-hydroxybenzotriazole (240 mg) in N,N-dimethylformamide (4.0 mL) was stirred at 0°C for 10 minutes. A solution of 1-[amino(3-bromophenyl)methyl]cyclopentanol (300 mg) in N,N-dimethylformamide (3.0 mL) was added to the reaction mixture and stirred at room temperature for 3 hours. The reaction mixture was diluted with ethyl acetate, washed sequentially with an aqueous saturated sodium hydrogen carbonate solution, water and saline, the organic layers were combined and dried over anhydrous magnesium sulfate. After filtering off the desiccating agent, the organic layer was concentrated under reduced pressure, and the residue was purified by column chromatography (silica gel cartridge, hexane/ethyl acetate = 67 : 33 to 0 : 100) to obtain N-[(3-bromophenyl)(1-hydroxycyclopentyl)methyl]-3-chloropyridine-2-carboxamide (250 mg).
(2) The title compound (170 mg) was obtained from N-[(3-bromophenyl)(1-hydroxycyclopentyl)methyl]-3-chloropyridine-2-carboxamide (220 mg) by the similar method as in Example 6.

### Example 17 (Enantiomer 1) 3-Chloro-N-[(4-chloropyridin-2-yl)(cis-4-fluoro-1-hydroxycyclohexyl)methyl]-2-(trifluoromethyl)pyridine-4-carboxamide

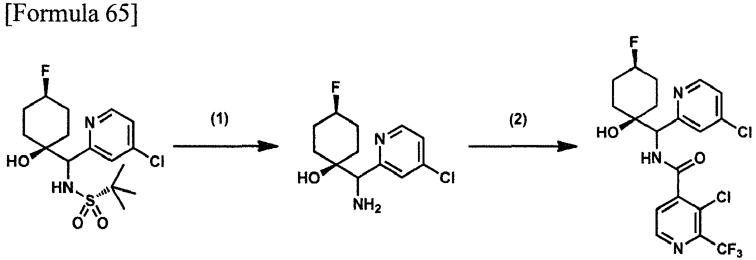

(1) A solution of 4.0 M hydrogen chloride in 1,4-dioxane (3.6 ml) was added dropwise to a solution of (diastereomer 1) (S)-N-[(4-chloropyridin-2-yl)(cis-4-fluoro-1-hydroxycyclohexyl)methyl]-2-methylpropane-2-sulfinamide (1.3 g) in methanol (15 ml), and stirred at room temperature for 1.5 hours. Triethylamine was added to the reaction mixture, concentrated under reduced pressure to obtain a crude product (2.9 g) containing cis-1-[amino(4-chloropyridin-2-yl)methyl]-4-fluorocyclohexanol.
(2) A solution of cis-1-[amino(4-chloropyridin-2-yl)methyl]-4-fluorocyclohexanol (100 mg), 3-chloro-2-(trifluoromethyl)isonicotinic acid (42 mg), 1-ethyl-3-(3-dimethylaminopropyl)carbodiimide hydrochloride (35 mg), 1-hydroxybenzotriazole (28 mg), triethylamine (43 µL) in N,N-dimethylformamide (1.0 mL) was stirred at room temperature overnight. The reaction mixture was purified by preparative HPLC to obtain the title compound (19 mg).

### Example 18-A (Enantiomer 1) 3-Chloro-N-[(6-chloropyridin-2-yl)(cis-4-fluoro-1-hydroxycyclohexyl)methyl]-2-(trifluoromethyl)pyridine-4-carboxamide

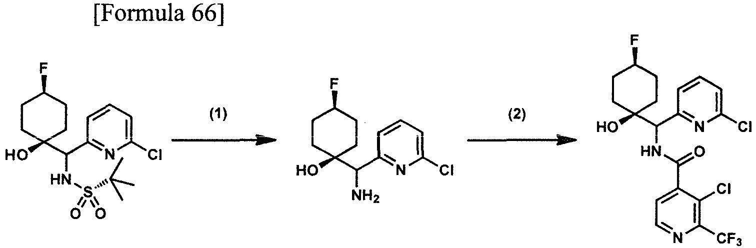

(1) A solution of 4.0 M hydrogen chloride in 1,4-dioxane (2.1 ml) was added dropwise under ice-bath cooling to a solution of (diastereomer 1) (S)-N-[(6-chloropyridin-2-yl)(cis-4-fluoro-1-hydroxycyclohexyl)methyl]-2-methylpropane-2-sulfinamide (1.0 g) in methanol (55 ml), and stirred for 2 hours. Triethylamine was added to the reaction mixture, concentrated under reduced pressure to obtain a crude product (710 mg) containing (enantiomer 1) cis-1-[amino(6-chloropyridin-2-yl)methyl]-4-fluorocyclohexanol.
(2) N,N-Diisopropylethylamine (140 µL) and (enantiomer 1) cis-1-[amino(6-chloropyridin-2-yl)methyl]-4-fluorocyclohexanol (71 mg) were added to a solution of 3-chloro-2-(trifluoromethyl)isonicotinic acid (62 mg) and O-(7-azabenzotriazol-1-yl)-N,N,N',N'-tetramethyluronium hexafluorophosphoric acid (130 mg) in chloroform (1.0 mL), and stirred at room temperature overnight. The reaction mixture was concentrated under reduced pressure. The residue was purified by preparative HPLC to obtain the title compound (79 mg).

### Example 18-B (Enantiomer 1) 5-Chloro-N-[(6-chloropyridin-2-yl)(4,4-difluoro-1-hydroxycyclohexyl)methyl]-2-(trifluoromethyl)pyridine-4-carboxamide

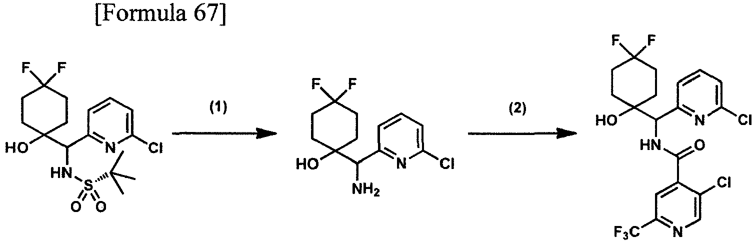

(1) A solution of 4.0 M hydrogen chloride in 1,4-dioxane (340 µL) was added dropwise under ice-bath cooling to a solution of (diastereomer 1) (S)-N-[(6-chloropyridin-2-yl)(4,4-difluoro-1-hydroxycyclohexyl)methyl]-2-methylpropane-2-sulfinamide (150 mg) in methanol (5.0 ml), and stirred at room temperature for 2 hours. Triethylamine was added to the reaction mixture, concentrated under reduced pressure to obtain a crude product containing (enantiomer 1) 1-[amino(6-chloropyridin-2-yl)methyl]-4,4-difluorocyclohexanol.
(2) N,N-Diisopropylethylamine (41 µL) and 1-[amino(6-chloropyridin-2-yl)methyl]-4,4-difluorocyclohexanol (a 1/5 amount of the crude product obtained in the reaction (1)) were added to a solution of 5-chloro-2-(trifluoromethyl)isonicotinic acid (19 mg) and O-(7-azabenzotriazol-1-yl)-N,N,N',N'-tetramethyluronium hexafluorophosphoric acid (36 mg) in chloroform (1.0 mL) and N,N-dimethylformamide (0.50 ml), and stirred at room temperature overnight. The reaction mixture was concentrated under reduced pressure. The residue was purified by preparative HPLC to obtain the title compound (22 mg).

### Example 19 (Enantiomer 1) N-[(2-Chloro-3-fluorophenyl)(cis-4-fluoro-1-hydroxycyclohexyl)methyl]-6-methyl-3-(pyrimidin-2-yl)pyridine-2-carboxamide

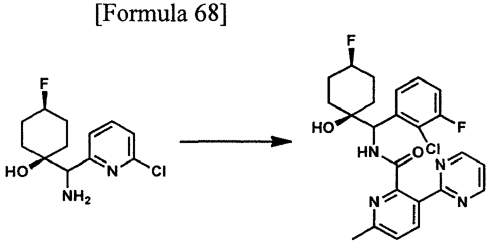

A solution of 6-methyl-3-(pyrimidin-2-yl)picolinic acid (17 mg), N,N-diisopropylethylamine (51 µl) and 1.7 M 2,4,6-tripropyl-1,3,5,2,4,6-trioxatriphosphinane 2,4,6-trioxide in N,N-dimethylformamide (85 µl) was added to a solution of (enantiomer 1) cis-1-[amino(2-chloro-3-fluorophenyl)methyl]-4-fluorocyclohexanol (20 mg) inN,N-dimethylformamide (1.0 ml), and stirred at room temperature overnight. The reaction mixture was purified by preparative HPLC to obtain the title compound (7.4 mg).

### Example 20 (Enantiomer 1) 3-Chloro-N-[(2-chloro-3-fluorophenyl)(cis-4-fluoro-1-hydroxycyclohexyl)methyl]-N-methyl-4-(trifluoromethyl)pyridine-2-carboxamide

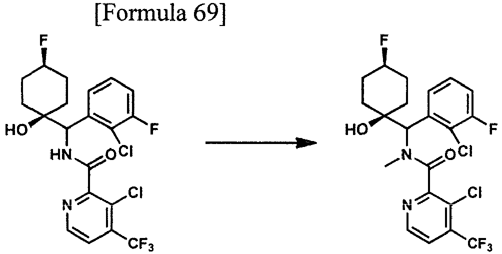

60% Sodium hydride (2.5 mg) was added under ice-bath cooling to a solution of (enantiomer 1) 3-chloro-N-[(2-chloro-3-fluorophenyl)(cis-4-fluoro-1-hydroxycyclohexyl)methyl]-4-(trifluoromethyl)pyridine-2-carboxamide (30 mg) in N,N-dimethylformamide (200 µl), and stirred for 30 minutes. Iodomethane (4.0 µl) was added to the reaction mixture and stirred for 3 hours. Water was added to the reaction mixture, after extraction with chloroform, drying was carried out over anhydrous sodium sulfate, followed by concentration under reduced pressure. The residue was purified by preparative HPLC to obtain the title compound (7.0 mg).

Table 1 shows the compounds illustrated in Examples 1 to 20, the structural formulae and names of the compounds synthesized by the similar method, and instrument data thereof. Each number shown in the column Example in the Table shows that the compound was synthesized by the similar method as in the Example of the above Examples 1 to 20 shown by the number.

In Table 1, the abbreviations of columns and solvents used are described as follows.
Columns used
Daicel CHIRALPAK AD3: AD3
Daicel CHIRALPAK AS3: AS3
Daicel CHIRALPAK OD3: OD3
Daicel CHIRALPAK IA3: IA3
Daicel CHIRALPAKI IB3: IB3
Daicel CHIRALPAK ADH: AD-H
Daicel CHIRALPAK IE: IE
Daicel CHIRALPAK AS: AS
Solvents
n-hexane: Hex
Ethyl alcohol: EtOH
Isopropyl alcohol: IPA

**[Table 1-1]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **1** | **16** | 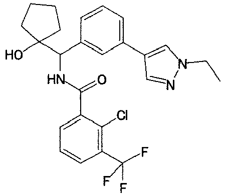 | 2-chloro-N-{[3-(1-ethyl-1H-pyrazol-4-yl)phenyl](1 -hydroxycyclopentyl)methyl}-3-(trifluoromethyl)benzamide | 492(M+H) | | | | 0.11 |
| **2** | **1** | 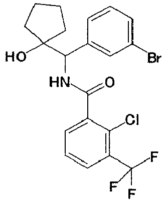 | N-[(3-bromophenyl)(1-hydroxycyclopentyl)methyl]-2-chloro-3-(trifluoromethyl)benzamide | 476(M+H) | | | | 0.67 |
| **3** | **6** | 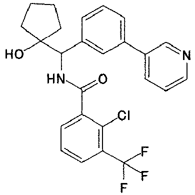 | 2-chloro-N-{(1-hydroxycyclopentyl)[3-(pyridin-3-yl)phenyl]methyl}-3-(trifluoromethyl)benzamide | 475(M+H) | | | | 0.36 |
| **4** | **6** | 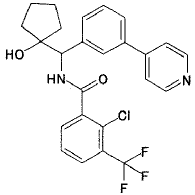 | 2-chloro-N-{(1-hydroxycyclopentyl)[3-(pyridin-4-yl)phenyl]methyl}-3-(trifluoromethyl)benzamide | 475(M+H) | | | | 0.40 |
| 5 | 6 | 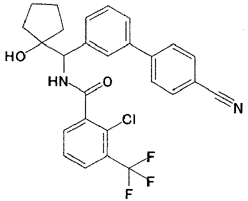 | 2-chloro-N-[(4'-cyanobiphenyl-3-yl)(1-hydroxycyclopentyl)methyl]-3-(trifluoromethyl)benzamide | 499(M+H) | | | | 0.88 |
| **6** | **6** | 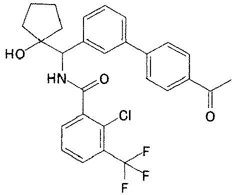 | N-[(4'-acetylbiphenyl-3-yl)(1-hydroxycyclopentyl)methyl]-2-chloro-3-(trifluoromethyl)benzamide | 516(M+H) | | | | 0.67 |

**[Table 1-2]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **7** | **6** | 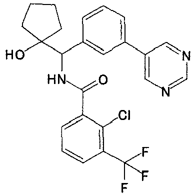 | 2-chloro-N-{(1-hydroxycyclopentyl)[3-(pyrimidin-5-yl)phenyl]methyl}-3-(trifluoromethyl)benzamide | 476(M+H) | | | | 0.29 |
| **8** | **8** | 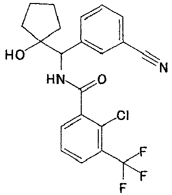 | 2-chloro-N-[(3-cyanophenyl)(1-hydroxycyclopentyl)methyl]-3-(trifluoromethyl)benzamide | 423(M+H) | | | | 0.40 |
| **9** | **6** | 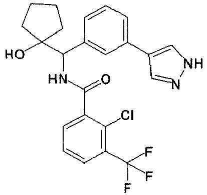 | 2-chloro-N-{(1-hydroxycyclopentyl)[3-(1H-pyrazol-4-yl)phenyl]methyl}-3-(trifluoromethyl)benzamide | 464(M+H) | | | | 0.11 |
| **10** | **6** | 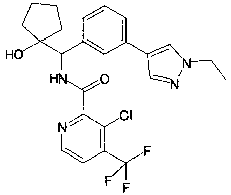 | 3-chloro-N-{(3-(1-ethyl-1H-pyrazol-4-yl)phenyl](1-hydroxycyclopentyl)methyl}-4-(trifluoromethyl)pyridine-2-carboxamide | 493(M+H) | | | | 0.013 |
| **11** | **1** | 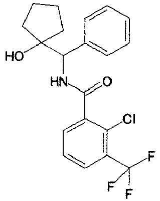 | 2-chloro-N-[(1-hydroxycyclopentyl)(phenyl) methyl]-3-(trifluoromethyl)benzamide | 398(M+H) | | | | 1.0 |
| **12** | **1** | 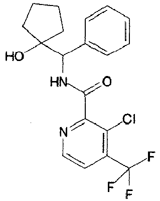 | 3-chloro-N-[(1-hydroxycyclopentyl)(phenyl) methyl]-4-(trifluoromethyl)pyridine-2-carboxamide | 399(M+H) | | | | 0.48 |

**[Table 1-3]**

| Compound No. | Example | Structure | Compound Name | (ESI pos.) m/z (ESI neg.) m/z | Optical isomer analysis conditions (Column used) (Solvent system) (Flow rate) | Optical isomer RT (min) | LCMS RT (min) condition | IC₅₀ (µM) |
|---|---|---|---|---|---|---|---|---|
| **13** | **6** | 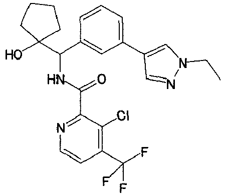 | (enantiomer 2) 3-chloro-N-{[3-(1-ethyl-1H-pyrazol-4-yl)phenyl](1-hydroxycyclopentyl)methyl}-4-(trifluoromethyl)pyridine-2-carboxamide | 493(M+H) | AD-H, 4.6*250 | 8.1,16.0 Faster | | 0.83 |
| | | | | | Hex: EtOH: IPA = 60 : 30:10 | | | |
| | | | | | 1mL/min | | | |
| **14** | **6** | 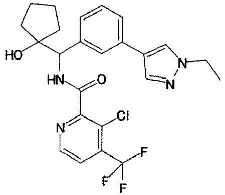 | (enantiomer 1) 3-chloro-N-{[3-(1-ethyl-1H-pyrazol-4-yl)phenyl](1-hydroxycyclopentyl)methyl}-4-(trifluoromethyl)pyridine-2-carboxamide | 493(M+H) | AD-H, 4.6*250 | 8.1,16.0 Later | | 0.013 |
| | | | | | Hex : EtOH : IPA = 60 : 30:10 | | | |
| | | | | | 1mL/min | | | |
| **15** | **6** | 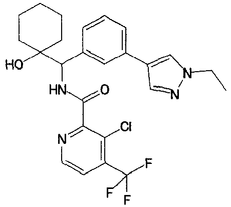 | 3-chloro-N-{[3-(1-ethyl-1H-pyrazol-4-yl)phenyl](1-hydroxycyclohexyl)methyl}-4-(trifluoromethyl)pyridine-2-carboxamide | 507(M+H) | | | | 0.0085 |
| **16** | **6** | 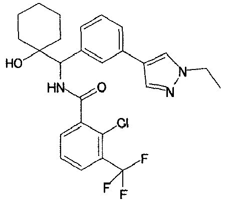 | 2-chloro-N-{[3-(1-ethyl-1H-pyrazol-4-yl)phenyl](1-hydroxycyclohexyl)methyl}-3-(trifluoromethyl)benzamide | 506(M+H) | | | | 0.044 |
| **17** | **6** | 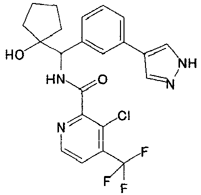 | 3-chloro-N-{(1-hydroxycyclopentyl)[3-(1H-pyrazol-4-yl)phenyl]methyl}-4-(trifluoromethyl)pyridine-2 - carboxamide | 465(M+H) | | | | 0.014 |
| **18** | **6** | 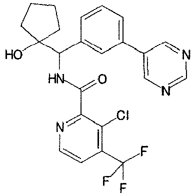 | 3-chloro-N-{(1-hydroxycyclopentyl)[3-(pyrimidin-5-yl)phenyl]methyl}-4-(trifluoromethyl)pyridine-2-carboxamide | 477(M+H) | | | | 0.092 |

**[Table 1-4]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **19** | **6** | 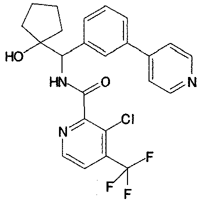 | 3-chloro-N-{(1-hydroxycyclopentyl)[3-(pyridin-4-yl)phenyl]methyl}-4-(trifluoromethyl)pyridine-2-carboxamide | 476(M+H) | | | | 0.034 |
| **20** | **6** | 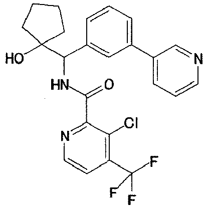 | 3-chloro-N-{(1-hydroxycyclopentyl)[3-(pyridin-3-yl)phenyl]melhyl}-4-(trifluoromethyl)pyridine-2-carboxamide | 476(M+H) | | | | 0.017 |
| **21** | **16** | 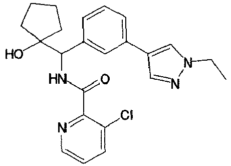 | 3-chloro-N-{[3-(1-ethyl-1 H-pyrazol-4-yl)phenyl](1-hydroxycyclopentyl)methyl} pyridine-2-carboxamide | 425(M+H) | | | | 4.6 |
| **22** | **16** | 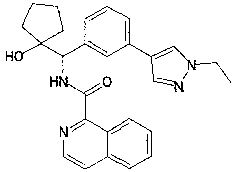 | N-{[3-(1-ethyl-1H-pyrazo!-4-yl)phenyl](1-hydroxycyclopentyl)methyl} isoquinoline-1-carboxamide | 441 (M+H) | | | | 0.11 |
| **23** | **8** | 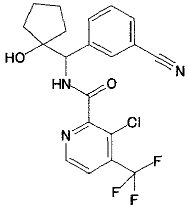 | 3-chloro-N-[(3-cyanophenyl)(1-hydroxycyclopentyl)methyl]-4-(trifluoromethyl)pyridine-2-carboxamide | 424(M+H) | | | | 0.30 |
| **24** | **6** | 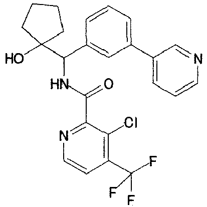 | (enantiomer 2) 3-hloro-N-{(1-hydroxycyclopentyl)[3-(pyridin-3-yl)phenyl]methyl}4-(trifluoromethyl)pyridine-2-carboxamide | 476(M+H) | AD-H, 4.6*250 | 10.7. 20.3 Faster | | 2.0 |
| | | | | | Hex: EtOH = 70 : 30 | | | |
| | | | | | 1mL/min | | | |

**[Table 1-5]**

| Compounds No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) conditions | IC₅₀ (µM) |
|---|---|---|---|---|---|---|---|---|
| **25** | **6** | 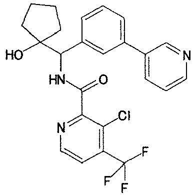 | (enantiomer 1) 3-chloro-N-{(1-hydroxycyclopentyl)[3-(pyridin-3-yl)phenyl]methyl}-4-(trifluoromethyl)pyridine-2-carboxamide | 476(M+H) | AD-H, 4.6*250 | 10.7, 20.3 Later | | 0.069 |
| | | | | | Hex : EtOH = 70 : 30 | | | |
| | | | | | 1mL/min | | | |
| **26** | **6** | 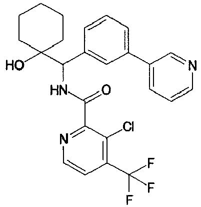 | 3-chloro-N-{(hydroxycyclohexyl)[3-(pyridin-3-yl)phenyl]methyl}-4-(trifluoromethyl)pyridine-2-carboxamide | 490(M+H) | | | | 0.080 |
| **27** | **6** | 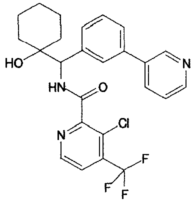 | (enantiomer 2) 3-chloro-N-{(1-hydroxycyclohexyl)[3-(pyridin-3-yl)phenyl]methyl}-4-(trifluoromethyl)pyridine-2-carboxamide | 490(M+H) | AD-H, 4.6*250 | 6.9,12.0 Faster | | 0.74 |
| | | | | | Hex: EtOH = 60 : 40 | | | |
| | | | | | 1mL/min | | | |
| **28** | **6** | 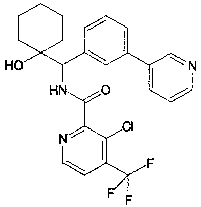 | (enantiomer 1) 3-chloro-N-{(1-hydroxycyclohexyl)[3-(pyridin-3-yl)phenyl]methyl}-4-(trifluoromethyl)pyridine-2-carboxamide | 490(M+H) | AD-H, 4.6*250 | 6.9,12.0 Later | | 0.02 |
| | | | | | Hex: EtOH = 60 : 40 | | | |
| | | | | | 1mL/min | | | |
| **29** | **1** | 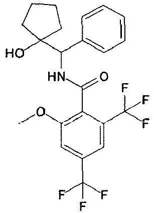 | N-[(1-hydroxycyclopentyl) (phenyl)methyl]-2-methoxy-4,6-bis(trifluoromethyl) benzamide | 462(M+H) | | | | 2.0 |
| **30** | **1** | 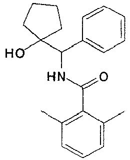 | N-[(1-hydroxycyclopentyl) (phenyl)methyl]-2,6-dimethylbenzamide | 324(M+H) | | | | 6.5 |

**[Table 1-6]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **31** | **1** | 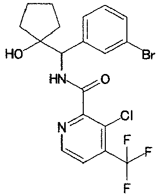 | N-[(3-bromophenyl)(1-hydroxycyclopentyl)methyl]-3-chloro-4-(trifluoromethyl)pyridine-2-carboxamide | 477(M+H) | | | 1.10, A | 0.17 |
| **32** | **1** | 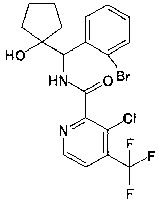 | N-[(2-bromophenyl)(1-hydroxycyclopentyl)methyl]-3-chloro-4-(trifluoromethyl)pyridine-2-carboxamide | 477(M+H) | | | 1.10, A | 0,060 |
| **33** | **2** | 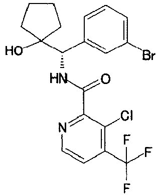 | N-[(S)-(3-bromophenyl)(1-hydroxycyclopentyl)methyl]-3-chloro-4-(trifluoromethyl)pyridine-2-carboxamide | 477(M+H) | | | 1.10, A | 0.082 |
| **34** | **1** | 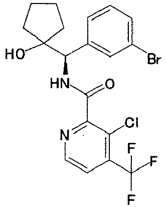 | N-((R)-(3-bromophenyl)(1-hydroxycyclopentyl)methyl]-3-chloro-4-(trifluoromethyl)pyridine-2-carboxamide | 477(M+H) | | | 1.10, A | 1.0 |
| **35** | **9** | 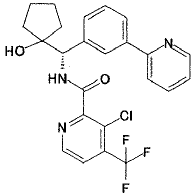 | 3-chloro-N-{(S)-(1-hydroxycyclopentyl)[3-(pyridin-2-yl)phenyl]methyl}-4-(trifluoromethyl)pyridine-2-carboxamide | 476(M+H) | | | 0.83, A | 0.074 |
| **36** | **9** | 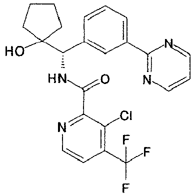 | 3-chloro-N-{(S)-(1-hydroxycyclopentyl)[3-(pyrimidin-2-yl)phenyl]methyl}-4-(trifluoromethyl)pyridine-2-carboxamide | 477(M+H) | | | 0.97, A | 0.13 |

**[Table 1-7]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **37** | **8** | 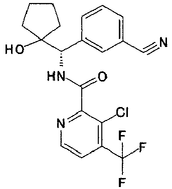 | 3-chloro-N-[(S)-(3-cyanophenyl)(1-hydroxycyclopentyl)methyl]-4-(trifluoromethyl)pyridine-2-carboxamide | 424(M+H) | | | 0.97, A | 0.26 |
| **38** | **3** | 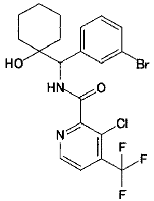 | N-[(3-bromophenyl)(1-hydroxycyclohexyl)methyl]-3-chloro-4-(trifluoromethyl)pyridine-2-carboxamide | 491(M+H) | | | 1.17, A | 0.060 |
| **39** | **2** | 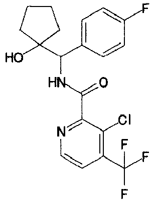 | 3-chloro-N-[(4-fluorophenyl)(1-hydroxycyclopentyl)methyl]4-(trifluoromethyl)pyridine-2-carboxamide | 417(M+H) | | | 1.01, A | 1.0 |
| **40** | **3** | 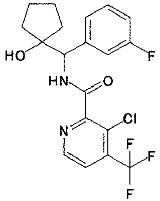 | 3-chloro-N-[(3-fluorophenyl)(1-hydroxycyclopentyl)methyl]-4-(trifluoromethyl)pyridine-2-carboxamide | 417(M+H) | | | 1.02, A | 0.11 |
| **41** | **3** | 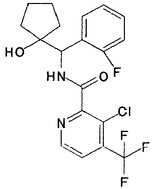 | 3-chloro-N-[(2-fluorophenyl)(1-hydroxycyclopentyl)methyl]-4-(trifluoromethyl)pyridine-2-carboxamide | 417(M+H) | | | 1.02, A | 0.18 |
| **42** | **3** | 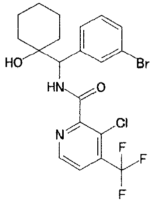 | (enantiomer 1) N-[(3-bromophenyl)(1-hydroxycyclohexyl)methyl]-3-chloro-4-(trifluoromethyl)pyridine-2-carboxamide | 491(M+H) | AD-H, 4.6*250 | 13.35, 18.16 Faster | 1.16, A | 0.038 |
| | | | | | Hex : IPA = 90 : 10 | | | |
| | | | | | 0.8mL/min | | | |

**[Table 1-8]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **43** | **3** | 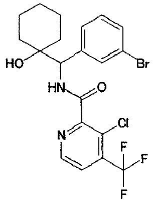 | (enantiomer 2) N-[(3-bromophenyl)(1-hydroxycyclohexyl)methyl]-3-chloro-4-(trifluoromethyl)pyridine-2-carboxamide | 491(M+H) | AD-H, 4.8*250 | 13.35, 18.16 Later | | 1.4 |
| | | | | | Hex : IPA = 90 :10 | | | |
| | | | | | 0.8mL/min | | | |
| **44** | **3** | 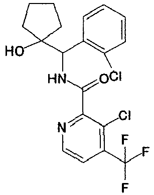 | 3-chloro-N-[(2-chlorophenyl)(1-hydroxycyclopentyl)methyl]-4-(trifluoromethyl)pyridine-2-carboxamide | 433(M+H) | | | 1.08, A | 0.066 |
| **45** | **3** | 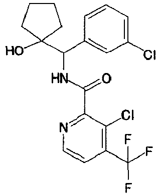 | 3-chloro-N-[(3-chlorophenyl)(1-hydroxycyclopentyl)methyl]-4-(trifluoromethyl)pyridine-2-carboxamide | 433(M+H) | | | 1.08, A | 0.090 |
| **46** | **3** | 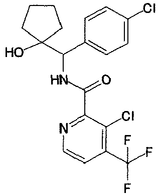 | 3-chloro-N-[(4-chlorophenyl)(1-hydroxycyclopentyl)methyl]-4-(trifluoromethyl)pyridine-2-carboxamide | 433(M+H) | | | 1.08, A | 1.7 |
| **47** | **3** | 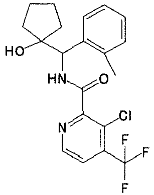 | 3-chloro-N-[(1-hydroxycyclopentyl)(2-methylphenyl)methyl]-4-(trifluoromethyl)pyridine-2-carboxamide | 413(M+H) | | | 1.07, A | 0.28 |
| **48** | **3** | 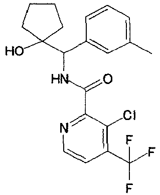 | 3-chloro-N-[(1-hydroxycyclopentyl)(3-methylphenyl)methyl]-4-(trifluoromethyl)pyridine-2-carboxamide | 413(M+H) | | | 1.06, A | 0.67 |

**[Table 1-9]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **49** | **3** | 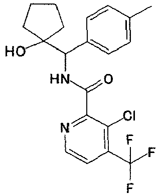 | 3-chloro-N-[(1-hydroxycyclopentyl)(4-methylphenyl)methyl]-4-(trifluoromethyl)pyridine-2-carboxamide | 413(M+H) | | | 1.06, A | 3.8 |
| **50** | **3** | 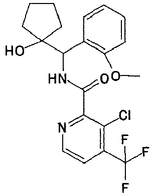 | 3-chloro-N-[(1-hydroxycyclopentyl)(2-methoxyphenyl)methyl]-4-(trifluoromethyl)pyridine-2-carboxamide | 429(M+H) | | | 1.02, A | 0.54 |
| **51** | **3** | 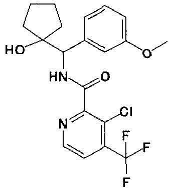 | 3-chloro-N-[(1-hydroxycyclopentyl)(3-methoxyphenyl)methyl]-4-(trifluoromethyl)pyridine-2-carboxamide | 429(M+H) | | | 1.00, A | 0.37 |
| **52** | **3** | 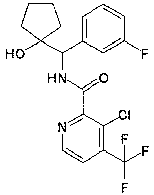 | (enantiomer 1) 3-chloro-N-[(3-fluorophenyl)(1-hydroxycyclopentyl)methyl}-4-(trifluoromethyl)pyridine-2-carboxamide | 417(M+H) | AD-H, 4.6*250 Hex : IPA= 90: 10 1mL/min | 15.23, 21.38 Faster | 1.03, A | 0.10 |
| **53** | **3** | 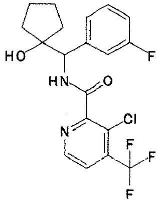 | (enantiomer 2) 3-chloro-N-[(3-fluorophenyl)(1-hydroxycyclopentyl)methyl]4-(trifluoromethyl)pyridine-2-carboxamide | 417(M+H) | AD-H, 4.6*250 Hex: IPA= 90: 10 1mL/min | 15.23, 21.38 Later | 1.03, A | 1.5 |
| **54** | **3** | 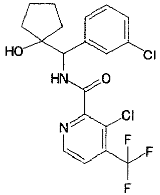 | (enantiomer 1) 3-chloro-N-[(3-chlorophenyl)(1-hydroxycyclopentyl)methyl]-4-(trifluoromethyl)pyridine-2-carboxamide | 433(M+H) | ①AD-H, 4.6*250 Hex IPA=80:20 1mL/min AD-3, 4.6'250 ② Hex IPA=80:20 1mL/min | ①6.50, 8.19 Faster ②5.49, 6.70 Faster | 1.09, A | 0.057 |

**[Table 1-10]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **55** | **3** | 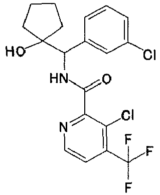 | (enantiomer 2) 3-chloro-N-[(3-chlorophenyl)(1-hydroxycyclopentyl)methyl]-4-(trifluoromethyl)pyridine-2-carboxamide | 433(M+H) | AD-H, 4.6*250 | 6.50, 8.19 Later | 1.09, A | 8.0 |
| | | | | | Hex: IPA = 80 : 20 | | | |
| | | | | | 1mL/min | | | |
| **56** | **3** | 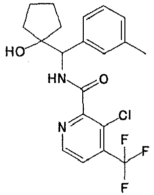 | (enantiomer 1) 3-chloro-N-[(1-hydroxycyclopentyl)(3-methylphenyl)methyl]-4-(trifluoromethyl)pyridine-2-carboxamide | 413(M+H) | AD-H, 4.6*250 | 14.13, 20.27 Faster | 1.06, A | 0.24 |
| | | | | | Hex: IPA= 90 :10 | | | |
| | | | | | 1mL/min | | | |
| **57** | **3** | 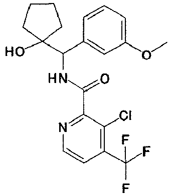 | (enantiomer 1) 3-chloro-N-[(1-hydroxycyclopentyl)(3-methoxyphenyl)methyl]-4-(trifluoromethyl)pyridine-2-carboxamide | 429(M+H) | AD-H, 4.6*250 | 6.04, 7.39 Faster | 1.01, A | 0.15 |
| | | | | | Hex: IPA = 70 : 30 | | | |
| | | | | | 1mL/min | | | |
| **58** | **2** | 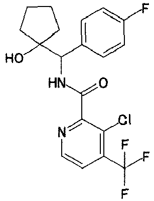 | (enantiomer 1) 3-chloro-N-[(4-fluorophenyl)(1-hydroxycyclopentyl)methyl]-4-(trifluoromethyl)pyridine-2-carboxamide | 417(M+H) | AD-H, 4.6*250 | 14.33, 28.34 Faster | 1.03, A | 0.76 |
| | | | | | Hex : IPA = 90 : 10 | | | |
| | | | | | 1mL/min | | | |
| **59** | **2** | 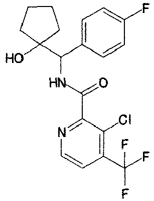 | (enantiomer2) 3-chloro-N-[(4-fluorophenyl)(1-hydroxycyclopentyl)methyl]-4-(trifluoromethyl)pyridine-2-carboxamide | 417(M+H) | AD-H, 4.6*250 | 14.33, 28.34 Later | 1.03, A | 9.5 |
| | | | | | Hex: IPA = 90 : 10 | | | |
| | | | | | 1mL/min | | | |
| **60** | **3** | 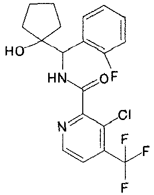 | (enantiomer 1) 3-chloro-N-[(2-fluorophenyl)(1-hydroxycyclopentyl)methyl]-4-(trifluoromethyl)pyridine-2-carboxamide | 417(M+H) | AD-H, 4.6*250 | 17.78, 19.95 Faster | 1.04, A | 0.20 |
| | | | | | Hex : IPA = 90 : 10 | | | |
| | | | | | 1mL/min | | | |

**[Table 1-11]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **61** | **3** | 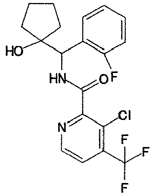 | (enantiomer 2) 3-chloro-N-[(2-fluorophenyl)(1-hydroxycyclopentyl)methyl]-4-(trifluoromethyl)pyridine-2-carboxamide | 417(M+H) | AD-H, 4.6*250 | 17.78, 19.95 Later | 1.04, A | 3.0 |
| | | | | | Hex : IPA = 90 :10 | | | |
| | | | | | 1mL/min | | | |
| **62** | **3** | 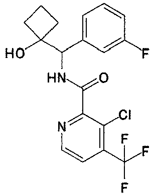 | 3-chloro-N-[(3-fluorophenyl)(1-hydroxycyclobutyl)methyl]-4-(trifluoromethyl)pyridine-2-carboxamide | 403(M+H) | | | 1.00, A | 2.1 |
| **63** | **3** | 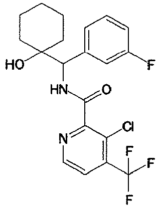 | 3-chloro-N-[(3-fluorophenyl)(1-hydroxycyclohexyl)methyl]-4-(trifluoromethyl)pyddine-2-carboxamide | 431 (M+H) | | | 1.10, A | 0.057 |
| **64** | **2** | 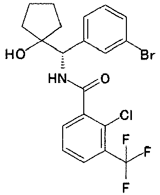 | N-[(S)-(3-bromophenyl)(1-hydroxycyclopentyl)methyl]-2-chloro-3-(trifluoromethyl)benzamide | 476(M+H) | AD-H, 4.6*250 | 11.93, 13.28 Later | 1.14, A | 0.075 |
| | | | | | Hex : IPA = 90 : 10 | | | |
| | | | | | 1mL/min | | | |
| **65** | **3** | 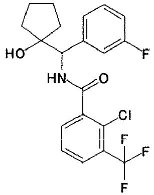 | 2-chloro-N-[(3-fluorophenyl)(1-hydroxycyclopentyl)methyl]-3-(trifluoromethyl)benzamide | 416(M+H) | | | 1.08, A | 0.27 |
| **66** | **3** | 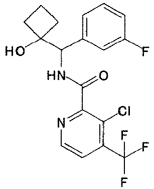 | (enantiomer 1) 3-chloro-N-[(3-fluorophenyl)(1-hydroxycyclobutyl)methyl]-4-(trifluoromethyl)pyridine-2-carboxamide | 403(M+H) | AD-H, 4.6*250 | 6.48, 8.15 Faster | 0.98, A | 0.69 |
| | | | | | Hex: IPA = 80: 20 | | | |
| | | | | | 1mL/min | | | |

**[Table 1-12]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **67** | **3** | 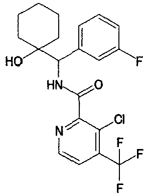 | (enantiomer 1) 3-chloro-N-[(3-fluorophenyl)(1-hydroxycyclohexyl)methyl]-4-(trifluoromethyl)pyridine-2-carboxamide | 431 (M+H) | AD-H, 4.6*250 | 6.52, 8.73 Faster | 1.08, A | 0.029 |
| | | | | | Hex : IPA = 80 : 20 | | | |
| | | | | | 1mL/min | | | |
| **68** | **3** | 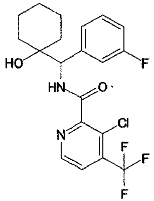 | (enantiomer 2) 3-chloro-N-[(3-fluorophenyl)(1-hydroxycyclohexyl)methyl]-4-(trifluoromethyl)pyridine-2-carboxamide | 431 (M+H) | AD-H, 4.6*250 | 6.52, 8.73 Later | 1.08, A | 2.6 |
| | | | | | Hex : IPA = 80 : 20 | | | |
| | | | | | 1mL/min | | | |
| **69** | **10** | 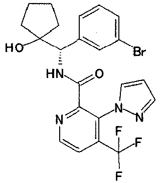 | N-[(S)-(3-bromophenyl)(1-hydroxycyclopentyl)methyl]-3-(1H-pyrazol-1-yl)-4-(trifluoromethyl)pyridine-2-carboxamide | 509(M+H) | | | 1.00, A | 1.4 |
| **70** | **10** | 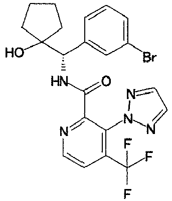 | N-[(S)-(3-bromophenyl)(1-hydroxycyclopentyl)methyl]-3-(2H-1,2,3-triazol-2-yl)-4-(trifluoromethyl)pyridine-2-carboxamide | 510(M+H) | | | 1.03, A | 1.2 |
| **71** | **10** | 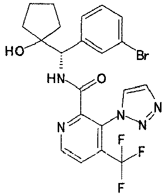 | N-[(S)-(3-bromophenyl)(1-hydroxycyclopentyl)methyl]-3-(1H-1,2,3-triazol-1-yl)-4-(trifluoromethyl)pyridine-2-carboxamide | 510(M+H) | | | 0.96, A | 5.7 |
| **72** | **3** | 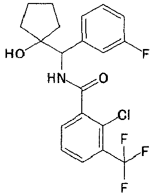 | (enantiomer 2) 2-chloro-N-[(3-fluorophenyl)(1-hydroxycyclopentyl)methyl]-3-(trifluoromethyl)benzamide | 416(M+H) | ①AD-H, 4.6*250 | ①23.99, 28.17 Later | 1.05, A | 7.1 |
| | | | | | Hex ; IPA = 95 : 5 | | | |
| | | | | | 1mL/min | | | |
| | | | | | ②AD-H, 4.6*250 | ②12.21, 12.95 Later | | |
| | | | | | Hex : EtOH = 95: 5 | | | |
| | | | | | 1mL/min | | | |

**[Table 1-13]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **73** | **3** | 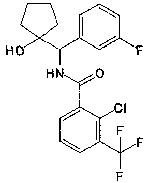 | (enanbomer 1) 2-chloro-N-[(3-fluorophenyl)(1-hydroxycyclopentyl)methyl]-3-(trifluoromethyl)benzamide | | ①AD-H, 4.6*250 | ①23.99, 28.17 Faster | 1.05, A | 0.22 |
| | | | | | Hex : IPA = ₉5 : 5 | | | |
| | | | | | 1mL/min | | | |
| | | | | 416(M+H) | ②AD-H, 4.6*250 | ②12.21, 12.95 Faster | | |
| | | | | | Hex: EtOH = 95 : 5 | | | |
| | | | | | 1mL/min | | | |
| 74 | **6** | 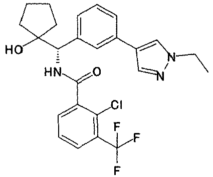 | 2-chloro-N-[(S)-[3-(1-ethyl-1H-pyrazol-4-yl)phenyl](1-hydroxycyclopentyl)methyl]-3-(trifluoromethyl)benzamide | 492(M+H) | | | 1.01, A | 0.043 |
| **75** | **3** | 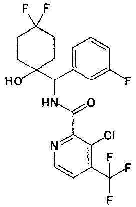 | 3-chloro-N-[(4,4-difluoro-1-hydroxycyclohexyl)(3-fluorophenyl)methyl]-4-(trifluoromethyl)pyridine-2-carboxamide | 467(M+H) | | | 1.04, A | 0.24 |
| **76** | **3** | 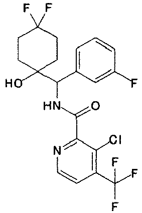 | (enantiomer 1) 3-chloro-N-[(4,4-difluoro-1-hydroxycyclohexyl)(3-fluorophenyl)methyl]-4-(trifluoromethyl)pyridine-2-carboxamide | 467(M+H) | AD-H, 4.6*250 | 7.18, 8.60 Faster | 1.04, A | 0.25 |
| | | | | | Hex : IPA = 80 : 20 | | | |
| | | | | | 1mL/min | | | |
| **77** | **3** | 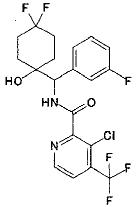 | (enantiomer 2) 3-chloro-N-[(4,4-difluoro-1-hydroxycyclohexyl)(3-fluorophenyl)methyl]-4-(trifluoromethyl)pyridine-2-carboxamide | 467(M+H) | AD-H, 4.6*250 | 7.18, 8.60 Later | 1.04, A | 1.2 |
| | | | | | Hex: IPA = 80 : 20 | | | |
| | | | | | 1mL/min | | | |
| **78** | **3** | 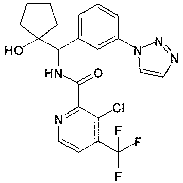 | 3-chloro-N-{(1-hydroxycyclopentyl)[3-(1H-1,2,3-triazol-1-yl)phenyl]methyl}-4-(trifluoromethyl)pyridine-2-carboxamide | 466(M+H) | | | 0.89, A | 0.45 |

**[Table 1-14]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **79** | **3** | 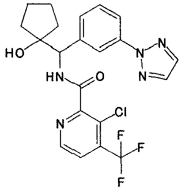 | 3-chloro-N-{(1-hydroxycyclopentyl)[3-(2H-1,2,3-triazol-2-yl)phenyl]methyl}-4-(trifluoromethyl)pyridine-2-carboxamide | 466(M+H) | | | 1.02, A | 0.31 |
| **80** | **3** | 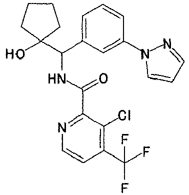 | 3-chloro-N-{(1-hydroxycyclopentyl)[3-(1H-pyrazol-1-yl)phenyl]methyl}-4-(trifluoromethyl)pyridine-2-carboxamide | 465(M+H) | | | 0.99, A | 0.16 |
| **81** | **3** | 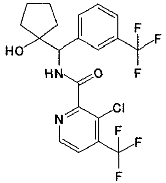 | 3-chloro-N-{(1-hydroxycyclopentyl)[3-(trifluoromethyl)phenyl]methyl} -4-(trifluoromethyl)pyridine-2-carboxamide | 467 (M+H) | | | 1.11, A | 0.96 |
| **82** | **3** | 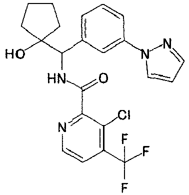 | (enantiomer 1) 3-chloro-N-{(1-hydroxycyclopentyl)[3-(1H-pyrazol-1-yl)phenyl]methyl}-4-(trifluoromethyl)pyridine-2-carboxamide | 465(M+H) | AD-H, 4.6*250 | 4.38, 7.02 Faster | 0.99, A | 0.089 |
| | | | | | Hex : IPA = 50 : 50 | | | |
| | | | | | 1mL/min | | | |
| **83** | **3** | 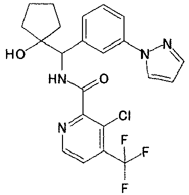 | (enantiomer 2) 3-chloro-N-{(1-hydroxycyclopentyl)[3-(1H-pyrazol-1-yl)phenyl]methyl}-4-(trifluoromethyl)pyridine-2-carboxamide | 465(M+H) | AD-H, 4.6*250 | 4.38, 7.02 Later | 0.99, A | 3.9 |
| | | | | | Hex : IPA = 50 : 50 | | | |
| | | | | | 1mL/min | | | |
| **84** | **3** | 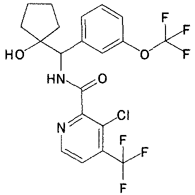 | 3-chloro-N-{(1-hydroxycyclopentyl)[3-(trifluoromethoxy)phenyl] methyl}-4-(trifluoromethyl)pyridine-2-carboxamide | 483(M+H) | | | 1.14, A | 2.6 |

**[Table 1-15]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT(min) condition | IC₅₀ (µM) |
|---|---|---|---|---|---|---|---|---|
| **85** | **3** | 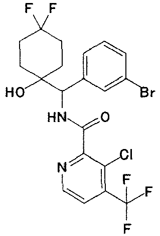 | N-[(3-bromophenyl)(4,4-difluoro-1-hydroxycyclohexyl)methyl]-3-chloro-4-(trifluoromethyl)pyridine-2-carboxamide | 527(M+H) | | | 1.13, A | 0.32 |
| **86** | **6** | 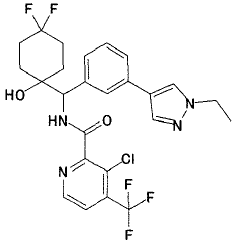 | 3-chloro-N-{(4,4-difluoro-1-hydroxycydohexyl)(3-(1 -ethyl-1H-pyrazol-4-yl)phenyl]methyl}-4-(trifluoromethyl)pyridine-2-carboxamide | 543(M+H) | | | 1.03, A | 0.073 |
| **87** | **3** | 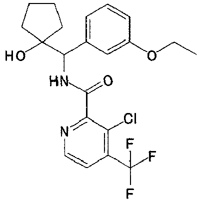 | 3-chloro-N-[(3-ethoxyphenyl)(1-hydroxycyclopentyl)methyl]-4-(trifluoromethyl)pyridine-2-carboxamide | 465(M+Na) | | | 1.07, A | 0.30 |
| **88** | **3** | 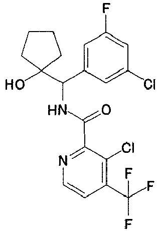 | 3-chloro-N-[(3-chloro-5-fluorophenyl)(1-hydroxycyclopentyl)methyl]-4-(trifluoromethyl)pyridine-2-carboxamide | 451 (M+H) | | | 1.14, A | 0.18 |
| **89** | **3** | 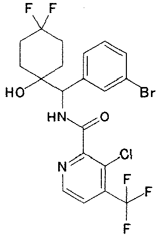 | (enantiomer 2) N-[(3-bromophenyl)(4,4-difluoro-1-hydroxycyclohexyl)methyl]-3-chloro-4-(trifluoromethyl)pyridine-2-carboxamide | 527(M+H) | | 8.48, 9.10 Faster | 1.11, A | 1.1 |
| | | | | | AD-3, 4,6*150 | | | |
| | | | | | Hex: EtOH = Hex:EtOH=90:10 1mL/min | | | |

**[Table 1-16]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **90** | **3** | 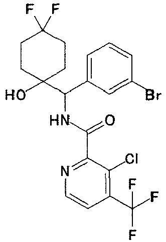 | (enantiomer 1) N-[(3-bromophenyl)(4,4-difluoro-1-hydroxycyclohexyl)methyl]-3-chloro-4-(trifluoromethyl)pyridine-2-carboxamide | 527(M+H) | AD-3, 4.6*150 | 8.48, 9.10 Later | 1.11, A | 0.30 |
| | | | | | Hex : EtOH = 90 :10 | | | |
| | | | | | 1mL/min | | | |
| **91** | **6** | 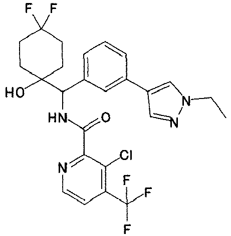 | (enantiomer 2) 3-chloro-N-{(4,4-difluoro-1-hydroxycyclohexyl)[3-(1-ethyl-1H-pyrazol-4-yl)phenyl]methyl}-4-(trifluoromethyl)pyridine-2-carboxamide | 543(M+H) | AD-H, 4.6*250 | 2.91, 4.29 Faster | 1.01, A | 3.8 |
| | | | | | Hex: IPA = 50 : 50 | | | |
| | | | | | 1mL/min | | | |
| **92** | **6** | 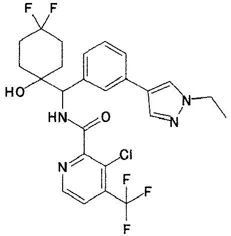 | (enantiomer 1) 3-chloro-N-{(4,4-difluoro-1-hydroxycyclohexyl)[3-(1-ethyl-1H-pyrazol-4-yl)phenyl]methyl}-4-(trifluoromethyl)pyridine-2-carboxamide | 543(M+H) | AD-H, 4.6*250 | 2.91, 4.29 Later | 1.01, A | 0.027 |
| | | | | | Hex: IPA = 50 : 50 | | | |
| | | | | | 1mL/min | | | |
| **93** | **3** | 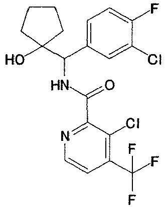 | 3-chloro-N-{(3-chloro-4-fluorophenyl)(1-hydroxycyclopentyl)methyl}-4-(trifluoromethyl)pyridine-2-carboxamide | 451(M+H) | | | 1.09, A | 0.43 |
| **94** | **3** | 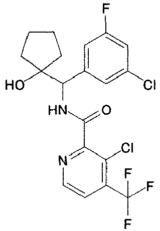 | (enantiomer 1) 3-chloro-N-[(3-chloro-5-fluorophenyl)(1-hydroxycylopentyl)methyl]-4-(trifluoromethyl)pyridine-2-carboxamide | 451(M+H) | AS, 4.6*250 | 3.68, 4.54 Later | 1.12, A | 0.11 |
| | | | | | Hex: EtOH = 80: 20 | | | |
| | | | | | 1mL/min | | | |

**[Table 1-17]**

| Compound No. | Example | Structure | Compound Name | (ESI pos.) m/z (ESI neg.) m/z | Optical isomer analysis conditions (Column used) (Solvent system) (Flow rate) | Optical isomer LCMS analysis RT (min) RT condition (min) | | IC₅₀ (µm) |
|---|---|---|---|---|---|---|---|---|
| **95** | **3** | 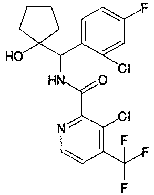 | 3-chloro-N-[(2-chloro-4-fluorophenyl)(1-hydroxycyclopentyl)methyl]-4-(trifluoromethyl)pyridine-2-carboxamide | 451 (M+H) | | | 1.10, A | 0.13 |
| **96** | **3** | 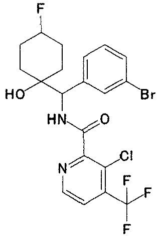 | N-[(3-bromophenyl)(4-fluoro-1-hydroxycyclohexyl)methyl]-3-chloro-4-(trifluoromethyl)pyridine-2-carboxamide | 509(M+H) | | | 1.08, A | 0.25 |
| **97** | **3** | 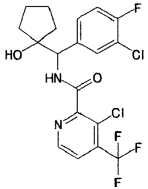 | (enantiomer 1) 3-chloro-N-[(3-chloro-4-fluorophenyl)(1-hydroxycyclopentyl)methyl]4-(trifluoromethyl)pyridine-2-carboxamide | 451 (M+H) | AS, 4.6*250 | 3.73, 5.01 Later | 1.11, A | 0.27 |
| | | | | | Hex: EtOH = 80 : 20 | | | |
| | | | | | 1mL/min | | | |
| **98** | **3** | 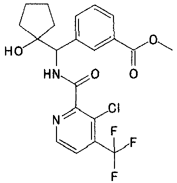 | methyl 3-[({[3-chloro-4-(trifluoromethyl)pyridin-2-yl]carbonyl}amino)(1-hydroxycyclopentyl)methyl] benzoate | 479(M+Na) | | | 1.01, A | 0.59 |
| **99** | **3** | 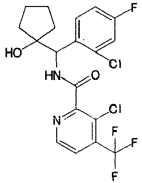 | (enantiomer 2) 3-chloro-N-[(2-chloro-4-fluorophenyl)(1-hydroxycyclopentyl)methyl]-4-(trifluoromethyl)pyridine-2-carboxamide | 451 (M+H) | AS, 4.6*250 | 8.53, 10.15 Faster | 1.12, A | 5.2 |
| | | | | | Hex : EtOH = 95 : 5 | | | |
| | | | | | 1mL/min | | | |
| **100** | **3** | 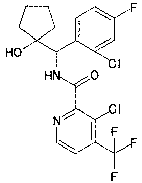 | (enantiomer 1) 3-chloro-N-[(2-chloro-4-fluorophenyl)(1-hydroxycyclopentyl)methyl]-4-(trifluoromethyl)pyridine-2-carboxamide | 451 (M+H) | AS, 4.6*250 | 8.53, 10.15 Later | 1.12, A | 0.059 |
| | | | | | Hex: EtOH = 95 : 5 | | | |
| | | | | | 1mL/min | | | |

**[Table 1-18]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **101** | **3** | 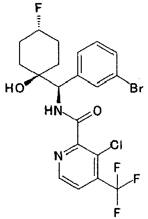 | N-[(R)-(3-bromophenyl)(trans-4-fluoro-1-hydroxycyclohexyl)methyl]-3-chloro-4-(trifluoromethyl)pyridine-2-carboxamide | 509(M+H) | AS, 4.6*250 | 4,47, 5.80 Faster | 1.07, A | 1.5 |
| | | | | | Hex: EtOH = BO : 20 | | | |
| | | | | | 1mL/min | | | |
| **102** | **3** | 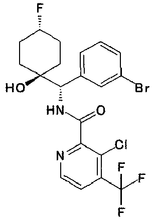 | N-[(S)-(3-bromophenyl)(trans-4-fluoro-1-hydroxycyclohexyl)methyll-3-chloro-4-(trifluoromethyl)pyridine-2-carboxamide | 509(M+H) | AS, 4.6*250 | 4.47, 5.80 Later | 1.07, A | 0.15 |
| | | | | | Hex: EtOH = 80 : 20 | | | |
| | | | | | 1mL/min | | | |
| **103** | **3** | 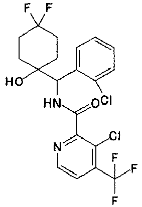 | 3-chloro-N-[(2-chlorophenyl)(4,4-difluoro-1-hydroxycyclohexyl)methyl]-4-(trifluoromethyl)pyridine-2-carboxamide | 483(M+H) | | | 1.09, A | 0.25 |
| **104** | **13** | 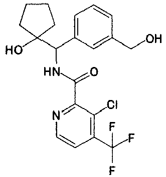 | 3-chloro-N-{(1-hydroxycyclopentyl)[3-(hydroxymethyl)phenyl]methyl} -4-(trifluoromethyl)pyridine-2-carboxamide | 429(M+H) | | | 0.83, A | 0.92 |
| **105** | **15** | 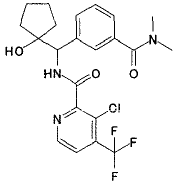 | 3-chloro-N-{[3-(dimethylcarbamoyl)phenyl](1-hydroxycyclopentyl)methyl}4-(trifluoromethyl)pyridine-2-carboxamide | 470(M+H) | | | 0.85, A | 1.6 |
| **106** | **15** | 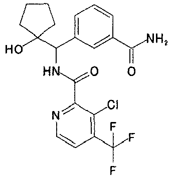 | N-[(3-carbamoylphenyl)(1-hydroxycyclopentyl)methyl]-3-chloro-4-(trifluoromethyl)pyridine-2-carboxamide | 442(M+H) | | | 0.78, A | 0.90 |

**[Table 1-19]**

| Compound No. | Example | Structure | Compound Name | (ESI pos.) m/z (ESI neg.) m/z | Optical isomer analysis conditions (Column used) (Solvent system) (Flow rate) | optical isomer analysis RT (min) | LCMS RT (min) condition | IC₅₀ (µM) |
|---|---|---|---|---|---|---|---|---|
| **107** | **3** | 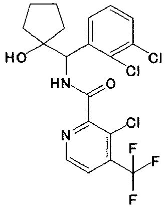 | 3-chloro-N-[(2,3-dichlorophenyl)(1-hydroxycyclopentyl)methyl]-4-(trifluoromethyl)pyridine-2-carboxamide | 467(M+H) | | | 1.16, A | 0.039 |
| **108** | **3** | 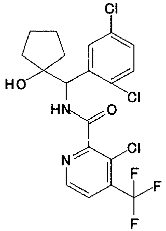 | 3-chloro-N-[(2,5-dichlorophenyl)(1-hydroxycyclopentyl)methyl]-4-(trifluoromethyl)pyridine-2-carboxamide | 467(M+H) | | | 1.17, A | 0.068 |
| **109** | **3** | 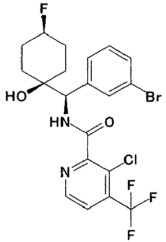 | N-[(R)-(3-bromophenyl)(cis-4-fluoro-1-hydroxycyclohexyl)methyl]-3-chloro-4-(trifluoromethyl)pyridine-2-carboxamide | 509(M+H) | AS, 4.6*250 | 4.60, 6.13 Faster | | 6.1 |
| | | | | | Hex : IPA = 80 : 20 | | | |
| | | | | | 1mL/min | | | |
| **110** | **3** | 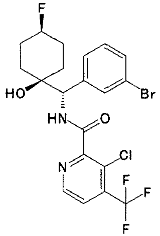 | N-[(S)-(3-bromophenyl)(cis-4-fluoro-1-hydroxycyclohexyl)methyl]-3-chloro-4-(trifluoromethyl)pyridine-2-carboxamide | 509(M+H) | AS, 4.6*250 | 4.60, 6.13 Later | 1.08, A | 0.095 |
| | | | | | Hex: IPA = 80 : 20 | | | |
| | | | | | 1mL/min | | | |
| **111** | **3** | 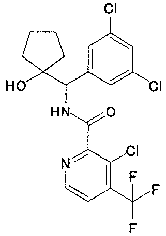 | 3-chloro-N-[(3,5-dichlorophenyl)(1-hydroxycyclopentyl)methyl]-4-(trifluoromethyl)pyridine-2-carboxamide | 467(M+H) | | | 1.19, A | 0.22 |

**[Table 1-20]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **112** | **14** | 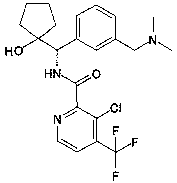 | 3-chloro-N-[{3-[(dimethylamino)methyl] phenyl}(1-hydroxycyclopentyl)methyl]-4-(trifluoromethyl)pyridine-2-carboxamide | 456(M+H) | | | 0.52, A | 1.8 |
| **113** | **3** | 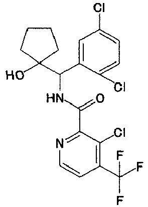 | (enantiomer2) 3-chloro-N-[(2,5-dichlorophenyl)(1-hydroxycyclopentyl)methyl]-4-(trifluoromethyl)pyridine-2-carboxamide | 467(M+H) | AD3, 4.6*150 | 3.91, 4.32 Faster | 1.17, A | 1.7 |
| | | | | | Hex : EtOH = 90 : 10 | | | |
| | | | | | 1mL/min | | | |
| **114** | **3** | 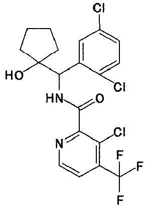 | (enantiomer 1) 3-chloro-N-[(2,5-dichlorophenyl)(1-hydroxycyclopentyl)methyl]-4-(trifluoromethyl)pyridine-2-carboxamide | 467(M+H) | AD3, 4.6*150 | 3.91, 4.32 Later | 1.17, A | 0.065 |
| | | | | | Hex : EtOH = 90 : 10 | | | |
| | | | | | 1mL/min | | | |
| **115** | **3** | 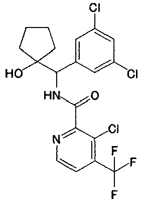 | (enantiomer 1) 3-chloro-N-[(3,5-dichlorophenyl)(1-hydroxycyclopentyl)methyl]-4-(trifluoromethyl)pyridine-2-carboxamide | 467(M+H) | AS, 4.6*250 | 3.68, 5.18 Later | 1.19, A | 0.059 |
| | | | | | Hex: EtOH = 80 : 20 | | | |
| | | | | | 1mL/min | | | |
| **116** | **3** | 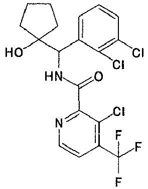 | (enantiomer 2) 3-chloro-N-[(2,3-dichlorophenyl)(1-hydroxycyclopentyl)methyl]-4-(trifluoromethyl)pyridine-2-carboxamide | 467(M+H) | IB3, 4.6*250 | 4.29, 5.13 Faster | 1.16, A | 6.0 |
| | | | | | Hex : IPA = 80 : 20 | | | |
| | | | | | 1mL/min | | | |
| **117** | **3** | 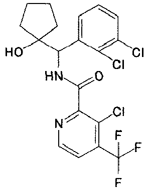 | (enantiomer 1) 3-chloro-N-[(2,3-dichlorophenyl)(1-hydroxycyclopentyl)methyl]-4-(trifluoromethyl)pyridine-2-carboxamide | 467(M+H) | IB3, 4.6*250 | 4.29, 5.13 Later | 1.16, A | 0.029 |
| | | | | | Hex : IPA = 80: 20 | | | |
| | | | | | 1mL/min | | | |

**[Table 1-21]**

| Compound No. | Example | Structure | Compound Name | (ESI pos.) m/z analysis (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **118** | **3** | 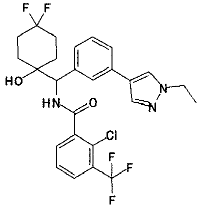 | 2-chloro-N-{(4,4-difluoro-1-hydroxycyclohexyl)[3-(1-ethyl-1H-pyrazol-4-yl)phenyl)methyl}-3-(trifluoromethyl)benzamide | 542(M+H) | | | 1.09, A | 0.26 |
| **119** | **3** | 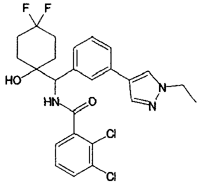 | 2,3-dichloro-N-{(4,4-difluoro-1-hydroxycyclohexyl)[3-(1-ethyl-1H-pyrazol-4-yl)phenyl]methyl}benzamide | 508(M+H) | | | 1.06, A | 0.47 |
| **120** | **3** | 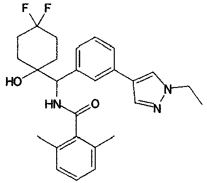 | N-{(4,4-difluoro-1-hydroxycyclohexyl)[3-(1 -ethyl-1H-pyrazol-4-yl)phenyl]methyl}-2,6-dimethylbenzamide | 468(M+H) | | | 0.96, A | 0.74 |
| **121** | **3** | 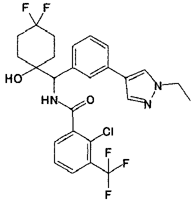 | (enantiomer 2)2-chloro-N-{(4,4-difluoro-1-hydroxycyclohexyl)[3-(1 -ethyl-1H-pyrazol-4-yl)phenyl]methyl}-3-(trifluoromethyl)benzamide | 542(M+H) | AD-H, 4.6*250 | 4.5,6.0 Faster | 1.03, A | 7.4 |
| | | | | | CO2 : EtOH = 70 : 30 | | | |
| | | | | | 5mL / min | | | |
| **122** | **3** | 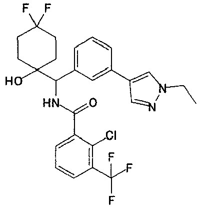 | (enantiomer 1) 2-chloro-N-{(4,4-difluoro-1-hydroxycyclohexyl)[3-(1-ethyl-1H-pyrazol-4-yl)phenyl]methyl}-3-(trifluoromethyl)benzamide | 542(M+H) | AD-H, 4.6*250 | 4.5,6.0 Later | 1.03, A | 0.22 |
| | | | | | CO2 : EtOH = 70 : 30 | | | |
| | | | | | 5mL/min | | | |
| **123** | **3** | 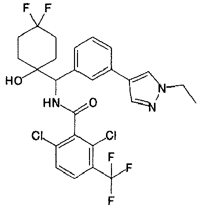 | 2,6-dichloro-N-{(4,4-difluoro-1-hydroxycyclohexyl)[3-(1-ethyl-1H-pyrazol-4-yl)phenyl]methyl}-3-(trifluoromethyl)benzamide | 576(M+H) | | | 1.06, A | 0.28 |

**[Table 1-22]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **124** | **3** | 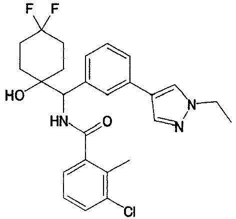 | 3-chloro-N-{(4,4-difluoro-1-hydroxycyclohexyl)[3-(1-ethyl-1H-pyrazol-4-yl)phenyl]methyl}-2-methylbenzamide | 488(M+H) | | | 1.03, A | 0.31 |
| **125** | **3** | 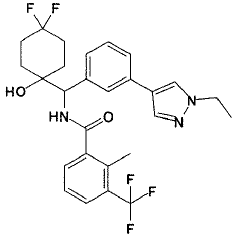 | N-{(4,4-difluoro-1-hydroxycyclohexyl)[3-(1-ethyl-1H-pyrazol-4-yl)phenyl]methyl}-2-methyl-3-(trifluoromethyl)benzamide | 522(M+H) | | | 1.05, A | 0.35 |
| **126** | **3** | 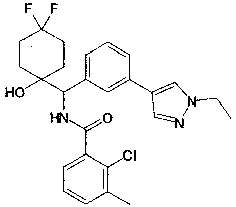 | 2-chloro-N-{(4,4-difluoro-1-hydroxycyclohexyl)[3-(1-ethyl-1H-pyrazol-4-yl)phenyl]methyl}-3-methylbenzamide | 488(M+H) | | | 0.99, A | 0.72 |
| **127** | **3** | 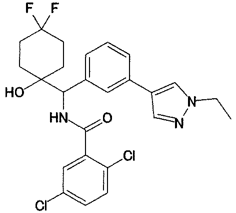 | 2,5-dichloro-N-{(4,4-difluoro-1-hydroxycyclohexyl)[3-(1 -ethyl-1H-pyrazol-4-yl)phenyl]methyl}benzamide | 508(M+H) | | | 1.03, A | 0.66 |
| **128** | **11** | 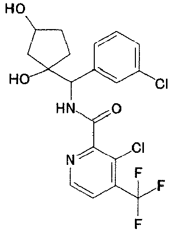 | 3-chloro-N-[(3-chlorophenyl)(1,3-dihydroxycyclopentyl)methyl]-4-(trifluoromethyl)pyridine-2-carboxamide | 449(M+H) | | | 0.90, A | 1.1 |

**[Table 1-23]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **129** | **3** | 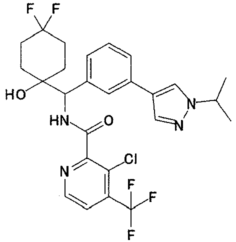 | 3-chloro-N-[(4,4-difluoro-1-hydroxycyclohexyl){3-[1-(propan-2-yl)-1H-pyrazol-4-yl]phenyl}methyl]-4-(trifluoromethyl)pyridine-2-carboxamide | 557(M+H) | | | 1.06, A | 0.099 |
| **130** | **12** | 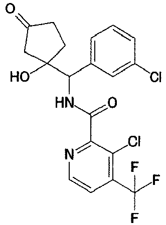 | 3-chloro-N-[(3-chlorophenyl)(1-hydroxy-3-oxocyclopentyl)methyl]-4-(trifluoromethyl)pyridine-2-carboxamide | 469(M+Na) | | | 0.93, A | 1.1 |
| **131** | **3** | 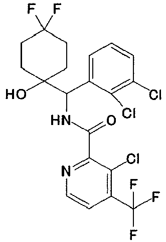 | 3-chloro-N-[(2,3-dichlorophenyl)(4,4-difluoro-1-hydroxycyclohexyl)methyl]-4-(trifluoromethyl)pyridine-2-carboxamide | 517(M+H) | | | 1.16, A | 0.16 |
| **132** | **3** | 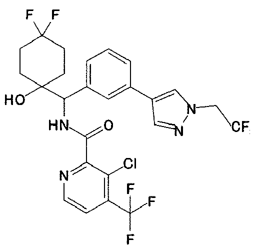 | 3-chloro-N-[(4,4-difluoro-1-hydroxycyclohexyl){3-[1-(2,2,2-trifluoroethyl)-1H-pyrazol-4-yl]phenyl}methyl]-4-(trifluoromethyl)pyridine-2-carboxamide | 597(M+H) | | | 1.06, A | 0.13 |
| **133** | **3** | 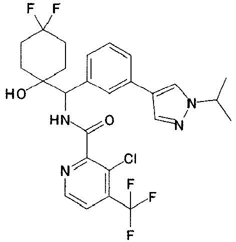 | (enantiomer 2) 3-chloro-N-[(4,4-difluoro-1-hydroxycyclohexyl){3-[1-(propan-2-yl)-1H-pyrazol-4-yl]phenyl}methyl]-4-(trifluoromethyl)pyridine-2-carboxamide | 557(M+H) | AD3, 4.6*150 | 3.45, 5.08 Faster | 1.06, A | 3.5 |
| | | | | | Hex : EtOH = 70 : 30 | | | |
| | | | | | 1mL/min | | | |

**[Table 1-24]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **134** | **3** | 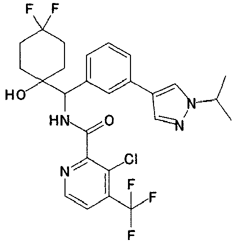 | (enantiomer 1) 3-chloro-N-[(4,4-difluoro-1-hydroxycyclohexyl){3-[1-(propan-2-yl)-1H-pyrazol-4-yl]phenyl}methyl]-4-(trifluoromethyl)pyridine-2-carboxamide | 557(M+H) | AD3, 4.6*150 | 3.45, 5.08 Later | 1.06, A | 0.031 |
| | | | | | Hex: EtOH = 70 : 30 | | | |
| | | | | | 1mL/min | | | |
| **135** | **3** | 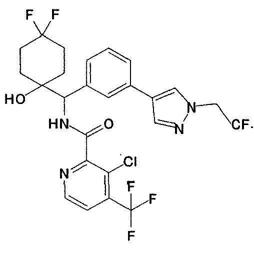 | (enantiomer 1) 3-chloro-N-[(4,4-difluoro-1-hydroxycyclohexyl){3-[1-(2,2,2-trifluoroethyl)-1 H-pyrazol-4-yl]phenyl}methyl]-4-(trifluoromethyl)pyridine-2-carboxamide | 597(M+H) | AD3, 4.6*150 | 4.29, 5.27 Later | 1.06, A | 0.072 |
| | | | | | Hex: EtOH = 70 : 30 | | | |
| | | | | | 1mL/min | | | |
| **136** | **3** | 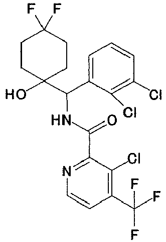 | (enantiomer 2) 3-chloro-N-[(2,3-dichlorophenyl)(4,4-difluoro-1-hydroxycyclohexyl)methyl]-4-(trifluoromethyl)pyridine-2-carboxamide | 517(M+H) | AD-3, 4.6*150 | 14.77, 17.26 Faster | | 3.7 |
| | | | | | Hex: EtOH = 95 : 5 | | | |
| | | | | | 1mL/min | | | |
| **137** | **3** | 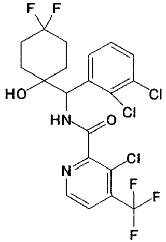 | (enantiomer 1) 3-chloro-N-[(2,3-dichlorophenyl)(4,4-difluoro-1-hydroxycyclohexyl)methyl]-4-(trifluoromethyl)pyridine-2-carboxamide | 517(M+H) | AD-3, 4.6*150 | 14.77, 17.26 Later | 1.16, A | 0.062 |
| | | | | | Hex: EtOH = 95: 5 | | | |
| | | | | | 1mL/min | | | |
| **138** | **5** | 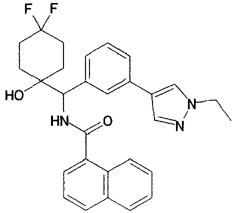 | N-{(4,4-difluoro-1-hydroxycyclohexyl)[3-(1 -ethyl-1H-pyrazol-4-yl)phenyl]methyl}naphthalene-1-carboxamide | 490(M+H) | | | 1.02, A | 0.84 |

**[Table 1-25]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **139** | **3** | 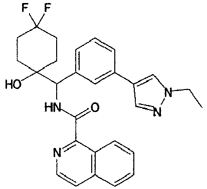 | N-((4,4-difluoro-1-hydroxycyclohexyl)[3-(1-ethyl-1H-pyrazol-4-yl)phenyl]methyl}isoquinoline-1-carboxamide | 491(M+H) | | | 1.05, A | 0.43 |
| **140** | **5** | 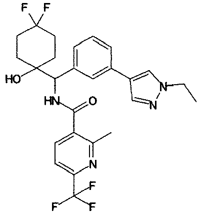 | N-{(4,4-difluoro-1-hydroxycyclohexyl)[3-(1 -ethyl-1H-pyrazol-4-yl)phenyl]methyl}-2-methyl-6-(trifluoromethyl)pyridine-3-carboxamide | 523(M+H) | | | 0.99, A | 3.3 |
| **141** | **3** | 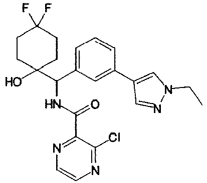 | 3-chloro-N-((4,4-difluoro-1-hydroxycyclohexyl)[3-(1-ethyl-1H-pyrazol-4-yl)phenyl]methyl}pyradine-2-carboxamide | 476(M+H) | | | 0.88, A | 3.1 |
| **142** | **3** | 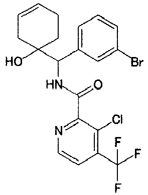 | N-[(3-bromophenyl)(1-hydroxycyclohex-3-en-1-yl)methyl]-3-chloro-4-(trifluoromethyl)pyridine-2-carboxamide | 489(M+H) | | | 1.13, A | 0.41 |
| **143** | **7** | 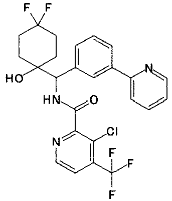 | 3-chloro-N-{(4,4-difluoro-1-hydroxycyclohexyl)[3-(pyridin-2-yl)phenyl]methyl}-4-(trifluoromethyl)pyridine-2-carboxamide | 526(M+H) | | | 0.92, A | 0.21 |
| **144** | **3** | 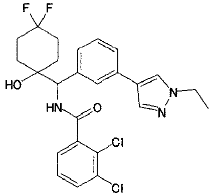 | (enantiomer 1) 2,3-dichloro-N-{(4,4-difluoro-1-hydroxycyclohexyl)[3-(1-ethyl-1H-pyrazol-4-yl)phenyl]methyl}benzamide | 508(M+H) | AD3, 4.6*150 | 3.17, 6.48 Faster | 1.02, A | 0.53 |
| | | | | | Hex : IPA = 50 : 50 | | | |
| | | | | | 1mL/min | | | |

**[Table 1-26]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **145** | **3** | 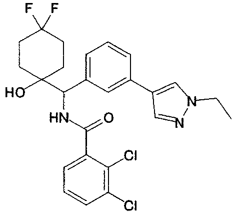 | (enantiomer 2) 2,3-dichloro-N-{(4,4-difluoro-1-hydroxycyclohexyl)[3-(1-ethyl-1H-pyrazol-4-yl)phenyl]methyl}benzamide | 508(M+H) | AD3, 4.6*150 | 3.17, 6.48 Later | | 0.67 |
| | | | | | Hex : IPA = 50 : 50 | | | |
| | | | | | 1mL/min | | | |
| **146** | **6** | 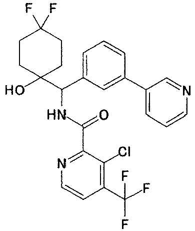 | 3-chloro-N-{(4,4-difluoro-1-hydroxycyclohexyl)[3-(pyridin-3-yl)phenyl]methyl}-4-(trifluoromethyl)pyridine-2-carboxamide | 526(M+H) | | | 0.77, A | 0.14 |
| **147** | **3** | 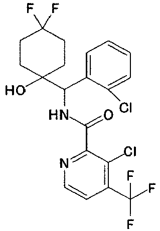 | (enantiomer 1) 3-chloro-N-[(2-chlorophenyl)(4,4-difluoro-1-hydroxycyclohexyl)methyl]-4-(trifluoromethyl)pyridine-2-carboxamide | 483(M+H) | IE+IE, 4.6*150*2 | 13.41, 14.04 Later | 1.11, A | 0.11 |
| | | | | | Hex: IPA = 70: 30 | | | |
| | | | | | 1mL/min | | | |
| **148** | **3** | 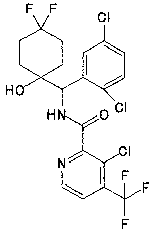 | 3-chloro-N-[(2,5-dichlorophenyl)(4,4-difluoro-1-hydroxycyclohexyl)methyl]-4-(trifluoromethyl)pyridine-2-carboxamide | 517(M+H) | | | 1.17, A | 0.26 |
| **149** | **3** | 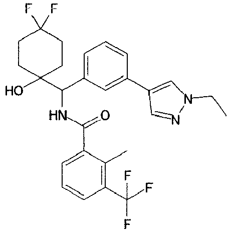 | (enantiomer 2) N-{(4,4-difluoro-1-hydroxycyclohexyl)[3-(1-ethyl-1H-pyrazol-4-yl)phenyl]methyl}-2-methyl-3-(trifluoromethyl)benzamide | 522(M+H) | AD3, 4.6*150 | 3.73, 6.76 Faster | 1.06, A | 2.6 |
| | | | | | Hex : EtOH = 80 : 20 | | | |
| | | | | | 1mL/min | | | |

**[Table 1-27]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **150** | **3** | 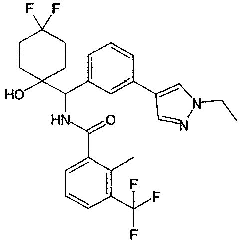 | (enantiomer 1) N-{(4,4-difluoro-1-hydroxycyclohexyl)[3-(1 -ethyl-1H-pyrazol-4-yl)phenyl]methyl}-2-methyl-3-(trifluoromethyl)benzamide | 522(M+H) | AD3, 4.6*150 | 3.73, 6.76 Later | 1.06, A | 0.84 |
| | | | | | Hex: EtOH = 80 : 20 | | | |
| | | | | | 1mL/min | | | |
| **151** | **3** | 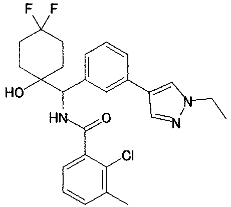 | (enantiomer 2) 2-chloro-N-{(4,4-difluoro-1-hydroxycyclohexyl)[3-(1 -ethyl-1H-pyrazol-4-yl)phenyl]methyl}-3-methylbenzamide | 488(M+H) | AD3, 4.6*150 | 5.09, 6.53 Faster | 1.00, A | 3.3 |
| | | | | | Hex: EtOH = 70 : 30 | | | |
| | | | | | 1mL/min | | | |
| **152** | **3** | 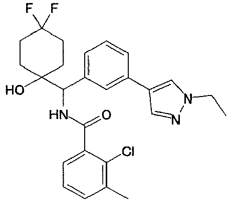 | (enantiomer 1) 2-chloro-N-{(4,4-difluoro-1-hydroxycyclohexyl)[3-(1 -ethyl-1H-pyrazol-4-yl)phenyl]methyl}-3-methylbenzamide | 488(M+H) | AD3, 4.6*150 | 5.09, 6.53 Later | 1.00, A | 1.3 |
| | | | | | Hex: EtOH = 70 : 30 | | | |
| | | | | | 1mL/min | | | |
| **153** | **2** | 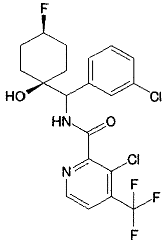 | 3-chloro-N-[(3-chlorophenyl)(cis-4-fluoro-1-hydroxycyclohexyl)methyl]-4-(trifluoromethyl)pyridine-2-carboxamide | 465(M+H) | | | 1.08, A | 0.098 |
| **154** | **4** | 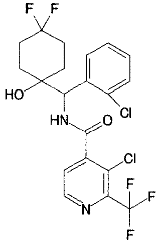 | (enantiomer 2) 3-chloro-N-[(2-chlorophenyl)(4,4-difluoro-1-hydroxycyclohexyl)methyl]-2-(trifluoromethyl)pyridine-4-carboxamide | 483(M+H) | | | 1.09, A | 0.50 |

**[Table 1-28]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **155** | **4** | 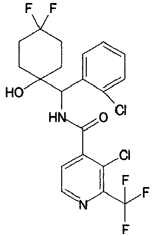 | (enantiomer 1) 3-chloro-N-[(2-chlorophenyl)(4,4-difluoro-1-hydroxycyclohexyl)methyl]-2-(trifluoromethyl)pyridine-4-carboxamide | 483(M+H) | AD3, 4.6*150 | 3.75, 4.39 Later | 1.09, A | 0.17 |
| | | | | | Hex : EtOH = 90 : 10 | | | |
| | | | | | 1mL/min | | | |
| **156** | **4** | 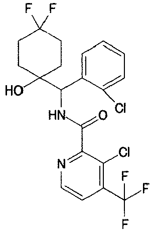 | (enantiomer 2) 3-chloro-N-[(2-chlorophenyl)(4,4-difluoro-1-hydroxycyclohexyl)methyl]-4-(trifluoromethyl)pyridine-2-carboxamide | 483(M+H) | | | 1.10, A | 1.8 |
| **157** | **3** | 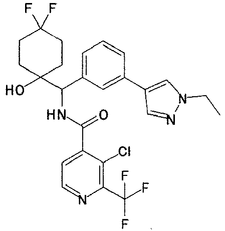 | 3-chloro-N-[(4,4-difluoro-1-hydroxycyclohexyl)[3-(1-ethyl-1H-pyrazol-4-yl)phenyl]methyl]-2-(trifluoromethyl)pyridine-4-carboxamide | 543(M+H) | | | 1.01, A | 0.16 |
| **158** | **2** | 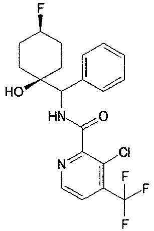 | 3-chloro-N-[(cis-4-fluoro-1-hydroxycyclohexyl)(phenyl) methyl]-4-(trifluoromethyl)pyridine-2-carboxamide | 431 (M+H) | | | 1.00, A | 0.35 |
| **159** | **9** | 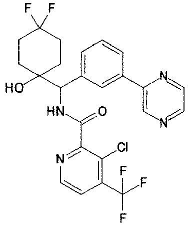 | 3-chloro-N-{(4,4-difluoro-1-hydroxycyclohexyl)[3-(pyrazin-2-yl)phenyl]methyl}-4-(trifluoromethyl)pyridine-2-carboxamide | 527(M+H) | | | 0.98, A | 0.32 |

**[Table 1-29]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **160** | **9** | 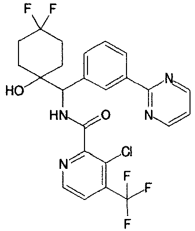 | 3-chloro-N-{(4,4-difluoro-1-hydroxycyclohexyl)[3-(pyrimidin-2-yl)phenyl]methyl}-4-(trifluoromethyl)pyridine-2-carboxamide | 527(M+H) | | | 1.01, A | 0.88 |
| **161** | **1,2** | 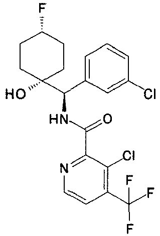 | 3-chloro-N-[(R)-(3-chlorophenyl)(cis-4-fluoro-1-hydroxycyclohexyl)methyl]-4-(trifluoromethyl)pyridine-2-carboxamide | 465(M+H) | AS, 4.6*250 | 5.37, 6.85 Faster | 1.07, A | 6.7 |
| | | | | | Hex : EtOH = 85 :15 | | | |
| | | | | | 1mL/min | | | |
| **162** | **1,2** | 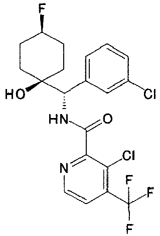 | 3-chloro-N-[(S)-(3-chlorophenyl)(cis-4-fluoro-1-hydroxycyclohexyl)methyl]-4-(trifluoromethyl)pyridine-2-carboxamide | 465(M+H) | AS, 4.6*250 | 5.37, 6.85 Later | 1.07, A | 0.065 |
| | | | | | Hex: EtOH = 85 : 15 | | | |
| | | | | | 1mL/min | | | |
| **163** | **2** | 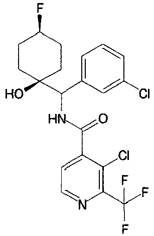 | 3-chloro-N-[(3-chlorophenyl)(cis-4-fluoro-1-hydroxycyclohexyl)methyl]-2-(trifluoromethyl)pyridine-4-carboxamide | 465(M+H) | | | 1.06, A | 0.39 |
| **164** | **4** | 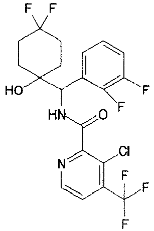 | (enantiomer 1) 3-chloro-N-[(4,4-difluoro-1-hydroxycyclohexyl)(2,3-difluorophenyl)methyl]-4-(trifluoromethyl)pyridine-2-carboxamide | 485(M+H) | | | 1.08, A | 0.12 |

**[Table 1-30]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **165** | **4** | 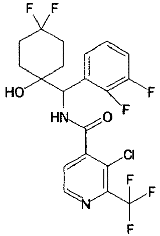 | (enantiomer 1) 3-chloro-N-[(4,4-difluoro-1-hydroxycyclohexyl)(2,3-difluorophenyl)methyl]-2-(trifluoromethyl)pyridine-4-carboxamide | 485(M+H) | | | 1.06, A | 0.21 |
| **166** | **4** | 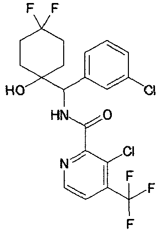 | (enantiomer 1) 3-chloro-N-[(3-chlorophenyl)(4,4-difluoro-1-hydroxycyclohexyl)methyl]-4-(trifluoromethyl)pyridine-2-carboxamide | 483(M+H) | | | 1.11, A | 0.18 |
| **167** | **4** | 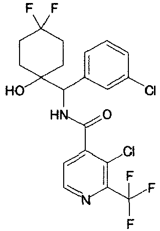 | (enantiomer 1) 3-chloro-N-[(3-chlorophenyl)(4,4-difluoro-1-hydroxycyclohexyl)methyl]-2-(trifluoromethyl)pyridine-4-carboxamide | 483(M+H) | | | 1.09, A | 0.21 |
| **168** | **4** | 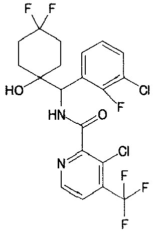 | (enantiomer 1) 3-chloro-N-[(3-chloro-2-fluorophenyl)(4,4-difluoro-1-hydroxycyclohexyl)methyl]-4-(trifluoromethyl)pyridine-2-carboxamide | 501(M+H) | | | 1.12, A | 0.057 |
| **169** | **4** | 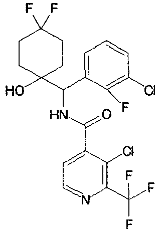 | (enantiomer 1) 3-chloro-N-[(3-chloro-2-fluorophenyl)(4,4-difluoro-1-hydroxycyclohexyl)methyl]-2-(trifluoromethyl)pyridine-4-carboxamide | 501(M+H) | | | 1.11, A | 0.095 |

**[Table 1-31]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **170** | **4** | 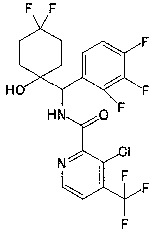 | 3-chloro-N-[(4,4-difluoro-1-hydroxycyclohexyl)(2,3,4-trifluorophenyl)methyl]-4-(trifluoromethyl)pyridine-2-carboxamide | 503(M+H) | | | 1.11, A | 0.63 |
| **171** | **4** | 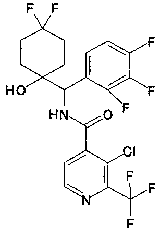 | 3-chloro-N-[(4,4-difluoro-1-hydroxycyclohexyl)(2,3,4-trifluorophenyl)methyl]-2-(trifluoromethyl)pyridine-4-carboxamide | 503(M+H) | | | 1.09, A | 1.1 |
| **172** | **3** | 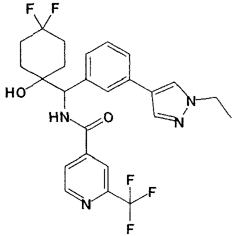 | N-{(4,4-difluoro-1-hydroxycyclohexyl)[3-{1-ethyl-1H-pyrazol-4-yl)phenyl]methyl}-2-(trifluoromethyl)pyridine-4-carboxamide | 509(M+H) | | | 0.99, A | 5.0 |
| **173** | **3** | 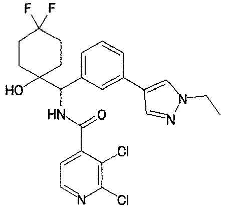 | 2,3-dichloro-N-{(4,4-difluoro-1-hydroxycyclohexyl)[3-(1 -ethyl-1H-pyrazol-4-yl)phenyl]methyl}pyridine-4-carboxamide | 509(M+H) | | | 0.95, A | 1.0 |
| **174** | **3** | 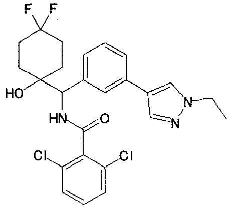 | 2,6-dichloro-N-{(4,4-difluoro-1-hydroxycyclohexyl)[3-(1-ethyl-1H-pyrazol-4-yl)phenyl]methyl}benzamide | 508(M+H) | | | 0.96, A | 1.1 |

**[Table 1-32]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **175** | **3** | 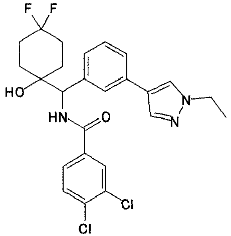 | 3,4-dichloro-N-{(4,4-difluoro-1-hydroxycyclohexyl)[3-(1-ethyl-1H-pyrazol-4-yl)phenyl]methyl}benzamide | 508(M+H) | | | 1.10, A | 4.0 |
| **176** | **3** | 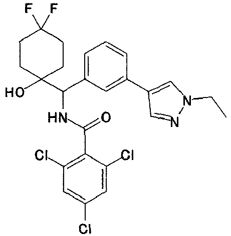 | 2,4,6-trichloro-N-{(4,4-difluoro-1-hydroxycyclohexyl)[3-(1-ethyl-1H-pyrazol-4-yl)phenyl]methyl}benzamide | 542(M+H) | | | 1.06, A | 1.0 |
| **177** | **4** | 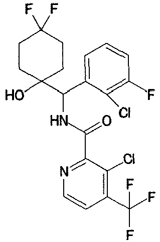 | (enantiomer 1) 3-chloro-N-[(2-chloro-3-fluorophenyl)(4,4-difluoro-1-hydroxycyclohexyl)methyl]-4-(trifluoromethyl)pyridine-2-carboxamide | 501(M+H) | | | 1.12, A | 0.10 |
| **178** | **4** | 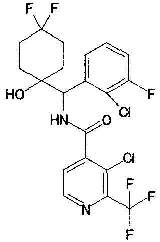 | (enantiomer 1) 3-chloro-N-[(2-chloro-3-fluorophenyl)(4,4-difluoro-1-hydroxycyclohexyl)methyl]-2-(trifluoromethyl)pyridine-4-carboxamide | 501(M+H) | | | 1.10, A | 0.13 |
| **179** | **3** | 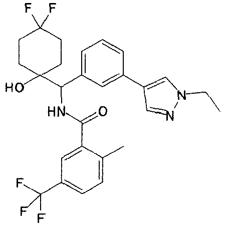 | N-{(4,4-difluoro-1-hydroxycyclohexyl)[3-(1-ethyl-1H-pyrazol-4-yl)phenyl]methyl}-2-methyl-5-(trifluoromethyl)benzamide | 522(M+H) | | | 1.06, A | 0.61 |

**[Table 1-33]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **180** | **3** | 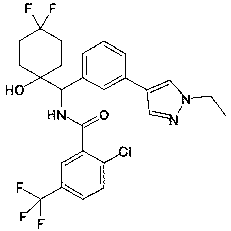 | 2-chloro-N-{(4,4-difluoro-1-hydroxycyclohexyl)[3-(1-ethyl-1H-pyrazol-4-yl)phenyl]methyl}-5-(trifluoromethyl)benzamide | 542(M+H) | | | 1.06, A | 0.21 |
| **181** | **3** | 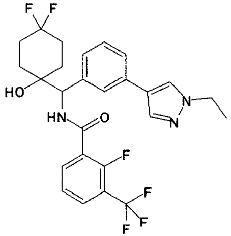 | N-[(4,4-difluoro-1-hydroxycyclohexyl)[3-(1-ethyl-1H-pyrazol-4-yl)phenyl]methyl}-2-fluoro-3-(trifluoromethyl)benzamide | 526(M+H) | | | 1.06, A | 1.5 |
| **182** | **3** | 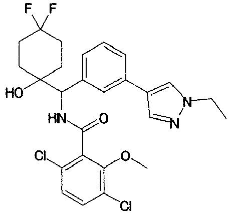 | 3,6-dichloro-N-[(4,4-difluoro-1-hydroxycyclohexyl)[3-(1-ethyl-1H-pyrazol-4-yl)phenyl]methyl}-2-methoxybenzamide | 538(M+H) | | | 1.02, A | 0.58 |
| **183** | **3** | 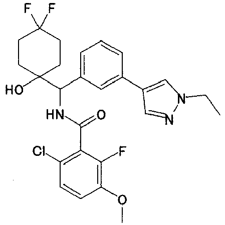 | 6-chloro-N-{(4,4-difluoro-1-hydroxycyclohexyl)[3-(1-ethyl-1H-pyrazol-4-yl)phenyl]methyl}-2-fluoro-3-methoxybenzamide | 522(M+H) | | | 0.96, A | 2.0 |
| **184** | **3** | 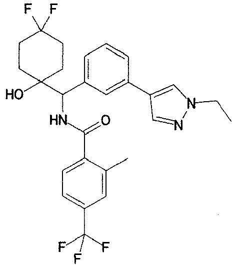 | N-{(4,4-difluoro-1-hydroxycyclohexyl)[3-(1-ethyl-1H-pyrazol-4-yl)phenyl]methyl}2-methyl-4-(trifluoromethyl)benzamide | 522(M+H) | | | 1.07, A | 0.86 |

**[Table 1-34]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **185** | **4** | 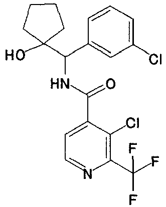 | (enantiomer 1) 3-chloro-N-[(3-chlorophenyl)(1-hydroxycyclopentyl)methyl]-2-(trifluoromethyl)pyridine-4-carboxamide | 433(M+H) | | | 1.08, A | 0.14 |
| **186** | **1** | 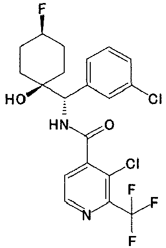 | 3-chloro-N-[(S)-(3-chlorophenyl)(cis-4-fluoro-1-hydroxycyclohexyl)methyl]-2-(trifluoromethyl)pyridine-4-carboxamide | 465(M+H) | | | 1.07, A | 0.15 |
| **187** | **1** | 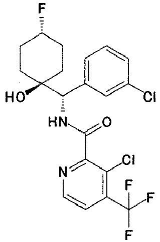 | 3-chloro-N-[(S)-(3-chlorophenyl)(trans-4-fluoro-1-hydroxycyclohexyl)methyl]-4-(trifluoromethyl)pyridine-2-carboxamide | 465(M+H) | | | 1.08, A | 0.21 |
| **188** | **3** | 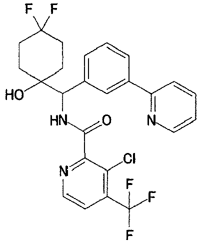 | (enantiomer 1) 3-chloro-N-{(4,4-difluoro-1-hydroxycyclohexyl)[3-(pyridin-2-yl)phenyl]methyl]-4-(trifluoromethyl)pyridine-2-carboxamide | 526(M+H) | | | 0.92, A | 0.073 |
| **189** | **4** | 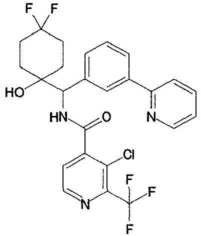 | (enantiomer 1) 3-chloro-N-{(4,4-difluoro-1-hydroxycyclohexyl)[3-(pyridin-2-yl)phenyl]methyl}-2-(trifluoromethyl)pyridine-4-carboxamide | 526(M+H) | | | 0.91, A | 0.20 |

**[Table 1-35]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **190** | **3** | 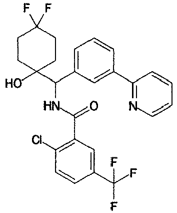 | (enantiomer 1) 2-chloro-N-[(4,4-difluoro-1-hydroxycyclohexyl)[3-(pyridin-2-yl)phenyl]methyl}-5-(trifluoromethyl)benzamide | 525(M+H) | | | 0.95, A | 0.49 |
| **191** | **5** | 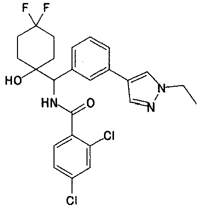 | 2,4-dichloro-N-{(4,4-difluoro-1-hydroxycyclohexyl)[3-(1-ethyl-1H-pyrazol-4-yl)phenyl]methyl}benzamide | 508(M+H) | | | 1.03, A | 1.3 |
| **192** | **5** | 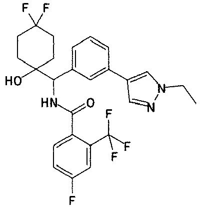 | N-{(4,4-difluoro-1-hydroxycyclohexyl)[3-(1-ethyl-1H-pyrazol-4-yl)phenyl]methyl}-4-fluoro-2-(trifluoromethyl)benzamide | 526(M+H) | | | 1.00, A | 0.34 |
| **193** | **3** | 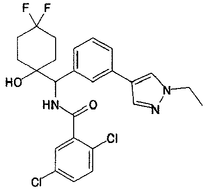 | (enantiomer 2) 2,5-dichloro-N-{(4,4-difluoro-1-hydroxycyclohexyl)[3-(1-ethyl-1H-pyrazol-4-yl)phenyl]methyl}benzamide | 508(M+H) | AD3, 4.6*150 | 2.35, 2.90 Faster | 1.03, A | 0.83 |
| | | | | | Hex : EtOH = 90 : 10 | | | |
| | | | | | 1mL/min | | | |
| **194** | **3** | 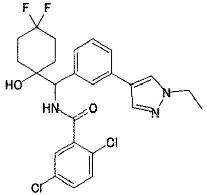 | (enantiomer 1) 2,5-dichloro-N-{(4,4-difluoro-1-hydroxycyclohexyl)[3-(1-ethyl-1H-pyrazol-4-yl)phenyl]methyl}benzamide | 508(M+H) | AD3, 4.6*150 | 2.35, 2.90 Later | 1.03, A | 0.76 |
| | | | | | Hex: EtOH = 90 : 10 | | | |
| | | | | | 1mL/min | | | |
| **195** | **5** | 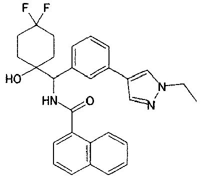 | (enantiomer 2) N-{(4,4-difluoro-1-hydroxycyclohexyl)[3-(1-ethyl-1H-pyrazol-4-yl)phenyl]methyl}naphthalene-1-carboxamide | 490(M+H) | AD3, 4.6*150 | 2.40, 3.46 Faster | 1.02, A | 1.1 |
| | | | | | Hex : EtOH = 10 90 | | | |
| | | | | | 1mL/min | | | |

**[Table 1-36]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **196** | **5** | 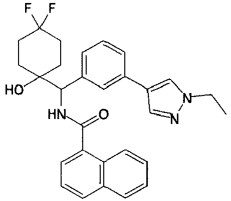 | (enantiomer 1) N-{(4,4-difluoro-1-hydroxycyclohexyl)[3-(1-ethyl-1H-pyrazol-4-yl)phenyl]methyl}naphthalene-1-carboxamide | 490(M+H) | AD3, 4.6*150 | 2.40, 3.46 Later | 1.02, A | 0.29 |
| | | | | | Hex : EtOH =10 : 90 | | | |
| | | | | | 1mL/min | | | |
| **197** | **2** | 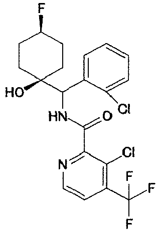 | (enantiomer 1) 3-chloro-N-[(2-chlorophenyl)(cis-4-fluoro-1-hydroxycyclohexyl)methyl]-4-(trifluoromethyl)pyridine-2-carboxamide | 465(M+H) | | | 1.07, A | 0.053 |
| **198** | **2** | 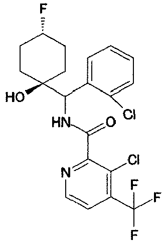 | (enantiomer 1) 3-chloro-N-[(2-chlorophenyl)(trans-4-fluoro-1-hydroxycyclohexyl)methyl]-4-(trifluoromethyl)pyridine-2-carboxamide | 465(M+H) | | | 1.06, A | 0.20 |
| **199** | **2** | 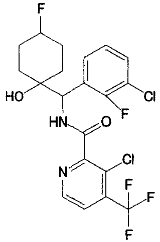 | (diastereomer 2, enantiomer 1) 3-chloro-N-[(3-chloro-2-fluorophenyl)(4-fluoro-1-hydroxycyclohexyl)methyl]-4-(trifluoromethyl)pyridine-2-carboxamide | 483(M+H) | | | 1.09, A | 0.082 |
| **200** | **2** | 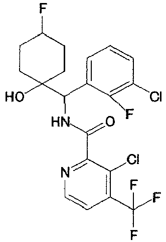 | (diastereomer 1, enantiomer 1) 3-chloro-N-[(3-chloro-2-fluorophenyl)(4-fluoro-1-hydroxycyclohexyl)methyl}-4-(trifluoromethyl)pyridine-2-carboxamide | 483(M+H) | | | 1.10, A | 0.064 |

**[Table 1-37]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **201** | **2** | 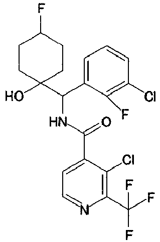 | (diastereomer 1, enantiomer 1) 3-chloro-N-[(3-chloro-2-fluorophenyl)(4-fluoro-1-hydroxycyclohexyl)methyl]-2-(trifluoromethyl)pyridine-4-carboxamide | 483(M+H) | | | 1.08, A | 0.13 |
| **202** | **4** | 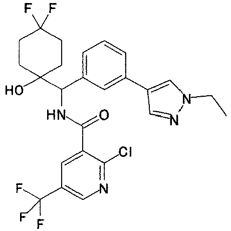 | 2-chloro-N-{(4,4-difluoro-1-hydroxycyclohexyl)[3-(1-ethyl-1H-pyrazol-4-yl)phenyl]methyl}-5-(trifluoromethyl)pyridine-3-carboxamide | 543(M+H) | | | 1.00, A | 0.90 |
| **203** | **3** | 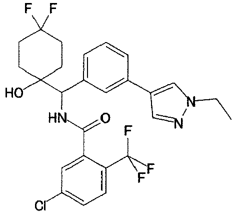 | 5-chloro-N-{(4,4-difluoro-1-hydroxycyclohexyl)[3-(1--ethyl-1H-pyrazol-4-yl)phenyl]methyl}-2-(trifluoromethyl)benzamide | 542(M+H) | | | 1.05, A | 0.73 |
| **204** | **2** | 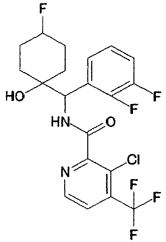 | (diastereomer 1, enantiomer 1) 3-chloro-N-[(2,3-difluorophenyl)(4-fluoro-1-hydroxycyclohexyl)methyl]-4-(trifluoromethyl)pyridine-2-carboxamide | 467(M+H) | | | 1.04, A | 0.10 |
| **205** | **3** | 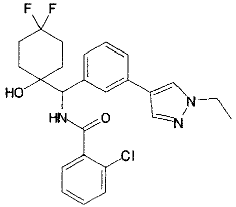 | 2-chloro-N-[(4,4-difluoro-1-hydroxycyclohexyl)[3-(1-ethyl-1H-pyrazol-4-yl)phenyl]methyl}benzamide | 474(M+H) | | | 0.94, A | 1.9 |

**[Table 1-38]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **206** | **4** | 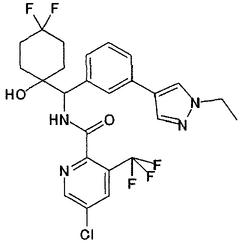 | 5-chloro-N-{(4,4-difluoro-1-hydroxycyclohexyl)[3-(1-ethyl-1H-pyrazol-4-yl)phenyl]methyl}-3-(trifluoromethyl)pyridine-2-carboxamide | 543(M+H) | | | 1.04, A | 8.7 |
| **207** | **3** | 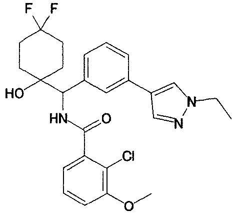 | 2-chloro-N-{(4,4-difluoro-1-hydroxycyclohexyl)[3-(1-ethyl-1H-pyrazol-4-yl)phenyl]methyl}-3-methoxybenzamide | 504(M+H) | | | 0.93, A | 8.8 |
| **208** | **3** | 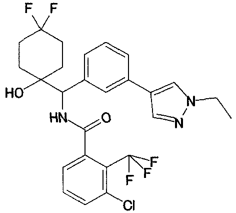 | 3-chloro-N-{(4,4-difluoro-1-hydroxycyclohexyl)[3-(1-ethyl-1H-pyrazol-4-yl)phenyl]methyl}-2-(trifluoromethyl)benzamide | 542(M+H) | | | 1.02, A | 1.3 |
| **209** | **3** | 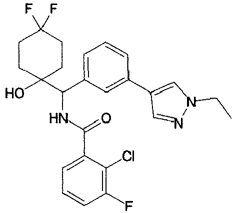 | 2-chloro-N-{(4,4-difluoro-1-hydroxycyclohexyl)[3-(1-ethyl-1H-pyrazol-4-yl)phenyl]methyl}-3-fluorobenzamide | 492(M+H) | | | 0.97, A | 1.2 |
| **210** | **2** | 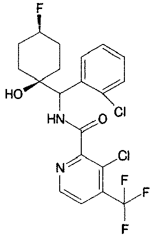 | (enantiomer 2) 3-chloro-N-[(2-chlorophenyl)(cis-4-fluoro-1-hydroxycyclohexyl)methyl]-4-(trifluoromethyl)pyridine-2-carboxamide | 465(M+H) | OD3, 4.6*250 | 19.2, 30.1 Faster | 1.07, A | 3.2 |
| | | | | | Hex: IPA = 94 : 6 | | | |
| | | | | | 1mL/min | | | |

**[Table 1-39]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **211** | **1** | 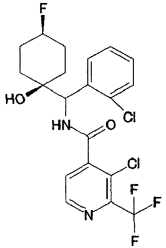 | (enantiomer 1) 3-chloro-N-[(2-chlorophenyl)(cis-4-fluoro-1-hydroxycyclohexyl)methyl]-2-(trifluoromethyl)pyridine-4-carboxamide | 463(M-H) | | | 1.06, A | 0.17 |
| **212** | **1** | 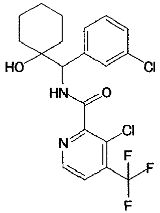 | 3-chloro-N-[(3-chlorophenyl)(1-hydroxycyclohexyl)methyl]-4-(trifluoromethyl)pyridine-2-carboxamide | | | | | 0,084 |
| **213** | **1** | 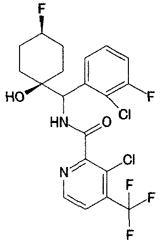 | (enantiomer 1) 3chloro-N-[(2-chloro-3-fluorophenyl)(cis-4-fluoro-1-hydroxycyclohexyl)methyl]-4-(trifluoromethyl)pyridine-2-carboxamide | 483(M+H) | IB3, 4.6*250 | 3.72, 4.21 Later | 1,10, A | 0.037 |
| | | | | | Hex : EtOH = 85 : 15 | | | |
| | | | | | 1mL/min | | | |
| **214** | **1** | 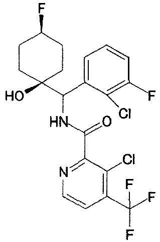 | (enantiomer 2) 3-chloro-N-[(2-chloro-3-fluorophenyl)(cis-4-fluoro-1-hydroxycyclohexyl)methyl]-4-(trifluoromethyl)pyridine-2-carboxamide | | IB3, 4.6*250 | 3.72, 4.21 Faster | | 0.45 |
| | | | | | Hex: EtOH = 85 : 15 | | | |
| | | | | | 1mL/min | | | |
| **215** | **5** | 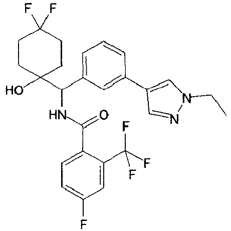 | (enantiomer 1) N-{(4,4-difluoro-1-hydroxycyclohexyl)[3-(1-ethyl-1H-pyrazol-4-yl)phenyl]methyl}-4-fluoro-2-(trifluoromethyl)benzamide | 526(M+H) | IA3, 4.6*150 Hex : IPA = | 2.60, 4.28 Faster | 1.01, A | 0.21 |
| | | | | | 50: 50 | | | |
| | | | | | 1mL/min | | | |

**[Table 1-40]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **216** | **5** | 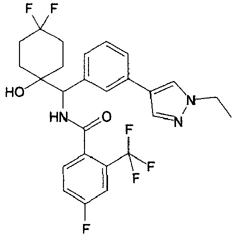 | (enantiomer 2) N-{(4,4-difluoro-1-hydroxycyclohexyl)[3-(1-ethyl-1H-pyrazol-4-yl)phenyl]methyl}-4-fluoro-2-(trifluoromethyl)benzamide | 526(M+H) | IA3, 4.6*150 | 2.60, 4.28 Later | 1.01, A | 5.5 |
| | | | | | Hex : IPA = 50: 50 | | | |
| | | | | | 1mL/min | | | |
| **217** | **3** | 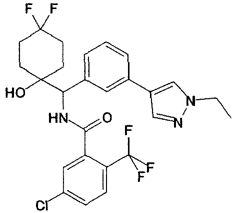 | (enantiomer 1) 5-chloro-N-{(4,4-difluoro-1-hydroxycyclohexyl)[3-(1-ethyl-1H-pyrazol-4-yl)phenyl]methyl}-2-(trifluoromethyl)benzamide | 542(M+H) | IA3, 4.6*150 | 3.13, 5.19 Faster | 1.07, A | 0.58 |
| | | | | | Hex: IPA = 50 : 50 | | | |
| | | | | | 1mL/min | | | |
| **218** | **3** | 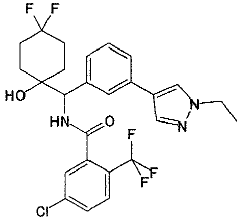 | (enantiomer 2) 5-chloro-N-[(4,4-difluoro-1-hydroxycyclohexyl)[3-(1-ethyl-1H-pyrazol-4-yl)phenyl]methyl}-2-(trifluoromethyl)benzamide | 542(M+H) | IA3, 4.6*150 | 3.13, 5.19 Later | 1.07, A | 5.9 |
| | | | | | Hex : IPA = 50 : 50 | | | |
| | | | | | 1mL/min | | | |
| **219** | **1** | 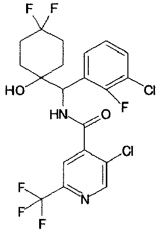 | (enantiomer 1) 5-chloro-N-[(3-chloro-2-fluorophenyl)(4,4-difluoro-1-hydroxycyclohexyl)methyl]-2-(trifluoromethyl)pyridine-4-carboxamide | 499(M-H) | | | 1.15, A | 0.92 |
| **220** | **2** | 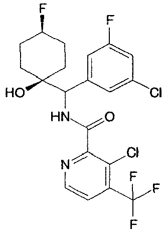 | (enantiomer 1) 3-chloro-N-[(3-chloro-5-fluorophenyl)(cis-4-fluoro-1-hydroxycyclohexyl)methyl]-4-(trifluoromethyl)pyridine-2-carboxamide | 483(M+H) | | | 1.12, A | 1.8 |

**[Table 1-41]**

| Compound No. | Example | Structure | Compound Name | (ESI pos.) m/z ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **221** | **3** | 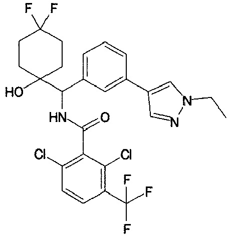 | (enantiomer 2) 2,6-dichloro-N-{(4,4-difluoro-1-hydroxycyclohexyl)[3-(1-ethyl-1H-pyrazol-4-yl)phenyl]methyl}-3-(trifluoromethyl)benzamide | 576(M+H) | AS3, 4.6*250 | 5.59, 7.85 Faster | 1.08, A | 5.6 |
| | | | | | Hex : EtOH = 85 : 15 | | | |
| | | | | | 1mL/min | | | |
| **222** | **3** | 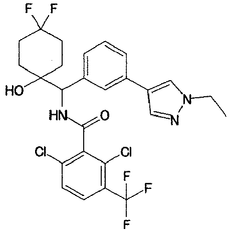 | (enantiomer 1) 2,6-dichloro-N-{(4,4-difluoro-1-hydroxycyclohexyl)[3-(1-ethyl-1H-pyrazol-4-yl)phenyl]methyl}-3-(trifluoromethyl)benzamide | 576(M+H) | AS3, 4.6*250 | 5.59, 7.85 Later | 1.08, A | 0.19 |
| | | | | | Hex: EtOH = 85 : 15 | | | |
| | | | | | 1mL/min | | | |
| **223** | **1** | 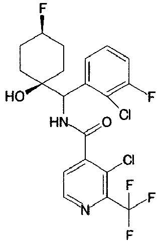 | (enantiomer 1) 3-chloro-N-[(2-chloro-3-fluorophenyl)(cis-4-fluoro-1-hydroxycyclohexyl)methyl]-2-(trifluoromethyl)pyridine-4-carboxamide | 483(M+H) | | | 1.09, A | 0.056 |
| **224** | **1** | 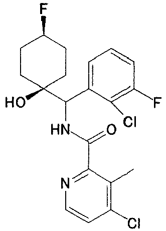 | (enantiomer 1) 4-chloro-N-[(2-chloro-3-fluorophenyl)(cis-4-fluoro-1-hydroxycyclohexyl)methyl]-3-methylpyridine-2-carboxamide | 429(M+H) | | | 1.15, A | 0.27 |
| **225** | **1** | 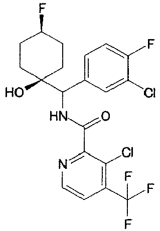 | (enantiomer 1) 3-chloro-N-[(3-chloro-4-fluorophenyl)(cis-4-fluoro-1-hydroxycyclohexyl)methyl]-4-(trifluoromethyl)pyridine-2-carboxamide | | | | | 0.39 |

**[Table 1-42]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **226** | **1** | 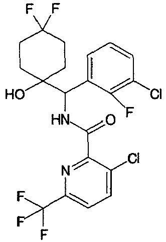 (enantiomer | (enantiomer 1) 3-chloro-N-[(3-chloro-2-fluorophenyl)(4,4-difluoro-1-hydroxycyclohexyl)methyl]-6-(trifluoromethyl)pyridine-2-carboxamide | | | | | 1.3 |
| **227** | **1** | 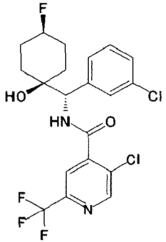 | 5-chloro-N-[(S)-(3-chlorophenyl)(cis-4-fluoro-1-hydroxycyclohexyl)methyl]-2-(trifluoromethyl)pyridine-4-carboxamide | | | | | 0.51 |

**[Table 1-43]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **228** | **1** | 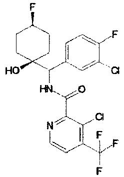 | (enantiomer 2) 3-chloro-N-[(3-chloro-4-fluorophenyl)(cis-4-fluoro-1-hydroxycyclohexyl)methyl]-4-(trifluoromethyl)pyridine-2-carboxamide | 483(M+H) | | | 1.11, A | 4.2 |
| **229** | **1** | 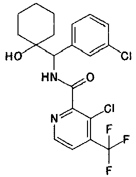 | (enantiomer 1) 3-chloro-N-[(3 chlorophenyl)(1-hydroxycyclohexyl)methyl]-4-(trifluoromethyl)pyridine-2-carboxamide | 447(M+H) | AD3. 4.6*250 | 4.83, 6.11 Faster | 1.20, A | 0.028 |
| | | | | | Hex : IPA = 85 : 15 | | | |
| | | | | | 1 mL / min | | | |
| **230** | **1** | 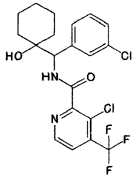 | (enantiomer 2) 3-chloro-N-[(3-chlorophenyl)(1-hydroxycyclohexyl)methyl]-4-(trifluoromethyl)pyridine-2-carboxamide | 447(M+H) | AD3, 4.6*250 | 4.83, 6.11 Later | 1.20, A | 2.8 |
| | | | | | Hex : IPA = 85 : 15 | | | |
| | | | | | 1mL/min | | | |
| **231** | **18** | 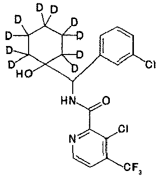 | (enantiomer 1) 3-chloro-N-{(3-chlorophenyl)[1-hydroxy(²H₁₀)cyclohexyl] methyl}-4-(trifluoromethyl)pyridine-2-carboxamide | 457(M+H) | AD3, 4.6*250 | 4.84, 6.23 Faster | 1.19, A | 0.040 |
| | | | | | Hex : IPA = 85 : 15 | | | |
| | | | | | 1 mL/min | | | |
| **232** | **18** | 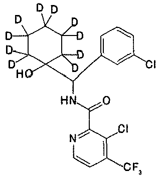 | (enantiomer 2) 3-chloro-N-{(3-chlorophenyl)[1-hydroxy(²H₁₀)cyclohexyl] methyl}-4-(trifluoromethyl)pyridine-2-carboxamide | 457(M+H) | AD3, 4.6*250 | 4.84, 6.23 Later | 1.19,A | 1.4 |
| | | | | | Hex : IPA = 85 : 15 | | | |
| | | | | | 1 mL / min | | | |
| **233** | **4** | 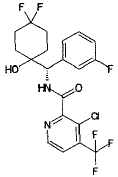 | (enantiomer 1) 3-chloro-N-[(4,4-difluoro-1-hydroxycyclohexyl)(3-fluorophenyl)methyl]-4-(trifluoromethyl)pyridine-2-carboxamide | 467(M+H) | | | 1.08. A | 0.12 |
| **234** | **3** | 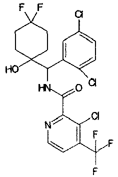 | (enantiomer 1) 3-chloro-N-[(2,5-dichlorophenyl)(4,4-difluoro-1-hydroxycyclohexyl)methyl]-4-(trifluoromethyl)pyridine-2-carboxamide | 517(M+H) | AS3, 46*250 | 13.21, 18.92 Faster | 1.20, A | 0.28 |
| | | | | | Hex : IPA = 92 : 8 | | | |
| | | | | | 1mL/min | | | |

**[Table 1-44]**

| Compound No. | Example | Structure | Compound Name | (ESI pos.) m/z (ESI neg.) m/z | Optical isomer analysis conditions (Column used (Solvent system) (Flow rate) | Optical isomer analysis RT (min) | LCMS RT (min) condition | IC₅₀ (µM) |
|---|---|---|---|---|---|---|---|---|
| **235** | **3** | 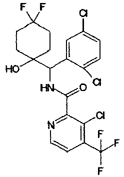 | (enantiomer 2) 3-chloro-N-[(2.5-dichlorophenyl)(4,4-difluoro-1-hydroxycyclohexyl)methyl]-4-(trifluoromethyl)pyridine-2-carboxamide | 517(M+H) | AS3, 4.6*250 | 13.21, 18.92 Later | 1.20, A | 0.74 |
| | | | | | Hex :IPA = 92 : 8 | | | |
| | | | | | 1 mL / min | | | |
| **236** | **1** | 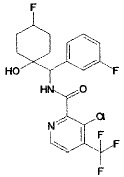 | (diastereomer 1, enantiomer 1) 3-chloro-N-[(cis-4-fluoro-1-hydroxycyclohexyl)(3-fluorophenyl)methyl]-4-(trifluoromethyl)pyridine-2-carboxamide | | | | | 0.10 |
| **237** | **4** | 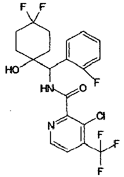 | (enantiomer 1) 3-chloro-N-[4,4-difluoro-1-hydroxycyclohexyl)(2-fluorophenyl)methyl]-4-(trifluoromethyl)pyridine-2-carboxamide | 467(M+H) | | | 1.10, A | 0.25 |
| **238** | **1** | 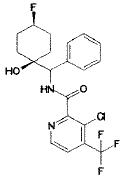 | (enantiomer 1) 3-chloro-N-[(cis-4-fluoro-1-hydroxycyclohexyl)(phenyl) methyl]-4-(trifluoromethyl) pyridine-2-carboxamide | 431 (M+H) | | | 1.01, A | 0.22 |
| **239** | **1** | 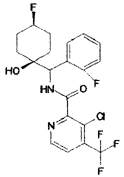 | (enantiomer 1) 3chloro-N-[(cis-4-fluoro-1-hydroxycyclohexyl)(2-fluorophenyl)methyl]-4-(trifluoromethyl)pyridine-2-carboxamide | 449(M+H) | | | 1.06, A | 0.39 |
| **240** | **1** | 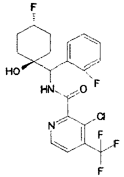 | (enantiomer 1) 3-chloro-N-[(trans-4-fluoro-1-hydroxycyclohexyl)(2-fluorophenyl)methyl]-4-(trifluoromethyl)pyridine-2-carboxamide | 449(M+H) | | | 1.04, A | 0.23 |
| **241** | **1** | 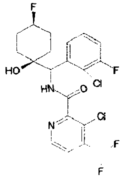 | (enantiomer 1) 3-chloro-N-[(2-chloro-3-fluorophenyl)(cis-4-fluoro-1-hydroxycyclohexyl)methyl]-4-(difluoromethyl)pyridine-2-carboxamide | 465(M+H) | | | 1.06, A | 0.11 |

**[Table 1-45]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **242** | **17** | 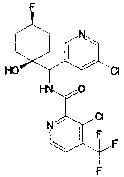 | (enantiomer 1) 3-chloro-N-[(5-chloropyridin-3-yl)(cis-4-fluoro-1-hydroxycyclohexyl)methyl]-4-(trifluoromethyl)pyridine-2-carboxamide | 466(M+H) | | | 0.96, A | 0.24 |
| **243** | **17** | 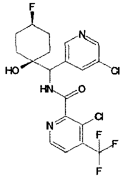 | (enantiomer 2) 3-chloro-N-[(5-chloropyridin-3-yl)(cis-4-fluoro-1-hydroxycyclohexyl)methyl]-4-(trifluoromethyl)pyridine-2-carboxamide | 466(M+H) | | | 0.96, A | 1.5 |
| **244** | **1** | 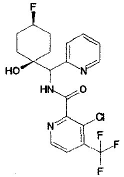 | (enantiomer 1) 3-chloro-N-[(cis-4-fluoro-1-hydroxycyclohexyl)(pyridin-2-yl)methyl]-4-(trifluoromethyl)pyridine-2-carboxamide | 432(M+H) | | | 0.83, A | 1.0 |
| **245** | **1** | 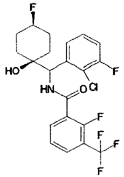 | (enantiomer 1) N-[(2-chloro-3-fluorophenyl)(cis-4-fluoro-1-hydroxycyclohexyl)methyl]-2-fluoro-3-(trifluoromethyl)benzamide | 466(M+H) | | | 1.14, A | 0.99 |
| **246** | **1** | 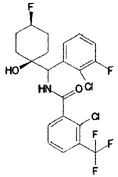 | (enantiomer 1) 2-chloro-N-[(2-chloro-3-fluorophenyl)(cis-4-fluoro-1-hydroxycyclohexyl)methyl]-3-(trifluoromethyl)benzamide | 482(M+H) | | | 1.12, A | 0.10 |
| **247** | **1** | 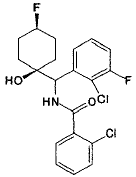 | (enantiomer 1) 2-chloro-N-[(2-chloro-3-fluorophenyl)(cis-4-fluoro-1-hydroxycyclohexyl)methyl] benzamide | 414(M+H) | | | 1.02, A | 1.0 |
| **248** | **1** | 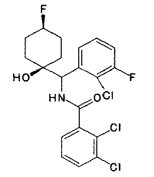 | (enantiomer 1) 2,3-dichloro-N-[(2-chloro-3-fluorophenyl)(cis-4-fluoro-1-hydroxycyclohexyl)methyl] benzamide | 448(M+H) | | | 1.09, A | 0.90 |

**[Table 1-46]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **249** | **2** | 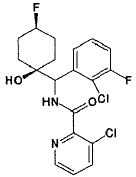 | (enantiomer 1) 3-chloro-N-[(2-chloro-3-fluorophenyl)(cis-4-fluoro-1-hydroxycyclohexyl)methyl] pyridine-2-carboxamide | 415(M+H) | | | 0.98, A | 1.2 |
| **250** | **2** | 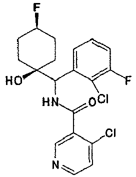 | (enantiomer 1) 4-chloro-N-[(2-chloro-3-fluorophenyl)(cis-4-fluoro-1-hydroxycyclohexyl)methyl] pyridine-3-carboxamide | 415(M+H) | | | 0.88, A | 1.9 |
| **251** | **1** | 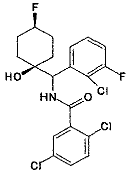 | (enantiomer 1) 2,5-dichloro-N-[(2-chloro-3-fluorophenyl)(cis-4-fluoro-1-hydroxycyclohexyl)methyl] benzamide | 448(M+H) | | | 1.12, A | 0.43 |
| **252** | **1** | 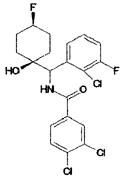 | (enantiomer 1) 3,4-dichloro-N-[(2-chloro-3-fluorophenyl)(cis-4-fluoro-1-hydroxycyclohexyl)methyl] benzamide | 448(M+H) | | | 1.19, A | 4.7 |
| **253** | **1** | 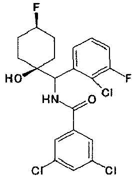 | (enantiomer 1) 3,5-dichloro-N-((2-chloro-3-fluorophenyl)(cis-4-fluoro-1-hydroxycyclohexyl)methyl] benzamide | 448(M+H) | | | 1.22, A | 8.3 |
| **254** | **1** | 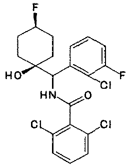 | (enantiomer 1) 2,6-dichloro-N-[(2-chloro-3-fluorophenyl)(cis-4-fluoro-1-hydroxycyclohexyl)methyl] benzamide | 448(M+H) | | | 105, A | 1.0 |
| **255** | **1** | 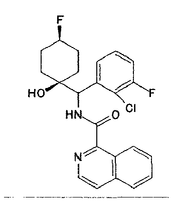 | (enantiomer 1) N-[(2-chloro-3-fluorophenyl)(cis-4-fluoro-1-hydroxycyclohexyl)methyl] isoquinoline-1-carboxamidea | 431 (M+H) | | | 1.16, A | 0.12 |

**[Table 1-47]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **256** | **1** | 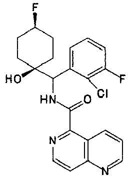 | (enantiomer 1) N-[(2-chloro-3-fluorophenyl)(cis-4-fluoro-1-hydroxycyclohexyl)methyl]-1,6-naphthyridin-5-carboxamide | 432(M+H) | | | 1.02, A | 3.1 |
| **257** | **17** | 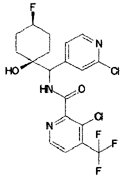 | (enantiomer 1) N-[(2-chlorophenyl)(4,4-difluoro-1-hydroxycyclohexyl)methyl]-3-(2H-1,2,3-triazol-2-yl)-5-(trifluoromethyl)pyridine-2-carboxamide | 466(M+H) | | | 0.95, A | 0.44 |
| **258** | **17** | 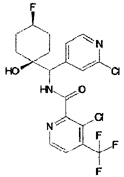 | (enantiomer 2) N-[(2-chlorophenyl)(4,4-difluoro-1-hydroxycyclohexyl)methyl]-2-(2H-1,2,3-triazol-2-yl)-6-(trifluoromethyl)pyridine-3-carboxamide | 466(M+H) | | | 0.95, A | 3.0 |
| **259** | **1** | 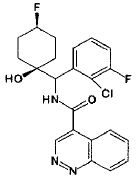 | (enantiomer 1) N-[(2-chloro-3-fluorophenyl)(cis-4-fluoro-1-hydroxycyclohexyl)methyl] cinnoline-4-carboxamide | 432(M+H) | | | 0.93, A | 1.0 |
| **260** | **1** | 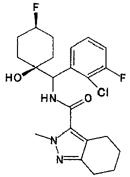 | (enantiomer 1) N-[(2-chloro-3-fluorophenyl)(cis-4-fluoro-1-hydroxycyclohexyl)methyl]-2-methyl-4,5,6,7-tetrahydro-2H-indazol-3-carboxamide | 438(M+H) | | | 1.04, A | 1.7 |
| **261** | **1** | 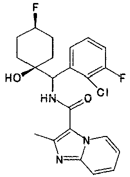 | (enantiomer 1) N-[(2-chloro-3-fluorophenyl)(cis-4-fluoro-1-hydroxycyclohexyl)methyl]-2-methylimidazo[1,2-a]pyridine-3-carboxamide | 434(M+H) | | | 0.66. A | 7.4 |
| **262** | **1** | 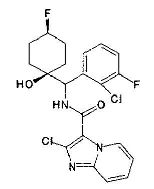 | (enantiomer 1) 2-chloro-N-[(2-chloro-3-fluorophenyl)(cis-4-fluoro-1-hydroxycyclohexyl)methyl] imidazo[1,2-a]pyridine-3-carboxamide | 454(M+H) | | | 1.08, A | 8.8 |

**[Table 1-48]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **263** | **1** | 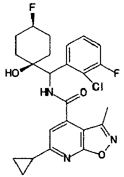 | (enantiomer 1) N-[(2-chloro-3-fluorophenyl)(cis-4-fluoro-1-hydroxycyclohexyl)methyl]-6-cyclopropyl-3-methyl[1,2]oxazolo[5,4-b]pyridine-4-carboxamide | 476(M+H) | | | 1.11, A | 0.99 |
| **264** | **1** | 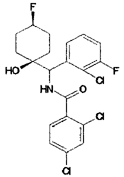 | (enantiomer 1) 2,4-dichloro-N-[(2-chloro-3-fluorophenyl)(cis-4-fluoro-1-hydroxycyclohexyl)methyl] benzamide | 448(M+H) | | | 1.12, A | 0.91 |
| **265** | **17** | 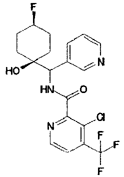 | (enantiomer 1) 3-chloro-N-[(cis-4-fluoro-1-hydroxycyclohexyl)(pyridin-3-yl)methyl]-4-(trifluoromethyl)pyridine-2-carboxamide | 432(M+H) | | | 0.54, A | 0.89 |
| **266** | **17** | 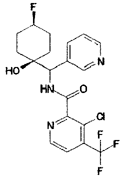 | (enantiomer 2) 3-chloro-N-[(cis-4-fluoro-1-hydroxycyclohexyl)(pyridin-3 yl)methyl]-4-(trifluoromethyl)pyridine-2-carboxamide | | | | | 1.6 |
| **267** | **1** | 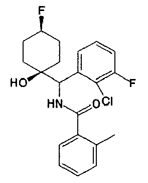 | (enantiomer 1) N-[(2-chloro-3-fluorophenyl)(cis-4-fluoro-1-hydroxycyclohexyl)methyl]-2-methylbenzamide | 394(M+H) | | | 1.03, A | 1.9 |
| **268** | **1** | 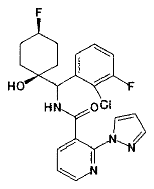 | (enantiomer 1) N-[(2-chloro-3-fluorophenyl)(cis-4-fluoro-1-hydroxycyclohexyl)methyl]-2-(1H-pyrazol-1-yl)pyridine-3-carboxamide | 447(M+H) | | | 0.90, A | 1.7 |
| **269** | **1** | 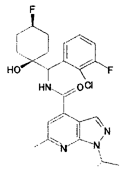 | (enantiomer 1) N-[(2-chloro-3-fluorophenyl)(cis-4-fluoro-1-hydroxycyclohexyl)methyl]-6-methyl-1-(propan-2-yl)-1H-pyrazolo[3,4-b]pyridine-4-carboxamide | 477(M+H) | | | 1.12, A | 1.7 |

**[Table 1-49]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **270** | **1** | 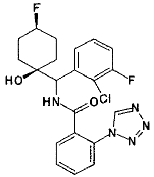 | (enantiomer 1) N-[(2-chloro-3-fluorophenyl)(cis-4-fluoro-1-hydroxycyclohexyl)methyl]-2-(1H-tetrazol-1-yl)benzamide | 448(M+H) | | | 0.87,A | 2.4 |
| **271** | **1** | 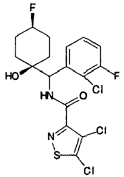 | (enantiomer 1) 4,5-dichloro-N-[(2-chloro-3-fluorophenyl)(cis-4-fluoro-1-hydroxycyclohexyl)methyl]-1,2-thiazol-3-carboxamide | 455(M+H) | | | 1.17, A | 1.1 |
| **272** | **1** | 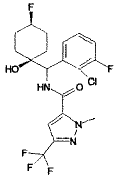 | (enantiomer 1) N-[(2-chloro-3-fluorophenyl)(cis-4-fluoro-1-hydroxycyclohexyl)methyl]-1-methyl-3-(trifluoromethyl)-1H-pyrazole-5-carboxamide | 452(M+H) | | | 1.12, A | 2.5 |
| **273** | **1** | 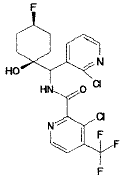 | (enantiomer 1) 3-chloro-N-[(2-chloropyridin-3-yl)(cis-4-fluoro-1-hydroxycyclohexyl)methyl]-4-(trifluoromethyl)pyridine-2-carboxamide | 466(M+H) . | | | 0.93, A | 0.33 |
| **274** | **17** | 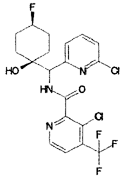 | (enantiomer 1) 3-chloro-N-[(6-chloropyridin-2-yl)(cis-4-fluoro-1-hydroxycyclohexyl)methyl]-4-(trifluoromethyl)pyridine-2-carboxamide | 466(M+H) | | | 1.04. A | 0.077 |
| **275** | **1** | 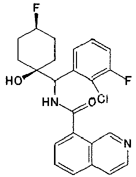 | (enantiomer 1) N-[(2-chloro-3-fluorophenyl)(cis-4-fluoro-1-hydroxycyclohexyl)methyl] isoquinoline-8-carboxamide | 431(M+H) | | | 0.64, A | 1.6 |
| **276** | **1** | 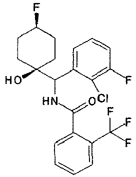 | (enantiomer 1) N-[(2-chloro-3-fluorophenyl)(cis-4-fluoro-1-hydroxycyclohexyl)methyl]-2-(trifluoromethyl)benzamide | 448(M+H) | | | 1.04. A | 1.6 |

**[Table 1-50]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **277** | **17** | 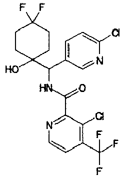 | (enantiomer 1) 3-chloro-N-[(6-chloropyridin-3-yl)(4,4-difluoro-1-hydroxycyclohexyl)methyl]-4-(trifluoromethyl)pyridine-2-carboxamide | 484(M+H) | | | 0.99, A | 0.64 |
| **278** | **17** | 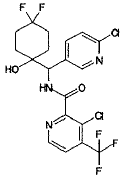 | (enantiomer 2) 3-chloro-N-[(6-chloropyridin-3-yl)(4,4-difluoro-1-hydroxycyclohexyl)methyl]-4-(trifluoromethyl)pyridine-2-carboxamide | 484(M+H) | | | 0.99, A | 2.7 |
| **279** | **1** | 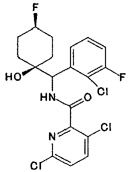 | (enantiomer 1) 3,6-dichloro-N-[(2-chloro-3-fluorophenyl)(cis-4-fluoro-1-hydroxycyclohexyl)methyl] pyridine-2-carboxamide | 449(M+H) | | | 1.08, A | 3.9 |
| **280** | **1** | 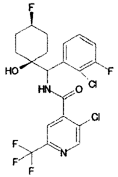 | (enantiomer 1) 5-chloro-N-[(2-chloro-3-fluorophenyl)(cis-4-fluoro-1-hydroxycyclohexyl)methyl]-2-(trifluoromethyl)pyridine-4-carboxamide | 483(M+H) | | | 1.11, A | 0.15 |
| **281** | **1** | 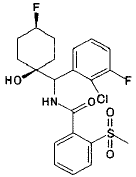 | (enantiomer 1) N-[(2-chloro-3-fluorophenyl)(cis-4-fluoro-1-hydroxycyclohexyl)methyl]-2-(methylsulfonyl)benzamide | 458(M+H) | | | 0.91, A | 2.1 |
| **282** | **1** | 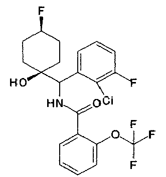 | (enantiomer 1) N-[(2-chloro-3-fluorophenyl)(cis-4-fluoro-1-hydroxycyclohexyl)methyl]-2-(trifluoromethoxy)benzamide | 464(M+H) | | | 1.10, A | 3.4 |
| **283** | **1** | 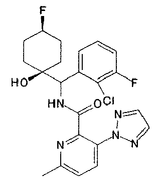 | (enantiomer 1) N-[(2-chloro-3-fluorophenyl)(cis-4-fluoro-1-hydroxycyclohexyl)methyl]-6-methyl-3-(2H-1,2,3-triazol-2-yl)pyridine-2-carboxamide | 462(M+H) | | | 0.93, A | 1.1 |

**[Table 1-51]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **284** | **19** | 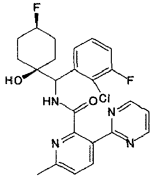 | (enantiomer 1) N-[(2-chloro-3-fluorophenyl)(cis-4-fluoro-1-hydroxycyclohexyl)methyl]-6-methyl-3-(pyrimidin-2-yl)pyridine-2-carboxamide | 473(M+H) | | | 0.87, A | 1.9 |
| **285** | **1** | 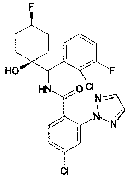 | (enantiomer 1) 4-chloro-N-[(2-chloro-3-fluorophenyl)(cis-4-fluoro-1-hydroxycyclohexyl)methyl]-2-(2H-1,2,3-triazol-2-yl)benzamide | 481 (M+H) | | | 0.95, A | 2.0 |
| **286** | **17** | 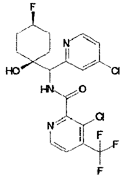 | (enantiomer 2) 3-chloro-N-[(4-chloropyridin-2-yl)(cis-4-fluoro-1-hydroxycyclohexyl)methyl]-4-(trifluoromethyl)pyridine-2-carboxamide | 466(M+H) | | | 1.03, A | 0.14 |
| **287** | **1** | 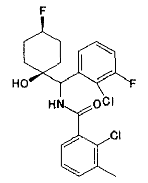 | (enantiomer 1) 2-chloro-N-[(2-chloro-3-fluorophenyl)(cis-4-fluoro-1-hydroxycyclohexyl)methyl]-3-methylbenzamide | 428(M+H) | | | 1.07, A | 0.96 |
| **288** | **1** | 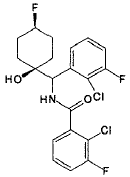 | (enantiomer 1) 2-chloro-N-[(2-chloro-3-fluorophenyl)(cis-4-fluoro-1-hydroxycyclohexyl)methyl]-3-fluorobenzamide | 432(M+H) | | | 1.04, A | 1.0 |
| **289** | **1** | 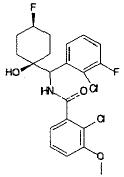 | (enantiomer 1) 2-chloro-N-[(2-chloro-3-fluorophenyl)(cis-4-fluoro-1-hydroxycyclohexyl)methyl]-3-methoxybenzamide | 444(M+H) | | | 1.01, A | 1.6 |
| **290** | **1** | 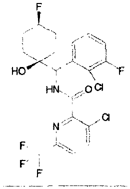 | (enantiomer 1) 3-chloro-N-[(2-chloro-3-fluorophenyl)(cis-4-fluoro-1-hydroxycyclohexyl)methyl]-6-(trifluoromethyl)pyridine-2-carboxamide | 483(M+H) | | | 1.11, A | 0.90 |

**[Table 1-52]**

| Compound No. | Example | Structure | Compound Name | (ESI pos.) m/z (ESI neg.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **291** | **17** | 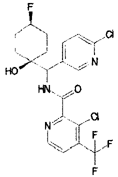 | (enantiomer 1) 3-chloro-N-[(6-chloropyridin-3-yl)(cis-4-fluoro-1-hydroxycyclohexyl]methyl]-4-(trifluoromethyl)pyridine-2-carboxamide | 466(M+H) | | | 0.95, A | 0.89 |
| **292** | **1** | 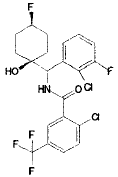 | (enantiomer 1) 2-chloro-N-[(2-chloro-3-fluorophenyl)(cis-4-fluoro-1-hydroxycyclohexyl)methyl]-5-(trifluoromethyl)benzamide | 482(M+H) | | | 1.14, A | 0.12 |
| **293** | **1** | 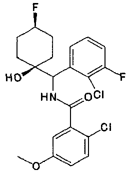 | (enantiomer 1) 2-chloro-N-[(2-chloro-3-fluorophenyl)(cis-4-fluoro-1-hydroxycyclohexyl)methyl]-5-methoxybenzamide | 444(M+H) | | | 1.05, A | 0.45 |
| **294** | **1** | 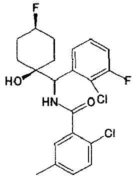 | (enantiomer 1) 2-chloro-N-[(2-chloro-3-fluorophenyl)(cis-4-fluoro-1-hydroxycyclohexyl)methyl]-5-methylbenzamide | 428(M+H) | | | 1.08, A | 0.99 |
| **295** | **1** | 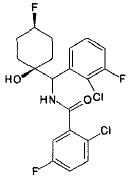 | (enantiomer 1) 2-chloro-N-[(2-chloro-3-fluorophenyl)(cis-4-fluoro-1-hydroxycyclohexyl)methyl]-5-fluorobenzamide | 432(M+H) | | | 1.05, A | 2.1 |
| **296** | **1** | 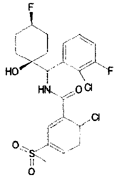 | (enantiomer 1) 2-chloro-N-[(2-chloro-3-fluorophenyl)(cis-4-fluoro-1-hydroxycyclohexyl)methyl]-5-(methylsulfonyl)benzamide | 492(M⁺H) | | | 0.95, A | 3.5 |
| **297** | **1** | 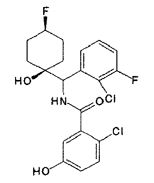 | (enantiomer 1) 2-chloro-N-[(2-chloro-3-fluorophenyl)(cis-4-fluoro-1-hydroxycyclohexyl)methyl]-5-hydroxybenzamide | 430(M+H) | | | 1.00, A | 1.5 |

**[Table 1-53]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **298** | **1** | 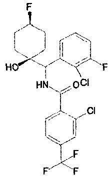 | (enantiomer 1) 2-chloro-N-[(2-chloro-3-fluorophenyl)(cis-4-fluoro-1-hydroxycyclohexyl)methyl]-4-(trifluoromethyl)benzamide | 482(M+H) | | | 1.20, A | 0.41 |
| **299** | **1** | 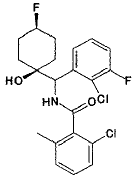 | (enantiomer 1) 2-chloro-N-[(2-chloro-3-fluorophenyl)(cis4-fluoro-1-hydroxycyclohexyl)methyl]-6 methylbenzamide | 428(M+H) | | | 1.10, A | 0.61 |
| **300** | **1** | 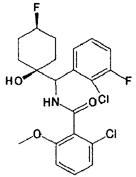 | (enantiomer 1) 2-chloro-N-[(2-chloro-3-fluorophenyl)(cis-4-fluoro-1-hydroxycyclohexyl)methyl]-6-methoxybenzamide | 444(M+H) | | | 1.06, A | 1.2 |
| **301** | **1** | 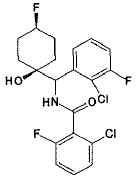 | (enantiomer 1) 2-chloro-N-[(2-chloro-3-fluorophenyl)(cis-4-fluoro-1-hydroxycyclohexyl)methyl]-6-fluorobenzamide | 432(M+H) | | | 1.08, A | 1.1 |
| **302** | **1** | 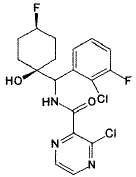 | (enantiomer 1) 3-chloro-N-[(2-chloro-3-fluorophenyl)(cis-4-fluoro-1-hydroxycyclohexyl)methyl]pyrazi ne-2-carboxamide | 416(M+H) | | | 1.01, A | 5.5 |
| **303** | **17** | 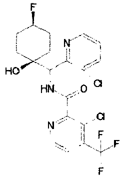 | (enantiomer 1) 3-chloro-N-[(3-chloropyridin-2-yl)(cis-4-fluoro-1-hydroxycyclohexyl)methyl]-4-(trifluoromethyl)pyridine-2-carboxamide | 466(M+H) | | | 1.10, A | 4.0 |
| **304** | **2** | 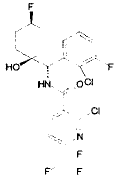 | (enantiomer 1) 2-chloro-N-[(2-chloro-3-fluorophenyl)(cis-4-fluoro-1-hydroxycyclohexyl)methyl]-6-(trifluoromethyl)pyridine-3-carboxamide | 481(M-H) | | | 1.15, A | 0.29 |

**[Table 1-54]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **305** | **2** | 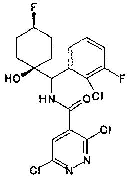 | (enantiomer 1) 3,6-dichloro-N-[(2-chloro-3-fluorophenyl)(cis-4-fluoro-1-hydroxycyclohexyl) methyl]pyridazine-4-carboxamide | 448(M-H) | | | 1.06, A | 1.0 |
| **306** | **1** | 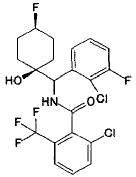 | (enantiomer 1) 2-chloro-N-[(2-chloro-3-fluorophenyl)(cis-4-fluoro-1-hydroxycyclohexyl)methyl]-6-(trifluoromethyl)benzamide | 504(M+Na) | | | 1.09, A | 0.67 |
| **307** | **1** | 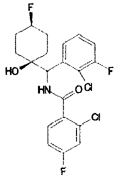 | (enantiomer 1) 2-chloro-N-[(2-chloro-3-fluorophenyl)(cis-4-fluoro-1-hydroxycyclohexyl)methyl]-4-fluorobenzamide | 432(M+H) . | | | 1.08, A | 1.3 |
| **308** | **17** | 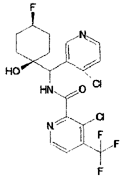 | (enantiomer 1) 3-chloro-N-[(4-chloropyridin-3-yl)(cis-4-fluoro-1-hydroxycyclohexyl)methyl]-4-(trifluoromethyl)pyridine-2-carboxamide | 466(M+H) | | | 0.96, A | 0.38 |
| **309** | **17** | 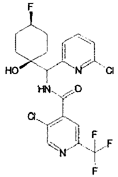 | (enantiomer 1) 5-chloro-N-[(6-chloropyridin-2-yl)(cis-4-fluoro-1-hydroxycyclohexyl)methyl]-2-(trifluoromethyl)pyridine-4-carboxamide | 466(M+H) | | | 1.03, A | 0.55 |
| **310** | **17** | 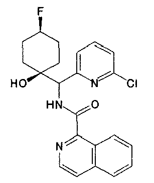 | (enantiomer 1) N-[(6-chloropyridin-2-yl)(cis-4-fluoro-1-hydroxycyclohexyl)methyl] isoquinoline-1-carboxamide | 414(M+H) | | | 1.08, A | 0.55 |
| **311** | **17** | 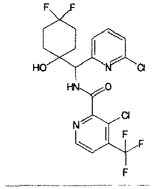 | (enantiomer 1) 3-chloro-N-[(6-chloropyridin-2-yl)(4,4-difluoro-1-hydroxycyclohexyl)methyl]-4-(trifluoromethyl)pyridine-2-carboxamide | 484(M+H) | | | 1.06, A | 0.077 |

**[Table 1-55]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **312** | **1** | 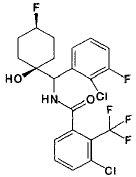 | (enantiomer 1) 3-chloro-N-[(2-chloro-3-fluorophenyl)(cis-4-fluoro-1-hydroxycyclohexyl)methyl]-2-(trifluoromethyl)benzamide | 480(M-H) | | | 1.16, A | 0.89 |
| **313** | **1** | 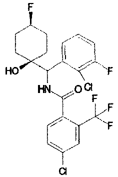 | (enantiomer 1) 4-chloro-N-[(2-chloro-3-fluorophenyl)(cis-4-fluoro-1-hydroxycyclohexyl)methyl]-2-(trifluoromethyl)benzamide | 482(M+H) | | | 1.18, A | 6.4 |
| **314** | **1** | 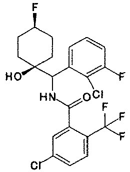 | (enantiomer 1) 5-chloro-N-[(2-chloro-3-fluorophenyl)(cis-4-fluoro-1-hydroxycyclohexyl)methyl]-2-(trifluoromethyl)benzamide | 480(M-H) | | | 1.18, A | 0.42 |
| **315** | **1** | 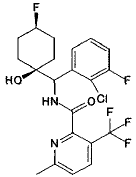 | (enantiomer 1) N-[(2-chloro-3-fluorophenyl)(cis-4-fluoro-1-hydroxycyclohexyl)methyl]-6-methyl-3 (trifluoromethyl)pyridine-2-carboxamide | 463(M+H) | | | 1.12, A | 0.82 |
| **316** | **1** | 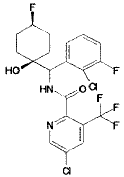 | (enantiomer 1) 5-chloro-N-[(2-chloro-3-fluorophenyl)(cis-4-fluoro-1-hydroxycyclohexyl)methyl]-3-(trifluoromethyl)pyridine-2-carboxamide | 483(M+H) | | | 1.18, A | 5.8 |
| **317** | **1** | 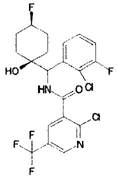 | (enantiomer 1) 2-chloro-N-[(2-chloro-3-fluorophenyl)(cis-4-fluoro-1-hydroxycyclohexyl)methyl]-5-(trifluoromethyl)pyridine-3-carboxamide | 481(M-H) | | | 1.14, A | 0.15 |
| **318** | **17** | 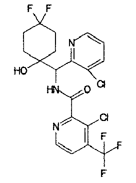 | (enantiomer 1) 3-chloro-N-[(3-chloropyridin-2-yl)(4,4-difluoro-1-hydroxycyclohexyl)methyl]-4-(trifluoromethyl)pyridine-2-carboxamide | 484(M+H) | | | 1.15, A | 0.24 |

**[Table 1-56]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **319** | **17** | 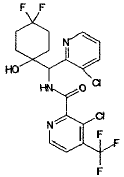 | (enantiomer 2) 3-chloro-N-[(3-chloropyridin-2-yl)(4,4-difluoro-1-hydroxycyclohexyl)methyl]-4-(trifluoromethyl)pyridine-2-carboxamide | 484(M+H) | | | 1.15, A | 1.8 |
| **320** | **2** | 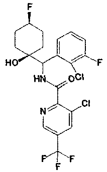 | (enantiomer 1) 3-chloro-N-[(2-chloro-3-fluorophenyl)(cis-4-fluoro-1-hydroxycyclohexyl)methyl]-5-(trifluoromethyl)pyridine-2-carboxamide | 483(M+H) | | | 1.18, A | 1.0 |
| **321** | **1** | 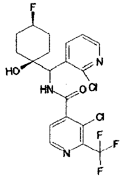 | (enantiomer 1) 3-chloro-N-[(2-chloropyridin-3-yl)(cis-4-fluoro-1-hydroxycyclohexyl)methyl]-2-(trifluoromethyl)pyridine-4-carboxamide | 466(M+H) | | | 0.98, A | 0.59 |
| **322** | **1** | 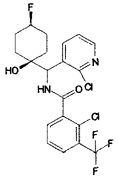 | (enantiomer 1) 2-chloro-N-[(2-chloropyridin-3-yl)(cis-4-fluoro-1-hydroxycyclohexyl)methyl]-3-(trifluoromethyl)benzamide | 465(M+H) | | | 1.02, A | 0.35 |
| **323** | **1** | 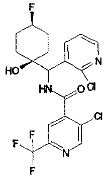 | (enantiomer 1) 5-chloro-N-[(2-chloropyridin-3-yl)(cis-4-fluoro-1-hydroxycyclohexyl)methyl]-2-(trifluoromethyl)pyridine-4-carboxamide | 466(M+H) | | | 1.00, A | 0.20 |
| **324** | **1** | 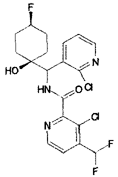 | (enantiomer 1) 3-chloro-N-[(2-chloropyridin-3-yl)(cis-4-fluoro-1-hydroxycyclohexyl)methyl]-4-(difluoromethyl)pyridine-2-carboxamide | 448(M+H) | | | 0.92, A | 1.2 |
| **325** | **1** | 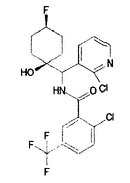 | (enantiomer 1) 2-chloro-N-[(2-chloropyridin-3-yl)(cis-4-fluoro-1-hydroxycyclohexyl)methyl]-5-(trifluoromethyl)benzamide | 465(M+H) | | | 1.04, A | 0.43 |

**[Table 1-57]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **326** | **1** | 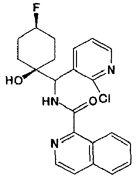 | (enantiomer 1) N-[(2-chloropyridin-3-yl)(cis-4-fluoro-1-hydroxycyclohexyl)methyl] isoquinoline-1-carboxamide | 414(M+H) | | | 1.03, A | 1.1 |
| **327** | **17** | 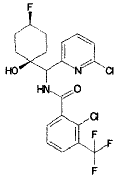 | (enantiomer 1) 2-chloro-N-[(6-chloropyridin-2-yl(cis-4-fluoro-1-hydroxycyclohexyl)methyl]-3-(trifluoromethyl)benzamide | 465(M+H) | | | 1.05, A | 0.081 |
| **328** | **17** | 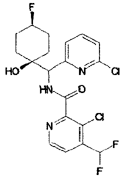 | (enantiomer 1) 3-chloro-N-[(6-chloropyridin-2-yl)(cis-4-fluoro-1-hydroxycyclohexyl)methyl]-4-(difluoromethyl)pyridine-2-carboxamide | 448(M+H) | | | 0.97, A | 0.20 |
| **329** | **17** | 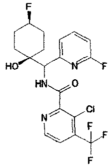 | (enantiomer 1) 3-chloro-N-[(cis-4-fluoro-1-hydroxycyclohexyl)(6-fluoropyridin-2-yl)methyl]-4-(trifluoromethyl)pyridine-2-carboxamide | 450(M+H) | | | 0.97, A | 0.14 |
| **330** | **17** | 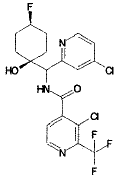 | (enantiomer 1) 3-chloro-N-[(4-chloropyridin-2-yl)(cis-4-fluoro-1-hydroxycyclohexyl)methyl]-2-(trifluoromethyl)pyridine-4-carboxamide | 466(M+H) | | | 1.07, A | 0.056 |
| **331** | **17** | 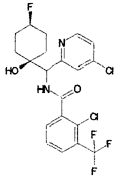 | (enantiomer 1) 2-chloro-N-[(4-chloropyridin-2-yl)(cis-4-fluoro-1-hydroxycyclohexyl)methyl]-3-(trifluoromethyl)benzamide | 465(M+H) | | | 1.13, A | 0.088 |
| **332** | **17** | 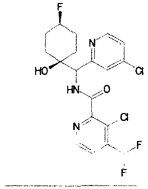 | (enantiomer 1) 3-chloro-N-[(4-chloropyridin-2-yl)(cis-4-fluoro-1-hydroxycyclohexyl)methyl]-4-(difluoromethyl)pyridine-2-carboxamide | 448(M+H) | | | 1.03, A | 1.4 |

**[Table 1-58]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **333** | **17** | 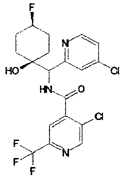 | (enantiomer 1) 5-chloro-N-[(4-chloropyridin-2-yl)(cis-4-fluoro-1-hydroxycyclohexyl)methyl]-2-(trifluoromethyl)pyridine-4-carboxamide | 466(M+H) | | | 1.09, A | 1.7 |
| **334** | **17** | 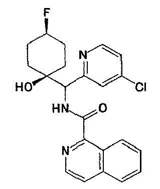 | (enantiomer 1) N-[(4-chloropyridin-2-yl)(cis-4-fluoro-1-hydroxycyclohexyl)methyl] isoquinoline-1-carboxamide | 414(M+H) | | | 1.15, A | 0.90 |
| **335** | **17** | 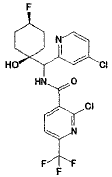 | (enantiomer 1) 2-chloro-N-[(4-chloropyridin-2-yl)(cis-4-fluoro-1-hydroxycyclohexyl)methyl]-6-(trifluoromethyl)pyridine-3-carboxamide | 466(M+H) | | | 1.08, A | 0.27 |
| **336** | **1** | 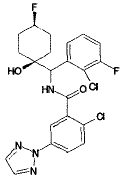 | (enantiomer 1) 2-chloro-N-[(2-chloro-3-fluorophenyl)(cis-4-fluoro-1-hydroxycyclohexyl)methyl]-5-(2H-1,2,3-triazol-2-yl)benzamide | 481(M+H) | | | 1.13, A | 0.69 |
| **337** | **1** | 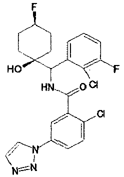 | (enantiomer 1) 2-chloro-N-[(2-chloro-3-fluorophenyl)(cis-4-fluoro-1-hydroxycyclohexyl)methyl]-5-(1H-1,2,3-triazol-1-yl)benzamide | 481(M+H) | | | 1.02, A | 1.9 |
| **338** | **18** | 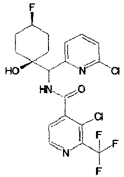 | (enantiomer 1) 3-chloro-N-[(6-chloropyridin-2-yl)(cis-4-fluoro-1-hydroxycyclohexyl)methyl]-2-(trifluoromethyl)pyridine-4-carboxamide | 466(M+H) | | | 1.07, A | 0.096 |
| **339** | **18** | 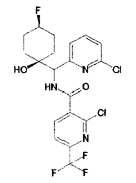 | (enantiomer 1) 2-chloro-N-[(6-chloropyridin-2-yl)(cis-4-fluoro-1-hydroxycyclohexyl)methyl]-6-(trifluoromethyl)pyridine-3-carboxamide | 466(M+H) | | | 1.09, A | 0.14 |

**[Table 1-59]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **340** | **2** | 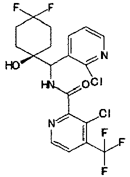 | (enantiomer 1) 3-chloro-N-[(2-chloropyridin-3-yl)(4,4-difluoro-1-hydroxycyclohexyl)methyl]-4-(trifluoromethyl)pyridine-2-carboxamide | 484(M+H) | | | 1.04, A | 0.32 |
| **341** | **2** | 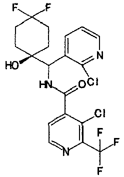 | (enantiomer 1) 3-chloro-N-[(2-chloropyridin-3-yl)(4,4-difluoro-1-hydroxycyclohexyl)methyl]-2-(trifluoromethyl)pyridine-4-carboxamide | 484(M+H) | | | 1.03, A | 0.85 |
| **342** | **2** | 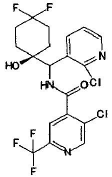 | (enantiomer 1) 5-chloro-N-[(2-chloropyridin-3-yl)(4,4-difluoro-1-hydroxycyclohexyl)methy]-2-(trifluoromethyl)pyridine-4-carboxamide | 484(M+H) | | | 1.05, A | 1.2 |
| **343** | **2** | 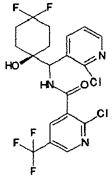 | (enantiomer 1) 5-chloro-N-[(2-chloropyridin-3-yl)(4,4-difluoro-1-hydroxycyclohexyl)methyl]-2-(trifluoromethyl)pyridine-4-carboxamide | 484(M+H) | | | 1.03, A | 1.7 |
| **344** | **2** | 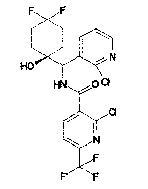 | (enantiomer 1) 2-chloro-N-[(2-chloropyridin-3-yl)(4,4-difluoro-1-hydroxycyclohexyl)methyl]-6-(trifluoromethyl)pyridine-3 carboxamide | 484(M+H) | | | 1.04, A | 2.3 |
| **345** | **2** | 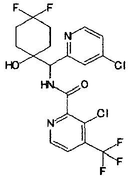 | (enantiomer 1) 3-chloro-N-[(4-chloropyridin-2-yl)(4,4-difluoro-1-hydroxycyclohexyl)methyl]-4-(trifluoromethyl)pyridine-2-carboxamide | 484(M+H) | | | 1.15, A | 0.27 |
| **346** | **2** | 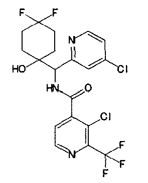 | (enantiomer 1) 3-chloro-N-[(4-chloropyridin-2-yl)(4,4-difluoro-1-hydroxycyclohexyl)methyl]-2-(trifluoromethyl)pyridine-4-carboxamide | 484(M+H) | | | 1.12, A | 0.36 |

**[Table 1-60]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **347** | **17** | 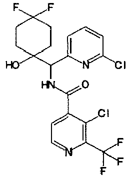 | (enantiomer 1) 3-chloro-N-[(6-chloropyridin-2-yl)(4,4-difluoro-1-hydroxycyclohexyl)methyl]-2-(trifluoromethyl)pyridine-4-carboxamide | 484(M+H) | | | 1.13, A | 0.38 |
| **348** | **2** | 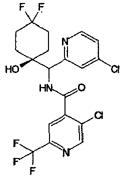 | (enantiomer 1) 5-chloro-N-[(4-chloropyridin-2-yl)(4,4-difluoro-1-hydroxycyclohexyl)methyl]-2-(trifluoromethyl)pyridine-4-carboxamide | 484(M+H) | | | 1.16, A | 0.49 |
| **349** | **2** | 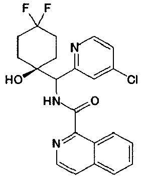 | (enantiomer 1) N-[(4-chloropyridin-2-yl)(4,4-difluoro-1-hydroxycyclohexyl)methyl] isoquinoline-1-carboxamide | 432(M+H) | | | 1.22, A | 1.1 |
| **350** | **2** | 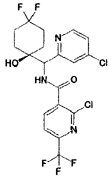 | (enantiomer 1) 2-chloro-N-[(4-chloropyridin-2-yl)(4,4-difluoro-1-hydroxycyclohexyl)methyl]-6-(trifluoromethyl)pyridine-3-carboxamide | 484(M+H) | | | 1.14, A | 0.62 |
| **351** | **2** | 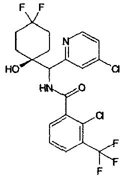 | (enantiomer 1) 2-chloro-N-[(4-chloropyridin-2-yl)(4,4-difluoro-1-hydroxycyclohexyl)methyl]-3-(trifluoromethyl)benzamide | 483(M+H) | | | 1.18, A | 0.15 |
| **352** | **18** | 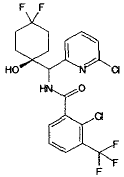 | (enantiomer 1) 2-chloro-N-[(6-chloropyridin-2-yl)(4,4-difluoro-1-hydroxycyclohexyl)methyl]-3-(trifluoromethyl)benzamide | 483(M+H) | | | 1.18, A | 0.15 |
| **353** | **18** | 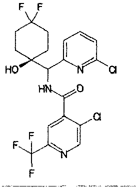 | (enantiomer 1) 5-chloro-N-[(6-chloropyridin-2-yl)(4,4-difluoro-1-hydroxycyclohexyl)methyl]-2-(trifluoromethyl)pyridine-4-carboxamide | 484(M+H) | | | 1.15, A | 0.15 |

**[Table 1-61]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **354** | **18** | 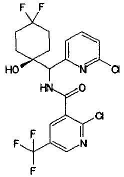 | (enantiomer 1) 2-chloro-N-[(6-chloropyridin-2-yl)(4,4-difluoro-1-hydroxycyclohexyl)methyl]-5-(trifluoromethyl)pyridine-3-carboxamide | 484(M+H) | | | 1.13, A | 0.22 |
| **355** | **18** | 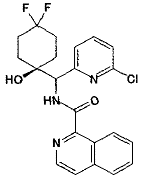 | (enantiomer 1) N-[(6-chloropyridin-2-yl)(4,4-difluoro-1-hydroxycyclohexyl)methyl] isoquinoline-1-carboxamide | 432(M+H) | | | 1.21, A | 0.13 |
| **356** | **18** | 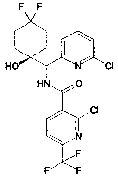 | (enantiomer 1) 2-chloro-N-[(6-chloropyridin-2-yl)(4,4-difluoro-1-hydroxycyclohexyl)methyl]-6-(trifluoromethyl)pyridine-3-carboxamide | 484(M+H) | | | 1.14, A | 0.25 |
| **357** | **2** | 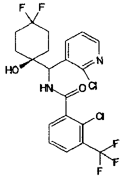 | (enantiomer 1) 2-chloro-N-[(2-chloropyridin-3-yl)(4,4-difluoro-1-hydroxycyclohexyl)methyl]-3-(trifluoromethyl)benzamide | 483(M+H) | | | 1.07, A | 0.33 |
| **358** | **2** | 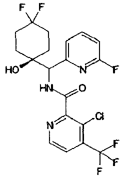 | (enantiomer 1) 3-chloro-N-[(4,4-difluoro-1-hydroxycyclohexyl)(6-fluoropyridin-2-yl)methyl]-4-(trifluoromethyl)pyridine-2-carboxamide | 468(M+H) | | | 1.06, A | 0.15 |
| **359** | **2** | 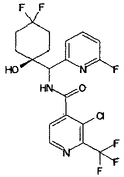 | (enantiomer 1) 3-chloro-N-[(4,4-difluoro-1-hydroxycyclohexyl)(6-fluoropyridin-2-yl)methyl]-2-(trifluoromethyl)pyridine-4-carboxamide | 468(M+H) | | | 1.03, A | 0.25 |
| **360** | **2** | 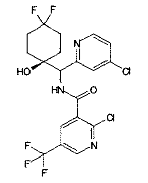 | (enantiomer 1) 2-chloro-N-[(4-chloropyridin-2-yl)(4,4-difluoro-1-hydroxycyclohexyl)methyl]-5-(trifluoromethyl)pyridine-3-carboxamide | 484(M+H) | | | 1.15, A | 0.52 |

**[Table 1-62]**

| Compound No. | Example | Structure | Compound Name | (ESI pos.) m/z (ESI pos.) m/2 | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **361** | **17** | 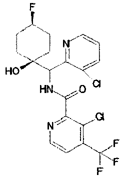 | (enantiomer 1) 3-chloro-N-[(3 chloropyridin-2-yl)(cis-4-fluoro-1-hydroxycyclohexyl)methyl]-4-(trifluoromethyl)pyridine-2-carboxamide | 466(M+H) | | | 1.11, A | 0.30 |
| **362** | **17** | 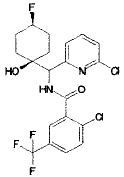 | (enantiomer 1) 2-chloro-N-[(S)-(6-chloropyridin-2-yl)(cis-4-fluoro-1-hydroxycyclohexyl)methyl]-5-(trifluoromethyl)benzamide | 465(M+H) | | | 1.16, A | 0.19 |
| **363** | **17** | 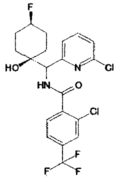 | (enantiomer 1) 2-chloro-N-[(6-chloropyridin-2-yl)(cis-4-fluoro-t-hydroxycyclohexyl)methyl]-4-(trifluoromethyl)benzamide | 465(M+H) | | | 1.18, A | 0.24 |
| **364** | **1** | 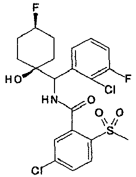 | (enantiomer 1) 5-chloro-N-[(2-chloro-3-fluorophenyl)(cis-4-fluoro-1-hydroxycyclohexyl)methyl]-2-(methylsulfonyl)benzamide | 490(M-H) | | | 1.09, A | 0.53 |
| **365** | **1** | 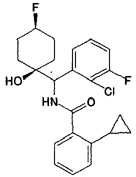 | (enantiomer 1) N-[(2-chloro-3-fluorophenyl)(cis-4-fluoro-1-hydroxycyclohexyl)methyl]-2-cyclopropylbenzamide | 420(M+H) | | | 1.15.A A | 2.8 |
| **366** | **2** | 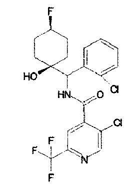 | (enantiomer 1) 5-chloro-N-[(2-chlorophenyl)(cis-4-fluoro-1-hydroxycyclohexyl)methyl]-2-(trifluoromethyl)pyridine-4-carboxamide | 463(M-H) | | | 1.16. A | 0.32 |
| **367** | **2** | 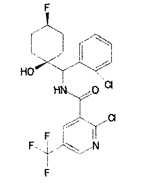 | (enanthiomer 1) 2-chloro-N-[(2-chlorophenyl)(cis-4-fluoro-1-hydroxycyclohexyl)methyl]-5-(trifluoromethyl)pyridine-3-carboxamide | 463(M-H) | | | 1.14. A | 0.38 |

**[Table 1-63]**

| Compound No. | Example | Structure | Compound Name | ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **368** | **2** | 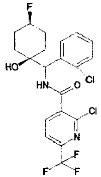 | (enantiomer 1) 2-chloro-N-[(2-chlorophenyl)(cis-4-fluoro-1-hydroxycyclohexyl)methyl]-6-(trifluoromethyl)pyridine-3-carboxamide | 463(M-H) | | | 1.14, A | 0.76 |
| **369** | **2** | 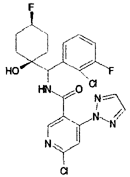 | (enantiomer 1) 6-chloro-N-[(2-chloro-3-fluorophenyl)(cis-4-fluoro-1-hydroxycyclohexyl)methyl]-4-(2H-1,2,3-triazol-2-yl)pyridine-3-carboxamide | 482(M+H) | | | 1.00, A | 1.2 |
| **370** | **2** | 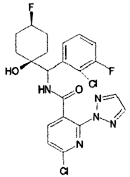 | (enantiomer 1) 6-chloro-N-[(2-chloro-3-fluorophenyl)(cis-4-fluoro-1-hydroxycyclohexyl)methyl]-2-(2H-1,2,3-triazol-2-yl)pyridine-3-carboxamide | 482(M+H) | | | 0.94, A | 3.0 |
| **371** | **2** | 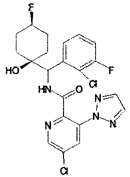 | (enantiomer 1) 5-chloro-N-[(2-chloro-3-fluorophenyl)(cis-4-fluoro-1-hydroxycyclohexyl)methyl]-3-(2H-1,2,3triazol-2-yl)pyridine-2-carboxamide | 482(M+H) | | | 1.02, A | 1.6 |
| **372** | **2** | 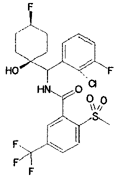 | (enantiomer 1) N-[(2-chloro-3 fluorophenyl)(cis-4-fluoro-1-hydroxycyclohexyl)methyl]-2-(methylsulfonyl)-5-(trifluoromethyl)benzamide | 548(M+Na) | | | 1.09, A | 0.041 |
| **373** | **2** | 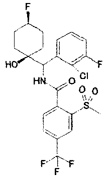 | (enantiomer 1) N-[(2-chloro-3 fluorophenyl)(cis-4-fluoro-1-hydroxycyclohexyl)methyl]-2-(methylsulfonyl)-4-(trifluoromethyl)benzamide | 548(M+Na) | | | 1.08, A | 0.10 |
| **374** | **1** | 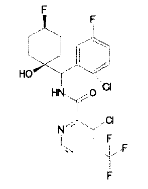 | (enantiomer 1) 3-chloro-N-[(2-chloro-5-fluorophenyl)(cis-4-fluoro-1-hydroxycyclohexyl)methyl]-4-(trifluoromethyl)pyridine-2-carboxamide | 483(M+H) | | | 1.17. A | 0.17 |

**[Table 1-64]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **375** | **1** | 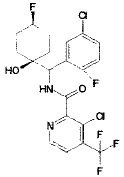 | (enantiomer 1) 3-chloro-N-[(5-chloro-2-fluorophenyl)(cis-4-fluoro-1-hydroxycyclohexyl)methyl]-4-(trifluoromethyl)pyridine-2-carboxamide | 483(M+H) | | | 1.17, A | 0.25 |
| **376** | **1** | 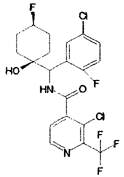 | (enantiomer 1) 3-chloro-N-[(5-chloro-2-fluorophenyl)(cis-4-fluoro-1-hydroxycyclohexyl)methyl]-2-(trifluoromethyl)pyridine-4-carboxamide | 483(M+H) | | | 1.15, A | 0.55 |
| **377** | **1** | 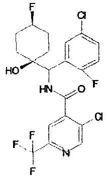 | (enantiomer 1) 5-chloro-N-[(5-chloro-2-fluorophenyl)(cis-4-fluoro-1-hydroxycyclohexyl)methyl]-2-(trifluoromethyl)pyridine-4-carboxamide | 483(M+H) | | | 1.17, A | 0.92 |
| **378** | **2** | 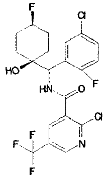 | (enantiomer 1) 2-chloro-N-[(5-chloro-2-fluorophenyl)(cis-4-fluoro-1-hydroxycyclohexyl)methyl]-5-(trifluoromethyl)pyridine-3-carboxamide | 481(M-H) | | | 1.16, A | 2.3 |
| **379** | **2** | 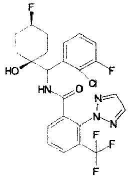 | (enantiomer 1) N-[(2-chloro-3-fluorophenyl)(cis-4-fluoro-1-hydroxycyclohexyl)methyl]-2-(2H-1,2,3-triazol-2-yl)-3-(trifluoromethyl)benzamide | 515(M+H) | | | 1.10, A | 0.79 |
| **380** | **2** | 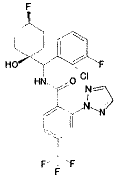 | (enantiomer 1) N-[(2-chloro-3-fluorophenyl)(cis-4-fluoro-1-hydroxycyclohexyl)methyl]-2-(2H-1,2,3-triazol-2-yl)-4-(trifluoromethyl)benzamide | 515(M+H) | | | 1.11, A | 0.27 |
| **381** | **2** | 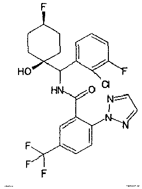 | (enantiomer 1) N-[-(2-chloro-3-fluorophenyl)(cis-4-fluoro-1-hydroxycyclohexyl)methyl]-2-(2H-1,2,3-triazol-2-yl)-5-(trifluoromethyl)benzamide | 515(M+H) | | | 1.15, A | 1.2 |

**[Table 1-65]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **382** | **20** | 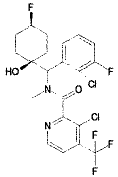 | (enantiomer 1) 3-chloro-N-[(2-chloro-3-fluorophenyl)(cis-4-fluoro-1-hydroxycyclohexyl)methyl]-N-methyl-4-(trifluoromethyl)pyridine-2-carboxamide | 497(M+H) | | | 1.20, A | 0.74 |
| **383** | **1** | 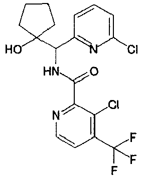 | 3-chloro-N-[(6-chloropyridin-2-yl)(1-hydroxycyclopentyl)methyl]-4-(trifluoromethyl)pyridine-2-carboxamide | 434(M+H) | | | 1.08, A | 0.021 |
| **384** | **17** | 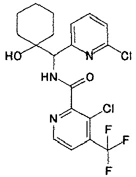 | (enantiomer 1) 3-chloro-N-[(6-chloropyridin-2-yl)(1-hydroxycyclohexyl)methyl]-4-(trifluoromethyl)pyridine-2-carboxamide | 448(M+H) | | | 1.19, A | 0.0094 |
| **385** | **17** | 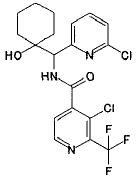 | (enantiomer 1) 3-chloro-N-[(6 chloropyridin-2-yl)(1-hydroxycyclohexyl)methyl]-2-(trifluoromethyl)pyridine-4-carboxamide | 448(M+H) | | | 1.16, A | 0.026 |
| **386** | **2** | 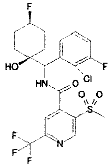 | (enantiomer 1) N-[(2-chloro-3-fluorophenyl)(cis-4-fluoro-1-hydroxycyclohexyl)methyl]-5-(methylsulfonyl)-2-(trifluoromethyl)pyridine-4-carboxamide | 549(M+Na) | | | 1.07, A | 0.035 |
| **387** | **2** | 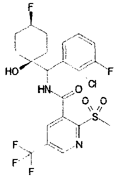 | (enantiomer 1)N-[(2-hloro-3-fluorophenyl)(cis-4-fluoro-1-hydroxycyclohexyl)methyl]-2-(methylsulfonyl)-5-(trifluoromethyl)pyridine-3-carboxamide | 549(M+Na) | | | 1.01, A | 0.036 |
| **388** | **2** | 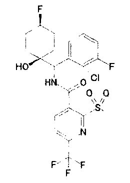 | (enantiomer 1) N-[(2-chloro-3-fluorophenyl)(cis-4-fluoro-1-hydroxycyclohexyl)methyl]-2-(methylsulfonyl)-6-(trifluoromethyl)pyridine-3-carboxamide | 549(M+Na) | | | 1.01, A | 0.26 |

**[Table 1-66]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **389** | **2** | 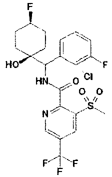 | (enantiomer 1) N-[(2-chloro-3-fluorophenyl)(cis-4-fluoro-1-hydroxycyclohexyl)methyl]-3-(methylsulfonyl)-5-(trifluoromethyl)pyridine-2-carboxamide | 549(M+Na) | | | 1.07, A | 1.8 |
| **390** | **2** | 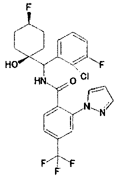 | (enantiomer 1) N-[(2-chloro-3 fluorophenyl)(cis-4-fluoro-1-hydroxycyclohexyl)methyl]-2-(1H-pyrazol-1-yl)-4-(trifluoromethyl)benzamide | 514(M+H) | | | 1.13, A | 0.13 |
| **391** | **2** | 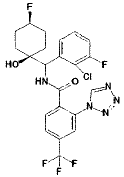 | (enantiomer 1) N-[(2-chloro-3-fluorophenyl)(cis-4-fluoro-1-hydroxycyclohexyl)methyl]-2-(1 H-tetrazol-1-yl)-4-(trifluoromethyl)benzamide | 538(M+Na) | | | 1.07, A | 0.027 |
| **392** | **2** | 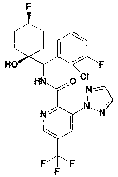 | (enantiomer 1) N-[(2-chloro-3-fluorophenyl)(cis-4-fluoro-1-hydroxycyclohexyl)methyl]-3-(2H-1,2,3-triazol-2-yl)-5-(trifluoromethyl)pyridine-2-carboxamide | 516(M+H) | | | 1.07, A | 0.11 |
| **393** | **2** | 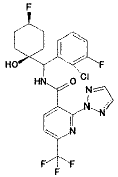 | (enantiomer 1) N-[(2-chloro-3-fluorophenyl)(cis-4-fluoro-1-hydroxycyclohexyl)methyl]-2-(2H-1,2,3-triazol-2-yl)-6-(trifluoromethyl)pyridine-3-carboxamide | 516(M+H) | | | 1.02, A | 0.040 |
| **394** | **2** | 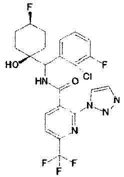 | (enantiomer 1) N-[(2-chloro-3-fluorophenyl)(cis-4-fluoro-1-hydroxycyclohexyl)methyl]-2-(1H-1,2,3-triazol-1-yl)-6-(trifluoromethyl)pyridine-3-carboxamide | 516(M+H) | | | 1.01, A | 1.3 |
| **395** | **4** | 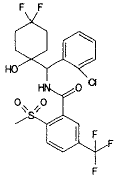 | (enantiomer 1) N-[(2-chlorophenyl)(4,4-difluoro-1-hydroxycyclohexyl)methyl]-2-(methylsulfonyl)-5-(trifluoromethyl)benzamide | 524(M-H) | | | 1.15, A | 0.33 |

**[Table 1-67]**

| Compound No. | Example | Structure | Compound Name | (ESI pos.) m/z (ESI neg.) m/z | (Column used) (Solvent system) (Flow rate) | Optical isomer analysis RT (min) | LCMS RT (min) condition | IC₅₀ (µM) |
|---|---|---|---|---|---|---|---|---|
| **396** | **17** | 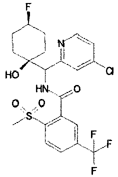 | (enantiomer 1) N-[(4-chloropyridin-2-yl)(cis-4-fluoro-1-hydroxycyclohexyl]methyl]-2-(methylsulfonyl)-5-(trifluoromethyl)benzamide | 509(M+H) | | | 1.06. A | 0.29 |
| **397** | **1** | 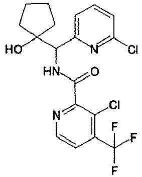 | (enantiomer 1) 3-chloro-N-[(6-chloropyridin-2-yl)(1-hydroxycyclopentyl)methyl]-4-(trifluoromethyl)pyridine-2-carboxamide | 434(M+H) | AD3, 4.6*250 | 3.96, 5.00 Faster | 1.08, A | 0.016 |
| | | | | | Hex : IPA = 80 : 20 | | | |
| | | | | | 1 mL / min | | | |
| **398** | **1** | 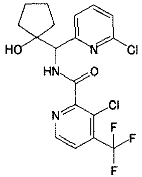 | (enantiomer 2) 3-chloro-N-[(6-chloropyridin-2-yl)(1-hydroxycyclopentyl)methyl]-4-(trifluoromethyl)pyridine-2-carboxamide | 434(M+H) | AD3, 4.6*250 | 3.96. 5.00 Later | 1.08, A | 7.5 |
| | | | | | Hex: IPA = 80: 20 | | | |
| | | | | | 1 mL / min | | | |
| **400** | **4** | 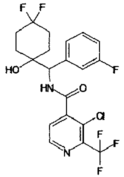 | (enantiomer 1) 3-chloro-N-[(4,4-difluoro-1-hydroxycyclohexyl)(3-fluorophenyl)methyl]-2-(trifluoromethyl)pyridine-4-carboxamide | 467(M+H) | | | 1.13, A | 0.23 |
| **401** | **4** | 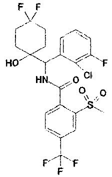 | (enantiomer 1) N-[(2-chloro-3-fluorophenyl)(4,4-difluoro-1-hydroxycyclohexyl)methyl]-2-(methylsulfonyl)-4-(trifluoromethyl)benzamide | 542(M-H) | | | 1.16, A | 0.23 |
| **402** | **4** | 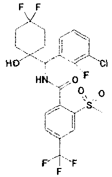 | (enantiomer 1) N-[(3-chloro-2-fluorophenyl)(4,4-difluoro-1-hydroxycyclohexyl)methyl]-2-(methylsulfonyl)-4-(trifluoromethyl)benzamide | 542(M-H) | | | 1.17. A | 0.45 |
| **403** | **4** | 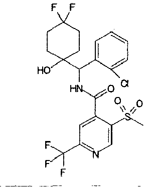 | (enantiomer 1) N-[(2-chlorophenyl)(4,4-difluoro-1-hydroxycyclohexyl)methyl]-5-(methylsulfonyl)-2-(trifluoromethyl)pyridine-4-carboxamide | 525(M-H) | | | 1.06, A | 0.12 |

**[Table 1-68]**

| Compound No. | Example | Structure | Compound Name | (ESI pos.) m/z (ESI neg.) m/z | Optical isomer analysis conditions (Column used) (Solvent system) (Flow rate) | Optical isomer analysis RT (min) | LCMS RT (min) condition | IC₅₀ (µM) |
|---|---|---|---|---|---|---|---|---|
| **404** | **4** | 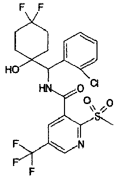 | (enantiomer 1) N-[(2-chlorophenyl)(4,4-difluoro-1-hydroxycyclohexyl)methyl]-2-(methylsulfonyl)-5-(trifluoromethyl)pyridine-3-carboxamide | 549(M+Na) | | | 1.01, A | 0.17 |
| **405** | **4** | 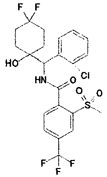 | (enantiomer 1) N-[(2-chlorophenyl)(4,4-difluoro-1-hydroxycyclohexyl)methyl]-2-(methylsulfonyl)-4-(trifluoromethyl)benzamide | 548(M+Na) | | | 1.07, A | 0.20 |
| **406** | **4** | 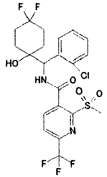 | (enantiomer 1) N-[(2-chlorophenyl)(4,4-difluoro-1-hydroxycyclohexyl)methyl]-2-(methylsulfonyl)-6-(trifluoromethyl)pyridine-3-carboxamide | 549(M+Na) | | | 1.00, A | 0.32 |
| **407** | **4** | 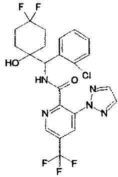 | (enantiomer 1) N-[(2-chlorophenyl)(4,4-difluoro-1-hydroxycyclohexyl)methyl]-3-(2H-1,2,3-triazol-2-yl)-5-(trifluoromethyl)pyridine-2-carboxamide | 516(M+H) | | | 1.14, A | 1.0 |
| **408** | **4** | 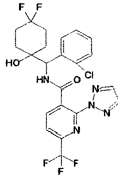 | (enantiomer 1) N-[(2-chlorophenyl)(4,4-difluoro-1-hydroxycyclohexyl)methyl]-2-(2H-1,2,3-triazol-2-yl)-6-(trifluoromethyl)pyridine-3-carboxamide | 516(M+H) | | | 1.10, A | 2.2 |
| **409** | **4** | 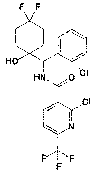 | (enantiomer 1) 2-chloro-N-[(2-chlorophenyl)(4,4-difluoro-1-hydroxycyclohexyl)methyl]-6-(trifluoromethyl)pyridine-3-carboxamide | 481 (M-H) | | | 1.18, A | 1.1 |

¹H-NMR data of the compounds shown in Table 1 are shown below.

### Compound 14

1H NMR (600 MHz, CHLOROFORM-d) d ppm 1.16 - 2.37 (m, 11 H) 4.21 (q, J=7.3 Hz, 2 H) 5.04 - 5.11 (m, 1 H) 7.27 - 7.78 (m, 7 H) 8.48 - 8.61 (m, 1 H) 8.68 (d, J=5.0 Hz, 1 H)

### Compound 21

1 H NMR (600 MHz, CHLOROFORM-d) d ppm 1.45 - 1.95 (m, 11 H) 4.20 (q, J=7.3 Hz, 2 H) 5.08 (d, J=9.2 Hz, 1 H) 7.28 - 7.43 (m, 4 H) 7.52 (s, 1 H) 7.62 - 7.67 (m, 1 H) 7.73 - 7.84 (m, 2 H) 8.53 (dd, J=4.6, 1.4 Hz, 1 H) 8.90 (d, J=9.2 Hz, 1 H)

### Compound 33

1H NMR (600 MHz, CHLOROFORM-d) d ppm 1.16 - 1.30 (m, 1 H) 1.67 - 1.96 (m, 7 H) 4.99 (d, J=8.7 Hz, 1 H) 7.18 - 7.26 (m, 1 H) 7.33 - 7.48 (m, 2 H) 7.55 - 7.64 (m, 1 H) 7.69-7.78 (m, 1 H) 8.50 - 8.72 (m, 2 H)

### Compound 35

1H NMR (600 MHz, CHLOROFORM-d) d ppm 1.19 - 1.98 (m, 8 H) 5.15 (d, J=8.7 Hz, 1 H) 7.23 - 8.12 (m, 8 H) 8.57 - 8.73 (m, 3 H)

### Compound 37

1 H NMR (600 MHz, CHLOROFORM-d) d ppm 1.15 - 1.24 (m, 1 H) 1.66 - 1.97 (m, 7 H) 5.02 (d, J=8.7 Hz, 1 H) 7.39 - 7.83 (m, 5 H) 8.60 - 8.73 (m, 2 H)

### Compound 42

1H NMR (600 MHz, CHLOROFORM-d) d ppm 1.13 - 1.71 (m, 9 H) 1.83 - 1.96 (m, 1 H) 4.99 (d, J=8.7 Hz, 1 H) 7.15 - 7.47 (m, 3 H) 7.54 - 7.62 (m, 1 H) 7.68 - 7.77 (m, 1 H) 8.50 (d, J=8.7 Hz, 1 H) 8.65 - 8.71 (m, 1 H)

### Compound 48

1 H NMR (600 MHz, CHLOROFORM-d) d ppm 1.16 - 1.24 (m, 1 H) 1.66 - 1.94 (m, 7 H) 2.35 (s, 3 H) 5.03 (d, J=9.1 Hz, 1 H) 7.05 - 7.25 (m, 4 H) 7.71 (d, J=5.0 Hz, 1 H) 8.52 (d, J=8.7 Hz, 1 H) 8.67 (d, J=5.0 Hz, 1 H)

### Compound 54

1H NMR (600 MHz, CHLOROFORM-d) d ppm 1.14 - 1.30 (m, 1 H) 1.67 - 1.96 (m, 7 H) 5.00 (d, J=9.1 Hz, 1 H) 7.20 - 7.49 (m, 4 H) 7.67 - 7.78 (m, 1 H) 8.51 - 8.73 (m, 2 H)

### Compound 57

1 H NMR (600 MHz, CHLOROFORM-d) d ppm 1.16 - 1.29 (m, 1 H) 1.67 - 1.97 (m, 7 H) 3.80 (s, 3 H) 5.03 (d, J=8.7 Hz, 1 H) 6.74 - 7.06 (m, 3 H) 7.21 - 7.32 (m, 1 H) 7.71 (d, J=5.0 Hz, 1 H) 8.46 - 8.70 (m, 2 H)

### Compound 66

1 H NMR (600 MHz, CHLOROFORM-d) d ppm 1.70 - 1.97 (m, 3 H) 2.05 - 2.40 (m, 3 H) 5.23 (d, J=8.7 Hz, 1 H) 6.95 - 7.05 (m, 1 H) 7.17 - 7.36 (m, 3 H) 7.67 - 7.78 (m, 1 H) 8.36 - 8.47 (m, 1 H) 8.61 - 8.73 (m, 1 H)

### Compound 69

1H NMR (600 MHz, CHLOROFORM-d) d ppm 1.09 - 1.23 (m, 1 H) 1.58 - 1.88 (m, 7 H) 4.85 (d, J=9.1 Hz, 1 H) 6.37 - 6.53 (m, 1 H) 7.09 - 7.91 (m, 7 H) 8.45 (d, J=8.3 Hz, 1 H) 8.91 (d, J=5.0 Hz, 1 H)

### Compound 76

1H NMR (600 MHz, CHLOROFORM-d) d ppm 1.27 - 1.38 (m, 1 H) 1.71 - 1.86 (m, 2 H) 1.89 - 2.20 (m, 5 H) 5.04 (d, J=9.1 Hz, 1 H) 7.01 - 7.40 (m, 4 H) 7.75 (d, J=5.0 Hz, 1 H) 8.46 - 8.57 (m, 1 H) 8.68 (d, J=5.0 Hz, 1 H)

### Compound 79

1H NMR (600 MHz, CHLOROFORM-d) d ppm 1.18 - 1.33 (m, 1 H) 1.69 - 1.99 (m, 7 H) 5.07 - 5.21 (m, 1 H) 7.41 - 7.53 (m, 2 H) 7.66 - 7.84 (m, 3 H) 7.97 - 8.08 (m, 1 H) 8.16 - 8.27 (m, 1 H) 8.58 - 8.74 (m, 2 H)

### Compound 90

1H NMR (600 MHz, CHLOROFORM-d) d ppm 1.24 - 2.20 (m, 8 H) 5.00 (d, J=9.1 Hz, 1 H) 7.20 - 7.59 (m, 4 H) 7.75 (d, J=4.5 Hz, 1 H) 8.44 - 8.57 (m, 1 H) 8.68 (d, J=5.0 Hz, 1 H)

### Compound 92

1H NMR (600 MHz, CHLOROFORM-d) d ppm 1.31 - 2.22 (m, 11 H) 4.21 (q, J=7.3 Hz, 2 H) 5.08 (d, J=9.1 Hz, 1 H) 7.19 - 7.25 (m, 1 H) 7.32 - 7.50 (m, 3 H) 7.62 - 7.81 (m, 3 H) 8.46 - 8.56 (m, 1 H) 8.68 (d, J=5.0 Hz, 1 H)

### Compound 102

1H NMR (600 MHz, CHLOROFORM-d) d ppm 1.01 - 1.14 (m, 1 H) 1.63 - 2.02 (m, 7 H) 4.73 - 5.03 (m, 2 H) 7.17 - 7.60 (m, 4 H) 7.74 (d, J=5.0 Hz, 1 H) 8.51 (d, J=9.1 Hz, 1 H) 8.63 - 8.72 (m, 1 H)

### Compound 104

1H NMR (600 MHz, CHLOROFORM-d) d ppm 1.07 - 2.00 (m, 8 H) 4.69 (s, 2 H) 4.98 - 5.12 (m, 1 H) 7.27 - 7.47 (m, 4 H) 7.71 (d, J=4.5 Hz, 1 H) 8.55 (d, J=8.7 Hz, 1 H) 8.66 (d, J=4.5 Hz, 1 H)

### Compound 105

1 H NMR (600 MHz, CHLOROFORM-d) d ppm 1.12 - 1.23 (m, 1 H) 1.66 - 1.98 (m, 7 H) 2.93 (s, 3 H) 3.08 (s, 3 H) 5.03 (d, J=8.7 Hz, 1 H) 7.31 - 7.40 (m, 2 H) 7.48 (d, J=7.4 Hz, 1 H) 7.51 (s, 1 H) 7.72 (d, J=5.0 Hz, 1 H) 8.53 - 8.59 (m, 1 H) 8.67 (d, J=5.0 Hz, 1 H)

### Compound 110

1H NMR (600 MHz, CHLOROFORM-d) d ppm 1.36 - 2.11 (m, 8 H) 4.51 - 4.72 (m, 1 H) 5.02 (d, J=9.1 Hz, 1 H) 7.18 - 7.62 (m, 4 H) 7.74 (d, J=4.5 Hz, 1 H) 8.46 - 8.73 (m, 2 H)

### Compound 112

1H NMR (600 MHz, CHLOROFORM-d) d ppm 1.08 - 1.24 (m, 1 H) 1.65 - 1.96 (m, 7 H) 2.12 - 2.31 (m, 6 H) 3.36 (d, J=12.5 Hz, 1 H) 3.47 (d, J=12.5 Hz, 2 H) 5.06 (d, J=8.7 Hz, 1 H) 7.23 (d, J=7.4 Hz, 1 H) 7.28 - 7.35 (m, 2 H) 7.39 (s, 1 H) 7.71 (d, J=5.0 Hz, 1 H) 8.51 (d, J=8.3 Hz, 1 H) 8.67 (d, J=5.0 Hz, 1 H)

### Compound 118

1H NMR (600 MHz, CHLOROFORM-d) d ppm 1.28 - 1.36 (m, 1 H) 1.51 - 1.59 (m, 4 H) 1.72 - 2.21 (m, 6 H) 4.22 (q, J=7.2 Hz, 2 H) 5.12 (d, J=8.7 Hz, 1 H) 7.02 (d, J=9.1 Hz, 1 H) 7.20 (d, J=7.4 Hz, 1 H) 7.35 - 7.49 (m, 4 H) 7.65 - 7.70 (m, 2 H) 7.76 - 7.81 (m, 2 H)

### Compound 128

1H NMR (600 MHz, CHLOROFORM-d) d ppm 1.73 - 2.23 (m, 6 H) 4.45 - 4.53 (m, 1 H) 4.97 (dd, J=8.7, 7.0 Hz, 1 H) 7.25 - 7.29 (m, 2 H) 7.34 - 7.39 (m, 1 H) 7.51 (d, J=5.8 Hz, 1 H) 7.73 (dd, J=5.0, 2.5 Hz, 1 H) 8.62 (br. s., 1 H) 8.68 (d, J=5.0 Hz, 1 H)

### Compound 130

1H NMR (600 MHz, CHLOROFORM-d) d ppm 1.61 - 2.62 (m, 6 H) 5.21 (d, J=8.7 Hz, 1 H) 7.24 - 7.46 (m, 4 H) 7.77 (t, J=5.4 Hz, 1 H) 8.53 - 8.62 (m, 1 H) 8.66 - 8.73 (m, 1 H)

### Compound 147

1H NMR (600 MHz, CHLOROFORM-d) d ppm 1.15 - 2.20 (m, 8 H) 5.67 (d, J=8.7 Hz, 1 H) 7.19 - 7.32 (m, 2 H) 7.38 - 7.52 (m, 2 H) 7.69 - 7.78 (m, 1 H) 8.55 - 8.71 (m, 2 H)

### Compound 155

1H NMR (600 MHz, CHLOROFORM-d) d ppm 1.16 - 1.24 (m, 1 H) 1.56 - 2.20 (m, 7 H) 5.61 - 5.74 (m, 1 H) 7.19 - 7.45 (m, 5 H) 7.59 - 7.63 (m, 1 H) 8.61 - 8.66 (m, 1 H)

### Compound 162

1 H NMR (600 MHz, CHLOROFORM-d) d ppm 1.34 - 2.08 (m, 8 H) 4.51 - 4.70 (m, 1 H) 4.98 - 5.07 (m, 1 H) 7.26 - 7.30 (m, 3 H) 7.40 - 7.42 (m, 1 H) 7.69 - 7.78 (m, 1 H) 8.45 - 8.56 (m, 1 H) 8.67 (d, J=5.0 Hz, 1 H)

### Compound 164

1 H NMR (600 MHz, CHLOROFORM-d) d ppm 1.34 - 1.43 (m, 1 H) 1.79 - 2.19 (m, 7 H) 5.42 (d, J=9.1 Hz, 1 H) 7.07 - 7.19 (m, 3 H) 7.73 - 7.78 (m, 1 H) 8.58 - 8.71 (m, 2 H)

### Compound 185

1H NMR (600 MHz, CHLOROFORM-d) d ppm 1.12 - 1.97 (m, 8 H) 5.02 (d, J=8.3 Hz, 1 H) 7.18 - 7.36 (m, 4 H) 7.39 - 7.44 (m, 1 H) 7.61 (d, J=4.5 Hz, 1 H) 8.65 (d, J=5.0 Hz, 1 H)

### Compound 186

1 H NMR (600 MHz, CHLOROFORM-d) d ppm 1.31 - 1.46 (m, 2 H) 1.67 - 1.80 (m, 2 H) 1.82 - 1.96 (m, 2 H) 1.96 - 2.11 (m, 2 H) 4.55 - 4.71 (m, 1 H) 5.05 (d, J=9.1 Hz, 1 H) 7.18 (d, J=8.7 Hz, 1 H) 7.23 - 7.36 (m, 3 H) 7.37 - 7.42 (m, 1 H) 7.59 (d, J=5.0 Hz, 1 H) 8.64 (d, J=5.0 Hz, 1 H)

### Compound 187

1 H NMR (600 MHz, CHLOROF ORM-d) d ppm 1.05 -1.11 (m, 1 H) 1.64 - 1.99 (m, 7 H) 4.77 - 4.89 (m, 1 H) 4.99 (d, J=8.7 Hz, 1 H) 7.24 - 7.31 (m, 3 H) 7.37 - 7.41 (m, 1 H) 7.74 (d, J=5.0 Hz, 1 H) 8.49 - 8.54 (m, 1 H) 8.68 (d, J=5.0 Hz, 1 H)

### Compound 188

1H NMR (600 MHz, CHLOROFORM-d) d ppm 1.36 (m, 1 H), 1.66 - 1.85 (m, 2 H), 1.89-2.25 (m, 5 H), 5.13 (d, J=9.1 Hz, 1 H), 7.24 - 7.29 (m, 1 H), 7.42 - 7.52 (m, 2 H), 7.68 - 7.74 (m, 2 H), 7.74 - 7.81 (m, 1 H), 7.87 (d, J=7.4 Hz, 1 H), 8.02 (s, 1 H), 8.59 - 8.70 (m, 3 H)

### Compound 197

1 H NMR (600 MHz, CHLOROFORM-d) d ppm 1.23 - 1.34 (m, 1 H) 1.55 - 1.81 (m, 3H) 1.82 - 2.07 (m, 3 H) 2.09 - 2.18 (m, 1 H) 4.51 - 4.67 (m, 1 H) 5.65 (d, J=9.1 Hz, 1 H) 7.20-7.31 (m, 2 H) 7.38 - 7.44 (m, 1 H) 7.50 - 7.55 (m, 1 H) 7.73 (d, J=4.5 Hz, 1 H) 8.56 - 8.70 (m, 2 H)

### Compound 198

1 H NMR (600 MHz, CHLOROFORM-d) d ppm 0.68 - 1.99 (m, 7 H) 2.07 - 2.17 (m, 1 H) 4.76 - 4.90 (m, 1 H) 5.64 (d, J=8.7 Hz, 1 H) 7.20 - 7.29 (m, 2 H) 7.39 - 7.47 (m, 2 H) 7.71 (d, J=4.5 Hz, 1 H) 8.65 (d, J=4.5 Hz, 1 H)

### Compound 199

1 H NMR (600 MHz, CHLOROFORM-d) d ppm 1.06 - 1.20 (m, 1 H) 1.64 - 2.03 (m, 7 H) 4.77 - 4.91 (m, 1 H) 5.39 (d, J=9.1 Hz, 1 H) 7.09 (t, J=8.1 Hz, 1 H) 7.27 - 7.38 (m, 2 H) 7.71 - 7.76 (m, 1 H) 8.55 - 8.70 (m, 2 H)

### Compound 200

1H NMR (600 MHz, CHLOROFORM-d) d ppm 1.31 - 2.11 (m, 8 H) 4.51 - 4.71 (m, 1 H) 5.43 (d, J=9.5 Hz, 1 H) 7.08 (t, J=8.3 Hz, 1 H) 7.30 - 7.40 (m, 2 H) 7.73 (d, J=5.0 Hz, 1 H) 8.55-8.71 (m, 2 H)

### Compound 201

1H NMR (600 MHz, CHLOROFORM-d) d ppm 1.33 - 1.41 (m, 1 H) 1.48 - 2.16 (m, 7 H) 4.55 - 4.70 (m, 1 H) 5.47 (d, J=8.7 Hz, 1 H) 7.10 - 7.16 (m, 1 H) 7.18 - 7.23 (m, 1 H) 7.27-7.33 (m, 1 H) 7.38 - 7.43 (m, 1 H) 7.60 - 7.63 (m, 1 H) 8.65 (d, J=5.0 Hz, 1 H)

### Compound 204

1 H NMR (600 MHz, CHLOROFORM-d) d ppm 0.74 - 2.18 (m, 8 H) 4.51 - 4.71 (m, 1 H) 5.45 (d, J=9.1 Hz, 1 H) 7.01 - 7.23 (m, 3 H) 7.74 (d, J=5.0 Hz, 1 H) 8.58 - 8.64 (m, 1 H) 8.68 (d, J=5.0 Hz, 1 H)

### Compound 210

1H NMR (600 MHz, CHLOROFORM-d) d ppm 1.23 - 1.34 (m, 1 H) 1.55 - 1.81 (m, 3 H) 1.82 - 2.07 (m, 3 H) 2.09 - 2.18 (m, 1 H) 4.51 - 4.67 (m, 1 H) 5.65 (d, J=9.1 Hz, 1 H) 7.20-7.31 (m, 2 H) 7.38 - 7.44 (m, 1 H) 7.50 - 7.55 (m, 1 H) 7.73 (d, J=4.5 Hz, 1 H) 8.56 - 8.70 (m, 2 H)

### Compound 212

1 H NMR (600 MHz, CHLOROFORM-d) d ppm 1.14 - 1.94 (m, 10 H) 5.00 (d, J=9.1 Hz, 1 H) 7.25 - 7.31 (m, 3 H) 7.42 (s, 1 H) 7.71 (d, J=4.5 Hz, 1 H) 8.50 (d, J=9.1 Hz, 1 H) 8.66 (d, J=4.5 Hz, 1 H)

### Compound 213

1H NMR (600 MHz, CHLOROFORM-d) d ppm 1.23 - 1.36 (1 H, m), 1.50 - 2.20 (7 H, m), 4.49 - 4.69 (1 H, m), 5.64 (1 H, d, J=9.1 Hz), 7.09 - 7.13 (1 H, m), 7.23 - 7.28 (1 H, m), 7.32 - 7.38 (1 H, m), 7.73 - 7.74 (1H, m), 8.62 - 8.67 (2 H, m)

### Compound 219

1H NMR (600 MHz, CHLOROFORM-d) d ppm 1.23 - 2.20 (8 H, m), 5.43 (1 H, d, J=8.7 Hz), 7.12 - 7.17 (1 H, m), 7.22 - 7.30 (1 H, m), 7.36 - 7.48 (1 H, m), 7.59 (1 H, d, J=8.7 Hz), 7.98 (1 H, s), 8.79 (1 H, s)

### Compound 220

1H NMR (600 MHz, CHLOROFORM-d) d ppm 1.23 - 2.18 (m, 8 H) 4.49 - 4.70 (m, 1 H) 5.60 (d, J=8.7 Hz, 1 H) 6.94 - 7.05 (m, 1 H) 7.11 - 7.20 (m, 1 H) 7.48 - 7.62 (m, 1 H) 7.67-7.80 (m, 1 H) 8.49 - 8.61 (m, 1 H) 8.61 - 8.76 (m, 1 H)

### Compound 225

1 H NMR (600 MHz, CHLOROFORM-d) d ppm 1.36 - 2.08 (8 H, m), 4.54 - 4.70 (1 H, m), 5.00 (1 H, d, J=9.5 Hz), 7.10 - 7.15 (1 H, m), 7.28 - 7.33 (1 H, m), 7.48 - 7.52 (1 H, m), 7.74 (1 H, d, J=5.0 Hz), 8.50 (1 H, d, J=9.1 Hz), 8.67 (1 H, d, J=5.0 Hz)

### Compound 226

1 H NMR (600 MHz, CHLOROFORM-d) d ppm 1.34 - 1.44 (1 H, m), 1.76 - 2.20 (7 H, m), 5.40 (1 H, d, J=9.1 Hz), 7.08 - 7.12 (1 H, m), 7.28 - 7.32 (1 H, m), 7.33 - 7.41 (1 H, m), 7.76 - 7.78 (1 H, m), 8.02 - 8.05 (1 H, m), 8.74 (1 H, d, J=9.1 Hz)

### Compound 227

1H NMR (600 MHz, CHLOROFORM-d) d ppm 1.19 - 2.14 (8 H, m), 4.57 - 4.72 (1 H, m), 5.06 (1 H, d, J=8.7 Hz), 7.23 - 7.36 (3 H, m), 7.42 (1 H, s), 7.49 - 7.58 (1 H, m), 7.97 (1 H, s), 8.79 (1 H, s)

### Compound 236

1H NMR (600 MHz, CHLOROFORM-d) d ppm 1.35 - 2.10 (m, 8 H) 4.54 - 4.69 (m, 1 H) 5.01 - 5.09 (m, 1 H) 7.01 (td, J=7.8,2.0 Hz, 1 H) 7.15 (dd, J=7.8, 2.0 Hz, 1 H) 7.18 (d, J=7.8 Hz, 1 H) 7.30 - 7.36 (m, 1 H) 7.74 (d, J=5.0 Hz, 1 H) 8.50 (m, 1 H) 8.67 (d, J=5.0 Hz, 1 H)

### Compound 266

1H NMR (600 MHz, CHLOROFORM-d) d ppm 0.75 - 2.10 (8 H, m) 4.54 - 4.75 (1 H, m) 5.10 (1 H, d, J=9.1 Hz) 7.27 - 7.35 (1 H, m) 7.71 - 7.83 (2 H, m) 8.51 - 8.61 (2 H, m) 8.64-8.75 (2 H, m)

### Compound 330

1H NMR (600 MHz, CHLOROFORM-d) d ppm 1.27 - 1.34 (1 H, m) 1.37 - 1.45 (1 H, m) 1.54 - 1.59 (1 H, m) 1.74 - 1.84 (1 H, m) 1.86 - 2.00 (3 H, m) 2.04 - 2.11 (1 H, m) 4.51 - 4.66 (1 H, m) 4.88 (1 H, br. s.) 5.05 (1 H, d, J=9.1 Hz) 7.27 - 7.29 (1 H, m) 7.32 - 7.37 (1 H, m) 7.48 - 7.50 (1 H, m) 7.53 - 7.58 (1 H, m) 8.44 - 8.48 (1 H, m) 8.60 - 8.64 (1 H, m)

### Compound 338

1 H NMR (600 MHz, CHLOROFORM-d) d ppm 1.23 - 2.13 (8 H, m), 3.70 (1 H, s), 4.48-4.67 (1 H, m), 5.07 (1 H, d, J=8.7 Hz), 7.25 (1 H, br. s.), 7.36 (2 H, dd, J=15.5, 8.1 Hz), 7.55 (1 H, d, J=4.5 Hz), 7.69 - 7.78 (1 H, m), 8.63 (1 H, d, J=4.5 Hz)

### Compound 353

1H NMR (600 MHz, CHLOROFORM-d) d ppm 1.22 - 2.27 (8 H, m), 4.30 (1 H, br. s.), 5.06 (1 H, d, J=8.7 Hz), 7.32 - 7.43 (2 H, m), 7.59 (1 H, d, J=8.7 Hz), 7.75 (1 H, t, J=7.6 Hz), 7.92 (1 H, s), 8.76 (1 H, s)

The optical rotation data of the compounds shown in Table 1 are shown below.

### Compound 165

[α]_{D}²⁶ +10.1° (C = 0.575, CHCl₃)

### Compound 166

[α]_{D}²⁶ -2.84° (C = 0.224, CHCl₃)

### Compound 168

[a]_{D}²⁴ -21.3° (C = 0.172, CHCl₃)

### Compound 177

[α]_{D}²⁶ -44.0° (C = 0.260, CHCl₃)

### Compound 188

[α]_{D}²⁶ +3.47° (C = 0.307, CHCl₃)

### Compound 204

[α]_{D}²⁴ -22.1° (C = 0.0116, CHCl₃)

### Test Example 1 [Glycine Uptake Inhibition Experiment]

A glycine uptake experiment was conducted in accordance with the method published in Neuron, 8, 927-935, 1992. In the experiment, T98G cells (glioma cells) expressing human type 1 glycine transporter (GlyT1) were used. The T98G cells were seeded in a 96-well plate at 2.0 × 10⁴ cells/well and cultured overnight in a CO₂ incubator. The test substance was dissolved in a 100% DMSO solution and then dissolved in a 10 mM HEPES buffer solution (pH 7.4) containing 150 mM sodium chloride, 1 mM calcium chloride, 5 mM potassium chloride, 1 mM magnesium chloride, 10 mM glucose and 0.2% bovine serum albumin. After removing the cell culture medium, the test substance was subjected to a 10-min pretreatment. Subsequently, the test substance and [³H] glycine (final concentration: 250 nM) were added to the cells and reaction was performed at room temperature for 15 minutes. After the end of the reaction, the extracellular fluid was aspirated with a manifold to remove excess labeled glycine present outside the cells, and then the cells were lysed with a 0.5 M aqueous sodium hydroxide solution. The glycine content in the cells was determined by measuring the radioactivity in the cell lysate with a liquid scintillation counter. Glycine uptake in the presence of 10 µM ALX5407 was defined as non-specific uptake, and the value calculated by subtracting the amount of the non-specific uptake from the total uptake in the absence of 10 µM ALX5407 was defined as specific uptake. In addition, glycine uptake inhibitory activity (IC₅₀ value) was calculated from an inhibition curve at the concentrations of each test substance ranging from 10⁻⁹ to 10⁻⁵ M.
It should be noted that ALX5407 is an HCl salt of N-[(3R)-3-([1,1'-biphenyl]-4-yloxy)-3-(4-fluorophenyl)propyl]-N-methylglycine. IC₅₀ values of the compounds of the present invention are shown in Table 1.

### INDUSTRIAL APPLICABILITY

The inventive compounds have glycine transporter (GlyT1)-inhibiti activity, and thus, are effective in the prevention or treatment of diseases associated with the glycine transporter which are, specifically, schizophrenia, Alzheimer's disease, cognitive impairment, dementia, anxiety disorders (e.g., generalized anxiety disorder, panic disorder, obsessive-compulsive disorder, social anxiety disorder, post-traumatic stress disorder, specific phobias, acute stress disorder), depression, drug dependence, spasm, tremor, pain, Parkinson's disease, attention deficit hyperactivity disorder, bipolar disorder, eating disorder, sleep disorders or the like.

## Claims

1. A compound represented by formula [IA] or a pharmaceutically acceptable salt thereof: 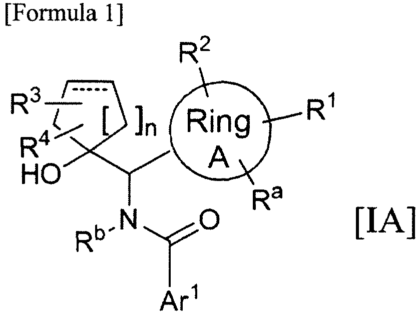 wherein the double line with one line being dotted represents a single bond or a double bond,
Ar¹ represents a naphthyl group, an isoquinolyl group, a naphtylidinyl group, a cinnolinyl group, a phenyl group, a pyrazinyl group, a pyrazolopyridyl group, an isothiazolyl group, a pyridazinyl group, a pyrazolyl group, a tetrahydroindazolyl group, an imidazopyridyl group, an isoxazolopyridyl group, (the phenyl group, the pirazinyl group, the pirazolopyridyl group, the isothiazolyl group, the pyridazinyl group, the pyrazolyl group, the tetrahydroindazolyl group, the imidazopyridyl group, and the isoxazolopyridyl group are optionally substituted with 1 to 3 substituents selected from substituent group 1A), or a pyridyl group substituted with 1 to 2 substituents selected from substituent group 1A,
substituent group 1A is the group consisting of a halogen atom, a C₁₋₆ alkyl group, a C₁₋₆ haloalkyl group, a C₁₋₆ alkoxy group, a C₁₋₆ haloalkoxy group, a C₃₋₆ cycloalkyl group, a C₁₋₆ alkylsulfonyl group, a hydroxy group, a pyrazolyl group, a triazolyl group, a tetrazolyl group, and a pyrimidinyl group,
ring A represents a phenyl group or a pyridyl group,
R¹ and R² are the same or different and are each a hydrogen atom, a halogen atom, a cyano group, a C₁₋₆ alkyl group optionally substituted with a substituent selected from substituent group 2A, a C₁₋₆ haloalkyl group, a C₁₋₆ alkoxy group, a C₁₋₆ haloalkoxy group, a C₂₋₇ alkoxycarbonyl group, a carbamoyl group optionally substituted with one or two C₁₋₆ alkyl groups, a phenyl group optionally substituted with a substituent selected from substituent group 3A, or a monocyclic heteroaryl group optionally substituted with a substituent selected from substituent group 4A,
substituent group 2A is the group consisting of a hydroxy group, and an amino group optionally substituted with one or two C₁₋₆ alkyl groups,
substituent group 3A is the group consisting of a cyano group and a C₁₋₆ alkanoyl group,
substituent group 4A is the group consisting of a C₁₋₆ alkyl group and a C₁₋₆ haloalkyl group,
R^{a} represents a hydrogen atom or a halogen atom,
R³ and R⁴ are the same or different and each represent a hydrogen atom, a halogen atom, or a hydroxy group, or together form an oxo group,
R^{b} represents a hydrogen atom or a C₁₋₆ alkyl group, and
n represents 0, 1, or 2.

2. The compound according to claim 1 or a pharmaceutically acceptable salt thereof wherein ring A is a pyridyl group,
R^{a} is a hydrogen atom, and
R^{b} is a hydrogen atom.

3. The compound according to claim 1 or 2 or a pharmaceutically acceptable salt thereof, wherein Ar¹ is a phenyl group optionally substituted with 1 to 3 substituents selected from substituent group 1A, or a pyridyl group substituted with 1 to 2 substituents selected from substituent group 1A.

4. The compound according to any one of claims 1 to 3 or a pharmaceutically acceptable salt thereof, wherein Ar¹ is a pyridyl group substituted with 1 to 2 substituents selected from substituent group 1 A.

5. The compound according to any one of claims 1 to 3 or a pharmaceutically acceptable salt thereof, wherein Ar¹ is a pyridyl group substituted with 1 to 2 substituents selected from a halogen atom, a C₁₋₆ haloalkyl group, a C₁₋₆ alkylsulfonyl group, a pyrazolyl group, and a triazolyl group.

6. The compound according to any one of claims 1 to 5 or a pharmaceutically acceptable salt thereof, wherein R¹ and R² are the same or different and are each a hydrogen atom, a halogen atom, or a monocyclic heteroaryl group optionally substituted with a substituent selected from substituent group 4A.

7. The compound according to any one of claims 1 to 6 or a pharmaceutically acceptable salt thereof, wherein R¹ and R² are the same or different and are each a hydrogen atom or a halogen atom.

8. The compound according to any one of claims 1 to 7 or a pharmaceutically acceptable salt thereof, wherein the double line with one line being dotted is a single bond, R³ and R⁴ are the same or different and are each a hydrogen atom or a halogen atom, and n is 1 or 2.

9. The compound according to claim 1 or a pharmaceutically acceptable salt thereof, wherein the compound represented by formula [IA] or a pharmaceutically acceptable salt thereof is represented by the following formula [I]: 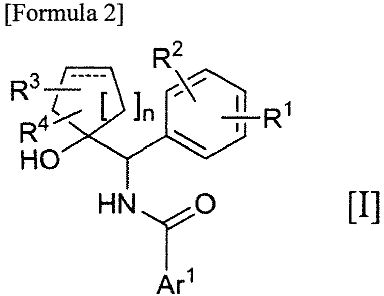 wherein
the double line with one line being dotted represents a single bond or a double bond,
Ar¹ represents a naphthyl group, an isoquinolyl group, a phenyl group optionally substituted with 1 to 3 substituents selected from substituent group 1, a pyrazinyl group optionally substituted with 1 to 2 substituents selected from substituent group 1, or a pyridyl group substituted with 1 to 2 substituents selected from substituent group 1,
substituent group 1 is the group consisting of a halogen atom, a C₁₋₆ alkyl group, a C₁₋₆ haloalkyl group, a C₁₋₆ alkoxy group, a pyrazolyl group, and a triazolyl group,
R¹ and R² are the same or different and each represent a hydrogen atom, a halogen atom, a cyano group, a C₁₋₆ alkyl group optionally substituted with a substituent selected from substituent group 2, a C₁₋₆ haloalkyl group, a C₁₋₆ alkoxy group, a C₁₋₆ haloalkoxy group, a C₂₋₇ alkoxycarbonyl group, a carbamoyl group optionally substituted with one or two C₁₋₆ alkyl groups, a phenyl group optionally substituted with a substituent selected from substituent group 3, or a monocyclic heteroaryl group optionally substituted with a substituent selected from substituent group 4,
substituent group 2 is the group consisting of a hydroxy group, and an amino group optionally substituted with one or two C₁₋₆ alkyl groups,
substituent group 3 is the group consisting of a cyano group and a C₁₋₆ alkanoyl group,
substituent group 4 is the group consisting of a C₁₋₆ alkyl group and a C₁₋₆ haloalkyl group,
R³ and R⁴ are the same or different and each represent a hydrogen atom, a halogen atom, or a hydroxy group, or together form an oxo group, and
n represents 0, 1, or 2.

10. The compound according to claim 9 or a pharmaceutically acceptable salt thereof, wherein Ar¹ is a phenyl group optionally substituted with 1 to 3 substituents selected from substituent group 1, or a pyridyl group substituted with 1 to 2 substituents selected from substituent group 1.

11. The compound according to claim 9 or a pharmaceutically acceptable salt thereof, wherein Ar¹ is a pyridyl group substituted with 1 to 2 substituents selected from substituent group 1.

12. The compound according to claim 9 or a pharmaceutically acceptable salt thereof, wherein Ar¹ is a pyridyl group substituted with 1 to 2 substituents selected from a halogen atom and a C₁₋₆ haloalkyl group.

13. The compound according to any one of claims 9 to 12 or a pharmaceutically acceptable salt thereof, wherein R¹ and R² are the same or different and are each a hydrogen atom, a halogen atom, or a monocyclic heteroaryl group optionally substituted with a substituent selected from substituent group 4.

14. The compound according to any one of claims 9 to 12, or a pharmaceutically acceptable salt thereof, wherein R¹ and R² are the same or different and are each a hydrogen atom or a halogen atom.

15. The compound according to any one of claims 9 to 14 or a pharmaceutically acceptable salt thereof, wherein the double line with one line being dotted is a single bond,
R³ and R⁴ are the same or different and are each a hydrogen atom or a halogen atom, and
n is 1 or 2.

16. The compound according to claim 1 or a pharmaceutically acceptable salt thereof selected from the group consisting of
3-chloro-N-{[3-(1-ethyl-1H-pyrazol-4-yl)phenyl](1-hydroxycyclohexyl)methyl}-4-(trifluoromethyl)pyridine-2-carboxamide,
3-chloro-N-[(6-chloropyridin-2-yl)(1-hydroxycyclohexyl)methyl]-4-(trifluoromethyl)pyridine-2-carboxamide,
3 -chloro-N-{[3-(1-ethyl-1H-pyrazol-4-yl)phenyl](1-hydroxycyclopentyl)methyl}-4-(trifluoromethyl)pyridine-2-carboxamide,
3-chloro-N-{[3-(1-ethyl-1H-pyrazol-4-yl)phenyl](1-hydroxycyclopentyl)methyl}-4-(trifluoromethyl)pyridine-2-carboxamide,
3-chloro-N-{(1-hydroxycyclopentyl)[3-(1H-pyrazol-4-yl)phenyl]methyl}-4-(trifluoromethyl)pyridine-2-carboxamide,
3-chloro-N-[(6-chloropyridin-2-yl)(1-hydroxycyclopentyl)methyl]-4-(trifluoromethyl)pyridine-2-carboxamide,
3-chloro-N-{(1-hydroxycyclopentyl)[3-(pyridin-3-yl)phenyl]methyl}-4-(trifluoromethyl)pyridine-2-carboxamide,
3-chloro-N-{(1-hydroxycyclohexyl)[3-(pyridin-3-yl)phenyl]methyl}-4-(trifluoromethyl)pyridine-2-carboxamide,
3-chloro-N-[(6-chloropyridin-2-yl)(1-hydroxycyclopentyl)methyl]-4-(trifluoromethyl)pyridine-2-carboxamide,
3-chloro-N-[(6-chloropyridin-2-yl)(1-hydroxycyclohexyl)methyl]-2-(trifluoromethyl)pyridine-4-carboxamide,
N-[(2-chloro-3-fluorophenyl)(cis-4-fluoro-1-hydroxycyclohexyl)methyl]-2-(1H-tetrazol-1-yl)-4-(trifluoromethyl)benzamide,
3-chloro-N-{(4,4-difluoro-1-hydroxycyclohexyl)[3-(1-ethyl-1H-pyrazol-4-yl)phenyl]methyl}-4-(trifluoromethyl)pyridine-2-carboxamide,
3-chloro-N-[(3-chlorophenyl)(1-hydroxycyclohexyl)methyl]-4-(trifluoromethyl)pyridine-2-carboxamide,
3-chloro-N-[(3-fluorophenyl)(1-hydroxycyclohexyl)methyl]-4-(trifluoromethyl)pyridine-2-carboxamide,
3-chloro-N-[(2,3-dichlorophenyl)(1-hydroxycyclopentyl)methyl]-4-(trifluoromethyl)pyridine-2-carboxamide,
3-chloro-N-[(4,4-difluoro-1-hydroxycyclohexyl){3-[1-(propan-2-yl)-1H-pyrazol-4-yl]phenyl} methyl]-4-(trifluoromethyl)pyridine-2-carboxamide,
3-chloro-N-{(1-hydroxycyclopentyl)[3-(pyridin-4-yl)phenyl]methyl}-4-(trifluoromethyl)pyridine-2-carboxamide,
N-[(2-chloro-3-fluorophenyl)(cis-4-fluoro-1-hydroxycyclohexyl)methyl]-5-(methylsulfonyl)-2-(trifluoromethyl)pyridine-4-carboxamide,
N-[(2-chloro-3-fluoropluenyl)(cis-4-fluoro-1-hydroxycyclohexyl)methyl]-2-(methylsulfonyl)-5-(trifluoromethyl)pyridine-3-carboxamide,
3-chloro-N-[(2-chloro-3-fluorophenyl)(cis-4-fluoro-1-hydroxycyclohexyl)methyl]-4-(trifluoromethyl)pyridine-2-carboxamide,
N-[(3-bromophenyl)(1-hydroxycyclohexyl)methyl]-3-chloro-4-(trifluoromethyl)pyridine-2-carboxamide,
3-chloro-N-[(2,3-dichlorophenyl)(1-hydroxycyclopentyl)methyl]-4-(trifluoromethyl)pyridine-2-carboxamide,
N-[(2-chloro-3-fluorophenyl)(cis-4-fluoro-1-hydroxycyclohexyl)methyl]-2-(2H-1,2,3-triazol-2-yl)-6-(trifluoromethyl)pyridine-3-carboxamide,
N-[(2-chloro-3-fluorophenyl)(cis-4-fluoro-1-hydroxycyclohexyl)methyl]-2-(methylsulfonyl)-5-(trifluoromethyl)benzamide,
2-chloro-N-[(S)-[3-(1-ethyl-1H-pyrazol-4-yl)phenyl](1-hydroxycyclopentyl)methyl]-3-(trifluoromethyl)benzamide,
2-chloro-N-{[3-(1-ethyl-1H-pyrazol-4-yl)phenyl](1-hydroxycyclohexyl)methyl}-3-(trifluoromethyl)benzamide,
3-chloro-N-[(2-chlorophenyl)(4-fluoro-1-hydroxycyclohexyl)methyl]-4-(trifluoromethyl)pyridine-2-carboxamide,
3-chloro-N-[(4-chloropyridin-2-yl)(cis-4-fluoro-1-hydroxycyclohexyl)methyl]-2-(trifluoromethyl)pyridine-4-carboxamide,
3-chloro-N-[(2-chloro-3-fluorophenyl)(cis-4-fluoro-1-hydroxycyclohexyl)methyl]-2-(trifluoromethyl)pyridine-4-carboxamide,
3-chloro-N-[(3-chlorophenyl)(1-hydroxycyclopentyl)methyl]-4-(trifluoromethyl)pyridine-2-carboxamide,
3-chloro-N-[(3-fluorophenyl)(1-hydroxycyclohexyl)methyl]-4-(trifluoromethyl)pyridine-2-carboxamide,
3-chloro-N-[(3-chloro-2-fluorophenyl)(4,4-difluoro-1-hydroxycyclohexyl)methyl)-4-(trifluoromethyl)pyridine-2-carboxamide,
3-chloro-N-[(2-chloro-4-fluorophenyl)(1-hydroxycyclopentyl)methyl]-4-(trifluoromethyl)pyridine-2-carboxamide,
3-chloro-N-[(3,5-dichlorophenyl)(1-hydroxycyclopentyl)methyl]-4-(trifluoromethyl)pyridine-2-carboxamide,
N-[(2-bromophenyl)(1-hydroxycyclopentyl)methyl]-3-chloro-4-(trifluoromethyl)pyridine-2-carboxamide,
N-[(3-bromophenyl)(1-hydroxycyclohexyl)methyl]-3 -chloro-4-(trifluoromethyl)pyridine-2-carboxamide,
3-chloro-N-[(2,3-dichlorophenyl)(4,4-difluoro-1-hydroxycyclohexyl)methyl]-4-(trifluoromethyl)pyridine-2-carboxamide,
3-chloro-N-[(3-chloro-2-fluorophenyl)(4-fluoro-1-hydroxycyclohexyl)methyl]-4-(trifluoromethyl)pyridine-2-carboxamide,
3-chloro-N-[(2,5-dichlorophenyl)(1-hydroxycyclopentyl)methyl]-4-(trifluoromethyl)pyridine-2-carboxamide,
3-chloro-N-[(S)-(3-chlorophenyl)(cis-4-fluoro-1-hydroxycyclohexyl)methyl]-4-(trifluoromethyl)pyridine-2-carboxamide,
3-chloro-N-[(2-chlorophenyl)(1-hydroxycyclopentyl)methyl]-4-(trifluoromethyl)pyridine-2-carboxamide,
3-chloro-N-[(2,5-dichlorophenyl)(1-hydroxycyclopentyl)methyl]-4-(trifluoromethyl)pyridine-2-carboxamide,
3-chloro-N-{(1-hydroxycyclopentyl)[3-(pyridin-3-yl)phenyl]methyl}-4-(trifluoromethyl)pyridine-2-carboxamide,
3-chloro-N-[(4,4-difluoro-1-hydroxycyclohexyl){3-[1-(2,2,2-trifluoroethyl)-1H-pyrazol-4-yl]phenyl}methyl]-4-(trifluoromethyl)pyridine-2-carboxamide,
3 -chloro-N-{(4,4-difluoro-1-hydroxycyclohexyl)[3-(1-ethyl-1H-pyrazol-4-yl)phenyl]methyl}-4-(trifluoromethyl)pyridine-2-carboxamide,
3-chloro-N-{(4,4-difluoro-1-hydroxycyclohexyl)[3-(pyridin-2-yl)phenyl]methyl}-4-(trifluoromethyl)pyridine-2-carboxamide,
3-chloro-N-{(S)-(1-hydroxycyclopentyl)[3-(pyridin-2-yl)phenyl]methyl}-4-(trifluoromethyl)pyridine-2-carboxamide,
N-[(S)-(3-bromophenyl)(1-hydroxycyclopentyl)methyl]-2-chloro-3-(trifluoromethyl)benzamide,
3-chloro-N-[(6-chloropyridin-2-yl)(4,4-difluoro-1-hydroxycyclohexyl)methyl]-4-(trifluoromethyl)pyridine-2-carboxamide,
3-chloro-N-[(6-chloropyridin-2-yl)(cis-4-fluoro-1-hydroxycyclohexyl)methyl]-4-(trifluoromethyl)pyridine-2-carboxamide,
3-chloro-N-{(1-hydroxycyclohexyl)[3-(pyridin-3-yl)phenyl]methyl}-4-(trifluoromethyl)pyridine-2-carboxamide,
2-chloro-N-[(6-chloropyridin-2-yl)(cis-4-fluoro-1-hydroxycyclohexyl)methyl]-3-(trifluoromethyl)benzamide,
N-[(S)-(3-bromophenyl)(1-hydroxycyclopentyl)methyl]-3-chloro-4-(trifluoromethyl)pyridine-2-carboxamide,
3-chloro-N-[(3-chloro-2-fluorophenyl)(4-fluoro-1-hydroxycyclohexyl)methyl]-4-(trifluoromethyl)pyridine-2-carboxamide,
3-chloro-N-[(3-chlorophenyl)(1-hydroxycyclohexyl)methyl]-4-(trifluoromethyl)pyridine-2-carboxamide,
2-chloro-N-[(4-chloropyridin-2-yl)(cis-4-fluoro-1-hydroxycyclohexyl)methyl]-3-(trifluoromethyl)benzamide,
3-chloro-N-{(1-hydroxycyclopentyl)[3-(1H-pyrazol-1-yl)phenyl]methyl}-4-(trifluoromethyl)pyridine-2-carboxamide,
3-chloro-N-[(3-chlorophenyl)(1-hydroxycyclopentyl)methyl]-4-(trifluoromethyl)pyridine-2-carboxamide,
3-chloro-N-{(1-hydroxycyclopentyl)[3-(pyrimidin-5-yl)phenyl]methyl}-4-(trifluoromethyl)pyridine-2-carboxamide,
N-[(S)-(3-bromophenyl)(cis-4-fluoro-1-hydroxycyclohexyl)methyl]-3-chloro-4-(trifluoromethyl)pyridine-2-carboxamide,
3-chloro-N-[(3-chloro-2-fluorophenyl)(4,4-difluoro-1-hydroxycyclohexyl)methyl]-2-(trifluoromethyl)pyridine-4-carboxamide,
3-chloro-N-[(6-chloropyridin-2-yl)(cis-4-fluoro-1-hydroxycyclohexyl)methyl]-2-(trifluoromethyl)pyridine-4-carboxamide,
3-chloro-N-[(3-fluorophenyl)(1-hydroxycyclopentyl)methyl]-4-(trifluoromethyl)pyridine-2-carboxamide,
3-chloro-N-[(3-chlorophenyl)(cis-4-fluoro-1-hydroxycyclohexyl)methyl]-4-(trifluoromethyl)pyridine-2-carboxamide,
2-chloro-N-[(2-chloro-3-fluorophenyl)(cis-4-fluoro-1-hydroxycyclohexyl)methyl]-3-(trifluoromethyl)benzamide,
3-chloro-N-[(4,4-difluoro-1-hydroxycyclohexyl){3-[1-(propan-2-yl)-1H-pyrazol-4-yl]phenyl}methyl]-4-(trifluoromethyl)pyridine-2-carboxamide,
3-chloro-N-[(2-chloro-3-fluorophenyl)(4,4-difluoro-1-hydroxycyclohexyl)methyl]-4-(trifluoromethyl)pyridine-2-carboxamide,
3-chloro-N-[(2,3-difluorophenyl)(4-fluoro-1-hydroxycyclohexyl)methyl]-4-(trifluoromethyl)pyridine-2-carboxamide,
3-chloro-N-[(cis-4-fluoro-1-hydroxycyclohexyl)(3-fluorophenyl)methyl]-4-(trifluoromethyl)pyridine-2-carboxamide,
N-[(2-chloro-3-fluorophenyl)(cis-4-fluoro-1-hydroxycyclohexyl)methyl]-2-(methylsulfonyl)-4-(trifluoromethyl)benzamide,
2-chloro-N-{[3-(1-ethyl-1H-pyrazol-4-yl)phenyl](1-hydroxycyclopentyl)methyl}-3-(trifluoromethyl)benzamide,
2-chloro-N-{(1-hydroxycyclopentyl)[3-(1H-pyrazol-4-yl)phenyl]methyl}-3-(trifluoromethyl)benzamide,
N-{[3-(1-ethyl-1H-pyrazol-4-yl)phenyl](1-hydroxycyclopentyl)methyl}isoquinoline-1-carboxamide,
3-chloro-N- [(3-fluorophenyl)(1-hydroxycyclopentyl)methyl]-4-(trifluoromethyl)pyridine-2-carboxamide,
3-chloro-N-[(3-chloro-5-fluorophenyl)(1-hydroxycyclopentyl)methyl]-4-(trifluoromethyl)pyridine-2-carboxamide,
3-chloro-N-[(2-chlorophenyl)(4,4-difluoro-1-hydroxycyclohexyl)methyl]-4-(trifluoromethyl)pyridine-2-carboxamide,
N-[(2-chloro-3-fluorophenyl)(cis-4-fluoro-1-hydroxycyclohexyl)methyl]-3-(2H-1,2,3-triazol-2-yl)-5-(trifluoromethyl)pyridine-2-carboxamide,
3-chloro-N-[(2-chloro-3-fluorophenyl)(cis-4-fluoro-1-hydroxycyclohexyl)methyl]-4-(difluoromethyl)pyridine-2-carboxamide,
3-chloro-N-[(4,4-difluoro-1-hydroxycyclohexyl)(2,3-difluorophenyl)methyl]-4-(trifluoromethyl)pyridine-2-carboxamide,
N-[(2-chlorophenyl)(4,4-difluoro-1-hydroxycyclohexyl)methyl]-5-(methylsulfonyl)-2-(trifluoromethyl)pyridine-4-carboxamide,
2-chloro-N-[(2-chloro-3-fluorophenyl)(cis-4-fluoro-1-hydroxycyclohexyl)methyl]-5-(trifluoromethyl)benzamide,
3-chloro-N-[(4,4-difluoro-1-hydroxycyclohexyl)(3-fluorophenyl)methyl]-4-(trifluoromethyl)pyridine-2-carboxamide,
N-[(2-chloro-3-fluorophenyl)(cis-4-fluoro-1-hydroxycyclohexyl)methyl]isoquinoline-1-carboxamide,
N-[(2-chloro-3-fluorophenyl)(cis-4-fluoro-1-hydroxycyclohexyl)methyl]-2-(1H-pyrazol-1-yl)-4-(trifluoromethyl)benzamide,
3-chloro-N-{(S)-(1-hydroxycyclopentyl)[3-(pyrimidin-2-yl)phenyl]methyl}-4-(trifluoromethyl)pyridine-2-carboxamide,
3-chloro-N-[(2-chloro-4-fluorophenyl)(1-hydroxycyclopentyl)methyl]-4-(trifluoromethyl)pyridine-2-carboxamide,
3-chloro-N-[(4,4-difluoro-1-hydroxycyclohexyl){3-[1-(2,2,2-trifluoroethyl)-1 H-pyrazol-4-yl]phenyl}methyl]-4-(trifluoromethyl)pyridine-2-carboxamide,
3-chloro-N-[(2-chloro-3-fluorophenyl)(4,4-difluoro-1-hydroxycyclohexyl)methyl]-2-(trifluoromethyl)pyridine-4-carboxamide,
3-chloro-N-[(3-chloro-2-fluorophenyl)(4-fluoro-l-hydroxycyclohexyl)methyl]-2-(trifluoromethyl)pyridine-4-carboxamide,
N-[(6-chloropyridin-2-yl)(4,4-difluoro-1-hydroxycyclohexyl)methyl]isoquinoline-1-carboxamide,
3-chloro-N-[(4-chloropyridin-2-yl)(cis-4-fluoro-1-hydroxycyclohexyl)methyl]-4-(trifluoromethyl)pyridine-2-carboxamide,
3-chloro-N-{(4,4-difluoro-1-hydroxycyclohexyl)[3-(pyridin-3-yl)phenyl]methyl}-4-(trifluoromethyl)pyridine-2-carboxamide,
3-chloro-N-[(3-chlorophenyl)(1-hydroxycyclopentyl)methyl]-2-(trifluoromethyl)pyridine-4-carboxamide,
2-chloro-N-[(6-chloropyridin-2-yl)(cis-4-fluoro-1-hydroxycyclohexyl)methyl]-6-(trifluoromethyl)pyridine-3-carboxamide,
3-chloro-N-[(cis-4-fluoro-1-hydroxycyclohexyl)(6-fluoropyridin-2-yl)methyl]-4-(trifluoromethyl)pyridine-2-carboxamide,
3-chloro-N-[(4,4-difluoro-1-hydroxycyclohexyl)(6-fluoropyridin-2-yl)methyl]-4-(trifluoromethyl)pyridine-2-carboxamide,
3-chloro-N-[(1-hydroxycyclopentyl)(3-methoxyphenyl)methyl]-4-(trifluoromethyl)pyridine-2-carboxamide,
N-[(S)-(3-bromophenyl)(trans-4-fluoro-1-hydroxycyclohexyl)methyl]-3-chloro-4-(trifluoromethyl)pyridine-2-carboxamide,
3-chloro-N-[(S)-(3-chlorophenyl)(cis-4-fluoro-1-hydroxycyclohexyl)methyl]-2-(trifluoromethyl)pyridine-4-carboxamide,
5-chloro-N-[(2-chloro-3-fluorophenyl)(cis-4-fluoro-1-hydroxycyclohexyl)methyl]-2-(trifluoromethyl)pyridine-4-carboxamide,
2-chloro-N-[(2-chloro-3-fluorophenyl)(cis-4-fluoro-1-hydroxycyclohexyl)methyl]-5-(trifluoromethyl)pyridine-3-carboxamide,
5-chloro-N-[(6-chloropyridin-2-yl)(4,4-difluoro-1-hydroxycyclohexyl)methyl]-2-(trifluoromethyl)pyridine-4-carboxamide,
2-chloro-N-[(6-chloropyridin-2-yl)(4,4-difluoro-1-hydroxycyclohexyl)methyl]-3-(trifluoromethyl)benzamide,
2-chloro-N-[(4-chloropyridin-2-yl)(4,4-difluoro-1-hydroxycyclohexyl)methyl]-3-(trifluoromethyl)benzamide,
3-chloro-N-{(1-hydroxycyclopentyl)[3-(1H-pyrazol-1-yl)phenyl]methyl}-4-(trifluoromethyl)pyridine-2-carboxamide,
3-chloro-N-[(2,3-dichlorophenyl)(4,4-difluoro-1-hydroxycyclohexyl)methyl]-4-(trifluoromethyl)pyridine-2-carboxamide,
3-chloro-N-(4,4-difluoro-1-hydroxycyclohexyl)[3-(-ethyl-1H-pyrazol-4-yl)phenyl]methyl}-2-(trifluoromethyl)pyridine-4-carboxamide,
3-chloro-N-[(2-chloro-5-fluorophenyl)(cis-4-fluoro-1-hydroxycyclohexyl)methyl]-4-(trifluoromethyl)pyridine-2-carboxamide,
N-[(2-chlorophenyl)(4,4-difluoro-1-hydroxycyclohexyl)methyl]-2-(methylsulfonyl)-5-(trifluoromethyl)pyridine-3-carboxamide,
N-[(3-bromophenyl)(1-hydroxycyclopentyl)methyl]-3-chloro-4-(trifluoromethyl)pyridine-2-carboxamide,
3-chloro-N-[(2-chlorophenyl)(4,4-difluoro-1-hydroxycyclohexyl)methyl]-2-(trifluoromethyl)pyridine-4-carboxamide,
3-chloro-N-[(2-chlorophenyl)(cis-4-fluoro-1-hydroxycyclohexyl)methyl]-2-(trifluoromethyl)pyridine-4-carboxamide,
3-chloro-N-[(2-fluorophenyl)(1-hydroxycyclopentyl)methyl]-4-(trifluoromethyl)pyridine-2-carboxamide,
3-chloro-N-[(3-chloro-5-fluorophenyl)(1-hydroxycyclopentyl)methyl]-4-(trifluoromethyl)pyridine-2-carboxamide,
3-chloro-N-[(3-chlorophenyl)(4,4-difluoro-1-hydroxycyclohexyl)methyl]-4-(trifluoromethyl)pyridine-2-carboxamide,
2,6-dichloro-N-{(4,4-difluoro-1-hydroxycyclohexyl)[3-(1-ethyl-1H-pyrazol-4-yl)phenyl]methyl}-3-(trifluoromethyl)benzamide,
2-chloro-N-[(S)-(6-chloropyridin-2-yl)(cis-4-fluoro-1-hydroxycyclohexyl)methyl]-5-(trifluoromethyl)benzamide,
3-chloro-N-[(2-fluorophenyl)(1-hydroxycyclopentyl)methyl]-4-(trifluoromethyl)pyridine-2-carboxamide,
3-chloro-N-{(4,4-difluoro-1-hydroxycyclohexyl)[3-(pyridin-2-yl)phenyl]methyl}-2-(trifluoromethyl)pyridine-4-carboxamide,
3-chloro-N-[(2-chlorophenyl)(4-fluoro-1-hydroxycyclohexyl)methyl]-4-(trifluoromethyl)pyridine-2-carboxamide,
5-chloro-N-[(2-chloropyridin-3-yl)(cis-4-fluoro-1-hydroxycyclohexyl)methyl]-2-(trifluoromethyl)pyridine-4-carboxamide,
3-chloro-N-[(6-chloropyridin-2-yl)(cis-4-fluoro-1-hydroxycyclohexyl)methyl]-4-(difluoromethyl)pyridine-2-carboxamide,
N-[(2-chlorophenyl)(4,4-difluoro-1-hydroxycyclohexyl)methyl]-2-(methylsulfonyl)-4-(trifluoromethyl)benzamide,
3-chloro-N-{(4,4-difluoro-1-hydroxycyclohexyl)[3-(pyridin-2-yl)phenyl]methyl}-4-(trifluoromethyl)pyridine-2-carboxamide,
3-chloro-N-[(4,4-difluoro-1-hydroxycyclohexyl)(2,3-difluorophenyl)methyl]-2-(trifluoromethyl)pyridine-4-carboxamide,
3-chloro-N-[(3-chlorophenyl)(4,4-difluoro-1-hydroxycyclohexyl)methyl]-2-(trifluoromethyl)pyridine-4-carboxamide,
2-chloro-N-{(4,4-difluoro-1-hydroxycyclohexyl)[3-(1-ethyl-1H-pyrazol-4-yl)phenyl]methyl}-5-(trifluoromethyl)benzamide,
3-chloro-N-[(S)-(3-chlorophenyl)(trans-4-fluoro-1-hydroxycyclohexyl)methyl]-4-(trifluoromethyl)pyridine-2-carboxamide,
N-{(4,4-difluoro-1-hydroxycyclohexyl)[3-(1-ethyl-1H-pyrazol-4-yl)phenyl]methyl}-4-fluoro-2-(trifluoromethyl)benzamide,
2-chloro-N-[(3-fluorophenyl)(1-hydroxycyclopentyl)methyl]-3-(trifluoromethyl)benzamide,
3-chloro-N-[(3,5-dichlorophenyl)(1-hydroxycyclopentyl)methyl]-4-(trifluoromethyl)pyridine-2-carboxamide,
2-chloro-N-{(4,4-difluoro-1-hydroxycyclohexyl)[3-(1-ethyl-1H-pyrazol-4-yl)phenyl]methyl} -3 -(trifluoromethyl)benzamide,
2-chloro-N-[(6-chloropyridin-2-yl)(4,4-difluoro-1-hydroxycyclohexyl)methyl]-5-(trifluoromethyl)pyridine-3-carboxamide,
3-chloro-N-[(cis-4-fluoro-1-hydroxycyclohexyl)(phenyl)methyl]-4-(trifluoromethyl)pyridine-2-carboxamide,
3-chloro-N-[(4,4-difluoro-1-hydroxycyclohexyl)(3-fluorophenyl)methyl]-2-(trifluoromethyl)pyridine-4-carboxamide,
N-[(2-chloro-3-fluorophenyl)(4,4-difluoro-1-hydroxycyclohexyl)methyl]-2-(methylsulfonyl)-4-(trifluoromethyl)benzamide,
3-chloro-N-[(trans-4-fluoro-l-hydroxycyclohexyl)(2-fluorophenyl)methyl]-4-(trifluoromethyl)pyridine-2-carboxamide,
3-chloro-N-[(5-chloropyridin-3-yl)(cis-4-fluoro-1-hydroxycyclohexyl)methyl]-4-(trifluoromethyl)pyridine-2-carboxamide,
3-chloro-N-[(1-hydroxycyclopentyl)(3-methyl phenyl)methyl]-4-(trifluoromethyl)pyridine-2-carboxamide,
3-chloro-N-[(4,4-difluoro-1-hydroxycyclohexyl)(3-fluorophenyl)methyl]-4-(trifluoromethyl)pyridine-2-carboxamide,
2-chloro-N-[(6-chloropyridin-2-yl)(cis-4-fluoro-1-hydroxycyclohexyl)methyl]-4-(trifluoromethyl)benzamide,
3-chloro-N-[(3-chloropyridin-2-yl)(4,4-difluoro-1-hydroxycyclohexyl)methyl]-4-(trifluoromethyl)pyridine-2-carboxamide,
3-chloro-N-[(4,4-difluoro-1-hydroxycyclohexyl)(6-fluoropyridin-2-yl)methyl]-2-(trifluoromethyl)pyridine-4-carboxamide,
3-chloro-N-[(5-chloro-2-fluorophenyl)(cis-4-fluoro-1-hydroxycyclohexyl)methyl]-4-(trifluoromethyl)pyridine-2-carboxamide,
3-chloro-N-[(4,4-difluoro-1-hydroxycyclohexyl)(3-fluorophenyl)methyl]-4-(trifluororaethyl)pyridine-2-carboxamide,
N-[(3-bromophenyl)(4-fluoro-1-hydroxycyclohexyl)methyl]-3-chloro-4-(trifluoromethyl)pyridine-2-carboxamide,
3-chloro-N-[(2-chlorophenyl)(4,4-difluoro-1-hydroxycyclohexyl)methyl]-4-(trifluoromethyl)pyridine-2-carboxamide,
2-chloro-N-[(6-chloropyridin-2-yl)(4,4-difluoro-1-hydroxycyclohexyl)methyl]-6-(trifluoromethyl)pyridine-3-carboxamide,
3-chloro-N-[4,4-difluoro-1-hydroxycyclohexyl)(2-fluorophenyl)methyl]-4-(trifluoromethyl)pyridine-2-carboxamide,
3-chloro-N-[(S)-(3-cyanophenyl)(1-hydroxycyclopentyl)methyl]-4-(trifluoromethyl)pyridine-2-carboxamide,
2-chloro-N-{(4,4-difluoro-1-hydroxycyclohexyl)[3-(1-ethyl-1H-pyrazol-4-yl)phenyl]methyl}-3-(trifluoromethyl)benzamide,
3-chloro-N-[(2,5-dichlorophenyl)(4,4-difluoro-1-hydroxycyclohexyl)methyl]-4-(trifluoromethyl)pyridine-2-carboxamide,
N-[(2-chloro-3-fluorophenyl)(cis-4-fluoro-1-hydroxycyclohexyl)methyl]-2-(methylsulfonyl)-6-(trifluoromethyl)pyridine-3-carboxamide,
3-chloro-N-[(4-chloropyridin-2-yl)(4,4-difluoro-1-hydroxycyclohexyl)methyl]-4-(trifluoromethyl)pyridine-2-carboxamide,
N-[(2-chloro-3-fluorophenyl)(cis-4-fluoro-1-hydroxycyclohexyl)methyl]-2-(2H-1,2,3-triazol-2-yl)-4-(trifluoromethyl)benzamide,
2-chloro-N-[(4-chloropyridin-2-yl)(cis-4-fluoro-1-hydroxycyclohexyl)methyl]-6-(trifluoromethyl)pyridine-3-carboxamide,
2-chloro-N-[(3-fluorophenyl)(1-hydroxycyclopentyl)methyl]-3-(trifluoromethyl)benzamide,
3-chloro-N-[(3-chloro-4-fluorophenyl)(1-hydroxycyclopentyl)methyl]-4-(trifluoromethyl)pyridine-2-carboxamide,
4-chloro-N-[(2-chloro-3-fluorophenyl)(cis-4-fluoro-1-hydroxycyclohexyl)methyl]-3-methyl pyridine-2-carboxamide,
3-chloro-N-[(1-hydroxycyclopentyl)(2-methyl phenyl)methyl]-4-(trifluoromethyl)pyridine-2-carboxamide,
2,6-dichloro-N-{(4,4-difluoro-1-hydroxycyclohexyl)[3-(1-ethyl-1H-pyrazol-4-yl)phenyl]methyl}-3-(trifluoromethyl)benzamide,
3-chloro-N-[(2,5-dichlorophenyl)(4,4-difluoro-1-hydroxycyclohexyl)methyl]-4-(trifluoromethyl)pyridine-2-carboxamide,
2-chloro-N-[(2-chloro-3-fluorophenyl)(cis-4-fluoro-1-hydroxycyclohexyl)methyl]-6-(trifluoromethyl)pyridine-3-carboxamide,
N-[(4-chloropyridin-2-yl)(cis-4-fluoro-1-hydroxycyclohexyl)methyl]-2-(methylsulfonyl)-5-(trifluoromethyl)benzamide,
2-chloro-N-{(1-hydroxycyclopentyl)[3-(pyrimidin-5-yl)phenyl]methyl}-3-(trifluoromethyl)benzamide,
N-{(4,4-difluoro-1-hydroxycyclohexyl)[3-(1-ethyl-1H-pyrazol-4-yl)phenyl]methyl}naphthalene-1-carboxamide,
3-chloro-N-[(3-cyanophenyl)(1-hydroxycyclopentyl)methyl]-4-(trifluoromethyl)pyridine-2-carboxamide,
3-chloro-N-[(3-ethoxyphenyl)(1-hydroxycyclopentyl)methyl]-4-(trifluoromethyl)pyridine-2-carboxamide,
N-[(3-bromophenyl)(4,4-difluoro-1-hydroxycyclohexyl)methyl]-3-chloro-4-(trifluoromethyl)pyridine-2-carboxamide,
3-chloro-N-[(3-chloropyridin-2-yl)(cis-4-fluoro-1-hydroxycyclohexyl)methyl]-4-(trifluoromethyl)pyridine-2-carboxamide,
3 -chloro-N-{(1-hydroxycyclopentyl)[3-(2H-1,2,3-triazol-2-yl)phenyl]methyl}-4-(trifluoromethyl)pyridine-2-carboxamide,
3-chloro-N-{(4,4-difluoro-1-hydroxycyclohexyl)[3-(1-ethyl-1H-pyrazol-4-yl)phenyl]methyl} -2-methylbenzamide,
5-chloro-N-[(2-chlorophenyl)(cis-4-fluoro-1-hydroxycyclohexyl)methyl]-2-(trifluoromethyl)pyridine-4-carboxamide,
N-[(3-bromophenyl)(4,4-difluoro-1-hydroxycyclohexyl)methyl]-3-chloro-4-(trifluoromethyl)pyridine-2-carboxamide,
3-chloro-N-{(4,4-difluoro-1-hydroxycyclohexyl)[3-(pyrazin-2-yl)phenyl]methyl}-4-(trifluoromethyl)pyridine-2-carboxamide,
N-[(2-chlorophenyl)(4,4-difluoro-1-hydroxycyclohexyl)methyl]-2-(methylsulfonyl)-6-(trifluoromethyl)pyridine-3-carboxamide,
3-chloro-N-[(2-chloropyridin-3-yl)(4,4-difluoro-1-hydroxycyclohexyl)methyl]-4-(trifluoromethyl)pyridine-2-carboxamide,
3-chloro-N-[(2-chloropyridin-3-yl)(cis-4-fluoro-1-hydroxycyclohexyl)methyl]-4-(trifluoromethyl)pyridine-2-carboxamide,
2-chloro-N-[(2-chloropyridin-3-yl)(4,4-difluoro-1-hydroxycyclohexyl)methyl]-3-(trifluoromethyl)benzamide,
N-[(2-chlorophenyl)(4,4-difluoro-1-hydroxycyclohexyl)methyl]-2-(methylsulfonyl)-5-(trifluoromethyl)benzamide,
N-{(4,4-difluoro-1-hydroxycyclohexyl)[3-(1-ethyl-1H-pyrazol-4-yl)phenyl]methyl} -4-fluoro-2-(trifluoromethyl)benzamide,
N-{(4,4-difluoro-1-hydroxycyclohexyl)[3-(1-ethyl-1H-pyrazol-4-yl)phenyl]methyl}-2-methyl-3-(trifluoromethyl)benzamide,
3-chloro-N-[(cis-4-fluoro-1-hydroxycyclohexyl)(phenyl)methyl]-4-(trifluoromethyl)pyridine-2-carboxamide,
2-chloro-N-[(2-chloropyridin-3-yl)(cis-4-fluoro-1-hydroxycyclohexyl)methyl]-3-(trifluoromethyl)benzamide,
3-chloro-N-[(4-chloropyridin-2-yl)(4,4-difluoro-1-hydroxycyclohexyl)methyl]-2-(trifluoromethyl)pyridine-4-carboxamide,
2-chloro-N-{(1-hydroxycyclopentyl)[3-(pyridin-3-yl)phenyl]methyl}-3-(trifluoromethyl)benzamide,
3-chloro-N-[(1-hydroxycyclopentyl)(3-methoxyphenyl)methyl]-4-(trifluoromethyl)pyridine-2-carboxamide,
3-chloro-N-[(4-chloropyridin-3-yl)(cis-4-fluoro-1-hydroxycyclohexyl)methyl]-4-(trifluoromethyl)pyridine-2-carboxamide,
3-chloro-N-[(6-chloropyridin-2-yl)(4,4-difluoro-1-hydroxycyclohexyl)methyl]-2-(trifluoromethyl)pyridine-4-carboxamide,
2-chloro-N-[(2-chlorophenyl)(cis-4-fluoro-1-hydroxycyclohexyl)methyl]-5-(trifluoromethyl)pyridine-3-carboxamide,
3-chloro-N-[(3-chlorophenyl)(cis-4-fluoro-1-hydroxycyclohexyl)methyl]-2-(trifluoromethyl)pyridine-4-carboxamide,
3-chloro-N-[(3-chloro-4-fluorophenyl)(cis-4-fluoro-1-hydroxycyclohexyl)methyl]-4-(trifluoromethyl)pyridine-2-carboxamide,
3-chloro-N-[(cis-4-fluoro-1-hydroxycyclohexyl)(2-fluorophenyl)methyl]-4-(trifluoromethyl)pyridine-2-carboxamide,
2-chloro-N-{(1-hydroxycyclopentyl)[3-(pyridin-4-yl)phenyl]methyl}-3-(trifluoromethyl)benzamide,
2-chloro-N-[(3-cyanophenyl)(1-hydroxycyclopentyl)methyl]-3-(trifluoromethyl)benzamide,
2-chloro-N-[(2-chloro-3-fluorophenyl)(cis-4-fluoro-1-hydroxycyclohexyl)methyl]-4-(trifluoromethyl)benzamide,
N-[(3-bromophenyl)(1-hydroxycyclohexa-3-en-1-yl)methyl]-3-chloro-4-(trifluoromethyl)pyridine-2-carboxamide,
5-chloro-N-[(2-chloro-3-fluorophenyl)(cis-4-fluoro-1-hydroxycyclohexyl)methyl]-2-(trifluoromethyl)benzamide,
2,5-dichloro-N-[(2-chloro-3-fluorophenyl)(cis-4-fluoro-1-hydroxycyclohexyl)methyl]benzamide,
3-chloro-N-[(3-chloro-4-fluorophenyl)(1-hydroxycyclopentyl)methyl] -4-(trifluoromethyl)pyridine-2-carboxamide,
N-{(4,4-difluoro-1-hydroxycyclohexyl)[3-(1-ethyl-1H-pyrazol-4-yl)phenyl]methyl}isoquinoline-1-carboxamide,
2-chloro-N-[(2-chloropyridin-3-yl)(cis-4-fluoro-1-hydroxycyclohexyl)methyl]-5-(trifluoromethyl)benzamide,
N-[(2-chlorophenyl)(4,4-difluoro-1-hydroxycyclohexyl)methyl]-3-(2H-1,2,3-triazol-2-yl)-5-(trifluoromethyl)pyridine-2-carboxamide,
2-chloro-N-[(2-chloro-3-fluorophenyl)(cis-4-fluoro-1-hydroxycyclohexyl)methyl]-5-methoxybenzamide,
N-[(3-chloro-2-fluorophenyl)(4,4-difluoro-1-hydroxycyclohexyl)methyl]-2-(methylsulfonyl)-4-(trifluoromethyl)benzamide,
3-chloro-N-{(1-hydroxycyclopentyl)[3-(1H-1,2,3-triazol-1-yl)phenyl]methyl}-4-(trifluoromethyl)pyridine-2-carboxamide,
3-chloro-N-[(2-chloro-3-fluorophenyl)(cis-4-fluoro-1-hydroxycyclohexyl)methyl]-4-(trifluoromethyl)pyridine-2-carboxamide,
2,3-dichloro-N-{(4,4-difluoro-1-hydroxycyclohexyl)[3-(1-ethyl-1H-pyrazol-4-yl)phenyl]methyl}benzamide,
3-chloro-N-[(1-hydroxycyclopentyl)(phenyl)methyl]-4-(trifluoromethyl)pyridine-2-carboxamide,
5-chloro-N-[(4-chloropyridin-2-yl)(4,4-difluoro-1-hydroxycyclohexyl)methyl]-2-(trifluoromethyl)pyridine-4-carboxamide,
2-chloro-N-{(4,4-difluoro-1-hydroxycyclohexyl)[3-(pyridin-2-yl)phenyl]methyl}-5-(trifluoromethyl)benzamide,
3-chloro-N-[(2-chlorophenyl)(4,4-difluoro-1-hydroxycyclohexyl)methyl]-2-(trifluoromethyl)pyridine-4-carboxamide.

17. A pharmaceutical composition comprising, as an active ingredient, the compound or pharmaceutically acceptable salt thereof according to any one of claims 1 to 16.

18. An agent for preventing or treating diseases of schizophrenia, Alzheimer's disease, cognitive impairment, dementia, anxiety disorders, depression, drug dependence, spasm, tremor, pain, Parkinson's disease, attention deficit hyperactivity disorder, bipolar disorder, eating disorder, or sleep disorders, which comprises, as an active ingredient, the compound or pharmaceutically acceptable salt thereof according to any one of claims 1 to 16.
